# EUROPEAN PATENT SPECIFICATION

(11) **EP 2 663 650 B1**
(45) Date of publication and mention of the grant of the patent: **09.11.2016**
(21) Application number: 12700803.5
(22) Date of filing: 11.01.2012
(51) Int. Cl.: C12Q 1/68

(54) **METHODS FOR PREDICTING THE OUTCOME OF A CANCER IN A PATIENT BY ANALYSING GENE EXPRESSION**
VERFAHREN ZUR VORHERSAGE DES AUSGANGS VON KREBS DURCH ANALYSE DER GENEXPRESSION
PROCÉDÉS DE PRÉVISION DU RÉSULTAT D'UN CANCER CHEZ UN PATIENT PAR L'ANALYSE DE L'EXPRESSION GÉNIQUE

(30) Priority: 11.01.2011 EP 11305024; 20.01.2011 US 201161434514 P
(43) Date of publication of application: 20.11.2013
(73) Proprietor: INSERM - Institut National de la Santé et de la Recherche Médicale, 75013 Paris (FR); Université Paris Descartes, 75006 Paris (FR); Assistance Publique Hôpitaux De Paris, 75004 Paris 4 (FR)
(72) Inventor: GALON, Jérôme, F-75006 Paris (FR); PAGES, Franck, F-75006 Paris (FR); MLECNIK, Bernhard, F-75006 Paris (FR); FRIDMAN, Herve, F-75006 Paris (FR)
(74) Representative: Cabinet Plasseraud
(86) International application number: PCT/EP2012/050358
(87) International publication number: WO 2012/095448

(56) References cited:
- WO-A1-2005/111076
- WO-A1-2007/045996
- WO-A1-2008/046182
- WO-A1-2010/144192
- WO-A2-02/09573
- WO-A2-2005/054508
- WO-A2-2005/071419

## Description

### FIELD OF THE INVENTION:

The present invention relates to a method for predicting the outcome of a cancer in a patient by analysing gene expression in a sample obtained from said patient.

### BACKGROUND OF THE INVENTION:

Cancer remains a serious public health problem in developed countries. Accordingly, to be most effective, cancer treatment requires not only early detection and treatment or removal of the malignancy, but a reliable assessment of the severity of the malignancy and a prediction of the likelihood of cancer recurrence. The stage of a cancer indicates how far a cancer has spread. Staging is important because treatment is often decided according to the stage of a cancer. To date, cancers are generally classified according to the UICC-TNM system. The TNM (for "Tumor-Node-Metastasis") classification system uses the size of the tumor, the presence or absence of tumor in regional lymph nodes, and the presence or absence of distant metastases, to assign a stage to the tumor. The TNM system developed from the observation that patients with small tumours have better prognosis than those with tumours of greater size at the primary site. In general, patients with tumours confined to the primary site have better prognosis than those with regional lymph node involvement, which in turn is better than for those with distant spread of disease to other organs. Accordingly, cancer can be generally divided into four stages. Stage I is very localized cancer with no cancer in the lymph nodes. Stage II cancer has spread to the lymph nodes. Stage III cancer has spread near to where the cancer started. Stage IV cancer has spread to another part of the body. The assigned stage is used as a basis for selection of appropriate therapy and for prognostic purposes.

The above TNM classifications, although they are to be useful, are imperfect and do not allow a reliable prognosis of the outcome of the cancers. Recently, Galon et al. suggested that analysing the expression of genes related to the adaptive immune response within the tumour may be suitable for predicting the outcome of a cancer in a patient (WO2007045996). Thus they provides list of genes and combination thereof that may be useful for the prognosis of patients for progression of cancer. However the methods depicted in said document fail to point out particular combinations of genes that provide a better performance than the TNM classification does for predicting the outcome of a cancer in a patient.

### SUMMARY OF THE INVENTION:

The present invention relates to a method for predicting the outcome of a cancer as defined by the claims

### DETAILED DESCRIPTION OF THE INVENTION:

International patent application WO2007045996 pertains to a general selection of the most important genes (∼300) describing the tumour micro-environment that was done without considering combinations among them. The possible number of non redundant clusters of around 15 out of 300 genes described in international patent application WO2007045996 exceeds 1.0E+37. To restrict the number of clusters, only 15 genes were used that remained highly logrank significant after 100x cross-validation on a cohort of 77 Stage I/II/III stage patients. Accordingly the present invention focuses on the predictive accuracy of a multivariate cox model based on an accumulative number of gene combinations rather than on the importance of single genes. More particularly the present invention relates to various gene clusters for spredicting the outcome of a cancer in the patient (Table 1 to Table 15). They demonstrated, by determining the ROC curves for each of said cluster, that most of them provide greater sensitivities and selectivities than the UICC-TNM classification does.

Accordingly, the present invention relates to a method for predicting the outcome of a cancer in a patient comprising a step consisting of determining the expression level of a gene cluster consisting of the 15 genes as disclosed in Tab. A.

All the genes pertaining to the invention are known per se, and are listed in the below Tables A:

**Table A: list of the genes according to the invention**

| **Gene** | **Name** | **Gene ID** |
|---|---|---|
| GNLY | granulysin | 10578 |
| PF4 | platelet factor | 5196 |
| LAG3 | lymphocyte-activation gene 3 | 3902 |
| CXCL13 | chemokine (C-X-C motif) ligand 13 | 10563 |
| CXCL9 | chemokine (C-X-C motif) ligand 9 | 4283 |
| REN | renin | 8972 |
| ITGAE | integrin, alpha E (antigen CD103, human mucosal lymphocyte antigen 1; alpha polypeptide) | 3682 |
| CCL5 | chemokine (C-C motif) ligand 5 | 6352 |
| TSLP | thymic stromal lymphopoietin | 85480 |
| IL17A | interleukin 17A | 3605 |
| PROM1 | prominin 1 | 8842 |
| IHH | Indian hedgehog | 3549 |
| GZMH | granzyme H (cathepsin G-like 2, protein h-CCPX) | 2999 |
| IFNG | interferon, gamma | 3548 |
| VEGFA | vascular endothelial growth factor A | 7422 |

The term "gene cluster" refers to the 15, genes of Table A.

Also disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising one gene selected from the group consisting of GNLY, LAG3, CXCL13, PF4, CXCL9, REN, ITGAE, CCL5, TSLP, IL17A, GZMH, PROM1, IHH, and IFNG.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising GLNY.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising LAG3.

In particular, disclosed is a method for predicting the outcome of a cancer comprising a step consisting of determining the expression level of a gene cluster consisting of at least 3 genes in a sample obtained from said patient, wherein said gene cluster is selected from the group consisting of clusters depicted in Table 3-15 comprising CXCL13.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising PF4.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising CXCL9.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising REN.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising ITGAE.

In disclosed is a method for predicting the outcome of a cancer in a patienr comprising a step consisting of determining the expression level of a gene cluster consisting of at least 3 genes in a sample obtained from said patient, wherein said gene cluster is selected from the group consisting of clusters depicted in Table 3-15 comprising CCL5.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising TSLP.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising IL17A.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising GZMH.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising PROM1.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising IHH.

In particular, disclosed is a method for predicting the outcome of a cancer in a patient comprising a step consisting of determining the expression level of a gene cluster consisting of at least 3 genes in a sample obtained from said patient, wherein said gene cluster is selected from the group consisting of clusters depicted in Table 3-15 comprising IFNG.

Disclosed is a method for predicting the outcome of a cancer comprising a step consisting of determining the expression level of a gene cluster consisting of at least 3 genes in a sample obtained from said patient, wherein said 3 genes are selected from the group consisting of GNLY, PF4, LAG3, CXCL13, CXCL9, REN, ITGAE, CCL5, TSLP, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises GLNY and 2 genes selected from the group consiting of PF4, LAG3, CXCL13, CXCL9, REN, ITGAE, CCL5, TSLP, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises PF4 and 2 genes selected from the group consiting of GNLY, LAG3, CXCL13, CXCL9, REN, ITGAE, CCL5, TSLP, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises LAG3 and 2 genes selected from the group consiting of PF4, GNLY, CXCL13, CXCL9, REN, ITGAE, CCL5, TSLP, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises CXCL13 and 2 genes selected from the group consiting of PF4, LAG3, GNLY, CXCL9, REN, ITGAE, CCL5, TSLP, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises CXCL9 and 2 genes selected from the group consiting of PF4, LAG3, CXCL13, GNLY, REN, ITGAE, CCL5, TSLP, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises REN and 2 genes selected from the group consiting of PF4, LAG3, CXCL13, CXCL9, GNLY, ITGAE, CCL5, TSLP, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises ITGAE and 2 genes selected from the group consiting of PF4, LAG3, CXCL13, CXCL9, REN, GNLY, CCL5, TSLP, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises CCL5 and 2 genes selected from the group consiting of PF4, LAG3, CXCL13, CXCL9, REN, ITGAE, GNLY, TSLP, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises TSLP and 2 genes selected from the group consiting of PF4, LAG3, CXCL13, CXCL9, REN, ITGAE, CCL5, GNLY, IL17A, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises IL17A and 2 genes selected from the group consiting of PF4, LAG3, CXCL13, CXCL9, REN, ITGAE, CCL5, TSLP, GNLY, PROM1, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises PROM1 and 2 genes selected from the group consiting of PF4, LAG3, CXCL13, CXCL9, REN, ITGAE, CCL5, TSLP, IL17A, GNLY, IHH, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises IHH and 2 genes selected from the group consiting of PF4, LAG3, CXCL13, CXCL9, REN, ITGAE, CCL5, TSLP, IL17A, PROM1, GNLY, GZMH, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises GZMH and 2 genes selected from the group consiting of PF4, LAG3, CXCL13, CXCL9, REN, ITGAE, CCL5, TSLP, IL17A, PROM1, IHH, GNLY, IFNG, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises IFNG and 2 genes selected from the group consiting of PF4, LAG3, CXCL13, CXCL9, REN, ITGAE, CCL5, TSLP, IL17A, PROM1, IHH, GZMH, GNLY, and VEGFA.

In particular, disclosed is a method for predicting the outcome of a cancer in a patient comprising a step consisting of determining the expression level of a gene cluster consisting of at least 3 genes in a sample obtained from said patient, wherein said gene cluster comprises VEGFA and 2 genes selected from the group consiting of PF4, LAG3, CXCL13, CXCL9, REN, ITGAE, CCL5, TSLP, IL17A, PROM1, IHH, GZMH, IFNG, and GNLY.

As used herein, the term "patient" denotes a mammal. In a preferred embodiment of the invention, a patient according to the invention is a human.

The term "sample" means any tissue sample derived from the patient. Said tissue sample is obtained for the purpose of the in vitro evaluation. The sample can be fresh, frozen, fixed (e.g., formalin fixed), or embedded (e.g., paraffin embedded). In a particular embodiment the sample results from biopsy performed in the tumour sample of the patient. For example an endoscopical biopsy performed in the bowel of the patient affected by a colorectal cancer.

### Gene clusters based on GNLY:

An aspect of the disclosure relates to a method for predicting the outcome of a cancer in a patient comprising a step consisting of determining the expression level of a gene cluster in a sample obtained from said patient, wherein said gene cluster comprises GNLY.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of PF4, LAG3, CXCL13, CXCL9, REN, ITGAE, CCL5, TSLP, IL17A, PROM1, IHH, GZMH, IFNG, and VEGFA.

In a particular embodiment, the gene cluster consists in the combination of 2 genes. For example, said gene cluster may be selected according to any combination described in Table 2 which comprises GNLY. In a particular embodiment, the gene cluster consists in the combination GNLY + PF4.

In a particular embodiment, the gene cluster consists in the combination of 3 genes. For example, said gene cluster may be selected according to any combination described in Table 3 which comprises GNLY. In a particular embodiment, the gene cluster consists in the combination GNLY + PF4 + REN.

In a particular embodiment, the gene cluster consists in the combination of 4 genes. For example, said gene cluster may be selected according to any combination described in Table 4 which comprises GNLY. In a particular embodiment, the gene cluster consists in the combination GNLY + PF4 + IL17A + REN.

In a particular embodiment, the gene cluster consists in the combination of 5 genes. For example, said gene cluster may be selected according to any combination described in Table 5 which comprises GNLY. In a particular embodiment, the gene cluster consists in the combination IFNG + GNLY + PF4 + IL17A + REN.

In a particular embodiment, the gene cluster consists of in the combination of 6 genes. For example, said gene cluster may be selected according to any combination described in Table 6 which comprises GNLY. In a particular embodiment, the gene cluster consists in the combination IFNG + GNLY + ITGAE + PF4 + IL17A + REN.

In a particular embodiment, the gene cluster consists in the combination of 7 genes. For example, said gene cluster may be selected according to any combination described in Table 7 which comprises GNLY. In a particular embodiment, the gene cluster consists in the combination IFNG + GNLY + ITGAE + CCL5 + PF4 + IL17A + REN.

In a particular embodiment, the gene cluster consists in the combination of 8 genes. For example, said gene cluster may be selected according to any combination described in Table 8 which comprises GNLY. In a particular embodiment, the gene cluster consists in the combination IFNG + GNLY + ITGAE + CCL5 + PF4 + IL17A + REN + PROM1.

In a particular embodiment, the gene cluster consists in the combination of 9 genes. For example, said gene cluster may be selected according to any combination described in

Table 9 which comprises GNLY. In a particular embodiment, the gene cluster consists in the combination GZMH + IFNG + GNLY + ITGAE + CCL5 + PF4 + IL17A + REN + PROM1.

In a particular embodiment, the gene cluster consists in the combination of 10 genes. For example, said gene cluster may be selected according to any combination described in Table 10 which comprises GNLY. In a particular embodiment, the gene cluster consists in the combination GZMH + IFNG + GNLY + ITGAE + CCL5 + PF4 + IL17A + REN + PROM1 + VEGFA.

In a particular embodiment, the gene cluster consists in the combination of 11 genes. For example, said gene cluster may be selected according to any combination described in Table 11 which comprises GNLY. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises GNLY. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises GNLY. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises GNLY. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on LAG3:

An aspect of the disclosure relates to a method for predicting the outcome of a cancer in a patient comprising a step consisting of determining the expression level of a gene cluster in a sample obtained from said patient, wherein said gene cluster comprises LAG3.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, CXCL13, CXCL9, REN, ITGAE, CCL5, TSLP, IL17A, PROM1, IHH, GZMH, IFNG, and VEGFA.

In a particular embodiment, the gene cluster consists in the combination of 2 genes. For example, said gene cluster may be selected according to any combination described in Table 2 which comprises LAG3. In a particular embodiment, the gene cluster consists in the combination GNLY + LAG3.

In a particular embodiment, the gene cluster consists in the combination of 3 genes. For example, said gene cluster may be selected according to any combination described in Table 3 which comprises LAG3. In a particular embodiment, the gene cluster consists in the combination LAG3 + PF4 + REN.

In a particular embodiment, the gene cluster consists in the combination of 4 genes. For example, said gene cluster may be selected according to any combination described in Table 4 which comprises LAG3. In a particular embodiment, the gene cluster consists in the combination GNLY + LAG3 + PF4 + REN.

In a particular embodiment, the gene cluster consists in the combination of 5 genes. For example, said gene cluster may be selected according to any combination described in Table 5 which comprises LAG3. In a particular embodiment, the gene cluster consists in the combination GNLY + LAG3 + PF4 + IL17A + REN.

In a particular embodiment, the gene cluster consists of in the combination of 6 genes. For example, said gene cluster may be selected according to any combination described in Table 6 which comprises LAG3. In a particular embodiment, the gene cluster consists in the combination IFNG + GNLY + LAG3 + PF4 + IL17A + REN.

In a particular embodiment, the gene cluster consists in the combination of 7 genes. For example, said gene cluster may be selected according to any combination described in Table 7 which comprises LAG3. In a particular embodiment, the gene cluster consists in the combination IFNG + GNLY + LAG3 + CCL5 + PF4 + IL17A + REN.

In a particular embodiment, the gene cluster consists in the combination of 8 genes. For example, said gene cluster may be selected according to any combination described in Table 8 which comprises LAG3. In a particular embodiment, the gene cluster consists in the combination IFNG + GNLY + LAG3 + ITGAE + CCL5 + PF4 + IL17A + REN.

In a particular embodiment, the gene cluster consists in the combination of 9 genes. For example, said gene cluster may be selected according to any combination described in Table 9 which comprises LAG3. In a particular embodiment, the gene cluster consists in the combination IFNG + GNLY + LAG3 + ITGAE + CCL5 + PF4 + IL17A + REN + PROM1.

In a particular embodiment, the gene cluster consists in the combination of 10 genes. For example, said gene cluster may be selected according to any combination described in Table 10 which comprises LAG3. In a particular embodiment, the gene cluster consists in the combination GZMH + IFNG + GNLY + LAG3 + ITGAE + CCL5 + PF4 + IL17A + REN + PROM1

In a particular embodiment, the gene cluster consists in the combination of 11 genes. For example, said gene cluster may be selected according to any combination described in Table 11 which comprises LAG3. In a particular embodiment, the gene cluster consists in the combination GZMH + IFNG + GNLY + LAG3 + ITGAE + CCL5 + PF4 + IL17A + REN + PROM1 + VEGFA.

In a particular embodiment, the gene cluster consists in the combination of 12 genes. For example, said gene cluster may be selected according to any combination described in Table 12 which comprises LAG3. In a particular embodiment, the gene cluster consists in the combination GZMH + IFNG + GNLY + LAG3 + ITGAE + CCL5 + PF4 + IL17A + REN + IHH + PROM1 + VEGFA.

In a particular embodiment, the gene cluster consists in the combination of 13 genes. For example, said gene cluster may be selected according to any combination described in Table 13 which comprises LAG3. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises LAG3. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on CXCL13:

An aspect of the disclosure relates to a method for predicting the outcome of a cancer in a patient comprising a step consisting of determining the expression level of a gene cluster in a sample obtained from said patient, wherein said gene cluster comprises CXCL13.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL9, REN, ITGAE, CCL5, TSLP, IL17A, PROM1, IHH, GZMH, IFNG, and VEGFA.

In a particular embodiment, the gene cluster consists in the combination of 2 genes. For example, said gene cluster may be selected according to any combination described in Table 2 which comprises CXCL13. In a particular embodiment, the gene cluster consists in the combination CXCL13 + PF4.

In a particular embodiment, the gene cluster consists in the combination of 3 genes. For example, said gene cluster may be selected according to any combination described in Table 3 which comprises CXCL13. In a particular embodiment, the gene cluster consists in the combination CXCL13 + GNLY + IL17A.

In a particular embodiment, the gene cluster consists in the combination of 4 genes. For example, said gene cluster may be selected according to any combination described in Table 4 which comprises CXCL13. In a particular embodiment, the gene cluster consists in the combination CXCL13 + GNLY + PF4 + REN.

In a particular embodiment, the gene cluster consists in the combination of 5 genes. For example, said gene cluster may be selected according to any combination described in Table 5 which comprises CXCL13. In a particular embodiment, the gene cluster consists in the combination CXCL13 + GNLY + PF4 + IL17A + REN.

In a particular embodiment, the gene cluster consists of in the combination of 6 genes. For example, said gene cluster may be selected according to any combination described in Table 6 which comprises CXCL13. In a particular embodiment, the gene cluster consists in the combination CXCL13 + GNLY + CCL5 + PF4 + IL17A + REN.

In a particular embodiment, the gene cluster consists in the combination of 7 genes. For example, said gene cluster may be selected according to any combination described in Table 7 which comprises CXCL13. In a particular embodiment, the gene cluster consists in the combination CXCL13 + GNLY + ITGAE + CCL5 + PF4 + IL17A + REN.

In a particular embodiment, the gene cluster consists in the combination of 8 genes. For example, said gene cluster may be selected according to any combination described in Table 8 which comprises CXCL13. In a particular embodiment, the gene cluster consists in the combination IFNG + CXCL13 + GNLY + ITGAE + CCL5 + PF4 + IL17A + REN.

In a particular embodiment, the gene cluster consists in the combination of 9 genes. For example, said gene cluster may be selected according to any combination described in Table 9 which comprises CXCL13. In a particular embodiment, the gene cluster consists in the combination IFNG + CXCL13 + GNLY + ITGAE + CCL5 + PF4 + IL17A + REN + PROM1

In a particular embodiment, the gene cluster consists in the combination of 10 genes. For example, said gene cluster may be selected according to any combination described in Table 10 which comprises CXCL13. In a particular embodiment, the gene cluster consists in the combination GZMH + IFNG + CXCL13 + GNLY + ITGAE + CCL5 + PF4 + IL17A + TSLP + REN + IHH + PROM1 + VEGFA.

In a particular embodiment, the gene cluster consists in the combination of 11 genes. For example, said gene cluster may be selected according to any combination described in Table 11 which comprises CXCL13. In a particular embodiment, the gene cluster consists in the combination GZMH + IFNG + CXCL13 + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises CXCL13. In a particular embodiment, the gene cluster consists in the combination GZMH + IFNG + CXCL13 + GNLY + ITGAE + CCL5 + PF4 + IL17A + REN + IHH + PROM1 + VEGFA.

In a particular embodiment, the gene cluster consists in the combination of 13 genes. For example, said gene cluster may be selected according to any combination described in Table 13 which comprises CXCL13. In a particular embodiment, the gene cluster consists in the combination GZMH + IFNG + CXCL13 + GNLY + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises CXCL13. In a particular embodiment, the gene cluster consists in the combination GZMH + IFNG + CXCL13 + GNLY + LAG3 + ITGAE + CCL5 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on PF4:

An aspect of the disclosure relates to a method for predicting the outcome of a cancer in a patient comprising a step consisting of determining the expression level of a gene cluster in a sample obtained from said patient, wherein said gene cluster comprises PF4.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, LAG3, CXCL13, CXCL9, REN, ITGAE, CCL5, TSLP, IL17A, PROM1, IHH, GZMH, IFNG, and VEGFA.

In a particular embodiment, the gene cluster consists in the combination of 2 genes. For example, said gene cluster may be selected according to any combination described in Table 2 which comprises PF4. In a particular embodiment, the gene cluster consists in the combination GNLY + PF4.

In a particular embodiment, the gene cluster consists in the combination of 3 genes. For example, said gene cluster may be selected according to any combination described in Table 3 which comprises PF4. In a particular embodiment, the gene cluster consists in the combination GNLY + PF4 + REN.

In a particular embodiment, the gene cluster consists in the combination of 4 genes. For example, said gene cluster may be selected according to any combination described in Table 4 which comprises PF4. In a particular embodiment, the gene cluster consists in the combination GNLY + PF4 + IL17A + REN.

In a particular embodiment, the gene cluster consists in the combination of 5 genes. For example, said gene cluster may be selected according to any combination described in Table 5 which comprises PF4. In a particular embodiment, the gene cluster consists in the combination IFNG + GNLY + PF4 + IL17A + REN.

In a particular embodiment, the gene cluster consists of in the combination of 6 genes. For example, said gene cluster may be selected according to any combination described in Table 6 which comprises PF4. In a particular embodiment, the gene cluster consists in the combination IFNG + GNLY + ITGAE + PF4 + IL17A + REN.

In a particular embodiment, the gene cluster consists in the combination of 7 genes. For example, said gene cluster may be selected according to any combination described in

Table 7 which comprises PF4. In a particular embodiment, the gene cluster consists in the combination IFNG + GNLY + ITGAE + CCL5 + PF4 + IL17A + REN.

In a particular embodiment, the gene cluster consists in the combination of 8 genes. For example, said gene cluster may be selected according to any combination described in Table 8 which comprises PF4. In a particular embodiment, the gene cluster consists in the combination IFNG + GNLY + ITGAE + CCL5 + PF4 + IL17A + REN + PROM1.

In a particular embodiment, the gene cluster consists in the combination of 9 genes. For example, said gene cluster may be selected according to any combination described in Table 9 which comprises PF4. In a particular embodiment, the gene cluster consists in the combination GZMH + IFNG + GNLY + ITGAE + CCL5 + PF4 + IL17A + REN + PROM1.

In a particular embodiment, the gene cluster consists in the combination of 10 genes. For example, said gene cluster may be selected according to any combination described in Table 10 which comprises PF4. In a particular embodiment, the gene cluster consists in the combination GZMH + IFNG + GNLY + ITGAE + CCL5 + PF4 + IL17A + REN + PROM1 + VEGFA.

In a particular embodiment, the gene cluster consists in the combination of 11 genes. For example, said gene cluster may be selected according to any combination described in Table 11 which comprises PF4. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises PF4. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises PF4. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises PF4. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on CXCL9:

An aspect of the disclosure relates to a method for predicting the outcome of a cancer in a patient comprising a step consisting of determining the expression level of a gene cluster in a sample obtained from said patient, wherein said gene cluster comprises CXCL9.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, REN, ITGAE, CCL5, TSLP, IL17A, PROM1, IHH, GZMH, IFNG, and VEGFA.

In a particular embodiment, the gene cluster consists in the combination of 2 genes. For example, said gene cluster may be selected according to any combination described in Table 2 which comprises CXCL9. In a particular embodiment, the gene cluster consists in the combination CXCL9 + PF4.

In a particular embodiment, the gene cluster consists in the combination of 3 genes. For example, said gene cluster may be selected according to any combination described in Table 3 which comprises CXCL9. In a particular embodiment, the gene cluster consists in the combination CXCL9 + PF4 + REN.

In a particular embodiment, the gene cluster consists in the combination of 4 genes. For example, said gene cluster may be selected according to any combination described in Table 4 which comprises CXCL9. In a particular embodiment, the gene cluster consists in the combination GNLY + CXCL9 + PF4 + REN.

In a particular embodiment, the gene cluster consists in the combination of 5 genes. For example, said gene cluster may be selected according to any combination described in Table 5 which comprises CXCL9. In a particular embodiment, the gene cluster consists in the combination GNLY + CXCL9 + PF4 + IL17A + REN.

In a particular embodiment, the gene cluster consists of in the combination of 6 genes. For example, said gene cluster may be selected according to any combination described in Table 6 which comprises CXCL9. In a particular embodiment, the gene cluster consists in the combination IFNG + GNLY + CXCL9 + PF4 + IL17A + REN.

In a particular embodiment, the gene cluster consists in the combination of 7 genes. For example, said gene cluster may be selected according to any combination described in Table 7 which comprises CXCL9. In a particular embodiment, the gene cluster consists in the combination IFNG + GNLY + CCL5 + CXCL9 + PF4 + IL17A + REN.

In a particular embodiment, the gene cluster consists in the combination of 8 genes. For example, said gene cluster may be selected according to any combination described in Table 8 which comprises CXCL9. In a particular embodiment, the gene cluster consists in the combination IFNG + GNLY + ITGAE + CCL5 + CXCL9 + PF4 + IL17A + REN.

In a particular embodiment, the gene cluster consists in the combination of 9 genes. For example, said gene cluster may be selected according to any combination described in Table 9 which comprises CXCL9. In a particular embodiment, the gene cluster consists in the combination GZMH + IFNG + GNLY + ITGAE + CCL5 + CXCL9 + PF4 + IL17A + REN.

In a particular embodiment, the gene cluster consists in the combination of 10 genes. For example, said gene cluster may be selected according to any combination described in Table 10 which comprises CXCL9. In a particular embodiment, the gene cluster consists in the combination GZMH + IFNG + GNLY + ITGAE + CCL5 + CXCL9 + PF4 + IL17A + REN + VEGFA.

In a particular embodiment, the gene cluster consists in the combination of 11 genes. For example, said gene cluster may be selected according to any combination described in Table 11 which comprises CXCL9. In a particular embodiment, the gene cluster consists in the combination GZMH + IFNG + GNLY + ITGAE + CCL5 + CXCL9 + PF4 + IL17A + REN + IHH + VEGFA.

In a particular embodiment, the gene cluster consists in the combination of 12 genes. For example, said gene cluster may be selected according to any combination described in Table 12 which comprises CXCL9. In a particular embodiment, the gene cluster consists in the combination GZMH + IFNG + GNLY + ITGAE + CCL5 + CXCL9 + PF4 + IL17A + TSLP + REN + IHH + VEGFA.

In a particular embodiment, the gene cluster consists in the combination of 13 genes. For example, said gene cluster may be selected according to any combination described in Table 13 which comprises CXCL9. In a particular embodiment, the gene cluster consists in the combination GZMH + IFNG + GNLY + ITGAE + CCL5 + CXCL9 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises CXCL9. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on REN:

An aspect of the disclosure relates to a method for predicting the outcome of a cancer in a patient comprising a step consisting of determining the expression level of a gene cluster in a sample obtained from said patient, wherein said gene cluster comprises REN.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, CXCL9, ITGAE, CCL5, TSLP, IL17A, PROM1, IHH, GZMH, IFNG, and VEGFA.

In a particular embodiment, the gene cluster consists in the combination of 2 genes. For example, said gene cluster may be selected according to any combination described in Table 2 which comprises REN. In a particular embodiment, the gene cluster consists in the combination GNLY + REN.

In a particular embodiment, the gene cluster consists in the combination of 3 genes. For example, said gene cluster may be selected according to any combination described in Table 3 which comprises REN. In a particular embodiment, the gene cluster consists in the combination GNLY + PF4 + REN.

In a particular embodiment, the gene cluster consists in the combination of 4 genes. For example, said gene cluster may be selected according to any combination described in Table 4 which comprises REN. In a particular embodiment, the gene cluster consists in the combination GNLY + PF4 + IL17A + REN.

In a particular embodiment, the gene cluster consists in the combination of 5 genes. For example, said gene cluster may be selected according to any combination described in Table 5 which comprises REN. In a particular embodiment, the gene cluster consists in the combination IFNG + GNLY + PF4 + IL17A + REN.

In a particular embodiment, the gene cluster consists of in the combination of 6 genes. For example, said gene cluster may be selected according to any combination described in Table 6 which comprises REN. In a particular embodiment, the gene cluster consists in the combination IFNG + GNLY + ITGAE + PF4 + IL17A + REN.

In a particular embodiment, the gene cluster consists in the combination of 7 genes. For example, said gene cluster may be selected according to any combination described in Table 7 which comprises REN. In a particular embodiment, the gene cluster consists in the combination IFNG + GNLY + ITGAE + CCL5 + PF4 + IL17A + REN.

In a particular embodiment, the gene cluster consists in the combination of 8 genes. For example, said gene cluster may be selected according to any combination described in Table 8 which comprises REN. In a particular embodiment, the gene cluster consists in the combination IFNG + GNLY + ITGAE + CCL5 + PF4 + IL17A + REN + PROM1.

In a particular embodiment, the gene cluster consists in the combination of 9 genes. For example, said gene cluster may be selected according to any combination described in Table 9 which comprises REN. In a particular embodiment, the gene cluster consists in the combination GZMH + IFNG + GNLY + ITGAE + CCL5 + PF4 + IL17A + REN + PROM1.

In a particular embodiment, the gene cluster consists in the combination of 10 genes. For example, said gene cluster may be selected according to any combination described in Table 10 which comprises REN. In a particular embodiment, the gene cluster consists in the combination GZMH + IFNG + GNLY + ITGAE + CCL5 + PF4 + IL17A + REN + PROM1 + VEGFA.

In a particular embodiment, the gene cluster consists in the combination of 11 genes. For example, said gene cluster may be selected according to any combination described in Table 11 which comprises REN. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises REN. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises REN. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises REN. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on ITGAE

An aspect of the disclosure relates to a method for predicting the outcome of a cancer in a patient comprising a step consisting of determining the expression level of a gene cluster in a sample obtained from said patient, wherein said gene cluster comprises ITGAE.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, CXCL9, REN, CCL5, TSLP, IL17A, PROM1, IHH, GZMH, IFNG, and VEGFA.

In a particular embodiment, the gene cluster consists in the combination of 2 genes. For example, said gene cluster may be selected according to any combination described in Table 2 which comprises ITGAE. In a particular embodiment, the gene cluster consists in the combination ITGAE + PF4.

In a particular embodiment, the gene cluster consists in the combination of 3 genes. For example, said gene cluster may be selected according to any combination described in Table 3 which comprises ITGAE. In a particular embodiment, the gene cluster consists in the combination ITGAE + PF4 + REN.

In a particular embodiment, the gene cluster consists in the combination of 4 genes. For example, said gene cluster may be selected according to any combination described in Table 4 which comprises ITGAE. In a particular embodiment, the gene cluster consists in the combination GNLY + ITGAE + PF4 + REN.

In a particular embodiment, the gene cluster consists in the combination of 5 genes. For example, said gene cluster may be selected according to any combination described in Table 5 which comprises ITGAE. In a particular embodiment, the gene cluster consists in the combination IFNG + GNLY + ITGAE + IL17A + REN.

In a particular embodiment, the gene cluster consists of in the combination of 6 genes. For example, said gene cluster may be selected according to any combination described in Table 6 which comprises ITGAE. In a particular embodiment, the gene cluster consists in the combination IFNG + GNLY + ITGAE + PF4 + IL17A + REN.

In a particular embodiment, the gene cluster consists in the combination of 7 genes. For example, said gene cluster may be selected according to any combination described in Table 7 which comprises ITGAE. In a particular embodiment, the gene cluster consists in the combination IFNG + GNLY + ITGAE + CCL5 + PF4 + IL17A + REN.

In a particular embodiment, the gene cluster consists in the combination of 8 genes. For example, said gene cluster may be selected according to any combination described in Table 8 which comprises ITGAE. In a particular embodiment, the gene cluster consists in the combination IFNG + GNLY + ITGAE + CCL5 + PF4 + IL17A + REN + PROM1.

In a particular embodiment, the gene cluster consists in the combination of 9 genes. For example, said gene cluster may be selected according to any combination described in Table 9 which comprises ITGAE. In a particular embodiment, the gene cluster consists in the combination GZMH + IFNG + GNLY + ITGAE + CCL5 + PF4 + IL17A + REN + PROM1.

In a particular embodiment, the gene cluster consists in the combination of 10 genes. For example, said gene cluster may be selected according to any combination described in Table 10 which comprises ITGAE. In a particular embodiment, the gene cluster consists in the combination GZMH + IFNG + GNLY + ITGAE + CCL5 + PF4 + IL17A + REN + PROM1 + VEGFA.

In a particular embodiment, the gene cluster consists in the combination of 11 genes. For example, said gene cluster may be selected according to any combination described in Table 11 which comprises ITGAE. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises ITGAE. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises ITGAE. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises ITGAE. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on CCL5

An aspect of the disclosure relates to a method for predicting the outcome of a cancer in a patient comprising a step consisting of determining the expression level of a gene cluster in a sample obtained from said patient, wherein said gene cluster comprises CCL5.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, CXCL9, REN, ITGAE, TSLP, IL17A, PROM1, IHH, GZMH, IFNG, and VEGFA.

In a particular embodiment, the gene cluster consists in the combination of 2 genes. For example, said gene cluster may be selected according to any combination described in Table 2 which comprises CCL5. In a particular embodiment, the gene cluster consists in the combination GNLY + CCL5.

In a particular embodiment, the gene cluster consists in the combination of 3 genes. For example, said gene cluster may be selected according to any combination described in Table 3 which comprises CCL5. In a particular embodiment, the gene cluster consists in the combination GNLY + CCL5 + PF4.

In a particular embodiment, the gene cluster consists in the combination of 4 genes. For example, said gene cluster may be selected according to any combination described in Table 4 which comprises CCL5. In a particular embodiment, the gene cluster consists in the combination GNLY + CCL5 + PF4 + REN.

In a particular embodiment, the gene cluster consists in the combination of 5 genes. For example, said gene cluster may be selected according to any combination described in Table 5 which comprises CCL5. In a particular embodiment, the gene cluster consists in the combination GNLY + CCL5 + PF4 + IL17A + REN.

In a particular embodiment, the gene cluster consists of in the combination of 6 genes. For example, said gene cluster may be selected according to any combination described in Table 6 which comprises CCL5. In a particular embodiment, the gene cluster consists in the combination IFNG + GNLY + CCL5 + PF4 + IL17A + REN.

In a particular embodiment, the gene cluster consists in the combination of 7 genes. For example, said gene cluster may be selected according to any combination described in

Table 7 which comprises CCL5. In a particular embodiment, the gene cluster consists in the combination IFNG + GNLY + ITGAE + CCL5 + PF4 + IL17A + REN.

In a particular embodiment, the gene cluster consists in the combination of 8 genes. For example, said gene cluster may be selected according to any combination described in Table 8 which comprises CCL5. In a particular embodiment, the gene cluster consists in the combination IFNG + GNLY + ITGAE + CCL5 + PF4 + IL17A + REN + PROM1.

In a particular embodiment, the gene cluster consists in the combination of 9 genes. For example, said gene cluster may be selected according to any combination described in Table 9 which comprises CCL5. In a particular embodiment, the gene cluster consists in the combination GZMH + IFNG + GNLY + ITGAE + CCL5 + PF4 + IL17A + REN + PROM1.

In a particular embodiment, the gene cluster consists in the combination of 10 genes. For example, said gene cluster may be selected according to any combination described in Table 10 which comprises CCL5. In a particular embodiment, the gene cluster consists in the combination GZMH + IFNG + GNLY + ITGAE + CCL5 + PF4 + IL17A + REN + PROM1 + VEGFA.

In a particular embodiment, the gene cluster consists in the combination of 11 genes. For example, said gene cluster may be selected according to any combination described in Table 11 which comprises CCL5. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises CCL5. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises CCL5. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises CCL5. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on TSLP

An aspect of the disclosure relates to a method for predicting the outcome of a cancer in a patient comprising a step consisting of determining the expression level of a gene cluster in a sample obtained from said patient, wherein said gene cluster comprises TSLP.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, CXCL9, REN, ITGAE, CCL5, IL17A, PROM1, IHH, GZMH, IFNG, and VEGFA.

In a particular embodiment, the gene cluster consists in the combination of 2 genes. For example, said gene cluster may be selected according to any combination described in Table 2 which comprises TSLP. In a particular embodiment, the gene cluster consists in the combination GNLY + TSLP.

In a particular embodiment, the gene cluster consists in the combination of 3 genes. For example, said gene cluster may be selected according to any combination described in Table 3 which comprises TSLP. In a particular embodiment, the gene cluster consists in the combination IFNG + GNLY + TSLP.

In a particular embodiment, the gene cluster consists in the combination of 4 genes. For example, said gene cluster may be selected according to any combination described in Table 4 which comprises TSLP. In a particular embodiment, the gene cluster consists in the combination GNLY + PF4 + TSLP + REN.

In a particular embodiment, the gene cluster consists in the combination of 5 genes. For example, said gene cluster may be selected according to any combination described in Table 5 which comprises TSLP. In a particular embodiment, the gene cluster consists in the combination GNLY + PF4 + IL17A + TSLP + REN.

In a particular embodiment, the gene cluster consists of in the combination of 6 genes. For example, said gene cluster may be selected according to any combination described in Table 6 which comprises TSLP. In a particular embodiment, the gene cluster consists in the combination IFNG + GNLY + PF4 + IL17A + TSLP + REN.

In a particular embodiment, the gene cluster consists in the combination of 7 genes. For example, said gene cluster may be selected according to any combination described in Table 7 which comprises TSLP. In a particular embodiment, the gene cluster consists in the combination IFNG + GNLY + ITGAE + PF4 + IL17A + TSLP + REN.

In a particular embodiment, the gene cluster consists in the combination of 8 genes. For example, said gene cluster may be selected according to any combination described in Table 8 which comprises TSLP. In a particular embodiment, the gene cluster consists in the combination IFNG + GNLY + ITGAE + CCL5 + PF4 + IL17A + TSLP + REN.

In a particular embodiment, the gene cluster consists in the combination of 9 genes. For example, said gene cluster may be selected according to any combination described in Table 9 which comprises TSLP. In a particular embodiment, the gene cluster consists in the combination IFNG + GNLY + ITGAE + CCL5 + PF4 + IL17A + TSLP + REN + PROM1.

In a particular embodiment, the gene cluster consists in the combination of 10 genes. For example, said gene cluster may be selected according to any combination described in Table 10 which comprises TSLP. In a particular embodiment, the gene cluster consists in the combination GZMH + IFNG + GNLY + ITGAE + CCL5 + PF4 + IL17A + TSLP + REN + PROM1.

In a particular embodiment, the gene cluster consists in the combination of 11 genes. For example, said gene cluster may be selected according to any combination described in Table 11 which comprises TSLP. In a particular embodiment, the gene cluster consists in the combination GZMH + IFNG + GNLY + ITGAE + CCL5 + PF4 + IL17A + TSLP + REN + PROM1 + VEGFA.

In a particular embodiment, the gene cluster consists in the combination of 12 genes. For example, said gene cluster may be selected according to any combination described in Table 12 which comprises TSLP. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises TSLP. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises TSLP. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on IL17A

An aspect of the disclosure relates to a method for predicting the outcome of a cancer in a patient comprising a step consisting of determining the expression level of a gene cluster in a sample obtained from said patient, wherein said gene cluster comprises IL17A.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, CXCL9, REN, ITGAE, CCL5, TSLP, PROM1, IHH, GZMH, IFNG, and VEGFA.

In a particular embodiment, the gene cluster consists in the combination of 2 genes. For example, said gene cluster may be selected according to any combination described in Table 2 which comprises IL17A. In a particular embodiment, the gene cluster consists in the combination GNLY + IL17A.

In a particular embodiment, the gene cluster consists in the combination of 3 genes. For example, said gene cluster may be selected according to any combination described in Table 3 which comprises IL17A. In a particular embodiment, the gene cluster consists in the combination GNLY + IL17A + REN.

In a particular embodiment, the gene cluster consists in the combination of 4 genes. For example, said gene cluster may be selected according to any combination described in Table 4 which comprises IL17A. In a particular embodiment, the gene cluster consists in the combination GNLY + PF4 + IL17A + REN.

In a particular embodiment, the gene cluster consists in the combination of 5 genes. For example, said gene cluster may be selected according to any combination described in Table 5 which comprises IL17A. In a particular embodiment, the gene cluster consists in the combination IFNG + GNLY + PF4 + IL17A + REN.

In a particular embodiment, the gene cluster consists of in the combination of 6 genes. For example, said gene cluster may be selected according to any combination described in Table 6 which comprises IL17A. In a particular embodiment, the gene cluster consists in the combination IFNG + GNLY + ITGAE + PF4 + IL17A + REN.

In a particular embodiment, the gene cluster consists in the combination of 7 genes. For example, said gene cluster may be selected according to any combination described in Table 7 which comprises IL17A. In a particular embodiment, the gene cluster consists in the combination IFNG + GNLY + ITGAE + CCL5 + PF4 + IL17A + REN.

In a particular embodiment, the gene cluster consists in the combination of 8 genes. For example, said gene cluster may be selected according to any combination described in Table 8 which comprises IL17A. In a particular embodiment, the gene cluster consists in the combination IFNG + GNLY + ITGAE + CCL5 + PF4 + IL17A + REN + PROM1.

In a particular embodiment, the gene cluster consists in the combination of 9 genes. For example, said gene cluster may be selected according to any combination described in Table 9 which comprises IL17A. In a particular embodiment, the gene cluster consists in the combination GZMH + IFNG + GNLY + ITGAE + CCL5 + PF4 + IL17A + REN + PROM1.

In a particular embodiment, the gene cluster consists in the combination of 10 genes. For example, said gene cluster may be selected according to any combination described in Table 10 which comprises IL17A. In a particular embodiment, the gene cluster consists in the combination GZMH + IFNG + GNLY + ITGAE + CCL5 + PF4 + IL17A + REN + PROM1 + VEGFA.

In a particular embodiment, the gene cluster consists in the combination of 11 genes. For example, said gene cluster may be selected according to any combination described in Table 11 which comprises IL17A. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises IL17A. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises IL17A. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises IL17A. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on GZMH

An aspect of the disclosure relates to a method for predicting the outcome of a cancer in a patient comprising a step consisting of determining the expression level of a gene cluster in a sample obtained from said patient, wherein said gene cluster comprises GZMH.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, CXCL9, REN, ITGAE, CCL5, TSLP, PROM1, IHH, IL17A, IFNG, and VEGFA.

In a particular embodiment, the gene cluster consists in the combination of 2 genes. For example, said gene cluster may be selected according to any combination described in Table 2 which comprises GZMH. In a particular embodiment, the gene cluster consists in the combination GZMH + GNLY.

In a particular embodiment, the gene cluster consists in the combination of 3 genes. For example, said gene cluster may be selected according to any combination described in Table 3 which comprises GZMH. In a particular embodiment, the gene cluster consists in the combination GZMH + GNLY + REN.

In a particular embodiment, the gene cluster consists in the combination of 4 genes. For example, said gene cluster may be selected according to any combination described in Table 4 which comprises GZMH. In a particular embodiment, the gene cluster consists in the combination GZMH + GNLY + PF4 + REN.

In a particular embodiment, the gene cluster consists in the combination of 5 genes. For example, said gene cluster may be selected according to any combination described in Table 5 which comprises GZMH. In a particular embodiment, the gene cluster consists in the combination GZMH + GNLY + PF4 + IL17A + REN.

In a particular embodiment, the gene cluster consists of in the combination of 6 genes. For example, said gene cluster may be selected according to any combination described in Table 6 which comprises GZMH. In a particular embodiment, the gene cluster consists in the combination GZMH + IFNG + GNLY + PF4 + IL17A + REN.

In a particular embodiment, the gene cluster consists in the combination of 7 genes. For example, said gene cluster may be selected according to any combination described in Table 7 which comprises GZMH. In a particular embodiment, the gene cluster consists in the combination GZMH + IFNG + GNLY + CCL5 + PF4 + IL17A + REN.

In a particular embodiment, the gene cluster consists in the combination of 8 genes. For example, said gene cluster may be selected according to any combination described in Table 8 which comprises GZMH. In a particular embodiment, the gene cluster consists in the combination GZMH + IFNG + GNLY + ITGAE + CCL5 + PF4 + IL17A + REN.

In a particular embodiment, the gene cluster consists in the combination of 9 genes. For example, said gene cluster may be selected according to any combination described in Table 9 which comprises GZMH. In a particular embodiment, the gene cluster consists in the combination GZMH + IFNG + GNLY + ITGAE + CCL5 + PF4 + IL17A + REN + PROM1.

In a particular embodiment, the gene cluster consists in the combination of 10 genes. For example, said gene cluster may be selected according to any combination described in Table 10 which comprises GZMH. In a particular embodiment, the gene cluster consists in the combination GZMH + IFNG + GNLY + ITGAE + CCL5 + PF4 + IL17A + REN + PROM1 + VEGFA.

In a particular embodiment, the gene cluster consists in the combination of 11 genes. For example, said gene cluster may be selected according to any combination described in Table 11 which comprises GZMH. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises GZMH. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises GZMH. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises GZMH. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on PROM1

An aspect of the disclosure relates to a method for predicting the outcome of a cancer in a patient comprising a step consisting of determining the expression level of a gene cluster in a sample obtained from said patient, wherein said gene cluster comprises PROM1.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, CXCL9, REN, ITGAE, CCL5, TSLP, GZMH, IHH, IL17A, IFNG, and VEGFA.

In a particular embodiment, the gene cluster consists in the combination of 2 genes. For example, said gene cluster may be selected according to any combination described in Table 2 which comprises PROM1. In a particular embodiment, the gene cluster consists in the combination GNLY + PROM1.

In a particular embodiment, the gene cluster consists in the combination of 3 genes. For example, said gene cluster may be selected according to any combination described in Table 3 which comprises PROM1. In a particular embodiment, the gene cluster consists in the combination GNLY + PF4 + PROM1.

In a particular embodiment, the gene cluster consists in the combination of 4 genes. For example, said gene cluster may be selected according to any combination described in Table 4 which comprises PROM1. In a particular embodiment, the gene cluster consists in the combination GNLY + PF4 + REN + PROM1.

In a particular embodiment, the gene cluster consists in the combination of 5 genes. For example, said gene cluster may be selected according to any combination described in Table 5 which comprises PROM1. In a particular embodiment, the gene cluster consists in the combination GNLY + PF4 + IL17A + REN + PROM1.

In a particular embodiment, the gene cluster consists of in the combination of 6 genes. For example, said gene cluster may be selected according to any combination described in Table 6 which comprises PROM1. In a particular embodiment, the gene cluster consists in the combination IFNG + GNLY + PF4 + IL17A + REN + PROM1.

In a particular embodiment, the gene cluster consists in the combination of 7 genes. For example, said gene cluster may be selected according to any combination described in Table 7 which comprises PROM1. In a particular embodiment, the gene cluster consists in the combination GNLY + ITGAE + CCL5 + PF4 + IL17A + REN + PROM1.

In a particular embodiment, the gene cluster consists in the combination of 8 genes. For example, said gene cluster may be selected according to any combination described in Table 8 which comprises PROM1. In a particular embodiment, the gene cluster consists in the combination IFNG + GNLY + ITGAE + CCL5 + PF4 + IL17A + REN + PROM1.

In a particular embodiment, the gene cluster consists in the combination of 9 genes. For example, said gene cluster may be selected according to any combination described in Table 9 which comprises PROM1. In a particular embodiment, the gene cluster consists in the combination GZMH + IFNG + GNLY + ITGAE + CCL5 + PF4 + IL17A + REN + PROM1.

In a particular embodiment, the gene cluster consists in the combination of 10 genes. For example, said gene cluster may be selected according to any combination described in Table 10 which comprises PROM1. In a particular embodiment, the gene cluster consists in the combination GZMH + IFNG + GNLY + ITGAE + CCL5 + PF4 + IL17A + REN + PROM1 + VEGFA.

In a particular embodiment, the gene cluster consists in the combination of 11 genes. For example, said gene cluster may be selected according to any combination described in Table 11 which comprises PROM1. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises PROM1. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises PROM1. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises PROM1. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on IHH

An aspect of the disclosure relates to a method for predicting the outcome of a cancer in a patient comprising a step consisting of determining the expression level of a gene cluster in a sample obtained from said patient, wherein said gene cluster comprises IHH

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, CXCL9, REN, ITGAE, CCL5, TSLP, GZMH, PROM1, IL17A, IFNG, and VEGFA.

In a particular embodiment, the gene cluster consists in the combination of 2 genes. For example, said gene cluster may be selected according to any combination described in Table 2 which comprises IHH. In a particular embodiment, the gene cluster consists in the combination GNLY + IHH

In a particular embodiment, the gene cluster consists in the combination of 3 genes. For example, said gene cluster may be selected according to any combination described in Table 3 which comprises IHH. In a particular embodiment, the gene cluster consists in the combination GNLY + PF4 + IHH

In a particular embodiment, the gene cluster consists in the combination of 4 genes. For example, said gene cluster may be selected according to any combination described in Table 4 which comprises IHH. In a particular embodiment, the gene cluster consists in the combination GNLY + PF4 + REN + IHH

In a particular embodiment, the gene cluster consists in the combination of 5 genes. For example, said gene cluster may be selected according to any combination described in Table 5 which comprises IHH. In a particular embodiment, the gene cluster consists in the combination GNLY + PF4 + IL17A + REN + IHH

In a particular embodiment, the gene cluster consists of in the combination of 6 genes. For example, said gene cluster may be selected according to any combination described in Table 6 which comprises IHH. In a particular embodiment, the gene cluster consists in the combination IFNG + GNLY + PF4 + IL17A + REN + IHH

In a particular embodiment, the gene cluster consists in the combination of 7 genes. For example, said gene cluster may be selected according to any combination described in Table 7 which comprises IHH. In a particular embodiment, the gene cluster consists in the combination IFNG + GNLY + CCL5 + PF4 + IL17A + REN + IHH

In a particular embodiment, the gene cluster consists in the combination of 8 genes. For example, said gene cluster may be selected according to any combination described in Table 8 which comprises IHH. In a particular embodiment, the gene cluster consists in the combination IFNG + GNLY + ITGAE + CCL5 + PF4 + IL17A + REN + IHH

In a particular embodiment, the gene cluster consists in the combination of 9 genes. For example, said gene cluster may be selected according to any combination described in Table 9 which comprises IHH. In a particular embodiment, the gene cluster consists in the combination IFNG + GNLY + ITGAE + CCL5 + PF4 + IL17A + REN + IHH + PROM1.

In a particular embodiment, the gene cluster consists in the combination of 10 genes. For example, said gene cluster may be selected according to any combination described in Table 10 which comprises IHH. In a particular embodiment, the gene cluster consists in the combination GZMH + IFNG + GNLY + ITGAE + CCL5 + PF4 + IL17A + REN + IHH + PROM1.

In a particular embodiment, the gene cluster consists in the combination of 11 genes. For example, said gene cluster may be selected according to any combination described in Table 11 which comprises IHH. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises IHH. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises IHH. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises IHH. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Gene clusters based on IFNG

An aspect of the disclosure relates to a method for predicting the outcome of a cancer in a patient comprising a step consisting of determining the expression level of a gene cluster in a sample obtained from said patient, wherein said gene cluster comprises IFNG.

The inventors have been indeed demonstrated that said gene provides a greater accuracy for predicting the outcome of a cancer than the UICC-TNM classification (i.e. the gene provides a ROC curve which results in a larger area under the curve than the one with TNM) (see Table 1).

In a particular embodiment, the gene cluster further comprises at least one gene selected from the group consisting of GNLY, PF4, LAG3, CXCL13, CXCL9, REN, ITGAE, CCL5, TSLP, GZMH, PROM1, IL17A, IHH, and VEGFA.

In a particular embodiment, the gene cluster consists in the combination of 2 genes. For example, said gene cluster may be selected according to any combination described in Table 2 which comprises IFNG. In a particular embodiment, the gene cluster consists in the combination IFNG + GNLY.

In a particular embodiment, the gene cluster consists in the combination of 3 genes. For example, said gene cluster may be selected according to any combination described in Table 3 which comprises IFNG. In a particular embodiment, the gene cluster consists in the combination IFNG + GNLY + IL17A.

In a particular embodiment, the gene cluster consists in the combination of 4 genes. For example, said gene cluster may be selected according to any combination described in Table 4 which comprises IFNG. In a particular embodiment, the gene cluster consists in the combination IFNG + GNLY + PF4 + REN.

In a particular embodiment, the gene cluster consists in the combination of 5 genes. For example, said gene cluster may be selected according to any combination described in Table 5 which comprises IFNG. In a particular embodiment, the gene cluster consists in the combination IFNG + GNLY + PF4 + IL17A + REN.

In a particular embodiment, the gene cluster consists of in the combination of 6 genes. For example, said gene cluster may be selected according to any combination described in Table 6 which comprises IFNG. In a particular embodiment, the gene cluster consists in the combination IFNG + GNLY + ITGAE + PF4 + IL17A + REN.

In a particular embodiment, the gene cluster consists in the combination of 7 genes. For example, said gene cluster may be selected according to any combination described in Table 7 which comprises IFNG. In a particular embodiment, the gene cluster consists in the combination IFNG + GNLY + ITGAE + CCL5 + PF4 + IL17A + REN.

In a particular embodiment, the gene cluster consists in the combination of 8 genes. For example, said gene cluster may be selected according to any combination described in Table 8 which comprises IFNG. In a particular embodiment, the gene cluster consists in the combination IFNG + GNLY + ITGAE + CCL5 + PF4 + IL17A + REN + PROM1.

In a particular embodiment, the gene cluster consists in the combination of 9 genes. For example, said gene cluster may be selected according to any combination described in Table 9 which comprises IFNG. In a particular embodiment, the gene cluster consists in the combination GZMH + IFNG + GNLY + ITGAE + CCL5 + PF4 + IL17A + REN + PROM1.

In a particular embodiment, the gene cluster consists in the combination of 10 genes. For example, said gene cluster may be selected according to any combination described in Table 10 which comprises IFNG. In a particular embodiment, the gene cluster consists in the combination GZMH + IFNG + GNLY + ITGAE + CCL5 + PF4 + IL17A + REN + PROM1 + VEGFA.

In a particular embodiment, the gene cluster consists in the combination of 11 genes. For example, said gene cluster may be selected according to any combination described in Table 11 which comprises IFNG. In a particular embodiment, the gene cluster consists in the combination GZMH + IFNG + GNLY + ITGAE + CCL5 + PF4 + IL17A + REN + IHH + PROM1 + VEGFA.

In a particular embodiment, the gene cluster consists in the combination of 12 genes. For example, said gene cluster may be selected according to any combination described in Table 12 which comprises IFNG. In a particular embodiment, the gene cluster consists in the combination GZMH + IFNG + GNLY + ITGAE + CCL5 + PF4 + IL17A + TSLP + REN + IHH + PROM1 + VEGFA.

In a particular embodiment, the gene cluster consists in the combination of 13 genes. For example, said gene cluster may be selected according to any combination described in Table 13 which comprises IFNG. In a particular embodiment, the gene cluster consists in the combination GZMH + IFNG + GNLY + LAG3 + ITGAE + CCL5 + PF4 + IL17A + TSLP + REN + IHH + PROM1 + VEGFA.

In a particular embodiment, the gene cluster consists in the combination of 14 genes. For example, said gene cluster may be selected according to any combination described in Table 14 which comprises IFNG. In a particular embodiment, the gene cluster consists in the combination GZMH + IFNG + GNLY + LAG3 + ITGAE + CCL5 + CXCL9 + PF4 + IL17A + TSLP + REN + IHH + PROM1 + VEGFA.

In a particular embodiment, the gene cluster consists in the combination of 15 genes. In a particular embodiment, the gene cluster consists in the combination GZMH + IFNG + CXCL13 + GNLY + LAG3 + ITGAE + CCL5 + CXCL9 + PF4 + IL17A + TSLP + REN + IHH + PROM1 + VEGFA.

### Techniques for measuring the expression level of the gene clusters:

Measuring the expression level of the gene clusters of the invention in the sample obtained form the patient can be performed by a variety of techniques well known in the art.

Typically, the expression level of a gene may be determined by determining the quantity of mRNA. Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from cumulus cells and subjecting the RNAs to amplification and hybridization to specific probes, more particularly by means of a quantitative or semi-quantitative RT-PCR.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210).

Expression level of a gene may be expressed as absolute expression level or normalized expression level. Typically, expression levels are normalized by correcting the absolute expression level of a gene by comparing its expression to the expression of a gene that is not a relevant for determining the cancer stage of the patient, e.g., a housekeeping gene that is constitutively expressed. Suitable genes for normalization include housekeeping genes such as the actin gene ACTB, ribosomal 18S gene, GUSB, PGK1 and TFRC. This normalization allows the comparison of the expression level in one sample, e.g., a patient sample, to another sample, or between samples from different sources.

### Reference levels

It is specifically contemplated that the invention can be used to evaluate differences between stages of the cancer, such as between hyperplasia, neoplasia, pre-cancer and cancer, or between a primary tumor and a metastasized tumor.

Therefore the methods of the invention further comprises a step consisting of comparing the expression level of one gene cluster (as above described) determined in the sample of the patient with a reference expression level of said gene cluster, wherein a difference between said expression levels is indicative of a the stage of the cancer in the patient.

The reference expression levels according to the invention are correlated with a specific cancer stage. The reference expression levels may thus consist in the expression level of said gene cluster in a tissue reference such a tissue representative of a non-cancerous stage or a tissue representative of a tissue representative of cancer stage. Accordingly, the reference levels may be predetermined by carrying out a method comprising the steps of a) providing at least one collection of tumor tissue samples selected from the group consisting of a collection of tumor tissue samples from cancer patients having different stages such as between hyperplasia, neoplasia, pre-cancer and cancer, or between a primary tumor and a metastasized tumour, b) determining for each tumor tissue sample comprised in a collection of tumor tissue samples provided at step a), the expression level of said gene clusters

Alternatively, the expression level determined for one gene cluster may be expressed as a score. Typically said score may be obtained by i) determining for every gene of the cluster their expression level in the sample ii) allocating for every gene a positive coefficient (e.g. +1) when the gene is associated with a good prognosis and a negative coefficient (e.g. - 1) when the gene is associated with a bad prognosis and iii) multiplying the expression level of one gene with the coefficient allocated in step ii) and iv) adding up all values determining at step iii) for obtaining said score. Typically a coefficient of +1 is allocated to the genes associated with a good prognosis, namely GNLY, CXCL13, CXCL9, LAG3, CCL5, GZMH, ITGAE and IFNG and a coefficient of -1 is allocated to the genes associates with a bad prognosis, namely PF4, REN, TSLP, IL17A, PROM1, IHH and VEGFA. Preferably, the expression levels are expressed as normalized expression levels as above described.

The advantage of said score is to make easier the comparison step with the reference levels that may be expressed as "cut-off values". For example the reference ("cut-off") value represents the score calculated for the gene cluster of interest in a tissue sample representative of a specific cancer stage. The cut-off values may also be predetermined by carrying out a method comprising the steps of:
a) selecting a gene cluster for which a reference value is to be determined;
b) providing a collection of tumor tissue samples from cancer patients;
c) providing, for each tumor sample provided at step b), information relating to the actual clinical outcome for the corresponding cancer patient;
d) providing a serial of arbitrary values for said gene cluster obtained at step a)
e) calculated the score of said gene cluster provided at step a) for each tumor tissue sample contained in the collection provided at step b)
f) classifying said tumor samples in two groups for one specific arbitrary value provided at step c), respectively :
   (i) a first group comprising tumor samples that exhibit a score for said gene cluster that is lower than the said arbitrary value contained in the said serial of values;
   (ii) a second group comprising tumor samples that exhibit a score for said gene cluster that is higher than said arbitrary value contained in the said serial of values;
      whereby two groups of tumor samples are obtained for the said specific value, wherein the tumors samples of each group are separately enumerated;
g) calculating the statistical significance between (i) the score for said gene cluster obtained at step e) and (ii) the actual clinical outcome of the patients from which tumor samples contained in the first and second groups defined at step f) derive;
h) reiterating steps f) and g) until every arbitrary value provided at step d) is tested;
i) setting the said reference value ("cut-off" value) as consisting of the arbitrary value for which the highest statistical significance (most significant) has been calculated at step g). As it is disclosed above, said method allows the setting of a single "cut-off" value permitting discrimination between bad and good outcome prognosis. Practically, high statistical significance values (e.g. low P values) are generally obtained for a range of successive arbitrary values, and not only for a single arbitrary value. Thus, in one alternative embodiment of the method of determining "cut-off" values above, a minimal statistical significance value (minimal threshold of significance, e.g. maximal threshold P value) is arbitrarily set and the range of arbitrary values for which the statistical significance value calculated at step g) is higher (more significant, e.g. lower P value) are retained, whereby a range of values is provided. Said range of values consist of a "cut-off" value according to the invention. According to this specific embodiment of a "cut-off" value, bad or good clinical outcome prognosis can be determined by comparing the score obtained for the gene cluster with the range of values delimiting said "cut-off" value. In certain embodiments, a cut-off value consisting of a range of values for the considered gene cluster, consists of a range of values centred on the value for which the highest statistical significance value is found (e.g. generally the minimum P value which is found). Typically, the cut-off values as above described are determined from a cohort of patient that has a sufficient size enough for allowing a reproducible and accurate discrimination between patients with good prognosis and patients with bad prognosis.

In particular embodiment, the cut-off values as described above may be reported in a table, so that the physician can compare the score obtained for a patient with said values and can easily determined the cancer stage for said patient.

### Cancers:

The methods of the invention may be performed for any type of cancers selected from the group consisting of adrenal cortical cancer, anal cancer, bile duct cancer (e.g. periphilar cancer, distal bile duct cancer, intrahepatic bile duct cancer), bladder cancer, bone cancer (e.g. osteoblastoma, osteochrondroma, hemangioma, chondromyxoid fibroma, osteosarcoma, chondrosarcoma, fibrosarcoma, malignant fibrous histiocytoma, giant cell tumor of the bone, chordoma, lymphoma, multiple myeloma), brain and central nervous system cancer (e.g. meningioma, astocytoma, oligodendrogliomas, ependymoma, gliomas, medulloblastoma, ganglioglioma, Schwannoma, germinoma, craniopharyngioma), breast cancer (e.g. ductal carcinoma in situ, infiltrating ductal carcinoma, infiltrating, lobular carcinoma, lobular carcinoma in, situ, gynecomastia), Castleman disease (e.g. giant lymph node hyperplasia, angiofollicular lymph node hyperplasia), cervical cancer, colorectal cancer, endometrial cancer (e.g. endometrial adenocarcinoma, adenocanthoma, papillary serous adnocarcinroma, clear cell), esophagus cancer, gallbladder cancer (mucinous adenocarcinoma, small cell carcinoma), gastrointestinal carcinoid tumors (e.g. choriocarcinoma, chorioadenoma destruens), Hodgkin's disease, non-Hodgkin's lymphoma, Kaposi's sarcoma, kidney cancer (e.g. renal cell cancer), laryngeal and hypopharyngeal cancer, liver cancer (e.g. hemangioma, hepatic adenoma, focal nodular hyperplasia, hepatocellular carcinoma), lung cancer (e.g. small cell lung cancer, non-small cell lung cancer), mesothelioma, plasmacytoma, nasal cavity and paranasal sinus cancer (e.g. esthesioneuroblastoma, midline granuloma), nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma (e.g. embryonal rhabdomyosarcoma, alveolar rhabdomyosarcoma, pleomorphic rhabdomyosarcoma), salivary gland cancer, skin cancer (e.g. melanoma, nonmelanoma skin cancer), stomach cancer, testicular cancer (e.g. seminoma, nonseminoma germ cell cancer), thymus cancer, thyroid cancer (e.g. follicular carcinoma, anaplastic carcinoma, poorly differentiated carcinoma, medullary thyroid carcinoma, thyroid lymphoma), vaginal cancer, vulvar cancer, and uterine cancer (e.g. uterine leiomyosarcoma). In a particular embodiment, the cancer is a colorectal cancer.

In a particular embodiment, the cancer is at Stage I, II, III, or IV as determined by the TNM classification.

Methods of the invention can be applied for monitoring the treatment (e.g., drug compounds) of the patient. For example, the effectiveness of an agent to affect the expression level of the gene cluster (as herein after described) can be monitored during treatments of patients receiving anti-cancer treatments.

The "anti-cancer treatment" that is referred to in the definition of step a) above relate to any type of cancer therapy undergone by the cancer patients previously to collecting the tumor tissue samples, including radiotherapy, chemotherapy and surgery, e.g. surgical resection of the tumor.

Accordingly, also disclosed is a method for monitoring the treatment of patient affected with a cancer, said method comprising the steps consisting of:
i) determining the stage of said cancer before said treatment by performing the method of the invention
ii) determining the stage of said cancer after said treatment by performing the method of the invention
iii) and comparing the stage determined a step i) with the stage determined at step ii) wherein a difference between said stages is indicative of the effectiveness of the treatment.

The present invention also relates to a method for predicting the outcome of a cancer in a patient, wherein said method may be used independently from conventional clinicopatholological cancer staging methods, and which method comprising determining the expression level of a gene cluster according to the invention.

### Kits:

Also disclosed are kits for performing the methods of the invention, wherein said kits comprise means for measuring the expression level of the gene clusters of the invention in the sample obtained from the patient. The kits may include probes, primers macroarrays or microarrays as above described.

For example, the kit may comprise a set of probes as above defined, usually made of DNA, and that may be pre-labelled. Alternatively, probes may be unlabelled and the ingredients for labelling may be included in the kit in separate containers. The kit may further comprise hybridization reagents or other suitably packaged reagents and materials needed for the particular hybridization protocol, including solid-phase matrices, if applicable, and standards.

Alternatively the kit may comprise amplification primers that may be pre-labelled or may contain an affinity purification or attachment moiety. The kit may further comprise amplification reagents and also other suitably packaged reagents and materials needed for the particular amplification protocol.

In a particular embodiment, the kit comprises means for determining the expression level of a gene cluster in a sample obtained from said patient, wherein said gene cluster comprises GNLY (or PF4, or LAG3, or CXCL13, or CXCL9, or REN, or ITGAE, or CCL5, or TSLP, or IL17A, or PROM1, or IHH, or GZMH, or IFNG, or VEGFA).

In a particular embodiment, the kit comprises means for determining the expression level of a gene represented by any combination described in Table 2, 3, 4, 5, 6, 7, 8, 9, 10, 11, 12, 13, or 14 which comprises GNLY (or PF4, or LAG3, or CXCL13, or CXCL9, or REN, or ITGAE, or CCL5, or TSLP, or IL17A, or PROM1, or IHH, or GZMH, or IFNG, or VEGFA).

The invention will be further illustrated by the following figures and examples. However, these examples and figures should not be interpreted in any way as limiting the scope of the present invention.

### FIGURES:

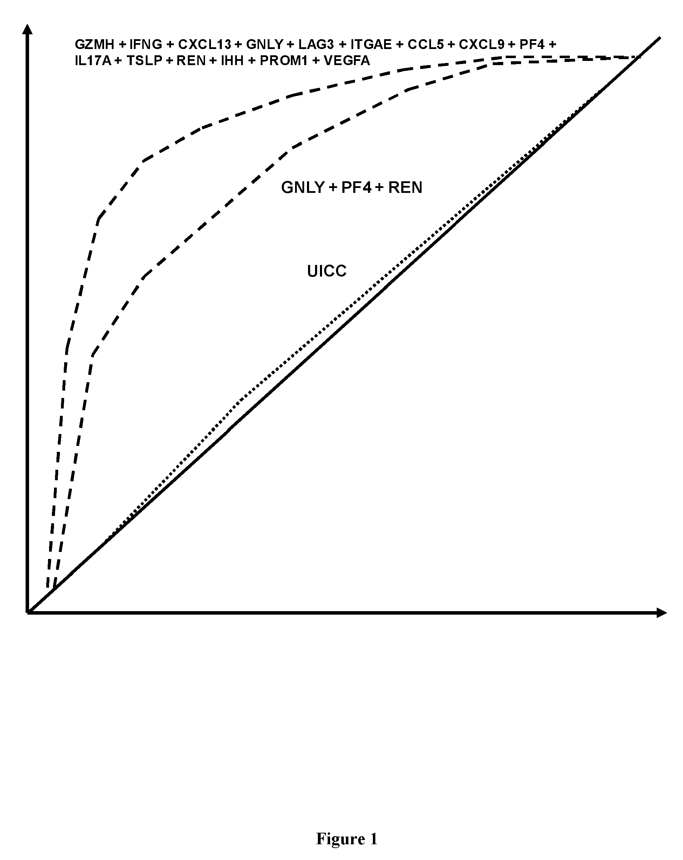

### Figure 1 show the ROC curve for cluster "GNLY + PF4 + REN" and cluster "GZMH + IFNG + CXCL13 + GNLY + LAG3 + ITGAE + CCL5 + CXCL9 + PF4 + IL17A + TSLP + REN + IHH + PROM1 + VEGFA"

### EXAMPLE:

### Material & Methods

**Patients and data:** The records of colorectal cancer (CRC) patients who underwent a primary resection of their tumor at the Laennec-HEGP Hospitals between 1996 and 2004 were reviewed and previously described (Galon et al. 2006). All frozen tumor samples (n=96) from UICC-TNM Stage I-III patients available from Laennec-HEGP Hospitals from 1996-2004, with sufficient RNA quality and quantity, were selected. The RNA samples analyzed were from 96 different patients. These patients were used for gene expression experiments (Taqman qPCR). The observation time in the cohorts was the interval between diagnosis and last contact (death or last follow-up). Data were censored at the last follow-up for patients without relapse, or death. The min:max values until progression/death or last follow-up were (0:136) months, respectively. Time to recurrence or disease-free time was defined as the interval from the date of surgery to confirmed tumor relapse date for relapsed patients and from the date of surgery to the date of last follow-up for disease-free patients.

Histopathological and clinical findings were scored according to the UICC-TNM staging system. Follow-up data were collected prospectively and updated. A secure Web-based database, TME.db (Tumor MicroEnvironment Database), was built on a 3-tier architecture using Java-2 Enterprise-Edition (J2EE) to integrate the clinical data and the data from high-throughput technologies. Ethical, Legal and Social Implications were approved by ethical review board.

**Statistical analysis:** All signatures were build based on 96 patients from a UICC-TNM Stage I-III CRC patient cohort and all 15 selected final genes. The predictive performance for each gene combination was assessed by the Harrel's concordance index (c-index) and time-dependent c-index (C_{τ} index) (R risksetROC package) and correspond to the area under the receiver operator curve (AUC) (c-index (Harrell FE et al. 1996) and iAUC (Heagerty PJ et al., 2005), respectively). A summary table for each possible model combination length from 1 to 15 genes was created and sorted by the highest C_{τ} index. Additionally each table shows the number of genes remaining significant in the cox model and a P-value (U-statistics, Pencina MJ et al., 2008) comparing the performance of all models of a certain number of genes with the best 15 gene combination, the one with the highest performance with the same number of genes (largest C_{τ} index) and the UICC criteria (R risksetROC and Hmisc package).

Time-dependent area under the ROC(t) (iAUC): Since the event occurrence is time-dependent, time-dependent ROC curves are more appropriate than conventional ones (Heagerty PJ et al., 2005). Heagerty PJ et al. proposed to summarize the discrimination potential of a risk score, estimated at the diagnosis/surgery time t=0, by calculating ROC curves for cumulative event occurrence by time t. From the ROC curve ROC(t) the integrated area under the curve over time (iAUC) can be calculated. The larger the iAUC at time t, the better is the predictability of the time to event (TTE) at time t as well as the average predictability of the TTE.

The concordance index (c-index): The concordance index (c-index) calculates the probability that, for a pair of randomly chosen comparable patients, the patient with the higher risk prediction will experience an event before the lower risk patient. The c-index is a generalization of the AUC(t) (iAUC), an can not represent the evolution of performance with respect to time (Harrell FE et al., 1996). The larger c-index, the better is the predictability of the time to event (TTE).

### Results

**Selection of the relevant genes:** In a cohort of 77 Stage I/II/III patients, we determined the logrank significance of each single marker using the minimal P-value approach for the dichotomization. The obtained P-values were corrected using Altman et al. (Altman, D. G., Lausen, B., Sauerbrei, W. & Schumacher, M. Dangers of using "optimal" cutpoints in the evaluation of prognostic factors. J Natl Cancer Inst, 1994;86:829-35.) and were additionally cross validated. The Hazard ratios obtained with the minimal P-value dichotomization were corrected using Hollaender et al. (Hollaender N, Sauerbrei W, Schumacher M. Confidence intervals for the effect of a prognostic factor after selection of an 'optimal' cutpoint. Stat Med, 2004;23(11):1701-13.). The final selection of the genes was based on the median cross validation P-value. We removed from the final gene signature genes which were not expressed (none of the determined values were larger than deltaCt > 32) although they where logrank significant. We finally end up with 15 markers, where 8 show a good out come when high expressed (i.e. GNLY, CXCL13, CXCL9, LAG3, CCL5, GZMH, ITGAE and IFNG) and 7 show a bad outcome when high expressed(i.e. PF4, REN, TSLP, IL17A, PROM1, IHH and VEGFA). All analyzes were performed using the R survival package.

**Time-dependent ROC Curves and iAUC (Time-dependent predictive accuracy) using Cox regression models for disease-free survival (DFS):** In the cohorts of Stage I/II, Stage I/II/III and Stage III patients using the 15 final selected genes we determined the time-dependent predictive accuracy (integrated Area Under the ROC curves, iAUC) (Heagerty PJ, Zheng Y. Survival model predictive accuracy and ROC curves. Biometrics, 2005, Mar;61(1):92-105.). All signatures show stable time-dependent ROC curves and none of the signatures violate the cox proportional hazards assumption.

The ROC and AUC curves were drawn based on the linear predictor of a fitted Cox regression model for the respective gene or gene combination were the gene data entered undichotimized into the model. Each circle in the iAUC plot indicates an event (relapse) of a patient. The ROC and AUC curves were calculated based on the LocalCox method in case the marker was violating the Hazards assumptions, otherwise the Cox method was used. The analyzes were performed using the R survival and risksetROC packages. All AUC characteristics for all combination markers are described in Table 1, 2, 3, 4, 5, 6, 7, 8, 9, 10, 11, 12, 13, and 14. The best combinations are described in Table 15.

**Correlation matrix cluster for the 17 final selected signature gene qPCR data clustered on 77patients (Stage I-III):** A high correlation cluster with the genes with good outcome and a high correlation cluster with the poor outcome genes. Negative correlation can be seen between those two clusters showing an inverse expression of those genes. A high correlation pattern between genes show a probable redundancy among those genes suggesting a possible replacement of one gene by another one gene. The following tables illustrate examples of said replacements:

| **Table B** | |
|---|---|
| 4 Marker Combination all miRs | c_tau |
| GNLY + **GZMH** + PF4 + REN | 0.7615 |
| GNLY + **ITGAE** + PF4 + REN | 0.7607 |

| **Table C** | |
|---|---|
| 4 Marker Combination all miRs | c_tau |
| CXCL13 + GNLY + **PF4** + REN | 0.7575 |
| CXCL13 + GNLY + **IL17A** + REN | 0.7539 |

| **Table D** | |
|---|---|
| 5 Marker Combination all miRs | c_tau |
| GNLY + **IFNG** + PF4 + IL17A + REN | 0.7876 |
| GNLY + **CCL5** + PF4 + IL17A + REN | 0.7759 |

| **Table E** | |
|---|---|
| 6 Marker Combination all miRs | c_tau |
| IFNG + GNLY + PF4 + IL17A + **IHH** + REN | 0.7877 |
| IFNG + GNLY + PF4 + IL17A + **TSLP** + REN | 0.7876 |

| **Table F** | |
|---|---|
| 7 Marker_Combination all patients | c_tau |
| IFNG + GNLY + **ITGAE** + CCL5 + PF4 + IL17A + REN | 0.7988 |
| IFNG + GNLY + **CXCL9** + CCL5 + PF4 + IL17A + REN | 0.7918 |

| **Table G** | |
|---|---|
| 8 Marker_Combination all patients | c_tau |
| IFNG + GNLY + LAG3 + CCL5 + **CXCL9** + PF4 + IL17A + REN | 0.79308 |
| IFNG + GNLY + LAG3 + CCL5 + **GZMH** + PF4 + IL17A + REN | 0.79290 |

Some of the genes are redundant and may share common biological functions which makes them replaceable among each other. This is of advantage in case some the genes are not expressed or are not measurable because of technical reasons.

| **Table 1: clusters of 1 gene** | |
|---|---|
| **Clusters of 1 gene** | **c_tau level** |
| GNLY | 0,68 |
| LAG3 | 0,65 |
| CXCL13 | 0,65 |
| PF4 | 0,65 |
| CXCL9 | 0,64 |
| REN | 0,64 |
| ITGAE | 0,63 |
| CCL5 | 0,6 |
| TSLP | 0,59 |
| IL17A | 0,58 |
| GZMH | 0,54 |
| PROM1 | 0,54 |
| IHH | 0,54 |
| IFNG | 0,52 |
| UICC | **0,52** |
| VEGFA | 0,5 |

| **Table 2: clusters of 2 genes** | |
|---|---|
| **Clusters of 2 genes** | **c_tau level** |
| GNLY + PF4 | 0,71 |
| IFNG + GNLY | 0,71 |
| GNLY + REN | 0,71 |
| GNLY + IL17A | 0,71 |
| CXCL13 + PF4 | 0,7 |
| GNLY + TSLP | 0,7 |
| PF4 + REN | 0,7 |
| GZMH + GNLY | 0,69 |
| CXCL13 + GNLY | 0,69 |
| CXCL9 + PF4 | 0,69 |
| GNLY + CCL5 | 0,69 |
| CXCL13 + IL17A | 0,69 |
| GNLY + LAG3 | 0,69 |
| ITGAE + PF4 | 0,69 |
| CXCL9 + REN | 0,68 |
| ITGAE + REN | 0,68 |
| GNLY + PROM1 | 0,68 |
| LAG3 + PF4 | 0,68 |
| GNLY + CXCL9 | 0,68 |
| GNLY + VEGFA | 0,68 |
| GNLY + ITGAE | 0,68 |
| GNLY + IHH | 0,68 |
| LAG3 + REN | 0,68 |
| IFNG + CXCL9 | 0,67 |
| CXCL13 + REN | 0,67 |
| LAG3 + IL17A | 0,67 |
| CCL5 + PF4 | 0,67 |
| LAG3 + TSLP | 0,67 |
| CXCL9 + IL17A | 0,67 |
| CXCL9 + TSLP | 0,67 |
| PF4 + TSLP | 0,67 |
| CXCL13 + LAG3 | 0,67 |
| CXCL13 + TSLP | 0,66 |
| PF4 + IL17A | 0,66 |
| GZMH + CXCL9 | 0,66 |
| LAG3 + CXCL9 | 0,66 |
| GZMH + PF4 | 0,66 |
| ITGAE + IL17A | 0,66 |
| ITGAE + TSLP | 0,66 |
| CXCL13 + ITGAE | 0,66 |
| CXCL13 + CXCL9 | 0,66 |
| LAG3 + ITGAE | 0,66 |
| LAG3 + PROM1 | 0,66 |
| CCL5 + REN | 0,66 |
| IFNG + LAG3 | 0,66 |
| IL17A + REN | 0,65 |
| LAG3 + IHH | 0,65 |
| IFNG + ITGAE | 0,65 |
| PF4 + PROM1 | 0,65 |
| LAG3 + VEGFA | 0,65 |
| GZMH + LAG3 | 0,65 |
| LAG3 + CCL5 | 0,65 |
| IFNG + PF4 | 0,65 |
| PF4 + IHH | 0,65 |
| TSLP + REN | 0,65 |
| CCL5 + CXCL9 | 0,65 |
| CXCL13 + PROM1 | 0,65 |
| REN + PROM1 | 0,65 |
| CXCL13 + VEGFA | 0,65 |
| PF4 + VEGFA | 0,65 |
| ITGAE + CXCL9 | 0,65 |
| CXCL13 + IHH | 0,65 |
| CXCL9 + VEGFA | 0,64 |
| GZMH + CXCL13 | 0,64 |
| GZMH + ITGAE | 0,64 |
| CXCL9 + PROM1 | 0,64 |
| GZMH + REN | 0,64 |
| CXCL9 + IHH | 0,64 |
| CXCL13 + CCL5 | 0,64 |
| IFNG + CXCL13 | 0,64 |
| ITGAE + VEGFA | 0,64 |
| ITGAE + PROM1 | 0,64 |
| ITGAE + IHH | 0,64 |
| REN + VEGFA | 0,64 |
| IFNG + REN | 0,64 |
| REN + IHH | 0,64 |
| ITGAE + CCL5 | 0,64 |
| CCL5 + TSLP | 0,63 |
| CCL5 + IL17A | 0,63 |
| IFNG + CCL5 | 0,62 |
| GZMH + CCL5 | 0,61 |
| IL17A + TSLP | 0,61 |
| GZMH + TSLP | 0,61 |
| CCL5 + PROM1 | 0,6 |
| CCL5 + IHH | 0,6 |
| GZMH + IL17A | 0,6 |
| CCL5 + VEGFA | 0,6 |
| TSLP + PROM1 | 0,6 |
| IFNG + TSLP | 0,59 |
| TSLP + VEGFA | 0,59 |
| TSLP + IHH | 0,59 |
| IFNG + IL17A | 0,59 |
| IL17A + IHH | 0,59 |
| IL17A + PROM1 | 0,59 |
| IL17A + VEGFA | 0,58 |
| GZMH + PROM1 | 0,55 |
| GZMH + IHH | 0,55 |
| IHH + PROM1 | 0,55 |
| IFNG + PROM1 | 0,54 |
| IFNG + IHH | 0,54 |
| GZMH + IFNG | 0,54 |
| GZMH + VEGFA | 0,54 |
| IHH + VEGFA | 0,54 |
| PROM 1 + VEGFA | 0,54 |
| IFNG + VEGFA | 0,52 |

| **Table 3: clusters of 3 genes:** | |
|---|---|
| **clusters of 3 genes** | **c_tau level** |
| GNLY + PF4 + REN | 0,75 |
| GNLY + IL17A + REN | 0,74 |
| IFNG + GNLY + IL17A | 0,74 |
| IFNG + GNLY + REN | 0,73 |
| CXCL13 + GNLY + IL17A | 0,73 |
| IFNG + GNLY + TSLP | 0,73 |
| IFNG + GNLY + PF4 | 0,73 |
| CXCL9 + PF4 + REN | 0,73 |
| GNLY + PF4 + IL17A | 0,73 |
| CXCL13 + PF4 + REN | 0,73 |
| LAG3 + PF4 + REN | 0,72 |
| CXCL13 + GNLY + PF4 | 0,72 |
| GNLY + PF4 + TSLP | 0,72 |
| CXCL13 + PF4 + IL17A | 0,72 |
| ITGAE + PF4 + REN | 0,72 |
| IFNG + GNLY + ITGAE | 0,72 |
| GNLY + IL17A + TSLP | 0,72 |
| GNLY + PF4 + IHH | 0,72 |
| GZMH + GNLY + REN | 0,72 |
| GNLY + CCL5 + PF4 | 0,72 |
| GNLY + PF4 + PROM1 | 0,72 |
| GNLY + TSLP + REN | 0,72 |
| IFNG + GNLY + VEGFA | 0,72 |
| IFNG + GNLY + CXCL9 | 0,72 |
| GZMH + GNLY + PF4 | 0,72 |
| GZMH + GNLY + IL17A | 0,72 |
| GNLY + CCL5 + REN | 0,72 |
| GNLY + CXCL9 + PF4 | 0,71 |
| GNLY + REN + PROM1 | 0,71 |
| GNLY + PF4 + VEGFA | 0,71 |
| GNLY + CXCL9 + IL17A | 0,71 |
| GNLY + LAG3 + PF4 | 0,71 |
| GNLY + ITGAE + PF4 | 0,71 |
| GNLY + LAG3 + IL17A | 0,71 |
| IFNG + GNLY + PROM1 | 0,71 |
| GNLY + CCL5 + IL17A | 0,71 |
| IFNG + GNLY + CCL5 | 0,71 |
| GNLY + ITGAE + REN | 0,71 |
| GNLY + ITGAE + IL17A | 0,71 |
| GZMH + IFNG + GNLY | 0,71 |
| GNLY + IL17A + PROM1 | 0,71 |
| GNLY + LAG3 + REN | 0,71 |
| IFNG + GNLY + IHH | 0,71 |
| IFNG + GNLY + LAG3 | 0,71 |
| CXCL13 + PF4 + TSLP | 0,71 |
| CCL5 + PF4 + REN | 0,71 |
| GNLY + IL17A + VEGFA | 0,71 |
| CXCL13 + GNLY + REN | 0,71 |
| GNLY + CXCL9 + REN | 0,71 |
| GNLY + REN + VEGFA | 0,71 |
| GNLY + REN + IHH | 0,71 |
| GZMH + GNLY + TSLP | 0,71 |
| GNLY + IL17A + IHH | 0,71 |
| CXCL13 + GNLY + TSLP | 0,71 |
| CXCL13 + CXCL9 + PF4 | 0,71 |
| GNLY + CCL5 + TSLP | 0,71 |
| CXCL13 + ITGAE + PF4 | 0,71 |
| CXCL13 + PF4 + PROM1 | 0,71 |
| GZMH + GNLY + CXCL9 | 0,71 |
| CXCL13 + LAG3 + PF4 | 0,71 |
| GZMH + CXCL13 + PF4 | 0,7 |
| GZMH + PF4 + REN | 0,7 |
| IFNG + CXCL13 + GNLY | 0,7 |
| CXCL13 + PF4 + VEGFA | 0,7 |
| CXCL9 + PF4 + TSLP | 0,7 |
| CXCL13 + IL17A + REN | 0,7 |
| CXCL13 + PF4 + IHH | 0,7 |
| CXCL13 + CCL5 + PF4 | 0,7 |
| GNLY + LAG3 + TSLP | 0,7 |
| GNLY + TSLP + PROM1 | 0,7 |
| GNLY + CXCL9 + TSLP | 0,7 |
| GNLY + ITGAE + TSLP | 0,7 |
| GNLY + TSLP + VEGFA | 0,7 |
| PF4 + REN + PROM1 | 0,7 |
| CXCL9 + PF4 + IL17A | 0,7 |
| IFNG + CXCL13 + PF4 | 0,7 |
| GZMH + CXCL13 + GNLY | 0,7 |
| PF4 + TSLP + REN | 0,7 |
| GZMH + GNLY + ITGAE | 0,7 |
| PF4 + IL17A + REN | 0,7 |
| IFNG + CXCL9 + PF4 | 0,7 |
| LAG3 + IL17A + REN | 0,7 |
| CXCL9 + IL17A + REN | 0,7 |
| GNLY + TSLP + IHH | 0,7 |
| GNLY + CCL5 + CXCL9 | 0,7 |
| CXCL13 + GNLY + CCL5 | 0,7 |
| IFNG + CXCL9 + REN | 0,7 |
| IFNG + PF4 + REN | 0,7 |
| ITGAE + IL17A + REN | 0,7 |
| GZMH + GNLY + PROM1 | 0,7 |
| GNLY + LAG3 + CCL5 | 0,7 |
| CXCL9 + PF4 + VEGFA | 0,7 |
| PF4 + REN + IHH | 0,7 |
| PF4 + REN + VEGFA | 0,7 |
| ITGAE + PF4 + TSLP | 0,7 |
| CXCL9 + PF4 + IHH | 0,7 |
| GZMH + GNLY + LAG3 | 0,7 |
| CCL5 + CXCL9 + PF4 | 0,7 |
| GZMH + CXCL9 + PF4 | 0,7 |
| ITGAE + PF4 + IL17A | 0,7 |
| GZMH + GNLY + VEGFA | 0,7 |
| CXCL9 + PF4 + PROM1 | 0,69 |
| GZMH + CXCL9 + REN | 0,69 |
| LAG3 + PF4 + TSLP | 0,69 |
| GZMH + GNLY + IHH | 0,69 |
| CXCL13 + GNLY + LAG3 | 0,69 |
| CXCL13 + GNLY + PROM1 | 0,69 |
| GZMH + GNLY + CCL5 | 0,69 |
| ITGAE + CXCL9 + PF4 | 0,69 |
| IFNG + CXCL9 + TSLP | 0,69 |
| CXCL13 + LAG3 + IL17A | 0,69 |
| CXCL13 + GNLY + VEGFA | 0,69 |
| GNLY + ITGAE + CCL5 | 0,69 |
| CXCL13 + GNLY + CXCL9 | 0,69 |
| LAG3 + PF4 + IL17A | 0,69 |
| LAG3 + CXCL9 + PF4 | 0,69 |
| IFNG + ITGAE + REN | 0,69 |
| CXCL13 + GNLY + ITGAE | 0,69 |
| ITGAE + PF4 + IHH | 0,69 |
| CXCL13 + GNLY + IHH | 0,69 |
| GNLY + CCL5 + PROM1 | 0,69 |
| CXCL9 + TSLP + REN | 0,69 |
| ITGAE + PF4 + VEGFA | 0,69 |
| LAG3 + REN + PROM1 | 0,69 |
| CXCL13 + IL17A + TSLP | 0,69 |
| CXCL13 + CXCL9 + IL17A | 0,69 |
| LAG3 + PF4 + IHH | 0,69 |
| CXCL13 + ITGAE + IL17A | 0,69 |
| CCL5 + CXCL9 + REN | 0,69 |
| ITGAE + TSLP + REN | 0,69 |
| ITGAE + CXCL9 + REN | 0,69 |
| GNLY + LAG3 + PROM1 | 0,69 |
| LAG3 + ITGAE + REN | 0,69 |
| GNLY + CCL5 + VEGFA | 0,69 |
| IFNG + LAG3 + REN | 0,69 |
| ITGAE + PF4 + PROM1 | 0,69 |
| GNLY + CCL5 + IHH | 0,69 |
| CXCL9 + REN + PROM1 | 0,69 |
| LAG3 + ITGAE + PF4 | 0,69 |
| GZMH + ITGAE + REN | 0,69 |
| ITGAE + REN + VEGFA | 0,69 |
| IFNG + ITGAE + PF4 | 0,69 |
| ITGAE + REN + PROM1 | 0,69 |
| GNLY + LAG3 + CXCL9 | 0,69 |
| CXCL13 + IL17A + VEGFA | 0,69 |
| CXCL9 + REN + VEGFA | 0,69 |
| CXCL13 + IL17A + PROM1 | 0,69 |
| GNLY + LAG3 + IHH | 0,69 |
| GNLY + LAG3 + ITGAE | 0,69 |
| ITGAE + CCL5 + PF4 | 0,69 |
| GNLY + LAG3 + VEGFA | 0,69 |
| GZMH + ITGAE + PF4 | 0,69 |
| LAG3 + PF4 + PROM1 | 0,69 |
| GZMH + CXCL13 + IL17A | 0,69 |
| CXCL13 + CCL5 + IL17A | 0,69 |
| LAG3 + PF4 + VEGFA | 0,69 |
| IFNG + CXCL9 + IL17A | 0,69 |
| LAG3 + CXCL9 + REN | 0,69 |
| IFNG + LAG3 + TSLP | 0,68 |
| ITGAE + CCL5 + REN | 0,68 |
| CXCL13 + ITGAE + REN | 0,68 |
| LAG3 + CCL5 + REN | 0,68 |
| CXCL13 + LAG3 + REN | 0,68 |
| IFNG + CXCL13 + IL17A | 0,68 |
| GZMH + LAG3 + REN | 0,68 |
| CCL5 + PF4 + TSLP | 0,68 |
| CXCL13 + IL17A + IHH | 0,68 |
| IFNG + LAG3 + PF4 | 0,68 |
| LAG3 + TSLP + REN | 0,68 |
| GNLY + CXCL9 + PROM1 | 0,68 |
| ITGAE + REN + IHH | 0,68 |
| GNLY + ITGAE + PROM1 | 0,68 |
| CXCL13 + CXCL9 + REN | 0,68 |
| LAG3 + REN + VEGFA | 0,68 |
| GNLY + PROM1 + VEGFA | 0,68 |
| CXCL9 + REN + IHH | 0,68 |
| IFNG + ITGAE + CXCL9 | 0,68 |
| GNLY + ITGAE + CXCL9 | 0,68 |
| GNLY + CXCL9 + VEGFA | 0,68 |
| LAG3 + CCL5 + PF4 | 0,68 |
| GNLY + ITGAE + VEGFA | 0,68 |
| GNLY + IHH + PROM1 | 0,68 |
| GZMH + LAG3 + PF4 | 0,68 |
| CXCL13 + LAG3 + TSLP | 0,68 |
| GNLY + CXCL9 + IHH | 0,68 |
| GNLY + ITGAE + IHH | 0,68 |
| GNLY + IHH + VEGFA | 0,68 |
| LAG3 + IL17A + TSLP | 0,68 |
| IFNG + LAG3 + CXCL9 | 0,68 |
| CXCL13 + TSLP + REN | 0,68 |
| CCL5 + PF4 + IL17A | 0,68 |
| GZMH + CXCL9 + IL17A | 0,68 |
| LAG3 + CXCL9 + IL17A | 0,68 |
| GZMH + CXCL9 + TSLP | 0,68 |
| CXCL9 + IL17A + TSLP | 0,68 |
| LAG3 + CCL5 + CXCL9 | 0,68 |
| LAG3 + REN + IHH | 0,68 |
| LAG3 + IL17A + VEGFA | 0,68 |
| LAG3 + ITGAE + IL17A | 0,68 |
| GZMH + PF4 + IL17A | 0,68 |
| CXCL13 + REN + PROM1 | 0,68 |
| GZMH + PF4 + TSLP | 0,68 |
| IFNG + ITGAE + TSLP | 0,68 |
| CXCL13 + CXCL9 + TSLP | 0,68 |
| GZMH + IFNG + CXCL9 | 0,68 |
| LAG3 + CXCL9 + TSLP | 0,68 |
| CXCL13 + ITGAE + TSLP | 0,68 |
| IFNG + CXCL9 + PROM1 | 0,68 |
| IFNG + LAG3 + IL17A | 0,68 |
| CCL5 + IL17A + REN | 0,67 |
| CCL5 + CXCL9 + IL17A | 0,67 |
| PF4 + IL17A + TSLP | 0,67 |
| IFNG + CXCL9 + IHH | 0,67 |
| IFNG + CCL5 + CXCL9 | 0,67 |
| IFNG + CXCL9 + VEGFA | 0,67 |
| IFNG + LAG3 + ITGAE | 0,67 |
| CCL5 + PF4 + IHH | 0,67 |
| LAG3 + TSLP + VEGFA | 0,67 |
| LAG3 + IL17A + PROM1 | 0,67 |
| CXCL9 + IL17A + VEGFA | 0,67 |
| ITGAE + CXCL9 + IL17A | 0,67 |
| LAG3 + TSLP + PROM1 | 0,67 |
| LAG3 + ITGAE + TSLP | 0,67 |
| GZMH + LAG3 + CXCL9 | 0,67 |
| LAG3 + CCL5 + TSLP | 0,67 |
| CCL5 + PF4 + VEGFA | 0,67 |
| IFNG + CCL5 + PF4 | 0,67 |
| CXCL13 + REN + VEGFA | 0,67 |
| GZMH + ITGAE + CXCL9 | 0,67 |
| GZMH + LAG3 + IL17A | 0,67 |
| LAG3 + CCL5 + IL17A | 0,67 |
| CCL5 + PP4 + PROM1 | 0,67 |
| GZMH + CXCL13 + REN | 0,67 |
| LAG3 + IL17A + IHH | 0,67 |
| CCL5 + CXCL9 + TSLP | 0,67 |
| CXCL9 + TSLP + VEGFA | 0,67 |
| GZMH + LAG3 + TSLP | 0,67 |
| ITGAE + CXCL9 + TSLP | 0,67 |
| ITGAE + IL17A + TSLP | 0,67 |
| GZMH + CCL5 + PF4 | 0,67 |
| CXCL13 + CCL5 + REN | 0,67 |
| CXCL13 + LAG3 + PROM1 | 0,67 |
| PF4 + TSLP + PROM1 | 0,67 |
| IFNG + PF4 + IL17A | 0,67 |
| IFNG + ITGAE + IL17A | 0,67 |
| CXCL13 + LAG3 + ITGAE | 0,67 |
| IFNG + CXCL13 + REN | 0,67 |
| CXCL9 + IL17A + PROM1 | 0,67 |
| CXCL9 + TSLP + PROM1 | 0,67 |
| CXCL13 + LAG3 + CXCL9 | 0,67 |
| CXCL13 + LAG3 + CCL5 | 0,67 |
| IFNG + CXCL13 + LAG3 | 0,67 |
| LAG3 + TSLP + IHH | 0,67 |
| CXCL9 + IL17A + IHH | 0,67 |
| IFNG + PF4 + TSLP | 0,67 |
| ITGAE + IL17A + VEGFA | 0,67 |
| IFNG + CXCL13 + CXCL9 | 0,67 |
| CXCL9 + TSLP + IHH | 0,67 |
| PF4 + TSLP + VEGFA | 0,67 |
| GZMH + CXCL13 + CXCL9 | 0,67 |
| GZMH + CXCL13 + LAG3 | 0,67 |
| CXCL13 + LAG3 + VEGFA | 0,67 |
| PF4 + TSLP + IHH | 0,67 |
| CXCL13 + REN + IHH | 0,67 |
| CXCL13 + LAG3 + IHH | 0,67 |
| GZMH + LAG3 + ITGAE | 0,67 |
| IFNG + LAG3 + PROM1 | 0,67 |
| PF4 + IL17A + PROM1 | 0,67 |
| IFNG + CCL5 + REN | 0,67 |
| LAG3 + ITGAE + CCL5 | 0,67 |
| GZMH + CXCL9 + VEGFA | 0,67 |
| ITGAE + TSLP + VEGFA | 0,67 |
| IFNG + CXCL13 + ITGAE | 0,67 |
| CXCL13 + TSLP + PROM1 | 0,67 |
| LAG3 + CXCL9 + PROM1 | 0,67 |
| GZMH + CXCL9 + PROM1 | 0,67 |
| GZMH + PF4 + VEGFA | 0,67 |
| PF4 + IL17A + IHH | 0,67 |
| GZMH + ITGAE + IL17A | 0,66 |
| GZMH + PF4 + PROM1 | 0,66 |
| CCL5 + TSLP + REN | 0,66 |
| CCLS + REN + PROM1 | 0,66 |
| LAG3 + CXCL9 + VEGFA | 0,66 |
| IFNG + CXCL13 + TSLP | 0,66 |
| CXCL13 + TSLP + VEGFA | 0,66 |
| GZMH + CXCL9 + IHH | 0,66 |
| PF4 + IL17A + VEGFA | 0,66 |
| LAG3 + ITGAE + VEGFA | 0,66 |
| GZMH + CXCL13 + ITGAE | 0,66 |
| ITGAE + CCL5 + IL17A | 0,66 |
| LAG3 + ITGAE + PROM1 | 0,66 |
| LAG3 + CXCL9 + IHH | 0,66 |
| GZMH + CXCL13 + TSLP | 0,66 |
| GZMH + PF4 + IHH | 0,66 |
| CXCL13 + TSLP + IHH | 0,66 |
| GZMH + CCL5 + CXCL9 | 0,66 |
| LAG3 + ITGAE + CXCL9 | 0,66 |
| IFNG + LAG3 + IHH | 0,66 |
| LAG3 + ITGAE + IHH | 0,66 |
| CXCL13 + CCL5 + TSLP | 0,66 |
| GZMH + ITGAE + TSLP | 0,66 |
| GZMH + IFNG + PF4 | 0,66 |
| GZMH + CCL5 + REN | 0,66 |
| CXCL13 + CCL5 + CXCL9 | 0,66 |
| GZMH + IL17A + REN | 0,66 |
| LAG3 + CCL5 + PROM1 | 0,66 |
| CXCL13 + ITGAE + CXCL9 | 0,66 |
| ITGAE + IL17A + IHH | 0,66 |
| ITGAE + IL17A + PROM1 | 0,66 |
| ITGAE + CCL5 + TSLP | 0,66 |
| IL17A + TSLP + REN | 0,66 |
| ITGAE + TSLP + PROM1 | 0,66 |
| CXCL13 + CXCL9 + PROM1 | 0,66 |
| ITGAE + CCL5 + CXCL9 | 0,66 |
| GZMH + LAG3 + PROM1 | 0,66 |
| CXCL13 + ITGAE + CCL5 | 0,66 |
| LAG3 + PROM1 + VEGFA | 0,66 |
| ITGAE + TSLP + IHH | 0,66 |
| LAG3 + IHH + PROM1 | 0,66 |
| CXCL13 + ITGAE + PROM1 | 0,66 |
| CXCL13 + CXCL9 + VEGFA | 0,66 |
| IFNG + LAG3 + VEGFA | 0,66 |
| CXCL13 + ITGAE + VEGFA | 0,66 |
| CCL5 + REN + VEGFA | 0,66 |
| CXCL13 + CXCL9 + IHH | 0,66 |
| IL17A + REN + PROM1 | 0,66 |
| CXCL13 + ITGAE + IHH | 0,66 |
| GZMH + IFNG + LAG3 | 0,66 |
| IFNG + ITGAE + IHH | 0,66 |
| IFNG + LAG3 + CCL5 | 0,66 |
| IFNG + IL17A + REN | 0,66 |
| IFNG + ITGAE + PROM1 | 0,66 |
| TSLP + REN + PROM1 | 0,66 |
| IFNG + PF4 + PROM1 | 0,66 |
| CCL5 + REN + IHH | 0,66 |
| GZMH + LAG3 + IHH | 0,66 |
| IFNG + ITGAE + VEGFA | 0,66 |
| LAG3 + CCL5 + IHH | 0,66 |
| PF4 + IHH + PROM1 | 0,66 |
| LAG3 + IHH + VEGFA | 0,65 |
| IL17A + REN + VEGFA | 0,65 |
| IL17A + REN + IHH | 0,65 |
| GZMH + IFNG + ITGAE | 0,65 |
| IFNG + ITGAE + CCL5 | 0,65 |
| LAG3 + CCL5 + VEGFA | 0,65 |
| CCL5 + CXCL9 + VEGFA | 0,65 |
| GZMH + TSLP + REN | 0,65 |
| PF4 + PROM1 + VEGFA | 0,65 |
| GZMH + REN + PROM1 | 0,65 |
| GZMH + LAG3 + VEGFA | 0,65 |
| IFNG + PF4 + IHH | 0,65 |
| IFNG + PF4 + VEGFA | 0,65 |
| GZMH + LAG3 + CCL5 | 0,65 |
| IFNG + CCL5 + TSLP | 0,65 |
| CCL5 + CXCL9 + PROM1 | 0,65 |
| TSLP + REN + IHH | 0,65 |
| PF4 + IHH + VEGFA | 0,65 |
| TSLP + REN + VEGFA | 0,65 |
| ITGAE + CXCL9 + VEGFA | 0,65 |
| IFNG + TSLP + REN | 0,65 |
| GZMH + ITGAE + IHH | 0,65 |
| CCL5 + CXCL9 + IHH | 0,65 |
| CXCL13 + PROM1+ VEGFA | 0,65 |
| GZMH + ITGAE + VEGFA | 0,65 |
| IFNG + REN + PROM1 | 0,65 |
| GZMH + ITGAE + PROM1 | 0,65 |
| REN + PROM1 + VEGFA | 0,65 |
| REN + IHH + PROM1 | 0,65 |
| CXCL13 + IHH + PROM1 | 0,65 |
| ITGAE + CXCL9 + PROM1 | 0,65 |
| ITGAE + CXCL9 + IHH | 0,65 |
| CXCL9 + PROM1 + VEGFA | 0,65 |
| GZMH + CXCL13 + PROM1 | 0,65 |
| GZMH + ITGAE + CCL5 | 0,65 |
| CXCL13 + IHH + VEGFA | 0,65 |
| CXCL9 + IHH + VEGFA | 0,64 |
| GZMH + IFNG + REN | 0,64 |
| GZMH + CXCL13 + VEGFA | 0,64 |
| IFNG + CXCL13 + CCL5 | 0,64 |
| CCL5 + IL17A + TSLP | 0,64 |
| GZMH + CXCL13 + IHH | 0,64 |
| CXCL13 + CCL5 + PROM1 | 0,64 |
| CXCL9 + IHH + PROM1 | 0,64 |
| GZMH + REN + VEGFA | 0,64 |
| IFNG + CXCL13 + PROM1 | 0,64 |
| IFNG + CXCL13 + VEGFA | 0,64 |
| GZMH + CXCL13 + CCL5 | 0,64 |
| CXCL13 + CCL5 + VEGFA | 0,64 |
| GZMH + IFNG + CXCL13 | 0,64 |
| GZMH + REN + IHH | 0,64 |
| CXCL13 + CCL5 + IHH | 0,64 |
| IFNG + CXCL13 + IHH | 0,64 |
| ITGAE + PROM1 + VEGFA | 0,64 |
| ITGAE + CCL5 + IHH | 0,64 |
| ITGAE + CCL5 + PROM1 | 0,64 |
| ITGAE + IHH + VEGFA | 0,64 |
| ITGAE + CCL5 + VEGFA | 0,64 |
| ITGAE + IHH + PROM1 | 0,64 |
| GZMH + CCL5 + TSLP | 0,64 |
| IFNG + REN + VEGFA | 0,64 |
| REN + IHH + VEGFA | 0,64 |
| IFNG + REN + IHH | 0,64 |
| CCL5 + TSLP + PROM1 | 0,64 |
| CCL5 + TSLP + VEGFA | 0,64 |
| CCL5 + TSLP + IHH | 0,63 |
| GZMH + CCLS + IL17A | 0,63 |
| IFNG + CCL5 + IL17A | 0,63 |
| CCL5 + IL17A + VEGFA | 0,63 |
| CCL5 + IL17A + PROM1 | 0,63 |
| CCL5 + IL17A + IHH | 0,63 |
| GZMH + IL17A + TSLP | 0,62 |
| GZMH + IFNG + CCL5 | 0,62 |
| IFNG + CCL5 + PROM1 | 0,62 |
| IFNG + CCL5 + IHH | 0,62 |
| GZMH + CCL5 + PROM1 | 0,62 |
| IFNG + CCL5 + VEGFA | 0,62 |
| GZMH + CCL5 + IHH | 0,61 |
| IFNG + IL17A + TSLP | 0,61 |
| GZMH + CCL5 + VEGFA | 0,61 |
| IL17A + TSLP + PROM1 | 0,61 |
| GZMH + IFNG + TSLP | 0,61 |
| GZMH + TSLP + PROM1 | 0,61 |
| IL17A + TSLP + VEGFA | 0,61 |
| IL17A + TSLP + IHH | 0,61 |
| GZMH + TSLP + VEGFA | 0,61 |
| GZMH + TSLP + IHH | 0,61 |
| CCL5 + IHH + PROM1 | 0,6 |
| CCL5 + PROM1 + VEGFA | 0,6 |
| GZMH + IL17A + PROM1 | 0,6 |
| CCL5 + IHH + VEGFA | 0,6 |
| GZMH + IL17A + IHH | 0,6 |
| GZMH + IL17A + VEGFA | 0,6 |
| GZMH + IFNG + IL17A | 0,6 |
| IFNG + TSLP + PROM1 | 0,6 |
| TSLP + PROM1 + VEGFA | 0,6 |
| TSLP + IHH + PROM1 | 0,6 |
| IFNG + IL17A + PROM1 | 0,6 |
| IFNG + IL17A + IHH | 0,6 |
| IFNG + TSLP + VEGFA | 0,59 |
| IFNG + IL17A + VEGFA | 0,59 |
| IFNG + TSLP + IHH | 0,59 |
| TSLP + IHH + VEGFA | 0,59 |
| IL17A + IHH + PROM1 | 0,59 |
| IL17A + PROM1 + VEGFA | 0,59 |
| IL17A + IHH + VEGFA | 0,59 |
| GZMH + IHH + PROM1 | 0,56 |
| GZMH + PROM1 + VEGFA | 0,55 |
| GZMH + IFNG + PROM1 | 0,55 |
| IFNG + IHH + PROM1 | 0,55 |
| GZMH + IFNG + IHH | 0,55 |
| GZMH + IHH + VEGFA | 0,55 |
| IHH + PROM1 + VEGFA | 0,55 |
| IFNG + PROM1 + VEGFA | 0,54 |
| IFNG + IHH + VEGFA | 0,54 |
| GZMH + IFNG + VEGFA | 0,54 |

| **Table 4: clusters of 4 genes:** | |
|---|---|
| **clusters of 4 genes** | **c_tau level** |
| GNLY + PF4 + IL17A + REN | 0,77 |
| IFNG + GNLY + PF4 + REN | 0,76 |
| IFNG + GNLY + IL17A + REN | 0,76 |
| GNLY + PF4 + REN + PROM1 | 0,76 |
| GNLY + CCL5 + PF4 + REN | 0,76 |
| GNLY + PF4 + TSLP + REN | 0,76 |
| GZMH + GNLY + PF4 + REN | 0,76 |
| GNLY + ITGAE + PF4 + REN | 0,76 |
| GNLY + CXCL9 + PF4 + REN | 0,75 |
| GNLY + PF4 + REN + VEGFA | 0,75 |
| GNLY + PF4 + REN + IHH | 0,75 |
| GNLY + LAG3 + PF4 + REN | 0,75 |
| CXCL13 + GNLY + PF4 + REN | 0,75 |
| CXCL13 + GNLY + IL17A + REN | 0,75 |
| IFNG + CXCL13 + GNLY + IL17A | 0,75 |
| GNLY + IL17A + REN + IHH | 0,75 |
| CXCL13 + GNLY + CCL5 + IL17A | 0,75 |
| IFNG + GNLY + PF4 + IL17A | 0,74 |
| IFNG + GNLY + CXCL9 + IL17A | 0,74 |
| IFNG + GNLY + ITGAE + IL17A | 0,74 |
| IFNG + GNLY + IL17A + TSLP | 0,74 |
| GNLY + CXCL9 + IL17A + REN | 0,74 |
| GNLY + ITGAE + IL17A + REN | 0,74 |
| GNLY + IL17A + TSLP + REN | 0,74 |
| GZMH + GNLY + IL17A + REN | 0,74 |
| GNLY + LAG3 + IL17A + REN | 0,74 |
| GNLY + IL17A + REN + PROM 1 | 0,74 |
| IFNG + GNLY + PF4 + TSLP | 0,74 |
| IFNG + GNLY + LAG3 + IL17A | 0,74 |
| IFNG + GNLY + CCL5 + IL17A | 0,74 |
| IFNG + GNLY + TSLP + REN | 0,74 |
| GNLY + CCL5 + IL17A + REN | 0,74 |
| GNLY + IL17A + REN + VEGFA | 0,74 |
| GZMH + IFNG + GNLY + IL17A | 0,74 |
| IFNG + GNLY + IL17A + VEGFA | 0,74 |
| IFNG + GNLY + IL17A + PROM1 | 0,74 |
| IFNG + GNLY + ITGAE + REN | 0,74 |
| IFNG + GNLY + IL17A + IHH | 0,74 |
| CXCL13 + GNLY + PF4 + IL17A | 0,74 |
| GZMH + CXCL13 + GNLY + IL17A | 0,74 |
| CXCL13 + PF4 + IL17A + REN | 0,74 |
| IFNG + GNLY + REN + PROM1 | 0,74 |
| IFNG + GNLY + PF4 + IHH | 0,74 |
| IFNG + GNLY + REN + VEGFA | 0,74 |
| IFNG + GNLY + ITGAE + TSLP | 0,73 |
| IFNG + GNLY + CXCL9 + REN | 0,73 |
| IFNG + GNLY + CCL5 + REN | 0,73 |
| CXCL13 + GNLY + IL17A + TSLP | 0,73 |
| IFNG + GNLY + LAG3 + REN | 0,73 |
| GZMH + IFNG + GNLY + REN | 0,73 |
| CXCL13 + GNLY + LAG3 + IL17A | 0,73 |
| CXCL9 + PF4 + IL17A + REN | 0,73 |
| IFNG + GNLY + CXCL9 + TSLP | 0,73 |
| CXCL13 + GNLY + CXCL9 + IL17A | 0,73 |
| CXCL9 + PF4 + REN + VEGFA | 0,73 |
| IFNG + GNLY + PF4 + PROM1 | 0,73 |
| CXCL13 + GNLY + ITGAE + IL17A | 0,73 |
| IFNG + GNLY + CCL5 + PF4 | 0,73 |
| GNLY + CCL5 + PF4 + IL17A | 0,73 |
| IFNG + GNLY + TSLP + VEGFA | 0,73 |
| IFNG + GNLY + REN + IHH | 0,73 |
| CXCL13 + GNLY + IL17A + PROM1 | 0,73 |
| IFNG + GNLY + CXCL9 + PF4 | 0,73 |
| IFNG + GNLY + LAG3 + TSLP | 0,73 |
| CXCL13 + GNLY + IL17A + VEGFA | 0,73 |
| GZMH + CXCL9 + PF4 + REN | 0,73 |
| GNLY + CCL5 + CXCL9 + IL17A | 0,73 |
| CXCL9 + PF4 + REN + PROM1 | 0,73 |
| IFNG + GNLY + TSLP + PROM1 | 0,73 |
| IFNG + GNLY + PF4 + VEGFA | 0,73 |
| GZMH + IFNG + GNLY + TSLP | 0,73 |
| CXCL9 + PF4 + TSLP + REN | 0,73 |
| CXCL13 + GNLY + PF4 + TSLP | 0,73 |
| CCL5 + CXCL9 + PF4 + REN | 0,73 |
| IFNG + CXCL9 + PF4 + REN | 0,73 |
| GZMH + IFNG + GNLY + PF4 | 0,73 |
| CXCL13 + GNLY + CCL5 + PF4 | 0,73 |
| ITGAE + CXCL9 + PF4 + REN | 0,73 |
| IFNG + GNLY + LAG3 + PF4 | 0,73 |
| IFNG + GNLY + ITGAE + PF4 | 0,73 |
| IFNG + GNLY + CCL5 + TSLP | 0,73 |
| LAG3 + PF4 + IL17A + REN | 0,73 |
| CXCL13 + CXCL9 + PF4 + REN | 0,73 |
| GZMH + GNLY + PF4 + IL17A | 0,73 |
| GNLY + PF4 + IL17A + TSLP | 0,73 |
| CXCL9 + PF4 + REN + IHH | 0,73 |
| GNLY + PF4 + IL17A + IHH | 0,73 |
| LAG3 + CXCL9 + PF4 + REN | 0,73 |
| IFNG + GNLY + ITGAE + CCL5 | 0,73 |
| CXCL13 + GNLY + LAG3 + PF4 | 0,73 |
| CXCL13 + LAG3 + PF4 + REN | 0,73 |
| CXCL13 + ITGAE + PF4 + REN | 0,73 |
| LAG3 + PF4 + REN + PROM1 | 0,73 |
| IFNG + GNLY + TSLP + IHH | 0,73 |
| LAG3 + PF4 + REN + VEGFA | 0,73 |
| CXCL13 + GNLY + IL17A + IHH | 0,73 |
| GNLY + PF4 + IL17A + VEGFA | 0,73 |
| IFNG + CXCL13 + GNLY + PF4 | 0,73 |
| GZMH + GNLY + CXCL9 + IL17A | 0,73 |
| GNLY + LAG3 + PF4 + IL17A | 0,73 |
| ITGAE + PF4 + REN + VEGFA | 0,73 |
| GNLY + PF4 + IL17A + PROM1 | 0,73 |
| CXCL13 + PF4 + REN + PROM1 | 0,73 |
| GNLY + CCL5 + PF4 + TSLP | 0,73 |
| CXCL13 + PF4 + TSLP + REN | 0,73 |
| GNLY + ITGAE + CCL5 + IL17A | 0,73 |
| ITGAE + PF4 + IL17A + REN | 0,73 |
| GNLY + CXCL9 + PF4 + IL17A | 0,73 |
| GNLY + CCL5 + CXCL9 + PF4 | 0,73 |
| GNLY + ITGAE + PF4 + IL17A | 0,73 |
| CXCL13 + PF4 + IL17A + TSLP | 0,73 |
| GZMH + CXCL13 + PF4 + REN | 0,73 |
| GZMH + GNLY + PF4 + TSLP | 0,73 |
| CXCL13 + CCL5 + PF4 + IL17A | 0,73 |
| LAG3 + ITGAE + PF4 + REN | 0,73 |
| GZMH + CXCL13 + GNLY + PF4 | 0,73 |
| GZMH + GNLY + CXCL9 + REN | 0,73 |
| CXCL13 + GNLY + PF4 + PROM1 | 0,73 |
| GNLY + ITGAE + CCL5 + REN | 0,72 |
| GNLY + CCL5 + CXCL9 + REN | 0,72 |
| GZMH + GNLY + ITGAE + REN | 0,72 |
| CXCL13 + GNLY + PF4 + IHH | 0,72 |
| GNLY + PF4 + TSLP + PROM1 | 0,72 |
| ITGAE + PF4 + REN + PROM1 | 0,72 |
| CXCL13 + PF4 + REN + VEGFA | 0,72 |
| IFNG + GNLY + CCL5 + CXCL9 | 0,72 |
| CXCL13 + GNLY + ITGAE + PF4 | 0,72 |
| GZMH + LAG3 + PF4 + REN | 0,72 |
| ITGAE + PF4 + TSLP + REN | 0,72 |
| IFNG + CXCL13 + GNLY + REN | 0,72 |
| LAG3 + PF4 + TSLP + REN | 0,72 |
| LAG3 + CCL5 + PF4 + REN | 0,72 |
| GZMH + GNLY + TSLP + REN | 0,72 |
| IFNG + LAG3 + PF4 + REN | 0,72 |
| CXCL13 + PF4 + REN + IHH | 0,72 |
| GNLY + PF4 + TSLP + IHH | 0,72 |
| CXCL13 + GNLY + CXCL9 + PF4 | 0,72 |
| LAG3 + PF4 + REN + IHH | 0,72 |
| CXCL13 + GNLY + PF4 + VEGFA | 0,72 |
| GNLY + LAG3 + CCL5 + REN | 0,72 |
| GNLY + CXCL9 + PF4 + TSLP | 0,72 |
| CXCL13 + PF4 + IL17A + VEGFA | 0,72 |
| CXCL13 + CCL5 + PF4 + REN | 0,72 |
| GZMH + CXCL13 + PF4 + IL17A | 0,72 |
| GZMH + GNLY + REN + PROM1 | 0,72 |
| GNLY + PF4 + TSLP + VEGFA | 0,72 |
| CXCL13 + CXCL9 + PF4 + IL17A | 0,72 |
| GNLY + CCL5 + PF4 + PROM1 | 0,72 |
| ITGAE + PF4 + REN + IHH | 0,72 |
| IFNG + GNLY + ITGAE + IHH | 0,72 |
| IFNG + CXCL13 + GNLY + TSLP | 0,72 |
| GNLY + ITGAE + PF4 + TSLP | 0,72 |
| GNLY + CCL5 + TSLP + REN | 0,72 |
| IFNG + CXCL13 + PF4 + REN | 0,72 |
| GZMH + ITGAE + PF4 + REN | 0,72 |
| CXCL13 + ITGAE + PF4 + IL17A | 0,72 |
| GNLY + CCL5 + REN + PROM1 | 0,72 |
| CXCL13 + LAG3 + PF4 + IL17A | 0,72 |
| GNLY + LAG3 + PF4 + TSLP | 0,72 |
| GZMH + GNLY + IL17A + TSLP | 0,72 |
| CXCL13 + PF4 + IL17A + PROM1 | 0,72 |
| GZMH + IFNG + GNLY + ITGAE | 0,72 |
| GZMH + GNLY + ITGAE + IL17A | 0,72 |
| IFNG + GNLY + ITGAE + CXCL9 | 0,72 |
| GNLY + LAG3 + CCL5 + IL17A | 0,72 |
| GNLY + CXCL9 + IL17A + TSLP | 0,72 |
| GZMH + GNLY + LAG3 + REN | 0,72 |
| GNLY + CCL5 + PF4 + IHH | 0,72 |
| IFNG + GNLY + ITGAE + VEGFA | 0,72 |
| CXCL13 + PF4 + IL17A + IHH | 0,72 |
| IFNG + CXCL13 + PF4 + IL17A | 0,72 |
| IFNG + ITGAE + PF4 + REN | 0,72 |
| GNLY + TSLP + REN + PROM1 | 0,72 |
| GNLY + CCL5 + IL17A + TSLP | 0,72 |
| GNLY + ITGAE + CCL5 + PF4 | 0,72 |
| ITGAE + CCL5 + PF4 + REN | 0,72 |
| GZMH + GNLY + PF4 + IHH | 0,72 |
| GNLY + PF4 + IHH + PROM1 | 0,72 |
| IFNG + GNLY + ITGAE + PROM1 | 0,72 |
| GZMH + GNLY + PF4 + PROM1 | 0,72 |
| IFNG + GNLY + LAG3 + ITGAE | 0,72 |
| IFNG + GNLY + CXCL9 + VEGFA | 0,72 |
| GNLY + ITGAE + TSLP + REN | 0,72 |
| GZMH + GNLY + CXCL9 + PF4 | 0,72 |
| GNLY + ITGAE + IL17A + TSLP | 0,72 |
| GNLY + CXCL9 + PF4 + IHH | 0,72 |
| GNLY + ITGAE + PF4 + IHH | 0,72 |
| GNLY + LAG3 + IL17A + TSLP | 0,72 |
| GNLY + TSLP + REN + IHH | 0,72 |
| GNLY + LAG3 + PF4 + IHH | 0,72 |
| GZMH + GNLY + REN + VEGFA | 0,72 |
| GNLY + IL17A + TSLP + PROM1 | 0,72 |
| GZMH + GNLY + LAG3 + IL17A | 0,72 |
| GNLY + PF4 + IHH + VEGFA | 0,72 |
| CXCL13 + GNLY + TSLP + REN | 0,72 |
| GNLY + CXCL9 + TSLP + REN | 0,72 |
| GNLY + IL17A + TSLP + VEGFA | 0,72 |
| IFNG + GNLY + PROM1 + VEGFA | 0,72 |
| GZMH + GNLY + CXCL9 + TSLP | 0,72 |
| GZMH + GNLY + CCL5 + REN | 0,72 |
| GNLY + CCL5 + PF4 + VEGFA | 0,72 |
| GZMH + GNLY + CCL5 + PF4 | 0,72 |
| IFNG + GNLY + CXCL9 + PROM1 | 0,72 |
| GNLY + LAG3 + CCL5 + PF4 | 0,72 |
| GNLY + CXCL9 + PF4 + PROM1 | 0,72 |
| GZMH + GNLY + IL17A + PROM1 | 0,72 |
| GNLY + LAG3 + REN + PROM1 | 0,72 |
| GNLY + PF4 + PROM1 + VEGFA | 0,72 |
| IFNG + GNLY + IHH + VEGFA | 0,72 |
| GZMH + GNLY + REN + IHH | 0,72 |
| GNLY + CCL5 + CXCL9 + TSLP | 0,72 |
| GZMH + CXCL13 + GNLY + REN | 0,72 |
| GNLY + LAG3 + PF4 + PROM1 | 0,72 |
| GNLY + TSLP + REN + VEGFA | 0,72 |
| IFNG + GNLY + CCL5 + VEGFA | 0,72 |
| IFNG + GNLY + CXCL9 + IHH | 0,72 |
| GZMH + IFNG + GNLY + VEGFA | 0,72 |
| GZMH + GNLY + ITGAE + PF4 | 0,72 |
| GNLY + IL17A + TSLP + IHH | 0,72 |
| GNLY + ITGAE + PF4 + PROM1 | 0,72 |
| GNLY + REN + IHH + PROM1 | 0,72 |
| IFNG + GNLY + LAG3 + CXCL9 | 0,72 |
| CCL5 + PF4 + IL17A + REN | 0,72 |
| GNLY + LAG3 + TSLP + REN | 0,72 |
| GNLY + ITGAE + CCL5 + TSLP | 0,72 |
| IFNG + GNLY + LAG3 + VEGFA | 0,72 |
| GNLY + CXCL9 + IL17A + VEGFA | 0,72 |
| GZMH + GNLY + PF4 + VEGFA | 0,72 |
| GZMH + GNLY + IL17A + VEGFA | 0,72 |
| GZMH + IFNG + GNLY + CXCL9 | 0,72 |
| GZMH + GNLY + LAG3 + PF4 | 0,72 |
| GNLY + LAG3 + CCL5 + TSLP | 0,72 |
| IFNG + GNLY + LAG3 + CCL5 | 0,72 |
| IFNG + CXCL9 + PF4 + TSLP | 0,72 |
| GZMH + GNLY + IL17A + IHH | 0,72 |
| GNLY + CCL5 + REN + VEGFA | 0,72 |
| GNLY + CCL5 + REN + IHH | 0,71 |
| CXCL13 + GNLY + CCL5 + REN | 0,71 |
| GNLY + CXCL9 + PF4 + VEGFA | 0,71 |
| CXCL13 + GNLY + REN + PROM1 | 0,71 |
| GNLY + LAG3 + CXCL9 + PF4 | 0,71 |
| CCL5 + PF4 + REN + PROM1 | 0,71 |
| GNLY + ITGAE + REN + PROM1 | 0,71 |
| GNLY + CCL5 + IL17A + PROM1 | 0,71 |
| IFNG + GNLY + CCL5 + PROM1 | 0,71 |
| GZMH + GNLY + ITGAE + TSLP | 0,71 |
| GZMH + GNLY + CCL5 + IL17A | 0,71 |
| GNLY + CXCL9 + REN + PROM1 | 0,71 |
| GNLY + REN + PROM1 + VEGFA | 0,71 |
| GNLY + LAG3 + IL17A + PROM1 | 0,71 |
| GNLY + LAG3 + ITGAE + PF4 | 0,71 |
| GNLY + ITGAE + CXCL9 + PF4 | 0,71 |
| CXCL13 + CXCL9 + PF4 + TSLP | 0,71 |
| GNLY + ITGAE + PF4 + VEGFA | 0,71 |
| GNLY + ITGAE + CXCL9 + IL17A | 0,71 |
| GNLY + LAG3 + PF4 + VEGFA | 0,71 |
| GNLY + CXCL9 + IL17A + PROM1 | 0,71 |
| CXCL13 + GNLY + CCL5 + TSLP | 0,71 |
| GNLY + ITGAE + IL17A + VEGFA | 0,71 |
| GNLY + LAG3 + IL17A + VEGFA | 0,71 |
| GNLY + LAG3 + CXCL9 + IL17A | 0,71 |
| GNLY + ITGAE + IL17A + PROM1 | 0,71 |
| CXCL13 + PF4 + TSLP + PROM1 | 0,71 |
| CCL5 + PF4 + TSLP + REN | 0,71 |
| IFNG + GNLY + LAG3 + PROM1 | 0,71 |
| GNLY + LAG3 + ITGAE + IL17A | 0,71 |
| CCL5 + PF4 + REN + VEGFA | 0,71 |
| CXCL13 + LAG3 + PF4 + TSLP | 0,71 |
| GNLY + ITGAE + REN + VEGFA | 0,71 |
| IFNG + GNLY + LAG3 + IHH | 0,71 |
| CXCL13 + ITGAE + PF4 + TSLP | 0,71 |
| GZMH + CXCL13 + GNLY + TSLP | 0,71 |
| GNLY + CCL5 + IL17A + VEGFA | 0,71 |
| GNLY + LAG3 + ITGAE + REN | 0,71 |
| GNLY + IL17A + PROM1 + VEGFA | 0,71 |
| GZMH + IFNG + GNLY + PROM1 | 0,71 |
| GZMH + IFNG + GNLY + CCL5 | 0,71 |
| IFNG + GNLY + CCL5 + IHH | 0,71 |
| IFNG + CXCL13 + GNLY + VEGFA | 0,71 |
| CCL5 + PF4 + REN + IHH | 0,71 |
| IFNG + CXCL13 + GNLY + ITGAE | 0,71 |
| GNLY + CCL5 + IL17A + IHH | 0,71 |
| GNLY + ITGAE + CXCL9 + REN | 0,71 |
| GNLY + CXCL9 + IL17A + IHH | 0,71 |
| GNLY + LAG3 + CXCL9 + REN | 0,71 |
| GNLY + LAG3 + REN + VEGFA | 0,71 |
| GZMH + CXCL13 + PF4 + TSLP | 0,71 |
| GZMH + IFNG + GNLY + IHH | 0,71 |
| IFNG + CCL5 + PF4 + REN | 0,71 |
| CXCL13 + GNLY + LAG3 + REN | 0,71 |
| CXCL13 + PF4 + TSLP + VEGFA | 0,71 |
| GZMH + IFNG + GNLY + LAG3 | 0,71 |
| GNLY + ITGAE + REN + IHH | 0,71 |
| GNLY + CXCL9 + REN + VEGFA | 0,71 |
| IFNG + GNLY + IHH + PROM1 | 0,71 |
| GZMH + CCL5 + PF4 + REN | 0,71 |
| GNLY + LAG3 + IL17A + IHH | 0,71 |
| CXCL13 + GNLY + ITGAE + REN | 0,71 |
| CXCL13 + GNLY + REN + VEGFA | 0,71 |
| CXCL13 + CCL5 + PF4 + TSLP | 0,71 |
| GZMH + GNLY + LAG3 + TSLP | 0,71 |
| CXCL13 + GNLY + CXCL9 + REN | 0,71 |
| GNLY + IL17A + IHH + PROM1 | 0,71 |
| GNLY + LAG3 + REN + IHH | 0,71 |
| GNLY + CXCL9 + REN + IHH | 0,71 |
| IFNG + CXCL13 + PF4 + TSLP | 0,71 |
| GZMH + GNLY + TSLP + PROM1 | 0,71 |
| GNLY + REN + IHH + VEGFA | 0,71 |
| GZMH + GNLY + TSLP + VEGFA | 0,71 |
| GNLY + IL17A + IHH + VEGFA | 0,71 |
| GNLY + ITGAE + IL17A + IHH | 0,71 |
| GZMH + PF4 + IL17A + REN | 0,71 |
| GZMH + GNLY + CCL5 + CXCL9 | 0,71 |
| CXCL13 + LAG3 + IL17A + REN | 0,71 |
| GZMH + GNLY + CXCL9 + PROM1 | 0,71 |
| GZMH + GNLY + CCL5 + TSLP | 0,71 |
| CXCL13 + GNLY + TSLP + PROM1 | 0,71 |
| CXCL13 + PF4 + TSLP + IHH | 0,71 |
| CXCL13 + CCL5 + CXCL9 + PF4 | 0,71 |
| GNLY + CCL5 + TSLP + PROM1 | 0,71 |
| GZMH + CXCL9 + IL17A + REN | 0,71 |
| IFNG + CXCL9 + IL17A + REN | 0,71 |
| GZMH + PF4 + REN + PROM1 | 0,71 |
| CXCL9 + PF4 + IL17A + TSLP | 0,71 |
| CXCL13 + GNLY + LAG3 + TSLP | 0,71 |
| GNLY + LAG3 + CCL5 + CXCL9 | 0,71 |
| CXCL13 + GNLY + REN + IHH | 0,71 |
| GZMH + CXCL9 + PF4 + TSLP | 0,71 |
| CXCL13 + GNLY + CXCL9 + TSLP | 0,71 |
| IFNG + CXCL13 + GNLY + CXCL9 | 0,71 |
| GZMH + GNLY + TSLP + IHH | 0,71 |
| CXCL13 + GNLY + TSLP + VEGFA | 0,71 |
| CXCL13 + GNLY + ITGAE + TSLP | 0,71 |
| IFNG + CXCL9 + TSLP + REN | 0,71 |
| CXCL13 + CXCL9 + PF4 + PROM1 | 0,71 |
| CXCL9 + PF4 + IL17A + VEGFA | 0,71 |
| GZMH + GNLY + ITGAE + CXCL9 | 0,71 |
| IFNG + ITGAE + CXCL9 + REN | 0,71 |
| CXCL9 + PF4 + TSLP + VEGFA | 0,71 |
| CXCL13 + ITGAE + PF4 + PROM1 | 0,71 |
| LAG3 + IL17A + REN + VEGFA | 0,71 |
| GNLY + LAG3 + CCL5 + PROM1 | 0,71 |
| GZMH + CXCL13 + CXCL9 + PF4 | 0,71 |
| CCL5 + CXCL9 + PF4 + TSLP | 0,71 |
| GZMH + PF4 + REN + VEGFA | 0,71 |
| CXCL13 + CXCL9 + IL17A + REN | 0,71 |
| IFNG + CXCL13 + GNLY + PROM1 | 0,71 |
| CXCL13 + CXCL9 + PF4 + VEGFA | 0,71 |
| GZMH + GNLY + CXCL9 + IHH | 0,71 |
| GZMH + CXCL13 + ITGAE + PF4 | 0,71 |
| CXCL13 + ITGAE + IL17A + REN | 0,71 |
| GNLY + CCL5 + TSLP + VEGFA | 0,71 |
| CXCL13 + ITGAE + CCL5 + PF4 | 0,71 |
| LAG3 + CCL5 + IL17A + REN | 0,71 |
| GNLY + CCL5 + TSLP + IHH | 0,71 |
| CXCL13 + LAG3 + PF4 + PROM1 | 0,71 |
| IFNG + CXCL9 + PF4 + IL17A | 0,71 |
| CCL5 + CXCL9 + IL17A + REN | 0,71 |
| CXCL13 + ITGAE + PF4 + VEGFA | 0,71 |
| GZMH + PF4 + TSLP + REN | 0,71 |
| IFNG + PF4 + IL17A + REN | 0,71 |
| GZMH + GNLY + CXCL9 + VEGFA | 0,71 |
| CXCL13 + LAG3 + ITGAE + PF4 | 0,71 |
| CXCL9 + IL17A + REN + IHH | 0,71 |
| CXCL13 + ITGAE + PF4 + IHH | 0,71 |
| GZMH + CXCL13 + PF4 + PROM1 | 0,71 |
| CXCL13 + CXCL9 + PF4 + IHH | 0,71 |
| GZMH + PF4 + REN + IHH | 0,71 |
| CXCL13 + ITGAE + CXCL9 + PF4 | 0,71 |
| CCL5 + CXCL9 + PF4 + IL17A | 0,71 |
| CXCL13 + LAG3 + CXCL9 + PF4 | 0,71 |
| CXCL13 + GNLY + LAG3 + CCL5 | 0,71 |
| CXCL13 + PF4 + PROM1 + VEGFA | 0,71 |
| CXCL13 + LAG3 + PF4 + IHH | 0,71 |
| IFNG + CXCL13 + CXCL9 + PF4 | 0,71 |
| GZMH + CXCL13 + GNLY + PROM1 | 0,71 |
| ITGAE + CXCL9 + IL17A + REN | 0,71 |
| CXCL13 + LAG3 + PF4 + VEGFA | 0,71 |
| GZMH + CXCL13 + LAG3 + PF4 | 0,71 |
| PF4 + TSLP + REN + PROM1 | 0,71 |
| ITGAE + IL17A + REN + VEGFA | 0,71 |
| GZMH + GNLY + LAG3 + CXCL9 | 0,71 |
| IFNG + CXCL9 + PF4 + IHH | 0,71 |
| IFNG + LAG3 + IL17A + REN | 0,71 |
| GZMH + CXCL13 + GNLY + VEGFA | 0,71 |
| CXCL13 + GNLY + TSLP + IHH | 0,71 |
| LAG3 + CXCL9 + IL17A + REN | 0,7 |
| CXCL9 + IL17A + REN + VEGFA | 0,7 |
| GZMH + CXCL13 + GNLY + ITGAE | 0,7 |
| LAG3 + ITGAE + IL17A + REN | 0,7 |
| CXCL9 + PF4 + TSLP + PROM1 | 0,7 |
| IFNG + CXCL13 + GNLY + CCL5 | 0,7 |
| GZMH + IFNG + PF4 + REN | 0,7 |
| GNLY + CCL5 + CXCL9 + PROM1 | 0,7 |
| PF4 + IL17A + REN + PROM1 | 0,7 |
| CXCL13 + PF4 + IHH + PROM1 | 0,7 |
| GZMH + LAG3 + IL17A + REN | 0,7 |
| IFNG + CXCL13 + GNLY + LAG3 | 0,7 |
| CXCL13 + GNLY + ITGAE + CCL5 | 0,7 |
| CXCL13 + GNLY + CCL5 + PROM1 | 0,7 |
| GZMH + GNLY + ITGAE + IHH | 0,7 |
| IFNG + CXCL9 + PF4 + VEGFA | 0,7 |
| GNLY + LAG3 + TSLP + PROM1 | 0,7 |
| GZMH + CXCL13 + PF4 + VEGFA | 0,7 |
| CXCL13 + IL17A + TSLP + REN | 0,7 |
| GZMH + CXCL13 + GNLY + CXCL9 | 0,7 |
| GZMH + GNLY + ITGAE + PROM1 | 0,7 |
| LAG3 + IL17A + REN + PROM1 | 0,7 |
| CXCL13 + CCL5 + PF4 + PROM1 | 0,7 |
| GZMH + IFNG + CXCL13 + GNLY | 0,7 |
| PF4 + IL17A + TSLP + REN | 0,7 |
| GZMH + CXCL9 + PF4 + IL17A | 0,7 |
| CXCL13 + IL17A + REN + IHH | 0,7 |
| ITGAE + CXCL9 + PF4 + TSLP | 0,7 |
| CXCL13 + IL17A + REN + VEGFA | 0,7 |
| IFNG + PF4 + REN + PROM1 | 0,7 |
| CXCL9 + PF4 + IL17A + IHH | 0,7 |
| GZMH + CXCL13 + CCL5 + PF4 | 0,7 |
| CXCL13 + LAG3 + CCL5 + IL17A | 0,7 |
| CXCL9 + PF4 + TSLP + IHH | 0,7 |
| CXCL13 + IL17A + REN + PROM1 | 0,7 |
| GZMH + CXCL13 + PF4 + IHH | 0,7 |
| PF4 + TSLP + REN + IHH | 0,7 |
| CXCL9 + IL17A + TSLP + REN | 0,7 |
| CXCL13 + GNLY + CCL5 + CXCL9 | 0,7 |
| IFNG + CXCL13 + PF4 + PROM1 | 0,7 |
| IFNG + ITGAE + CXCL9 + PF4 | 0,7 |
| GNLY + LAG3 + ITGAE + TSLP | 0,7 |
| GNLY + CXCL9 + TSLP + PROM1 | 0,7 |
| GNLY + LAG3 + CXCL9 + TSLP | 0,7 |
| CXCL13 + GNLY + CCL5 + VEGFA | 0,7 |
| LAG3 + IL17A + REN + IHH | 0,7 |
| GZMH + CXCL13 + IL17A + REN | 0,7 |
| GNLY + ITGAE + TSLP + PROM1 | 0,7 |
| PF4 + IL17A + REN + IHH | 0,7 |
| CXCL13 + LAG3 + CCL5 + PF4 | 0,7 |
| CXCL13 + PF4 + IHH + VEGFA | 0,7 |
| IFNG + CXCL13 + ITGAE + PF4 | 0,7 |
| IFNG + CXCL13 + LAG3 + PF4 | 0,7 |
| PF4 + REN + IHH + PROM1 | 0,7 |
| IFNG + CXCL9 + PF4 + PROM1 | 0,7 |
| CXCL9 + PF4 + IL17A + PROM1 | 0,7 |
| GNLY + LAG3 + ITGAE + CCL5 | 0,7 |
| GNLY + ITGAE + CXCL9 + TSLP | 0,7 |
| ITGAE + CXCL9 + PF4 + IL17A | 0,7 |
| GNLY + LAG3 + TSLP + VEGFA | 0,7 |
| CXCL13 + CCL5 + PF4 + VEGFA | 0,7 |
| IFNG + PF4 + TSLP + REN | 0,7 |
| GNLY + CXCL9 + TSLP + VEGFA | 0,7 |
| ITGAE + PF4 + TSLP + VEGFA | 0,7 |
| GNLY + TSLP + PROM1 + VEGFA | 0,7 |
| GZMH + GNLY + LAG3 + ITGAE | 0,7 |
| IFNG + CCL5 + CXCL9 + PF4 | 0,7 |
| IFNG + ITGAE + IL17A + REN | 0,7 |
| CXCL13 + CCL5 + IL17A + REN | 0,7 |
| PF4 + IL17A + REN + VEGFA | 0,7 |
| GNLY + ITGAE + TSLP + VEGFA | 0,7 |
| ITGAE + CCL5 + CXCL9 + PF4 | 0,7 |
| GNLY + CXCL9 + TSLP + IHH | 0,7 |
| IFNG + CXCL13 + GNLY + IHH | 0,7 |
| IFNG + CXCL9 + REN + PROM1 | 0,7 |
| PF4 + REN + PROM1 + VEGFA | 0,7 |
| CXCL9 + IL17A + REN + PROM1 | 0,7 |
| LAG3 + CXCL9 + PF4 + TSLP | 0,7 |
| GZMH + CXCL13 + GNLY + LAG3 | 0,7 |
| LAG3 + IL17A + TSLP + REN | 0,7 |
| IFNG + CXCL13 + PF4 + VEGFA | 0,7 |
| ITGAE + PF4 + IL17A + VEGFA | 0,7 |
| GZMH + ITGAE + CXCL9 + REN | 0,7 |
| PF4 + TSLP + REN + VEGFA | 0,7 |
| IFNG + LAG3 + CXCL9 + REN | 0,7 |
| IFNG + CXCL13 + CCL5 + PF4 | 0,7 |
| IFNG + CXCL13 + IL17A + REN | 0,7 |
| CCL5 + CXCL9 + PF4 + VEGFA | 0,7 |
| CXCL13 + CCL5 + PF4 + IHH | 0,7 |
| GZMH + GNLY + ITGAE + VEGFA | 0,7 |
| ITGAE + PF4 + IL17A + TSLP | 0,7 |
| LAG3 + CXCL9 + PF4 + IL17A | 0,7 |
| GZMH + IFNG + CXCL9 + PF4 | 0,7 |
| GZMH + GNLY + ITGAE + CCL5 | 0,7 |
| GZMH + IFNG + CXCL9 + REN | 0,7 |
| GZMH + IFNG + CXCL13 + PF4 | 0,7 |
| GZMH + CXCL13 + GNLY + CCL5 | 0,7 |
| IFNG + CXCL13 + PF4 + IHH | 0,7 |
| IFNG + ITGAE + TSLP + REN | 0,7 |
| LAG3 + CCL5 + REN + PROM1 | 0,7 |
| GNLY + TSLP + IHH + PROM1 | 0,7 |
| IFNG + LAG3 + REN + PROM1 | 0,7 |
| GZMH + GNLY + LAG3 + PROM1 | 0,7 |
| GNLY + LAG3 + CCL5 + IHH | 0,7 |
| GNLY + ITGAE + TSLP + IHH | 0,7 |
| GZMH + ITGAE + IL17A + REN | 0,7 |
| IFNG + CCL5 + CXCL9 + REN | 0,7 |
| GNLY + LAG3 + TSLP + IHH | 0,7 |
| LAG3 + CCL5 + CXCL9 + REN | 0,7 |
| IFNG + LAG3 + CXCL9 + PF4 | 0,7 |
| GNLY + TSLP + IHH + VEGFA | 0,7 |
| GNLY + CCL5 + CXCL9 + VEGFA | 0,7 |
| GZMH + CXCL9 + PF4 + IHH | 0,7 |
| IFNG + ITGAE + PF4 + TSLP | 0,7 |

| **Table 5: clusters of 5 genes:** | |
|---|---|
| **Clusters of 5 genes** | **c_tau level** |
| IFNG + GNLY + PF4 + IL17A + REN | 0,78 |
| GNLY + CCL5 + PF4 + IL17A + REN | 0,77 |
| CXCL13 + GNLY + PF4 + IL17A + REN | 0,77 |
| GNLY + PF4 + IL17A + REN + IHH | 0,77 |
| GNLY + PF4 + IL17A + REN + PROM1 | 0,77 |
| GZMH + GNLY + PF4 + IL17A + REN | 0,77 |
| GNLY + PF4 + IL17A + TSLP + REN | 0,77 |
| IFNG + GNLY + ITGAE + IL17A + REN | 0,77 |
| GNLY + CXCL9 + PF4 + IL17A + REN | 0,77 |
| GNLY + ITGAE + PF4 + IL17A + REN | 0,77 |
| IFNG + GNLY + PF4 + REN + PROM1 | 0,77 |
| GNLY + PF4 + IL17A + REN + VEGFA | 0,77 |
| GNLY + LAG3 + PF4 + IL17A + REN | 0,77 |
| IFNG + GNLY + PF4 + TSLP + REN | 0,77 |
| IFNG + GNLY + CCL5 + PF4 + REN | 0,77 |
| IFNG + GNLY + CXCL9 + IL17A + REN | 0,77 |
| IFNG + GNLY + LAG3 + IL17A + REN | 0,77 |
| IFNG + GNLY + PF4 + REN + VEGFA | 0,77 |
| GZMH + IFNG + GNLY + PF4 + REN | 0,77 |
| GNLY + ITGAE + CCL5 + PF4 + REN | 0,76 |
| IFNG + GNLY + LAG3 + PF4 + REN | 0,76 |
| IFNG + GNLY + ITGAE + PF4 + REN | 0,76 |
| IFNG + GNLY + PF4 + REN + IHH | 0,76 |
| IFNG + CXCL13 + GNLY + IL17A + REN | 0,76 |
| IFNG + GNLY + IL17A + REN + IHH | 0,76 |
| GNLY + CCL5 + PF4 + REN + PROM1 | 0,76 |
| IFNG + GNLY + IL17A + TSLP + REN | 0,76 |
| IFNG + GNLY + IL17A + REN + PROM1 | 0,76 |
| IFNG + GNLY + CXCL9 + PF4 + REN | 0,76 |
| GNLY + CCL5 + CXCL9 + PF4 + REN | 0,76 |
| GZMH + IFNG + GNLY + IL17A + REN | 0,76 |
| IFNG + GNLY + IL17A + REN + VEGFA | 0,76 |
| IFNG + GNLY + CCL5 + IL17A + REN | 0,76 |
| GNLY + PF4 + TSLP + REN + PROM1 | 0,76 |
| CXCL13 + GNLY + CCL5 + PF4 + IL17A | 0,76 |
| GNLY + PF4 + REN + IHH + PROM1 | 0,76 |
| GNLY + CCL5 + PF4 + TSLP + REN | 0,76 |
| GZMH + GNLY + PF4 + REN + PROM1 | 0,76 |
| GNLY + ITGAE + PF4 + REN + PROM1 | 0,76 |
| GNLY + CXCL9 + PF4 + REN + PROM1 | 0,76 |
| GNLY + PF4 + REN + PROM1 + VEGFA | 0,76 |
| GNLY + LAG3 + PF4 + REN + PROM1 | 0,76 |
| GNLY + LAG3 + CCL5 + PF4 + REN | 0,76 |
| GZMH + GNLY + PF4 + TSLP + REN | 0,76 |
| GZMH + GNLY + ITGAE + PF4 + REN | 0,76 |
| GNLY + CCL5 + PF4 + REN + IHH | 0,76 |
| GNLY + CCL5 + PF4 + REN + VEGFA | 0,76 |
| GNLY + ITGAE + PF4 + TSLP + REN | 0,76 |
| GNLY + ITGAE + CCL5 + IL17A + REN | 0,76 |
| GZMH + GNLY + CCL5 + PF4 + REN | 0,76 |
| CXCL13 + GNLY + PF4 + REN + PROM1 | 0,76 |
| CXCL13 + GNLY + CCL5 + IL17A + REN | 0,76 |
| GZMH + GNLY + CXCL9 + PF4 + REN | 0,76 |
| IFNG + CXCL13 + GNLY + PF4 + REN | 0,76 |
| GNLY + CXCL9 + PF4 + TSLP + REN | 0,76 |
| GNLY + PF4 + TSLP + REN + VEGFA | 0,76 |
| GNLY + PF4 + TSLP + REN + IHH | 0,76 |
| GNLY + LAG3 + PF4 + TSLP + REN | 0,76 |
| CXCL13 + GNLY + ITGAE + CCL5 + IL17A | 0,76 |
| GZMH + GNLY + PF4 + REN + IHH | 0,76 |
| GZMH + GNLY + LAG3 + PF4 + REN | 0,76 |
| GZMH + GNLY + PF4 + REN + VEGFA | 0,76 |
| GNLY + ITGAE + PF4 + REN + VEGFA | 0,76 |
| GNLY + LAG3 + ITGAE + PF4 + REN | 0,76 |
| GNLY + ITGAE + PF4 + REN + IHH | 0,76 |
| GNLY + LAG3 + CXCL9 + PF4 + REN | 0,76 |
| GNLY + ITGAE + CXCL9 + PF4 + REN | 0,76 |
| CXCL13 + GNLY + CCL5 + PF4 + REN | 0,75 |
| GNLY + CXCL9 + PF4 + REN + IHH | 0,75 |
| GNLY + CXCL9 + PF4 + REN + VEGFA | 0,75 |
| GNLY + PF4 + REN + IHH + VEGFA | 0,75 |
| CXCL13 + GNLY + PF4 + TSLP + REN | 0,75 |
| GNLY + LAG3 + PF4 + REN + IHH | 0,75 |
| GZMH + CXCL13 + GNLY + IL17A + REN | 0,75 |
| GNLY + LAG3 + PF4 + REN + VEGFA | 0,75 |
| GNLY + LAG3 + CCL5 + IL17A + REN | 0,75 |
| CXCL13 + GNLY + LAG3 + PF4 + REN | 0,75 |
| GZMH + CXCL13 + GNLY + PF4 + REN | 0,75 |
| GNLY + CCL5 + CXCL9 + IL17A + REN | 0,75 |
| CXCL13 + GNLY + IL17A + REN + IHH | 0,75 |
| IFNG + GNLY + ITGAE + CCL5 + IL17A | 0,75 |
| IFNG + GNLY + CCL5 + CXCL9 + IL17A | 0,75 |
| CXCL13 + GNLY + PF4 + REN + VEGFA | 0,75 |
| CXCL13 + GNLY + ITGAE + PF4 + REN | 0,75 |
| IFNG + CXCL13 + GNLY + CCL5 + IL17A | 0,75 |
| CXCL13 + GNLY + CXCL9 + PF4 + REN | 0,75 |
| GZMH + GNLY + ITGAE + IL17A + REN | 0,75 |
| GZMH + GNLY + CXCL9 + IL17A + REN | 0,75 |
| CXCL13 + GNLY + LAG3 + PF4 + IL17A | 0,75 |
| CXCL13 + GNLY + PF4 + REN + IHH | 0,75 |
| IFNG + CXCL13 + GNLY + PF4 + IL17A | 0,75 |
| IFNG + GNLY + PF4 + IL17A + IHH | 0,75 |
| CXCL13 + GNLY + IL17A + TSLP + REN | 0,75 |
| GNLY + IL17A + TSLP + REN + IHH | 0,75 |
| CXCL13 + GNLY + LAG3 + IL17A + REN | 0,75 |
| CXCL13 + GNLY + IL17A + REN + PROM1 | 0,75 |
| CXCL13 + GNLY + LAG3 + CCL5 + IL17A | 0,75 |
| GNLY + CXCL9 + IL17A + REN + IHH | 0,75 |
| IFNG + GNLY + ITGAE + IL17A + TSLP | 0,75 |
| IFNG + CXCL13 + GNLY + ITGAE + IL17A | 0,75 |
| CXCL13 + GNLY + CCL5 + CXCL9 + IL17A | 0,75 |
| CXCL13 + GNLY + CXCL9 + IL17A + REN | 0,75 |
| IFNG + CXCL13 + GNLY + IL17A + TSLP | 0,75 |
| GNLY + ITGAE + IL17A + REN + IHH | 0,75 |
| IFNG + GNLY + PF4 + IL17A + TSLP | 0,75 |
| GNLY + IL17A + REN + IHH + PROM1 | 0,75 |
| IFNG + GNLY + ITGAE + CCL5 + REN | 0,75 |
| CXCL13 + GNLY + IL17A + REN + VEGFA | 0,75 |
| GZMH + IFNG + CXCL13 + GNLY + IL17A | 0,75 |
| IFNG + CXCL13 + GNLY + CXCL9 + IL17A | 0,75 |
| CXCL13 + GNLY + ITGAE + IL17A + REN | 0,75 |
| IFNG + GNLY + CXCL9 + IL17A + TSLP | 0,75 |
| GZMH + GNLY + IL17A + REN + IHH | 0,75 |
| IFNG + CXCL13 + GNLY + LAG3 + IL17A | 0,75 |
| IFNG + CXCL13 + GNLY + IL17A + VEGFA | 0,75 |
| CXCL13 + GNLY + CCL5 + IL17A + PROM1 | 0,75 |
| GZMH + CXCL13 + GNLY + CCL5 + IL17A | 0,75 |
| IFNG + CXCL13 + GNLY + IL17A + PROM1 | 0,75 |
| GNLY + LAG3 + IL17A + REN + IHH | 0,75 |
| CXCL13 + GNLY + CCL5 + IL17A + TSLP | 0,75 |
| GNLY + CCL5 + IL17A + REN + IHH | 0,75 |
| GNLY + IL17A + REN + IHH + VEGFA | 0,75 |
| GZMH + IFNG + GNLY + CXCL9 + IL17A | 0,75 |
| IFNG + GNLY + CXCL9 + PF4 + IL17A | 0,75 |
| IFNG + GNLY + ITGAE + IL17A + IHH | 0,75 |
| CXCL13 + GNLY + CCL5 + IL17A + VEGFA | 0,75 |
| GZMH + GNLY + LAG3 + IL17A + REN | 0,75 |
| IFNG + GNLY + ITGAE + CXCL9 + IL17A | 0,75 |
| IFNG + GNLY + ITGAE + TSLP + REN | 0,75 |
| IFNG + GNLY + CCL5 + PF4 + IL17A | 0,75 |
| IFNG + GNLY + LAG3 + IL17A + TSLP | 0,75 |
| IFNG + GNLY + CXCL9 + IL17A + VEGFA | 0,75 |
| IFNG + GNLY + CXCL9 + IL17A + PROM1 | 0,75 |
| IFNG + GNLY + PF4 + IL17A + VEGFA | 0,75 |
| GZMH + CXCL13 + GNLY + PF4 + IL17A | 0,75 |
| CXCL13 + GNLY + CCL5 + IL17A + IHH | 0,75 |
| IFNG + GNLY + ITGAE + PF4 + IL17A | 0,75 |
| IFNG + GNLY + LAG3 + PF4 + IL17A | 0,75 |
| GNLY + ITGAE + IL17A + TSLP + REN | 0,75 |
| GZMH + IFNG + GNLY + PF4 + IL17A | 0,75 |
| IFNG + CXCL13 + GNLY + IL17A + IHH | 0,75 |
| IFNG + GNLY + LAG3 + ITGAE + IL17A | 0,75 |
| IFNG + GNLY + ITGAE + IL17A + VEGFA | 0,75 |
| IFNG + GNLY + LAG3 + CXCL9 + IL17A | 0,74 |
| GNLY + CXCL9 + IL17A + TSLP + REN | 0,74 |
| IFNG + GNLY + ITGAE + IL17A + PROM1 | 0,74 |
| GZMH + IFNG + GNLY + ITGAE + IL17A | 0,74 |
| IFNG + GNLY + PF4 + IL17A + PROM1 | 0,74 |
| GZMH + GNLY + IL17A + TSLP + REN | 0,74 |
| IFNG + GNLY + IL17A + TSLP + VEGFA | 0,74 |
| IFNG + GNLY + CXCL9 + IL17A + IHH | 0,74 |
| GNLY + ITGAE + CXCL9 + IL17A + REN | 0,74 |
| IFNG + GNLY + CCL5 + IL17A + TSLP | 0,74 |
| GNLY + ITGAE + IL17A + REN + VEGFA | 0,74 |
| GZMH + IFNG + GNLY + IL17A + TSLP | 0,74 |
| CXCL13 + GNLY + CXCL9 + PF4 + IL17A | 0,74 |
| GNLY + CXCL9 + IL17A + REN + PROM1 | 0,74 |
| GZMH + GNLY + IL17A + REN + PROM1 | 0,74 |
| GNLY + CXCL9 + IL17A + REN + VEGFA | 0,74 |
| GNLY + IL17A + TSLP + REN + PROM1 | 0,74 |
| IFNG + GNLY + IL17A + TSLP + PROM1 | 0,74 |
| GNLY + LAG3 + IL17A + TSLP + REN | 0,74 |
| GNLY + ITGAE + IL17A + REN + PROM1 | 0,74 |
| CXCL13 + GNLY + ITGAE + PF4 + IL17A | 0,74 |
| GNLY + LAG3 + IL17A + REN + PROM1 | 0,74 |
| IFNG + GNLY + LAG3 + CCL5 + IL17A | 0,74 |
| GNLY + LAG3 + CXCL9 + IL17A + REN | 0,74 |
| GNLY + CCL5 + IL17A + TSLP + REN | 0,74 |
| IFNG + GNLY + PF4 + TSLP + IHH | 0,74 |
| GNLY + LAG3 + ITGAE + IL17A + REN | 0,74 |
| GNLY + IL17A + TSLP + REN + VEGFA | 0,74 |
| IFNG + GNLY + ITGAE + REN + PROM1 | 0,74 |
| GNLY + LAG3 + IL17A + REN + VEGFA | 0,74 |
| IFNG + GNLY + CCL5 + PF4 + TSLP | 0,74 |
| GZMH + GNLY + IL17A + REN + VEGFA | 0,74 |
| IFNG + GNLY + ITGAE + CCL5 + TSLP | 0,74 |
| GNLY + CCL5 + IL17A + REN + PROM1 | 0,74 |
| IFNG + GNLY + TSLP + REN + PROM1 | 0,74 |
| IFNG + GNLY + IL17A + TSLP + IHH | 0,74 |
| GZMH + IFNG + GNLY + PF4 + TSLP | 0,74 |
| GNLY + IL17A + REN + PROM1 + VEGFA | 0,74 |
| IFNG + GNLY + PF4 + TSLP + VEGFA | 0,74 |
| IFNG + GNLY + PF4 + TSLP + PROM1 | 0,74 |
| IFNG + GNLY + TSLP + REN + VEGFA | 0,74 |
| GZMH + IFNG + GNLY + CCL5 + IL17A | 0,74 |
| GZMH + CXCL13 + GNLY + IL17A + TSLP | 0,74 |
| CXCL13 + GNLY + PF4 + IL17A + TSLP | 0,74 |
| GNLY + CCL5 + CXCL9 + PF4 + IL17A | 0,74 |
| IFNG + GNLY + LAG3 + IL17A + VEGFA | 0,74 |
| IFNG + GNLY + CXCL9 + TSLP + REN | 0,74 |
| GZMH + IFNG + GNLY + LAG3 + IL17A | 0,74 |
| GZMH + GNLY + CCL5 + IL17A + REN | 0,74 |
| GZMH + IFNG + GNLY + ITGAE + REN | 0,74 |
| IFNG + GNLY + CCL5 + IL17A + VEGFA | 0,74 |
| IFNG + GNLY + LAG3 + PF4 + TSLP | 0,74 |
| IFNG + GNLY + LAG3 + TSLP + REN | 0,74 |
| IFNG + GNLY + LAG3 + IL17A + IHH | 0,74 |
| IFNG + GNLY + LAG3 + IL17A + PROM1 | 0,74 |
| IFNG + GNLY + CCL5 + TSLP + REN | 0,74 |
| CXCL13 + GNLY + PF4 + IL17A + IHH | 0,74 |
| IFNG + GNLY + ITGAE + REN + VEGFA | 0,74 |
| IFNG + GNLY + CCL5 + IL17A + PROM1 | 0,74 |
| GZMH + IFNG + GNLY + TSLP + REN | 0,74 |
| IFNG + GNLY + ITGAE + PF4 + IHH | 0,74 |
| CXCL13 + GNLY + PF4 + IL17A + VEGFA | 0,74 |
| GNLY + CCL5 + IL17A + REN + VEGFA | 0,74 |
| IFNG + GNLY + CXCL9 + PF4 + TSLP | 0,74 |
| GZMH + IFNG + GNLY + IL17A + VEGFA | 0,74 |
| IFNG + GNLY + ITGAE + PF4 + TSLP | 0,74 |
| IFNG + GNLY + LAG3 + ITGAE + REN | 0,74 |
| IFNG + GNLY + IL17A + PROM1 + VEGFA | 0,74 |
| GZMH + IFNG + GNLY + IL17A + PROM1 | 0,74 |
| IFNG + GNLY + CCL5 + CXCL9 + REN | 0,74 |
| GZMH + CXCL13 + GNLY + CXCL9 + IL17A | 0,74 |
| IFNG + GNLY + ITGAE + CXCL9 + REN | 0,74 |
| IFNG + GNLY + LAG3 + CCL5 + REN | 0,74 |
| IFNG + GNLY + CCL5 + IL17A + IHH | 0,74 |
| IFNG + GNLY + IL17A + IHH + VEGFA | 0,74 |
| CXCL13 + CXCL9 + PF4 + IL17A + REN | 0,74 |
| GZMH + IFNG + GNLY + IL17A + IHH | 0,74 |
| CXCL13 + GNLY + PF4 + IL17A + PROM1 | 0,74 |
| CXCL9 + PF4 + IL17A + REN + VEGFA | 0,74 |
| IFNG + GNLY + LAG3 + REN + PROM1 | 0,74 |
| IFNG + GNLY + CCL5 + CXCL9 + PF4 | 0,74 |
| IFNG + GNLY + REN + PROM1 + VEGFA | 0,74 |
| GZMH + CXCL13 + GNLY + LAG3 + IL17A | 0,74 |
| IFNG + GNLY + IL17A + IHH + PROM1 | 0,74 |
| GZMH + CXCL13 + GNLY + IL17A + VEGFA | 0,74 |
| IFNG + GNLY + TSLP + REN + IHH | 0,74 |
| GZMH + CXCL13 + GNLY + ITGAE + IL17A | 0,74 |
| GZMH + CXCL13 + GNLY + IL17A + PROM1 | 0,74 |
| CXCL13 + LAG3 + PF4 + IL17A + REN | 0,74 |
| IFNG + GNLY + CXCL9 + REN + PROM1 | 0,74 |
| IFNG + GNLY + CCL5 + REN + PROM1 | 0,74 |
| IFNG + GNLY + PF4 + IHH + VEGFA | 0,74 |
| CXCL13 + PF4 + IL17A + REN + IHH | 0,74 |
| GNLY + ITGAE + CCL5 + CXCL9 + IL17A | 0,74 |
| GNLY + ITGAE + CCL5 + TSLP + REN | 0,74 |
| GNLY + ITGAE + CCL5 + PF4 + IL17A | 0,74 |
| CXCL13 + PF4 + IL17A + REN + PROM1 | 0,74 |
| IFNG + GNLY + ITGAE + REN + IHH | 0,74 |
| CXCL13 + PF4 + IL17A + TSLP + REN | 0,74 |
| IFNG + GNLY + CXCL9 + PF4 + IHH | 0,74 |
| IFNG + GNLY + ITGAE + CCL5 + PF4 | 0,74 |
| GZMH + CXCL13 + PF4 + IL17A + REN | 0,74 |
| CXCL13 + ITGAE + PF4 + IL17A + REN | 0,74 |
| CXCL13 + PF4 + IL17A + REN + VEGFA | 0,74 |
| IFNG + GNLY + CCL5 + PF4 + IHH | 0,74 |
| IFNG + CXCL13 + GNLY + PF4 + TSLP | 0,74 |
| GZMH + CXCL13 + GNLY + IL17A + IHH | 0,74 |
| GNLY + LAG3 + CCL5 + REN + PROM1 | 0,74 |
| CXCL13 + GNLY + LAG3 + IL17A + TSLP | 0,74 |
| CXCL13 + GNLY + CCL5 + PF4 + TSLP | 0,74 |
| GNLY + CCL5 + CXCL9 + IL17A + VEGFA | 0,74 |
| IFNG + GNLY + PF4 + IHH + PROM1 | 0,74 |
| GZMH + IFNG + GNLY + REN + PROM1 | 0,74 |
| GZMH + CXCL9 + PF4 + IL17A + REN | 0,74 |
| IFNG + GNLY + CCL5 + CXCL9 + TSLP | 0,74 |
| GZMH + IFNG + GNLY + ITGAE + TSLP | 0,74 |
| LAG3 + PF4 + IL17A + REN + VEGFA | 0,74 |
| IFNG + GNLY + CCL5 + REN + VEGFA | 0,74 |
| GZMH + IFNG + GNLY + PF4 + IHH | 0,74 |
| IFNG + GNLY + CXCL9 + REN + VEGFA | 0,74 |
| GZMH + IFNG + GNLY + REN + VEGFA | 0,74 |
| CCL5 + CXCL9 + PF4 + IL17A + REN | 0,74 |
| CXCL13 + CCL5 + PF4 + IL17A + REN | 0,74 |
| IFNG + CXCL13 + PF4 + IL17A + REN | 0,74 |
| IFNG + GNLY + LAG3 + PF4 + IHH | 0,74 |
| LAG3 + CCL5 + CXCL9 + PF4 + REN | 0,74 |
| IFNG + GNLY + LAG3 + REN + VEGFA | 0,73 |
| GZMH + CXCL9 + PF4 + TSLP + REN | 0,73 |
| CXCL13 + GNLY + CXCL9 + IL17A + TSLP | 0,73 |
| GNLY + CCL5 + CXCL9 + IL17A + TSLP | 0,73 |
| IFNG + GNLY + ITGAE + TSLP + PROM1 | 0,73 |
| ITGAE + CXCL9 + PF4 + REN + VEGFA | 0,73 |
| CXCL9 + PF4 + IL17A + REN + IHH | 0,73 |
| IFNG + GNLY + REN + IHH + PROM1 | 0,73 |
| GNLY + LAG3 + CCL5 + CXCL9 + IL17A | 0,73 |
| GZMH + IFNG + GNLY + CCL5 + PF4 | 0,73 |
| IFNG + GNLY + ITGAE + TSLP + VEGFA | 0,73 |
| ITGAE + CXCL9 + PF4 + IL17A + REN | 0,73 |
| IFNG + GNLY + CCL5 + PF4 + PROM1 | 0,73 |
| GZMH + IFNG + GNLY + LAG3 + REN | 0,73 |
| IFNG + CXCL9 + PF4 + TSLP + REN | 0,73 |
| LAG3 + CXCL9 + PF4 + IL17A + REN | 0,73 |
| GZMH + ITGAE + CXCL9 + PF4 + REN | 0,73 |
| IFNG + GNLY + LAG3 + CXCL9 + PF4 | 0,73 |
| IFNG + CXCL9 + PF4 + IL17A + REN | 0,73 |
| CXCL13 + GNLY + ITGAE + IL17A + TSLP | 0,73 |
| CXCL13 + GNLY + IL17A + TSLP + PROM1 | 0,73 |
| GNLY + CCL5 + PF4 + IL17A + TSLP | 0,73 |
| IFNG + GNLY + LAG3 + CXCL9 + REN | 0,73 |
| CXCL9 + PF4 + IL17A + REN + PROM1 | 0,73 |
| GZMH + IFNG + GNLY + CCL5 + REN | 0,73 |
| CXCL9 + PF4 + IL17A + TSLP + REN | 0,73 |
| IFNG + GNLY + ITGAE + CXCL9 + TSLP | 0,73 |
| IFNG + GNLY + LAG3 + ITGAE + TSLP | 0,73 |
| IFNG + GNLY + ITGAE + TSLP + IHH | 0,73 |
| CCL5 + CXCL9 + PF4 + REN + VEGFA | 0,73 |
| CXCL13 + GNLY + CCL5 + PF4 + PROM1 | 0,73 |
| CXCL9 + PF4 + TSLP + REN + VEGFA | 0,73 |
| CCL5 + CXCL9 + PF4 + TSLP + REN | 0,73 |
| CXCL13 + GNLY + IL17A + TSLP + VEGFA | 0,73 |
| GZMH + IFNG + GNLY + CXCL9 + REN | 0,73 |
| GZMH + CXCL9 + PF4 + REN + VEGFA | 0,73 |
| GNLY + CCL5 + CXCL9 + PF4 + TSLP | 0,73 |
| GNLY + ITGAE + CCL5 + IL17A + TSLP | 0,73 |
| CXCL13 + GNLY + LAG3 + PF4 + TSLP | 0,73 |
| CCL5 + CXCL9 + PF4 + REN + PROM1 | 0,73 |
| IFNG + GNLY + ITGAE + CCL5 + CXCL9 | 0,73 |
| CXCL9 + PF4 + REN + PROM1 + VEGFA | 0,73 |
| IFNG + GNLY + LAG3 + CCL5 + TSLP | 0,73 |
| GNLY + ITGAE + CCL5 + REN + PROM1 | 0,73 |
| IFNG + CXCL9 + PF4 + REN + VEGFA | 0,73 |
| GZMH + GNLY + CCL5 + CXCL9 + IL17A | 0,73 |
| ITGAE + PF4 + IL17A + REN + VEGFA | 0,73 |
| GNLY + CCL5 + PF4 + IL17A + IHH | 0,73 |
| LAG3 + CXCL9 + PF4 + REN + VEGFA | 0,73 |
| IFNG + GNLY + REN + IHH + VEGFA | 0,73 |
| GZMH + LAG3 + CXCL9 + PF4 + REN | 0,73 |
| CXCL13 + GNLY + ITGAE + CXCL9 + IL17A | 0,73 |
| GZMH + CXCL9 + PF4 + REN + PROM1 | 0,73 |
| CXCL13 + GNLY + LAG3 + CXCL9 + IL17A | 0,73 |
| GZMH + GNLY + CXCL9 + PF4 + IL17A | 0,73 |
| CXCL13 + GNLY + CXCL9 + IL17A + VEGFA | 0,73 |
| GNLY + CCL5 + CXCL9 + IL17A + PROM1 | 0,73 |
| CXCL9 + PF4 + REN + IHH + VEGFA | 0,73 |
| GNLY + CCL5 + CXCL9 + TSLP + REN | 0,73 |
| LAG3 + PF4 + IL17A + REN + PROM1 | 0,73 |
| CXCL13 + GNLY + LAG3 + IL17A + VEGFA | 0,73 |
| IFNG + GNLY + CXCL9 + TSLP + VEGFA | 0,73 |
| IFNG + GNLY + CCL5 + PF4 + VEGFA | 0,73 |
| CXCL13 + GNLY + LAG3 + ITGAE + IL17A | 0,73 |
| IFNG + GNLY + CXCL9 + TSLP + PROM1 | 0,73 |
| IFNG + GNLY + LAG3 + PF4 + PROM1 | 0,73 |
| CXCL13 + GNLY + LAG3 + IL17A + PROM1 | 0,73 |
| IFNG + GNLY + LAG3 + ITGAE + PF4 | 0,73 |
| GZMH + IFNG + GNLY + CXCL9 + TSLP | 0,73 |
| GZMH + CXCL13 + GNLY + PF4 + TSLP | 0,73 |
| IFNG + GNLY + CCL5 + REN + IHH | 0,73 |
| IFNG + GNLY + LAG3 + CXCL9 + TSLP | 0,73 |
| CXCL9 + PF4 + TSLP + REN + PROM1 | 0,73 |
| IFNG + GNLY + CXCL9 + REN + IHH | 0,73 |
| CXCL13 + GNLY + CXCL9 + IL17A + PROM1 | 0,73 |
| IFNG + GNLY + PF4 + PROM1 + VEGFA | 0,73 |
| GNLY + CCL5 + PF4 + IL17A + PROM1 | 0,73 |
| IFNG + CXCL9 + PF4 + REN + PROM1 | 0,73 |
| IFNG + GNLY + ITGAE + PF4 + PROM1 | 0,73 |
| IFNG + CXCL13 + GNLY + ITGAE + REN | 0,73 |
| CXCL13 + GNLY + ITGAE + CCL5 + PF4 | 0,73 |
| LAG3 + PF4 + REN + PROM1 + VEGFA | 0,73 |
| CXCL13 + GNLY + CCL5 + PF4 + VEGFA | 0,73 |
| LAG3 + CXCL9 + PF4 + REN + PROM1 | 0,73 |
| LAG3 + CCL5 + PF4 + IL17A + REN | 0,73 |
| IFNG + ITGAE + CXCL9 + PF4 + REN | 0,73 |
| GZMH + IFNG + GNLY + PF4 + PROM1 | 0,73 |
| GZMH + GNLY + PF4 + IL17A + IHH | 0,73 |
| CXCL13 + GNLY + ITGAE + IL 17A + PROM1 | 0,73 |
| ITGAE + CCL5 + CXCL9 + PF4 + REN | 0,73 |
| IFNG + GNLY + TSLP + PROM1 + VEGFA | 0,73 |
| GZMH + GNLY + PF4 + IL17A + TSLP | 0,73 |
| GZMH + CXCL13 + GNLY + CCL5 + PF4 | 0,73 |
| CXCL13 + GNLY + ITGAE + IL17A + VEGFA | 0,73 |
| CXCL9 + PF4 + REN + IHH + PROM1 | 0,73 |
| IFNG + CCL5 + CXCL9 + PF4 + REN | 0,73 |
| GZMH + GNLY + ITGAE + TSLP + REN | 0,73 |
| IFNG + GNLY + CXCL9 + PF4 + PROM1 | 0,73 |
| GZMH + IFNG + CXCL9 + PF4 + REN | 0,73 |
| CXCL13 + GNLY + CCL5 + CXCL9 + PF4 | 0,73 |
| IFNG + GNLY + LAG3 + TSLP + PROM1 | 0,73 |
| IFNG + CXCL13 + GNLY + CXCL9 + REN | 0,73 |
| IFNG + GNLY + LAG3 + REN + IHH | 0,73 |
| CXCL13 + CXCL9 + PF4 + TSLP + REN | 0,73 |
| LAG3 + CCL5 + PF4 + REN + PROM1 | 0,73 |
| CXCL13 + GNLY + PF4 + TSLP + PROM1 | 0,73 |
| IFNG + GNLY + ITGAE + CCL5 + IHH | 0,73 |
| CXCL13 + CCL5 + CXCL9 + PF4 + IL17A | 0,73 |
| GNLY + CCL5 + CXCL9 + PF4 + PROM1 | 0,73 |
| CXCL9 + PF4 + TSLP + REN + IHH | 0,73 |
| IFNG + CXCL13 + GNLY + PF4 + IHH | 0,73 |
| IFNG + GNLY + LAG3 + PF4 + VEGFA | 0,73 |
| GZMH + IFNG + GNLY + REN + IHH | 0,73 |
| LAG3 + ITGAE + PF4 + REN + VEGFA | 0,73 |
| IFNG + CXCL13 + GNLY + TSLP + REN | 0,73 |
| GZMH + IFNG + GNLY + TSLP + VEGFA | 0,73 |
| GZMH + GNLY + CXCL9 + TSLP + REN | 0,73 |
| CXCL13 + GNLY + CCL5 + PF4 + IHH | 0,73 |
| LAG3 + ITGAE + PF4 + IL17A + REN | 0,73 |
| ITGAE + CXCL9 + PF4 + REN + PROM1 | 0,73 |
| GNLY + ITGAE + CCL5 + PF4 + TSLP | 0,73 |
| GZMH + GNLY + CCL5 + PF4 + IL17A | 0,73 |
| CXCL13 + CXCL9 + PF4 + REN + PROM1 | 0,73 |
| GNLY + LAG3 + CCL5 + PF4 + IL17A | 0,73 |
| IFNG + GNLY + CXCL9 + PF4 + VEGFA | 0,73 |
| GNLY + CCL5 + CXCL9 + PF4 + IHH | 0,73 |
| IFNG + LAG3 + CXCL9 + PF4 + REN | 0,73 |
| IFNG + GNLY + ITGAE + CXCL9 + PF4 | 0,73 |
| IFNG + GNLY + CCL5 + TSLP + PROM1 | 0,73 |
| ITGAE + CXCL9 + PF4 + TSLP + REN | 0,73 |
| GNLY + CCL5 + PF4 + IL17A + VEGFA | 0,73 |
| GZMH + CCL5 + CXCL9 + PF4 + REN | 0,73 |
| CXCL13 + LAG3 + PF4 + REN + PROM1 | 0,73 |
| IFNG + CXCL13 + GNLY + CCL5 + PF4 | 0,73 |
| GZMH + CXCL13 + CXCL9 + PF4 + REN | 0,73 |
| CXCL13 + GNLY + CXCL9 + PF4 + TSLP | 0,73 |
| GZMH + GNLY + CXCL9 + IL17A + TSLP | 0,73 |
| IFNG + GNLY + LAG3 + TSLP + VEGFA | 0,73 |
| IFNG + GNLY + CCL5 + TSLP + VEGFA | 0,73 |
| IFNG + CXCL13 + GNLY + LAG3 + PF4 | 0,73 |
| GNLY + CCL5 + CXCL9 + REN + PROM1 | 0,73 |
| CXCL13 + CXCL9 + PF4 + REN + VEGFA | 0,73 |
| GZMH + IFNG + GNLY + PF4 + VEGFA | 0,73 |
| GNLY + ITGAE + CCL5 + IL17A + PROM1 | 0,73 |
| CXCL13 + GNLY + ITGAE + PF4 + TSLP | 0,73 |
| GZMH + IFNG + GNLY + LAG3 + TSLP | 0,73 |
| ITGAE + CXCL9 + PF4 + REN + IHH | 0,73 |
| LAG3 + PF4 + IL17A + REN + IHH | 0,73 |
| IFNG + GNLY + CXCL9 + TSLP + IHH | 0,73 |
| IFNG + GNLY + LAG3 + CCL5 + PF4 | 0,73 |
| GZMH + CXCL9 + PF4 + REN + IHH | 0,73 |
| GZMH + IFNG + GNLY + CXCL9 + PF4 | 0,73 |
| CXCL13 + GNLY + IL17A + PROM1 + VEGFA | 0,73 |
| GZMH + IFNG + GNLY + LAG3 + PF4 | 0,73 |
| IFNG + CXCL9 + PF4 + REN + IHH | 0,73 |
| GZMH + IFNG + GNLY + TSLP + PROM1 | 0,73 |
| GZMH + LAG3 + PF4 + IL17A + REN | 0,73 |
| CXCL13 + GNLY + LAG3 + IL17A + IHH | 0,73 |
| GNLY + PF4 + IL17A + TSLP + PROM1 | 0,73 |
| CXCL13 + GNLY + PF4 + TSLP + VEGFA | 0,73 |
| GNLY + PF4 + IL17A + TSLP + VEGFA | 0,73 |
| GNLY + CCL5 + PF4 + TSLP + PROM1 | 0,73 |
| CCL5 + CXCL9 + PF4 + REN + IHH | 0,73 |
| GZMH + GNLY + CXCL9 + IL17A + VEGFA | 0,73 |
| CXCL13 + ITGAE + PF4 + REN + PROM1 | 0,73 |
| GNLY + ITGAE + CCL5 + PF4 + IHH | 0,73 |
| GZMH + GNLY + ITGAE + PF4 + IL17A | 0,73 |
| CXCL13 + CCL5 + CXCL9 + PF4 + REN | 0,73 |
| GNLY + PF4 + IL17A + TSLP + IHH | 0,73 |
| GNLY + CCL5 + CXCL9 + IL17A + IHH | 0,73 |
| CXCL13 + GNLY + LAG3 + CCL5 + PF4 | 0,73 |
| GNLY + LAG3 + ITGAE + CCL5 + REN | 0,73 |
| LAG3 + CXCL9 + PF4 + TSLP + REN | 0,73 |
| GNLY + LAG3 + CCL5 + CXCL9 + REN | 0,73 |
| IFNG + GNLY + ITGAE + PF4 + VEGFA | 0,73 |
| GZMH + GNLY + PF4 + IL17A + VEGFA | 0,73 |
| IFNG + LAG3 + PF4 + IL17A + REN | 0,73 |
| CXCL13 + GNLY + LAG3 + PF4 + PROM1 | 0,73 |
| GNLY + LAG3 + CCL5 + TSLP + REN | 0,73 |
| LAG3 + ITGAE + CXCL9 + PF4 + REN | 0,73 |
| GNLY + CXCL9 + PF4 + IL17A + TSLP | 0,73 |
| GNLY + ITGAE + PF4 + IL17A + IHH | 0,73 |
| GZMH + IFNG + GNLY + CCL5 + TSLP | 0,73 |
| CXCL13 + LAG3 + PF4 + TSLP + REN | 0,73 |
| GZMH + IFNG + GNLY + ITGAE + PF4 | 0,73 |
| GNLY + LAG3 + CCL5 + IL17A + TSLP | 0,73 |
| ITGAE + PF4 + REN + PROM1 + VEGFA | 0,73 |
| GZMH + GNLY + CXCL9 + REN + PROM1 | 0,73 |
| GNLY + CXCL9 + PF4 + IL17A + IHH | 0,73 |
| CXCL13 + LAG3 + CXCL9 + PF4 + REN | 0,73 |
| GNLY + PF4 + IL17A + IHH + PROM1 | 0,73 |
| GZMH + GNLY + LAG3 + PF4 + IL17A | 0,73 |
| LAG3 + ITGAE + PF4 + REN + PROM1 | 0,73 |
| CXCL13 + ITGAE + PF4 + TSLP + REN | 0,73 |
| LAG3 + PF4 + IL17A + TSLP + REN | 0,73 |
| CXCL13 + GNLY + CXCL9 + IL17A + IHH | 0,73 |
| GNLY + LAG3 + ITGAE + CCL5 + IL17A | 0,73 |
| CXCL13 + ITGAE + PF4 + REN + VEGFA | 0,73 |
| CXCL13 + GNLY + IL17A + TSLP + IHH | 0,73 |
| CXCL13 + ITGAE + CXCL9 + PF4 + REN | 0,73 |
| GZMH + LAG3 + PF4 + REN + PROM1 | 0,73 |
| IFNG + CXCL13 + GNLY + REN + PROM1 | 0,73 |
| ITGAE + PF4 + TSLP + REN + VEGFA | 0,73 |
| GNLY + ITGAE + PF4 + IL17A + TSLP | 0,73 |
| GNLY + LAG3 + PF4 + IL17A + IHH | 0,73 |
| GZMH + GNLY + ITGAE + REN + PROM1 | 0,73 |
| GNLY + PF4 + IL17A + IHH + VEGFA | 0,73 |
| IFNG + GNLY + LAG3 + TSLP + IHH | 0,73 |
| GZMH + GNLY + PF4 + IL17A + PROM 1 | 0,73 |
| IFNG + GNLY + LAG3 + ITGAE + CCL5 | 0,73 |
| GNLY + LAG3 + PF4 + IL17A + TSLP | 0,73 |
| CXCL13 + GNLY + LAG3 + PF4 + VEGFA | 0,73 |
| GZMH + GNLY + CXCL9 + PF4 + TSLP | 0,73 |
| CXCL13 + GNLY + PF4 + TSLP + IHH | 0,73 |
| IFNG + LAG3 + PF4 + REN + PROM1 | 0,73 |
| LAG3 + CXCL9 + PF4 + REN + IHH | 0,73 |
| GNLY + CXCL9 + PF4 + IL17A + VEGFA | 0,73 |
| CXCL13 + LAG3 + PF4 + REN + VEGFA | 0,73 |
| GNLY + LAG3 + CXCL9 + PF4 + IL17A | 0,73 |
| GNLY + ITGAE + CCL5 + IL17A + VEGFA | 0,73 |
| LAG3 + PF4 + REN + IHH + PROM1 | 0,73 |
| GZMH + CXCL13 + ITGAE + PF4 + REN | 0,73 |
| CXCL13 + GNLY + ITGAE + IL17A + IHH | 0,73 |
| CXCL13 + PF4 + TSLP + REN + PROM1 | 0,73 |
| CXCL13 + LAG3 + ITGAE + PF4 + REN | 0,73 |
| IFNG + CXCL13 + GNLY + PF4 + PROM1 | 0,73 |
| LAG3 + PF4 + TSLP + REN + VEGFA | 0,73 |
| IFNG + GNLY + ITGAE + CCL5 + PROM1 | 0,73 |
| GZMH + CXCL13 + LAG3 + PF4 + REN | 0,73 |
| IFNG + GNLY + TSLP + IHH + VEGFA | 0,73 |
| GZMH + LAG3 + ITGAE + PF4 + REN | 0,73 |

| **Table 6: clusters of 6 genes:** | |
|---|---|
| **clusters of 6 genes** | **c_tau level** |
| IFNG + GNLY + ITGAE + PF4 + IL17A + REN | 0,78 |
| IFNG + GNLY + PF4 + IL17A + REN + PROM1 | 0,78 |
| IFNG + GNLY + CCL5 + PF4 + IL17A + REN | 0,78 |
| IFNG + GNLY + PF4 + IL17A + REN + IHH | 0,78 |
| IFNG + GNLY + PF4 + IL17A + TSLP + REN | 0,78 |
| IFNG + GNLY + PF4 + IL17A + REN + VEGFA | 0,78 |
| IFNG + GNLY + LAG3 + PF4 + IL17A + REN | 0,78 |
| GZMH + IFNG + GNLY + PF4 + IL17A + REN | 0,78 |
| IFNG + GNLY + ITGAE + CCL5 + IL17A + REN | 0,78 |
| IFNG + GNLY + CXCL9 + PF4 + IL17A + REN | 0,78 |
| GNLY + ITGAE + CCL5 + PF4 + IL17A + REN | 0,78 |
| CXCL13 + GNLY + CCL5 + PF4 + IL17A + REN | 0,78 |
| IFNG + CXCL13 + GNLY + PF4 + IL17A + REN | 0,78 |
| GNLY + CCL5 + CXCL9 + PF4 + IL17A + REN | 0,78 |
| GNLY + LAG3 + CCL5 + PF4 + IL17A + REN | 0,78 |
| GZMH + GNLY + ITGAE + PF4 + IL17A + REN | 0,77 |
| GZMH + CXCL13 + GNLY + PF4 + IL17A + REN | 0,77 |
| CXCL13 + GNLY + LAG3 + PF4 + IL17A + REN | 0,77 |
| GNLY + CCL5 + PF4 + IL17A + REN + IHH | 0,77 |
| GNLY + CCL5 + PF4 + IL17A + REN + PROM1 | 0,77 |
| GZMH + GNLY + CXCL9 + PF4 + IL17A + REN | 0,77 |
| GNLY + PF4 + IL17A + REN + IHH + PROM1 | 0,77 |
| IFNG + GNLY + ITGAE + CCL5 + PF4 + REN | 0,77 |
| CXCL13 + GNLY + PF4+IL17A+REN + IHH | 0,77 |
| CXCL13 + GNLY + CXCL9 + PF4 + IL17A + REN | 0,77 |
| GNLY + CCL5 + PF4 + IL17A + TSLP + REN | 0,77 |
| GNLY + CXCL9 + PF4 + IL17A + REN + IHH | 0,77 |
| GNLY + PF4 + IL17A + TSLP + REN + IHH | 0,77 |
| GZMH + GNLY + PF4 + IL17A + REN + IHH | 0,77 |
| IFNG + GNLY + ITGAE + IL17A + TSLP + REN | 0,77 |
| GZMH + GNLY + PF4 + IL17A + TSLP + REN | 0,77 |
| GNLY + ITGAE + CCL5 + PF4 + REN + PROM1 | 0,77 |
| IFNG + GNLY + ITGAE + CXCL9 + IL17A + REN | 0,77 |
| GNLY + ITGAE + PF4 + IL17A + REN + IHH | 0,77 |
| GZMH + GNLY + CCL5 + PF4 + IL17A + REN | 0,77 |
| IFNG + GNLY + CCL5 + PF4 + REN + PROM1 | 0,77 |
| GNLY + CCL5 + PF4 + IL17A + REN + VEGFA | 0,77 |
| CXCL13 + GNLY + PF4 + IL17A + REN + PROM1 | 0,77 |
| CXCL13 + GNLY + PF4 + IL17A + TSLP + REN | 0,77 |
| GZMH + GNLY + PF4 + IL17A + REN + PROM1 | 0,77 |
| GNLY + PF4 + IL17A + TSLP + REN + PROM1 | 0,77 |
| GNLY + ITGAE + PF4 + IL17A + REN + PROM1 | 0,77 |
| GNLY + LAG3 + CXCL9 + PF4 + IL17A + REN | 0,77 |
| CXCL13 + GNLY + ITGAE + PF4 + IL17A + REN | 0,77 |
| GZMH + IFNG + GNLY + ITGAE + IL17A + REN | 0,77 |
| GNLY + ITGAE + PF4 + IL17A + TSLP + REN | 0,77 |
| CXCL13 + GNLY + PF4 + IL17A + REN + VEGFA | 0,77 |
| GNLY + CXCL9 + PF4 + IL17A + REN + PROM1 | 0,77 |
| GNLY + ITGAE + PF4 + IL17A + REN + VEGFA | 0,77 |
| GNLY + PF4 + IL17A + REN + PROM1 + VEGFA | 0,77 |
| GNLY + PF4 + IL17A + REN + IHH + VEGFA | 0,77 |
| GZMH + GNLY + LAG3 + PF4 + IL17A + REN | 0,77 |
| GNLY + LAG3 + PF4 + IL17A + REN + IHH | 0,77 |
| IFNG + GNLY + ITGAE + IL17A + REN + PROM1 | 0,77 |
| IFNG + GNLY + LAG3 + CCL5 + IL17A + REN | 0,77 |
| GNLY + CXCL9 + PF4 + IL17A + TSLP + REN | 0,77 |
| GNLY + CXCL9 + PF4 + IL17A + REN + VEGFA | 0,77 |
| GNLY + LAG3 + PF4 + IL17A + REN + PROM1 | 0,77 |
| GZMH + GNLY + PF4 + IL17A + REN + VEGFA | 0,77 |
| IFNG + GNLY + PF4 + TSLP + REN + PROM1 | 0,77 |
| IFNG + GNLY + ITGAE + IL17A + REN + VEGFA | 0,77 |
| GNLY + LAG3 + ITGAE + PF4 + IL17A + REN | 0,77 |
| GNLY + ITGAE + CXCL9 + PF4 + IL17A + REN | 0,77 |
| IFNG + GNLY + ITGAE + IL17A + REN + IHH | 0,77 |
| GNLY + PF4 + IL17A + TSLP + REN + VEGFA | 0,77 |
| IFNG + GNLY + LAG3 + ITGAE + IL17A + REN | 0,77 |
| GNLY + ITGAE + CCL5 + PF4 + TSLP + REN | 0,77 |
| GZMH + IFNG + GNLY + CCL5 + PF4 + REN | 0,77 |
| IFNG + GNLY + CCL5 + CXCL9 + IL17A + REN | 0,77 |
| GNLY + LAG3 + PF4 + IL17A + TSLP + REN | 0,77 |
| CXCL13 + GNLY + ITGAE + CCL5 + IL17A + REN | 0,77 |
| GZMH + IFNG + GNLY + PF4 + REN + PROM1 | 0,77 |
| IFNG + GNLY + CCL5 + PF4 + TSLP + REN | 0,77 |
| IFNG + GNLY + ITGAE + PF4 + REN + PROM1 | 0,77 |
| IFNG + GNLY + LAG3 + PF4 + REN + PROM1 | 0,77 |
| IFNG + GNLY + PF4 + REN + PROM1 + VEGFA | 0,77 |
| IFNG + GNLY + PF4 + REN + IHH + PROM1 | 0,77 |
| GNLY + LAG3 + PF4 + IL17A + REN + VEGFA | 0,77 |
| IFNG + GNLY + CXCL9 + IL17A + REN + IHH | 0,77 |
| IFNG + GNLY + PF4 + TSLP + REN + VEGFA | 0,77 |
| GNLY + CCL5 + CXCL9 + PF4 + REN + PROM1 | 0,77 |
| IFNG + GNLY + ITGAE + PF4 + TSLP + REN | 0,77 |
| GZMH + IFNG + GNLY + PF4 + TSLP + REN | 0,77 |
| GZMH + IFNG + GNLY + CXCL9 + IL17A + REN | 0,77 |
| IFNG + GNLY + CCL5 + CXCL9 + PF4 + REN | 0,77 |
| IFNG + CXCL13 + GNLY + ITGAE + IL17A + REN | 0,77 |
| IFNG + GNLY + PF4 + TSLP + REN + IHH | 0,77 |
| GNLY + LAG3 + CCL5 + PF4 + REN + PROM1 | 0,77 |
| IFNG + GNLY + LAG3 + CCL5 + PF4 + REN | 0,77 |
| IFNG + GNLY + LAG3 + PF4 + TSLP + REN | 0,77 |
| IFNG + GNLY + CXCL9 + IL17A + REN + VEGFA | 0,77 |
| GNLY + ITGAE + CCL5 + CXCL9 + PF4 + REN | 0,77 |
| IFNG + GNLY + CCL5 + PF4 + REN + VEGFA | 0,77 |
| IFNG + GNLY + CXCL9 + PF4 + REN + PROM1 | 0,77 |
| IFNG + GNLY + LAG3 + CXCL9 + IL17A + REN | 0,77 |
| IFNG + GNLY + LAG3 + IL17A + TSLP + REN | 0,77 |
| IFNG + GNLY + CCL5 + PF4 + REN + IHH | 0,77 |
| GNLY + ITGAE + CCL5 + CXCL9 + IL17A + REN | 0,77 |
| IFNG + GNLY + LAG3 + IL17A + REN + PROM1 | 0,77 |
| GZMH + IFNG + GNLY + LAG3 + IL17A + REN | 0,77 |
| GNLY + CCL5 + PF4 + TSLP + REN + PROM1 | 0,77 |
| IFNG + GNLY + LAG3 + IL17A + REN + VEGFA | 0,77 |
| IFNG + GNLY + LAG3 + ITGAE + PF4 + REN | 0,77 |
| IFNG + GNLY + LAG3 + IL17A + REN + IHH | 0,77 |
| IFNG + GNLY + CXCL9 + IL17A + REN + PROM1 | 0,77 |
| IFNG + GNLY + IL17A + TSLP + REN + IHH | 0,77 |
| GZMH + IFNG + GNLY + PF4 + REN + VEGFA | 0,77 |
| GNLY + CCL5 + CXCL9 + PF4 + TSLP + REN | 0,77 |
| IFNG + GNLY + CXCL9 + IL17A + TSLP + REN | 0,77 |
| GNLY + CCL5 + PF4 + REN + IHH + PROM1 | 0,77 |
| IFNG + GNLY + PF4 + REN + IHH + VEGFA | 0,77 |
| IFNG + GNLY + LAG3 + PF4 + REN + VEGFA | 0,77 |
| GZMH + IFNG + GNLY + ITGAE + PF4 + REN | 0,76 |
| GZMH + GNLY + ITGAE + CCL5 + PF4 + REN | 0,76 |
| GZMH + IFNG + GNLY + LAG3 + PF4 + REN | 0,76 |
| GZMH + IFNG + GNLY + PF4 + REN + IHH | 0,76 |
| IFNG + GNLY + LAG3 + CXCL9 + PF4 + REN | 0,76 |
| GNLY + LAG3 + ITGAE + CCL5 + IL17A + REN | 0,76 |
| IFNG + GNLY + ITGAE + PF4 + REN + VEGFA | 0,76 |
| IFNG + GNLY + ITGAE + PF4 + REN + IHH | 0,76 |
| CXCL13 + GNLY + ITGAE + CCL5 + PF4 + IL17A | 0,76 |
| IFNG + GNLY + CXCL9 + PF4 + TSLP + REN | 0,76 |
| GNLY + ITGAE + CCL5 + PF4 + REN + IHH | 0,76 |
| IFNG + CXCL13 + GNLY + CCL5 + IL17A + REN | 0,76 |
| CXCL13 + GNLY + CCL5 + IL17A + REN + IHH | 0,76 |
| IFNG + GNLY + LAG3 + PF4 + REN + IHH | 0,76 |
| IFNG + GNLY + CCL5 + IL17A + REN + IHH | 0,76 |
| IFNG + CXCL13 + GNLY + CXCL9 + IL17A + REN | 0,76 |
| GNLY + CCL5 + PF4 + REN + PROM1 + VEGFA | 0,76 |
| IFNG + CXCL13 + GNLY + IL17A + REN + VEGFA | 0,76 |
| IFNG + CXCL13 + GNLY + IL17A + REN + IHH | 0,76 |
| IFNG + CXCL13 + GNLY + IL17A + REN + PROM1 | 0,76 |
| GNLY + ITGAE + CCL5 + PF4 + REN + VEGFA | 0,76 |
| GZMH + IFNG + GNLY + IL17A + REN + IHH | 0,76 |
| GZMH + IFNG + CXCL13 + GNLY + IL17A + REN | 0,76 |
| GZMH + GNLY + CCL5 + PF4 + REN + PROM1 | 0,76 |
| IFNG + GNLY + ITGAE + CXCL9 + PF4 + REN | 0,76 |
| IFNG + GNLY + IL17A + REN + IHH + VEGFA | 0,76 |
| GNLY + LAG3 + ITGAE + CCL5 + PF4 + REN | 0,76 |
| IFNG + GNLY + IL17A + REN + IHH + PROM1 | 0,76 |
| IFNG + CXCL13 + GNLY + IL17A + TSLP + REN | 0,76 |
| IFNG + CXCL13 + GNLY + LAG3 + IL17A + REN | 0,76 |
| GZMH + IFNG + GNLY + IL17A + TSLP + REN | 0,76 |
| IFNG + GNLY + IL17A + TSLP + REN + VEGFA | 0,76 |
| IFNG + GNLY + IL17A + REN + PROM1 + VEGFA | 0,76 |
| IFNG + GNLY + CXCL9 + PF4 + REN + VEGFA | 0,76 |
| GNLY + LAG3 + CCL5 + CXCL9 + PF4 + REN | 0,76 |
| IFNG + CXCL13 + GNLY + ITGAE + CCL5 + IL17A | 0,76 |
| IFNG + GNLY + CXCL9 + PF4 + REN + IHH | 0,76 |
| GZMH + IFNG + GNLY + CCL5 + IL17A + REN | 0,76 |
| GNLY + CCL5 + CXCL9 + PF4 + REN + IHH | 0,76 |
| CXCL13 + GNLY + LAG3 + CCL5 + IL17A + REN | 0,76 |
| IFNG + GNLY + IL17A + TSLP + REN + PROM1 | 0,76 |
| GZMH + IFNG + GNLY + CXCL9 + PF4 + REN | 0,76 |
| GZMH + IFNG + GNLY + IL17A + REN + PROM1 | 0,76 |
| GZMH + GNLY + PF4 + TSLP + REN + PROM1 | 0,76 |
| IFNG + GNLY + CCL5 + IL17A + REN + VEGFA | 0,76 |
| GZMH + GNLY + ITGAE + PF4 + TSLP + REN | 0,76 |
| GNLY + CCL5 + CXCL9 + PF4 + REN + VEGFA | 0,76 |
| GZMH + CXCL13 + GNLY + CCL5 + PF4 + IL17A | 0,76 |
| GZMH + GNLY + CCL5 + CXCL9 + PF4 + REN | 0,76 |
| IFNG + GNLY + CCL5 + IL17A + TSLP + REN | 0,76 |
| GZMH + GNLY + ITGAE + PF4 + REN + PROM1 | 0,76 |
| CXCL13 + GNLY + CCL5 + CXCL9 + PF4 + IL17A | 0,76 |
| GNLY + ITGAE + PF4 + TSLP + REN + PROM1 | 0,76 |
| IFNG + CXCL13 + GNLY + CCL5 + PF4 + IL17A | 0,76 |
| CXCL13 + GNLY + CCL5 + PF4 + IL17A + PROM1 | 0,76 |
| GZMH + IFNG + GNLY + IL17A + REN + VEGFA | 0,76 |
| IFNG + CXCL13 + GNLY + PF4 + REN + PROM1 | 0,76 |
| GNLY + LAG3 + CCL5 + PF4 + TSLP + REN | 0,76 |
| GNLY + PF4 + TSLP + REN + IHH + PROM1 | 0,76 |
| IFNG + GNLY + CCL5 + IL17A + REN + PROM1 | 0,76 |
| GNLY + CXCL9 + PF4 + TSLP + REN + PROM1 | 0,76 |
| GNLY + CCL5 + PF4 + TSLP + REN + IHH | 0,76 |
| GNLY + PF4 + TSLP + REN + PROM1 + VEGFA | 0,76 |
| GNLY + ITGAE + CCL5 + IL17A + TSLP + REN | 0,76 |
| CXCL13 + GNLY + LAG3 + CCL5 + PF4 + IL17A | 0,76 |
| CXCL13 + GNLY + CCL5 + PF4 + IL17A + IHH | 0,76 |
| GNLY + LAG3 + CCL5 + CXCL9 + IL17A + REN | 0,76 |
| CXCL13 + GNLY + CCL5 + PF4 + IL17A + TSLP | 0,76 |
| CXCL13 + GNLY + CCL5 + PF4 + IL17A + VEGFA | 0,76 |
| GNLY + ITGAE + PF4 + REN + IHH + PROM1 | 0,76 |
| GZMH + GNLY + PF4 + REN + IHH + PROM1 | 0,76 |
| GNLY + LAG3 + PF4 + TSLP + REN + PROM1 | 0,76 |
| GNLY + LAG3 + PF4 + REN + IHH + PROM1 | 0,76 |
| GNLY + CXCL9 + PF4 + REN + IHH + PROM1 | 0,76 |
| GNLY + PF4 + REN + IHH + PROM1 + VEGFA | 0,76 |
| GZMH + GNLY + CXCL9 + PF4 + REN + PROM1 | 0,76 |
| GNLY + ITGAE + CCL5 + IL17A + REN + PROM1 | 0,76 |
| GZMH + GNLY + LAG3 + PF4 + REN + PROM1 | 0,76 |
| GNLY + CCL5 + PF4 + TSLP + REN + VEGFA | 0,76 |
| GZMH + GNLY + CCL5 + PF4 + TSLP + REN | 0,76 |
| GZMH + GNLY + PF4 + REN + PROM1 + VEGFA | 0,76 |
| GNLY + LAG3 + ITGAE + PF4 + REN + PROM1 | 0,76 |
| GNLY + ITGAE + PF4 + REN + PROM1 + VEGFA | 0,76 |
| GNLY + LAG3 + CXCL9 + PF4 + REN + PROM1 | 0,76 |
| GNLY + ITGAE + CXCL9 + PF4 + REN + PROM1 | 0,76 |
| GNLY + CXCL9 + PF4 + REN + PROM1 + VEGFA | 0,76 |
| GNLY + CCL5 + CXCL9 + IL17A + REN + IHH | 0,76 |
| GNLY + LAG3 + PF4 + REN + PROM1 + VEGFA | 0,76 |
| CXCL13 + GNLY + CCL5 + CXCL9 + IL17A + REN | 0,76 |
| GZMH + GNLY + CXCL9 + PF4 + TSLP + REN | 0,76 |
| GNLY + ITGAE + CCL5 + IL17A + REN + IHH | 0,76 |
| CXCL13 + GNLY + PF4 + TSLP + REN + PROM1 | 0,76 |
| GZMH + GNLY + PF4 + TSLP + REN + VEGFA | 0,76 |
| GNLY + LAG3 + CCL5 + PF4 + REN + VEGFA | 0,76 |
| GNLY + ITGAE + CCL5 + IL17A + REN + VEGFA | 0,76 |
| CXCL13 + GNLY + ITGAE + CCL5 + IL17A + PROM1 | 0,76 |
| IFNG + CXCL13 + GNLY + ITGAE + PF4 + REN | 0,76 |
| IFNG + CXCL13 + GNLY + PF4 + TSLP + REN | 0,76 |
| GZMH + GNLY + ITGAE + CXCL9 + PF4 + REN | 0,76 |
| GNLY + LAG3 + CCL5 + PF4 + REN + IHH | 0,76 |
| GZMH + GNLY + LAG3 + CCL5 + PF4 + REN | 0,76 |
| GZMH + GNLY + ITGAE + PF4 + REN + IHH | 0,76 |
| CXCL13 + GNLY + CCL5 + PF4 + REN + PROM1 | 0,76 |
| GNLY + CCL5 + PF4 + REN + IHH + VEGFA | 0,76 |
| GZMH + GNLY + PF4 + TSLP + REN + IHH | 0,76 |
| GNLY + ITGAE + PF4 + TSLP + REN + VEGFA | 0,76 |
| CXCL13 + GNLY + LAG3 + PF4 + REN + PROM1 | 0,76 |
| GZMH + GNLY + ITGAE + PF4 + REN + VEGFA | 0,76 |
| GZMH + CXCL13 + GNLY + PF4 + REN + PROM1 | 0,76 |
| GZMH + GNLY + LAG3 + ITGAE + PF4 + REN | 0,76 |
| GNLY + LAG3 + ITGAE + PF4 + TSLP + REN | 0,76 |
| GZMH + GNLY + ITGAE + CCL5 + IL17A + REN | 0,76 |
| GZMH + GNLY + CCL5 + PF4 + REN + IHH | 0,76 |
| IFNG + CXCL13 + GNLY + CXCL9 + PF4 + REN | 0,76 |
| GZMH + GNLY + CCL5 + PF4 + REN + VEGFA | 0,76 |
| CXCL13 + GNLY + ITGAE + CCL5 + PF4 + REN | 0,76 |
| GZMH + GNLY + LAG3 + PF4 + TSLP + REN | 0,76 |
| IFNG + CXCL13 + GNLY + PF4 + REN + VEGFA | 0,76 |
| GNLY + LAG3 + CCL5 + IL17A + REN + PROM1 | 0,76 |
| IFNG + GNLY + ITGAE + CCL5 + CXCL9 + IL17A | 0,76 |
| GZMH + CXCL13 + GNLY + CCL5 + IL17A + REN | 0,76 |
| GNLY + ITGAE + PF4 + TSLP + REN + IHH | 0,76 |
| GNLY + ITGAE + CXCL9 + PF4 + TSLP + REN | 0,76 |
| CXCL13 + GNLY + PF4 + REN + PROM1 + VEGFA | 0,76 |
| CXCL13 + GNLY + CCL5 + IL17A + REN + PROM1 | 0,76 |
| CXCL13 + GNLY + CCL5 + IL17A + TSLP + REN | 0,76 |
| CXCL13 + GNLY + ITGAE + PF4 + REN + PROM1 | 0,76 |
| CXCL13 + GNLY + CCL5 + IL17A + REN + VEGFA | 0,76 |
| GZMH + GNLY + CXCL9 + PF4 + REN + VEGFA | 0,76 |
| CXCL13 + GNLY + ITGAE + CCL5 + CXCL9 + IL17A | 0,76 |
| GZMH + GNLY + LAG3 + CXCL9 + PF4 + REN | 0,76 |
| GZMH + GNLY + CXCL9 + PF4 + REN + IHH | 0,76 |
| GZMH + CXCL13 + GNLY + IL17A + REN + IHH | 0,76 |
| CXCL13 + GNLY + CCL5 + CXCL9 + PF4 + REN | 0,76 |
| CXCL13 + GNLY + CCL5 + PF4 + TSLP + REN | 0,76 |
| IFNG + GNLY + ITGAE + CCL5 + IL17A + IHH | 0,76 |
| GNLY + CXCL9 + PF4 + TSLP + REN + VEGFA | 0,76 |
| GNLY + LAG3 + CXCL9 + PF4 + TSLP + REN | 0,76 |
| GZMH + IFNG + CXCL13 + GNLY + PF4 + REN | 0,76 |
| GZMH + CXCL13 + GNLY + ITGAE + CCL5 + IL17A | 0,76 |
| GNLY + LAG3 + CCL5 + IL17A + REN + IHH | 0,76 |
| GNLY + PF4 + TSLP + REN + IHH + VEGFA | 0,76 |
| CXCL13 + GNLY + ITGAE + CCL5 + IL17A + VEGFA | 0,76 |
| IFNG + CXCL13 + GNLY + CCL5 + CXCL9 + IL17A | 0,76 |
| IFNG + CXCL13 + GNLY + LAG3 + PF4 + REN | 0,76 |
| GNLY + CCL5 + CXCL9 + IL17A + REN + VEGFA | 0,76 |
| IFNG + GNLY + ITGAE + CCL5 + TSLP + REN | 0,76 |
| IFNG + GNLY + ITGAE + CCL5 + IL17A + TSLP | 0,76 |
| GNLY + CXCL9 + PF4 + TSLP + REN + IHH | 0,76 |
| IFNG + CXCL13 + GNLY + CCL5 + PF4 + REN | 0,76 |
| GNLY + LAG3 + ITGAE + CXCL9 + PF4 + REN | 0,76 |
| IFNG + CXCL13 + GNLY + LAG3 + PF4 + IL17A | 0,76 |
| GNLY + ITGAE + PF4 + REN + IHH + VEGFA | 0,76 |
| CXCL13 + GNLY + CCL5 + PF4 + REN + VEGFA | 0,76 |
| CXCL13 + GNLY + ITGAE + CCL5 + IL17A + TSLP | 0,76 |
| GNLY + LAG3 + PF4 + TSLP + REN + VEGFA | 0,76 |
| GZMH + GNLY + CXCL9 + IL17A + REN + IHH | 0,76 |
| CXCL13 + GNLY + LAG3 + ITGAE + CCL5 + IL17A | 0,76 |
| CXCL13 + GNLY + CXCL9 + PF4 + REN + PROM1 | 0,76 |
| GZMH + CXCL13 + GNLY + PF4 + TSLP + REN | 0,76 |
| GNLY + LAG3 + CCL5 + IL17A + TSLP + REN | 0,76 |
| GNLY + LAG3 + ITGAE + PF4 + REN + IHH | 0,76 |
| GNLY + LAG3 + ITGAE + PF4 + REN + VEGFA | 0,76 |
| GNLY + LAG3 + PF4 + TSLP + REN + IHH | 0,76 |
| GZMH + GNLY + PF4 + REN + IHH + VEGFA | 0,76 |
| GZMH + GNLY + LAG3 + PF4 + REN + IHH | 0,76 |
| GZMH + GNLY + LAG3 + PF4 + REN + VEGFA | 0,76 |
| CXCL13 + GNLY + PF4 + REN + IHH + PROM1 | 0,76 |
| GNLY + LAG3 + CXCL9 + PF4 + REN + IHH | 0,76 |
| GNLY + ITGAE + CXCL9 + PF4 + REN + VEGFA | 0,76 |
| GZMH + CXCL13 + GNLY + ITGAE + IL17A + REN | 0,76 |
| IFNG + CXCL13 + GNLY + PF4 + REN + IHH | 0,76 |
| CXCL13 + GNLY + LAG3 + CCL5 + PF4 + REN | 0,76 |
| CXCL13 + GNLY + LAG3 + PF4 + TSLP + REN | 0,76 |
| IFNG + GNLY + ITGAE + CCL5 + IL17A + VEGFA | 0,76 |
| GZMH + IFNG + CXCL13 + GNLY + CCL5 + IL17A | 0,76 |
| GNLY + LAG3 + CXCL9 + PF4 + REN + VEGFA | 0,76 |
| GNLY + ITGAE + CXCL9 + PF4 + REN + IHH | 0,76 |
| GZMH + CXCL13 + GNLY + IL17A + TSLP + REN | 0,76 |
| GZMH + CXCL13 + GNLY + CCL5 + PF4 + REN | 0,76 |
| CXCL13 + GNLY + ITGAE + CCL5 + IL17A + IHH | 0,76 |
| GNLY + CXCL9 + PF4 + REN + IHH + VEGFA | 0,76 |
| IFNG + GNLY + CCL5 + CXCL9 + IL17A + VEGFA | 0,75 |
| GNLY + CCL5 + CXCL9 + IL17A + TSLP + REN | 0,75 |
| GZMH + CXCL13 + GNLY + ITGAE + PF4 + REN | 0,75 |
| GNLY + CCL5 + CXCL9 + IL17A + REN + PROM1 | 0,75 |
| GZMH + GNLY + ITGAE + CXCL9 + IL17A + REN | 0,75 |
| CXCL13 + GNLY + LAG3 + IL17A + REN + IHH | 0,75 |
| CXCL13 + GNLY + ITGAE + PF4 + TSLP + REN | 0,75 |
| CXCL13 + GNLY + CCL5 + PF4 + REN + IHH | 0,75 |
| CXCL13 + GNLY + IL17A + TSLP + REN + IHH | 0,75 |
| GZMH + CXCL13 + GNLY + CXCL9 + IL17A + REN | 0,75 |
| GNLY + LAG3 + PF4 + REN + IHH + VEGFA | 0,75 |
| CXCL13 + GNLY + PF4 + TSLP + REN + VEGFA | 0,75 |
| GZMH + IFNG + GNLY + ITGAE + CCL5 + IL17A | 0,75 |
| CXCL13 + GNLY + CXCL9 + PF4 + TSLP + REN | 0,75 |
| GZMH + GNLY + CCL5 + CXCL9 + IL17A + REN | 0,75 |
| GZMH + CXCL13 + GNLY + IL17A + REN + VEGFA | 0,75 |
| IFNG + CXCL13 + GNLY + PF4 + IL17A + IHH | 0,75 |
| GZMH + GNLY + ITGAE + IL17A + REN + IHH | 0,75 |
| GZMH + GNLY + ITGAE + IL17A + TSLP + REN | 0,75 |
| IFNG + GNLY + CCL5 + CXCL9 + PF4 + IL17A | 0,75 |
| IFNG + CXCL13 + GNLY + LAG3 + CCL5 + IL17A | 0,75 |
| CXCL13 + GNLY + LAG3 + ITGAE + PF4 + REN | 0,75 |
| IFNG + GNLY + CCL5 + CXCL9 + IL17A + TSLP | 0,75 |
| IFNG + GNLY + ITGAE + CCL5 + PF4 + IL17A | 0,75 |
| IFNG + GNLY + ITGAE + CCL5 + IL17A + PROM1 | 0,75 |
| GZMH + CXCL13 + GNLY + CXCL9 + PF4 + REN | 0,75 |
| GZMH + CXCL13 + GNLY + LAG3 + IL17A + REN | 0,75 |
| IFNG + GNLY + ITGAE + PF4 + IL17A + IHH | 0,75 |
| IFNG + GNLY + LAG3 + CCL5 + CXCL9 + IL17A | 0,75 |
| GZMH + CXCL13 + GNLY + IL17A + REN + PROM1 | 0,75 |
| GZMH + CXCL13 + GNLY + LAG3 + PF4 + IL17A | 0,75 |
| GZMH + CXCL13 + GNLY + PF4 + REN + VEGFA | 0,75 |
| CXCL13 + GNLY + CXCL9 + IL17A + REN + IHH | 0,75 |
| GZMH + GNLY + LAG3 + CCL5 + IL17A + REN | 0,75 |
| CXCL13 + GNLY + LAG3 + PF4 + REN + VEGFA | 0,75 |
| CXCL13 + GNLY + IL17A + REN + IHH + PROM1 | 0,75 |
| CXCL13 + GNLY + IL17A + REN + IHH + VEGFA | 0,75 |
| GZMH + CXCL13 + GNLY + LAG3 + PF4 + REN | 0,75 |
| IFNG + CXCL13 + GNLY + CCL5 + IL17A + TSLP | 0,75 |
| GNLY + LAG3 + CCL5 + IL17A + REN + VEGFA | 0,75 |
| IFNG + GNLY + LAG3 + ITGAE + CCL5 + IL17A | 0,75 |
| CXCL13 + GNLY + LAG3 + CXCL9 + PF4 + REN | 0,75 |
| CXCL13 + GNLY + ITGAE + PF4 + REN + VEGFA | 0,75 |
| GZMH + IFNG + GNLY + CCL5 + CXCL9 + IL17A | 0,75 |
| IFNG + GNLY + CXCL9 + PF4 + IL17A + IHH | 0,75 |
| CXCL13 + GNLY + LAG3 + PF4 + IL17A + TSLP | 0,75 |
| GZMH + GNLY + LAG3 + ITGAE + IL17A + REN | 0,75 |
| IFNG + CXCL13 + GNLY + CCL5 + IL17A + VEGFA | 0,75 |
| GZMH + GNLY + CXCL9 + IL17A + TSLP + REN | 0,75 |
| GZMH + CXCL13 + GNLY + PF4 + REN + IHH | 0,75 |
| CXCL13 + GNLY + ITGAE + CXCL9 + PF4 + REN | 0,75 |
| GZMH + GNLY + CXCL9 + IL17A + REN + VEGFA | 0,75 |
| GZMH + GNLY + LAG3 + CXCL9 + IL17A + REN | 0,75 |
| CXCL13 + GNLY + PF4 + TSLP + REN + IHH | 0,75 |
| GNLY + CCL5 + IL17A + TSLP + REN + IHH | 0,75 |
| GZMH + GNLY + ITGAE + IL17A + REN + PROM1 | 0,75 |
| GZMH + IFNG + CXCL13 + GNLY + PF4 + IL17A | 0,75 |
| CXCL13 + GNLY + CXCL9 + PF4 + REN + VEGFA | 0,75 |
| GZMH + GNLY + ITGAE + IL17A + REN + VEGFA | 0,75 |
| IFNG + GNLY + CCL5 + CXCL9 + IL17A + PROM1 | 0,75 |
| IFNG + GNLY + LAG3 + ITGAE + CCL5 + REN | 0,75 |
| GNLY + CXCL9 + IL17A + REN + IHH + VEGFA | 0,75 |
| GZMH + GNLY + CXCL9 + IL17A + REN + PROM1 | 0,75 |
| GNLY + ITGAE + IL17A + TSLP + REN + IHH | 0,75 |
| IFNG + CXCL13 + GNLY + CXCL9 + PF4 + IL17A | 0,75 |
| IFNG + CXCL13 + GNLY + CCL5 + IL17A + PROM1 | 0,75 |
| IFNG + CXCL13 + GNLY + PF4 + IL17A + TSLP | 0,75 |
| IFNG + GNLY + CCL5 + CXCL9 + IL17A + IHH | 0,75 |
| GZMH + GNLY + IL17A + TSLP + REN + IHH | 0,75 |
| GNLY + CXCL9 + IL17A + TSLP + REN + IHH | 0,75 |
| CXCL13 + GNLY + LAG3 + PF4 + REN + IHH | 0,75 |
| CXCL13 + GNLY + LAG3 + ITGAE + PF4 + IL17A | 0,75 |
| CXCL13 + GNLY + LAG3 + PF4 + IL17A + PROM1 | 0,75 |
| CXCL13 + GNLY + LAG3 + CCL5 + CXCL9 + IL17A | 0,75 |
| GZMH + GNLY + LAG3 + IL17A + REN + IHH | 0,75 |
| CXCL13 + GNLY + ITGAE + IL17A + REN + IHH | 0,75 |
| CXCL13 + GNLY + LAG3 + PF4 + IL17A + VEGFA | 0,75 |
| IFNG + CXCL13 + GNLY + PF4 + IL17A + PROM1 | 0,75 |
| IFNG + CXCL13 + GNLY + PF4 + IL17A + VEGFA | 0,75 |
| CXCL13 + GNLY + PF4 + REN + IHH + VEGFA | 0,75 |
| CXCL13 + GNLY + LAG3 + CXCL9 + PF4 + IL17A | 0,75 |
| GNLY + IL17A + TSLP + REN + IHH + VEGFA | 0,75 |
| IFNG + CXCL13 + GNLY + ITGAE + PF4 + IL17A | 0,75 |
| GNLY + IL17A + TSLP + REN + IHH + PROM1 | 0,75 |
| CXCL13 + GNLY + ITGAE + PF4 + REN + IHH | 0,75 |
| CXCL13 + GNLY + LAG3 + IL17A + TSLP + REN | 0,75 |
| IFNG + GNLY + ITGAE + CCL5 + REN + PROM1 | 0,75 |
| IFNG + CXCL13 + GNLY + LAG3 + ITGAE + IL17A | 0,75 |
| IFNG + CXCL13 + GNLY + CCL5 + IL17A + IHH | 0,75 |
| GNLY + CXCL9 + IL17A + REN + IHH + PROM1 | 0,75 |
| IFNG + GNLY + PF4 + IL17A + TSLP + IHH | 0,75 |
| IFNG + GNLY + CCL5 + PF4 + IL17A + IHH | 0,75 |
| IFNG + GNLY + LAG3 + CXCL9 + PF4 + IL17A | 0,75 |
| GNLY + ITGAE + IL17A + REN + IHH + VEGFA | 0,75 |
| CXCL13 + GNLY + CXCL9 + PF4 + REN + IHH | 0,75 |
| GZMH + IFNG + GNLY + PF4 + IL17A + IHH | 0,75 |
| IFNG + GNLY + LAG3 + PF4 + IL17A + IHH | 0,75 |
| IFNG + GNLY + PF4 + IL17A + IHH + VEGFA | 0,75 |
| GZMH + CXCL13 + GNLY + LAG3 + CCL5 + IL17A | 0,75 |
| CXCL13 + GNLY + CXCL9 + IL17A + TSLP + REN | 0,75 |
| CXCL13 + GNLY + IL17A + TSLP + REN + PROM1 | 0,75 |
| GNLY + LAG3 + IL17A + TSLP + REN + IHH | 0,75 |
| GZMH + IFNG + GNLY + CXCL9 + IL17A + TSLP | 0,75 |
| CXCL13 + GNLY + LAG3 + CCL5 + IL17A + PROM1 | 0,75 |
| CXCL13 + GNLY + LAG3 + IL17A + REN + PROM1 | 0,75 |
| GZMH + IFNG + CXCL13 + GNLY + ITGAE + IL17A | 0,75 |
| CXCL13 + GNLY + CCL5 + CXCL9 + IL17A + VEGFA | 0,75 |
| IFNG + GNLY + ITGAE + IL17A + TSLP + VEGFA | 0,75 |
| CXCL13 + GNLY + LAG3 + CCL5 + IL17A + TSLP | 0,75 |
| CXCL13 + GNLY + LAG3 + PF4 + IL17A + IHH | 0,75 |
| CXCL13 + GNLY + LAG3 + CCL5 + IL17A + VEGFA | 0,75 |
| CXCL13 + GNLY + CCL5 + CXCL9 + IL17A + TSLP | 0,75 |
| GNLY + ITGAE + IL17A + REN + IHH + PROM1 | 0,75 |
| GZMH + IFNG + CXCL13 + GNLY + CXCL9 + IL17A | 0,75 |
| CXCL13 + GNLY + ITGAE + IL17A + TSLP + REN | 0,75 |
| IFNG + CXCL13 + GNLY + ITGAE + IL17A + TSLP | 0,75 |
| GZMH + GNLY + IL17A + REN + IHH + PROM1 | 0,75 |
| GNLY + ITGAE + CXCL9 + IL17A + REN + IHH | 0,75 |
| CXCL13 + GNLY + CCL5 + CXCL9 + IL17A + PROM1 | 0,75 |
| GZMH + IFNG + CXCL13 + GNLY + IL17A + TSLP | 0,75 |
| IFNG + GNLY + PF4 + IL17A + IHH + PROM1 | 0,75 |
| CXCL13 + GNLY + IL17A + TSLP + REN + VEGFA | 0,75 |
| CXCL13 + GNLY + LAG3 + CXCL9 + IL17A + REN | 0,75 |
| IFNG + CXCL13 + GNLY + CXCL9 + IL17A + TSLP | 0,75 |
| GNLY + LAG3 + IL17A + REN + IHH + PROM1 | 0,75 |
| IFNG + GNLY + CXCL9 + IL17A + TSLP + VEGFA | 0,75 |
| IFNG + CXCL13 + GNLY + LAG3 + CXCL9 + IL17A | 0,75 |
| IFNG + GNLY + CCL5 + PF4 + IL17A + TSLP | 0,75 |
| IFNG + CXCL13 + GNLY + ITGAE + IL17A + VEGFA | 0,75 |
| IFNG + GNLY + LAG3 + ITGAE + IL17A + TSLP | 0,75 |
| IFNG + GNLY + PF4 + IL17A + TSLP + VEGFA | 0,75 |
| GZMH + CXCL13 + GNLY + CCL5 + CXCL9 + IL17A | 0,75 |
| IFNG + GNLY + PF4 + IL17A + TSLP + PROM1 | 0,75 |
| GNLY + CCL5 + IL17A + REN + IHH + PROM1 | 0,75 |
| IFNG + GNLY + ITGAE + TSLP + REN + PROM1 | 0,75 |
| GNLY + LAG3 + CXCL9 + IL17A + REN + IHH | 0,75 |
| CXCL13 + GNLY + LAG3 + ITGAE + IL17A + REN | 0,75 |
| CXCL13 + GNLY + CXCL9 + IL17A + REN + PROM1 | 0,75 |
| IFNG + GNLY + ITGAE + PF4 + IL17A + TSLP | 0,75 |
| IFNG + GNLY + CXCL9 + PF4 + IL17A + TSLP | 0,75 |
| IFNG + CXCL13 + GNLY + ITGAE + IL17A + PROM1 | 0,75 |
| IFNG + CXCL13 + GNLY + LAG3 + IL17A + TSLP | 0,75 |
| GZMH + IFNG + GNLY + CXCL9 + PF4 + IL17A | 0,75 |
| GZMH + IFNG + GNLY + ITGAE + CXCL9 + IL17A | 0,75 |
| IFNG + GNLY + ITGAE + CXCL9 + IL17A + TSLP | 0,75 |
| IFNG + CXCL13 + GNLY + IL17A + TSLP + VEGFA | 0,75 |
| CXCL13 + GNLY + LAG3 + IL17A + REN + VEGFA | 0,75 |
| GZMH + GNLY + LAG3 + IL17A + TSLP + REN | 0,75 |
| GZMH + GNLY + IL17A + REN + IHH + VEGFA | 0,75 |
| IFNG + GNLY + ITGAE + CXCL9 + IL17A + VEGFA | 0,75 |
| IFNG + GNLY + LAG3 + CCL5 + IL17A + TSLP | 0,75 |
| IFNG + GNLY + ITGAE + CCL5 + PF4 + IHH | 0,75 |
| IFNG + CXCL13 + GNLY + LAG3 + IL17A + VEGFA | 0,75 |
| GZMH + CXCL13 + GNLY + PF4 + IL17A + IHH | 0,75 |
| IFNG + CXCL13 + GNLY + IL17A + TSLP + PROM1 | 0,75 |
| CXCL13 + GNLY + CXCL9 + IL17A + REN + VEGFA | 0,75 |
| GNLY + LAG3 + IL17A + REN + IHH + VEGFA | 0,75 |
| IFNG + GNLY + ITGAE + CCL5 + CXCL9 + REN | 0,75 |
| GZMH + IFNG + GNLY + ITGAE + CCL5 + REN | 0,75 |
| CXCL13 + GNLY + IL17A + REN + PROM1 + VEGFA | 0,75 |
| GZMH + IFNG + GNLY + ITGAE + IL17A + TSLP | 0,75 |
| IFNG + GNLY + LAG3 + PF4 + IL17A + TSLP | 0,75 |
| GZMH + CXCL13 + GNLY + CCL5 + IL17A + PROM1 | 0,75 |
| GNLY + IL17A + REN + IHH + PROM1 + VEGFA | 0,75 |
| CXCL13 + GNLY + ITGAE + IL17A + REN + PROM1 | 0,75 |
| IFNG + CXCL13 + GNLY + ITGAE + CXCL9 + IL17A | 0,75 |
| CXCL13 + GNLY + CCL5 + CXCL9 + IL17A + IHH | 0,75 |
| IFNG + GNLY + ITGAE + IL17A + TSLP + PROM1 | 0,75 |
| IFNG + GNLY + ITGAE + CCL5 + REN + VEGFA | 0,75 |
| GZMH + IFNG + GNLY + PF4 + IL17A + TSLP | 0,75 |
| IFNG + GNLY + LAG3 + ITGAE + CXCL9 + IL17A | 0,75 |
| IFNG + GNLY + LAG3 + CXCL9 + IL17A + TSLP | 0,75 |
| IFNG + GNLY + CXCL9 + PF4 + IL17A + VEGFA | 0,75 |
| GZMH + CXCL13 + GNLY + PF4 + IL17A + TSLP | 0,75 |
| IFNG + CXCL13 + GNLY + LAG3 + IL17A + PROM1 | 0,75 |
| GZMH + CXCL13 + GNLY + CCL5 + IL17A + TSLP | 0,75 |
| CXCL13 + GNLY + CCL5 + IL17A + TSLP + PROM1 | 0,75 |
| IFNG + CXCL13 + GNLY + CXCL9 + IL17A + VEGFA | 0,75 |
| GZMH + IFNG + CXCL13 + GNLY + IL17A + PROM1 | 0,75 |
| GZMH + GNLY + LAG3 + IL17A + REN + PROM1 | 0,75 |
| IFNG + GNLY + ITGAE + IL17A + TSLP + IHH | 0,75 |
| GNLY + LAG3 + ITGAE + IL17A + REN + IHH | 0,75 |
| GZMH + IFNG + CXCL13 + GNLY + LAG3 + IL17A | 0,75 |
| IFNG + GNLY + LAG3 + ITGAE + PF4 + IL17A | 0,75 |
| GZMH + IFNG + CXCL13 + GNLY + IL17A + VEGFA | 0,75 |
| GZMH + GNLY + CCL5 + IL17A + REN + IHH | 0,75 |
| CXCL13 + GNLY + LAG3 + CCL5 + IL17A + IHH | 0,75 |
| GNLY + CCL5 + IL17A + REN + IHH + VEGFA | 0,75 |
| IFNG + GNLY + CXCL9 + IL17A + TSLP + PROM1 | 0,75 |
| IFNG + GNLY + ITGAE + CXCL9 + IL17A + IHH | 0,75 |
| GZMH + IFNG + GNLY + ITGAE + IL17A + IHH | 0,75 |
| GZMH + IFNG + GNLY + CXCL9 + IL17A + VEGFA | 0,75 |
| CXCL13 + GNLY + ITGAE + IL17A + REN + VEGFA | 0,75 |
| GZMH + IFNG + GNLY + ITGAE + TSLP + REN | 0,75 |
| GNLY + ITGAE + CCL5 + CXCL9 + IL17A + VEGFA | 0,75 |
| CXCL13 + GNLY + ITGAE + CXCL9 + IL17A + REN | 0,75 |
| IFNG + CXCL13 + GNLY + CXCL9 + IL17A + PROM1 | 0,75 |
| CXCL13 + GNLY + CCL5 + IL17A + PROM1 + VEGFA | 0,75 |
| IFNG + GNLY + ITGAE + CCL5 + REN + IHH | 0,75 |
| CXCL13 + GNLY + CCL5 + IL17A + TSLP + VEGFA | 0,75 |
| IFNG + GNLY + ITGAE + CXCL9 + IL17A + PROM1 | 0,75 |
| IFNG + CXCL13 + GNLY + IL17A + PROM1 + VEGFA | 0,75 |
| IFNG + GNLY + CCL5 + PF4 + IL17A + PROM1 | 0,75 |
| GZMH + IFNG + GNLY + CCL5 + PF4 + IL17A | 0,75 |
| GZMH + IFNG + GNLY + CXCL9 + IL17A + IHH | 0,75 |
| IFNG + GNLY + ITGAE + CXCL9 + PF4 + IL17A | 0,75 |

| **Table 7: clusters of 7 genes:** | |
|---|---|
| **Clusters of 7 genes** | **c_tau level** |
| IFNG + GNLY + ITGAE + CCL5 + PF4 + IL17A + REN | 0,79 |
| IFNG + GNLY + CCL5 + CXCL9 + PF4 + IL17A + REN | 0,79 |
| GNLY + ITGAE + CCL5 + CXCL9 + PF4 + IL17A + REN | 0,79 |
| IFNG + GNLY + LAG3 + CCL5 + PF4 + IL17A + REN | 0,79 |
| GNLY + ITGAE + CCL5 + PF4 + IL17A + REN + PROM1 | 0,79 |
| CXCL13 + GNLY + ITGAE + CCL5 + PF4 + IL17A + REN | 0,79 |
| IFNG + GNLY + ITGAE + CCL5 + CXCL9 + IL17A + REN | 0,79 |
| GZMH + IFNG + GNLY + CCL5 + PF4 + IL17A + REN | 0,79 |
| IFNG + GNLY + LAG3 + ITGAE + CCL5 + IL17A + REN | 0,79 |
| IFNG + GNLY + CCL5 + PF4 + IL17A + REN + PROM1 | 0,78 |
| GZMH + IFNG + GNLY + ITGAE + PF4 + IL17A + REN | 0,78 |
| IFNG + GNLY + ITGAE + PF4 + IL17A + REN + VEGFA | 0,78 |
| IFNG + GNLY + CCL5 + PF4 + IL17A + REN + IHH | 0,78 |
| IFNG + GNLY + LAG3 + ITGAE + PF4 + IL17A + REN | 0,78 |
| IFNG + GNLY + ITGAE + PF4 + IL17A + REN + PROM1 | 0,78 |
| IFNG + GNLY + ITGAE + PF4 + IL17A + TSLP + REN | 0,78 |
| IFNG + GNLY + ITGAE + PF4 + IL17A + REN + IHH | 0,78 |
| GZMH + IFNG + GNLY + ITGAE + CCL5 + IL17A + REN | 0,78 |
| IFNG + GNLY + PF4 + IL17A + REN + IHH + PROM1 | 0,78 |
| IFNG + GNLY + CCL5 + PF4 + IL17A + TSLP + REN | 0,78 |
| IFNG + GNLY + LAG3 + PF4 + IL17A + REN + PROM1 | 0,78 |
| IFNG + GNLY + ITGAE + CCL5 + IL17A + TSLP + REN | 0,78 |
| GZMH + IFNG + GNLY + PF4 + IL17A + REN + PROM1 | 0,78 |
| IFNG + GNLY + PF4 + IL17A + TSLP + REN + PROM1 | 0,78 |
| IFNG + GNLY + CCL5 + PF4 + IL17A + REN + VEGFA | 0,78 |
| CXCL13 + GNLY + CCL5 + PF4 + IL17A + REN + IHH | 0,78 |
| GNLY + ITGAE + CCL5 + PF4 + IL17A + TSLP + REN | 0,78 |
| IFNG + GNLY + PF4 + IL17A + REN + PROM1 + VEGFA | 0,78 |
| IFNG + GNLY + LAG3 + PF4 + IL17A + REN + IHH | 0,78 |
| IFNG + GNLY + PF4 + IL17A + REN + IHH + VEGFA | 0,78 |
| GZMH + IFNG + GNLY + PF4 + IL17A + REN + IHH | 0,78 |
| IFNG + GNLY + PF4 + IL17A + TSLP + REN + IHH | 0,78 |
| IFNG + GNLY + LAG3 + PF4 + IL17A + TSLP + REN | 0,78 |
| IFNG + GNLY + LAG3 + CXCL9 + PF4 + IL17A + REN | 0,78 |
| IFNG + GNLY + ITGAE + CCL5 + IL17A + REN + VEGFA | 0,78 |
| GZMH + IFNG + GNLY + PF4 + IL17A + TSLP + REN | 0,78 |
| GZMH + IFNG + GNLY + CXCL9 + PF4 + IL17A + REN | 0,78 |
| IFNG + GNLY + ITGAE + CCL5 + IL17A + REN + PROM1 | 0,78 |
| IFNG + GNLY + CXCL9 + PF4 + IL17A + REN + VEGFA | 0,78 |
| GNLY + LAG3 + ITGAE + CCL5 + PF4 + IL17A + REN | 0,78 |
| IFNG + GNLY + CXCL9 + PF4 + IL17A + REN + IHH | 0,78 |
| GZMH + IFNG + GNLY + LAG3 + PF4 + IL17A + REN | 0,78 |
| IFNG + GNLY + LAG3 + PF4 + IL17A + REN + VEGFA | 0,78 |
| IFNG + GNLY + PF4 + IL17A + TSLP + REN + VEGFA | 0,78 |
| GZMH + IFNG + GNLY + PF4 + IL17A + REN + VEGFA | 0,78 |
| IFNG + GNLY + ITGAE + CXCL9 + PF4 + IL17A + REN | 0,78 |
| GNLY + ITGAE + CCL5 + PF4 + IL17A + REN + VEGFA | 0,78 |
| IFNG + GNLY + ITGAE + CCL5 + IL17A + REN + IHH | 0,78 |
| GZMH + GNLY + ITGAE + CCL5 + PF4 + IL17A + REN | 0,78 |
| IFNG + GNLY + CXCL9 + PF4 + IL17A + REN + PROM1 | 0,78 |
| CXCL13 + GNLY + CCL5 + CXCL9 + PF4 + IL17A + REN | 0,78 |
| GNLY + CCL5 + CXCL9 + PF4 + IL17A + REN + IHH | 0,78 |
| GNLY + ITGAE + CCL5 + PF4 + IL17A + REN + IHH | 0,78 |
| GNLY + LAG3 + CCL5 + CXCL9 + PF4 + IL17A + REN | 0,78 |
| IFNG + CXCL13 + GNLY + CCL5 + PF4 + IL17A + REN | 0,78 |
| IFNG + GNLY + CXCL9 + PF4 + IL17A + TSLP + REN | 0,78 |
| IFNG + CXCL13 + GNLY + ITGAE + CCL5 + IL17A + REN | 0,78 |
| CXCL13 + GNLY + LAG3 + CCL5 + PF4 + IL17A + REN | 0,78 |
| GNLY + CCL5 + CXCL9 + PF4 + IL17A + REN + VEGFA | 0,78 |
| CXCL13 + GNLY + CCL5 + PF4 + IL17A + REN + PROM1 | 0,78 |
| GZMH + CXCL13 + GNLY + CCL5 + PF4 + IL17A + REN | 0,78 |
| CXCL13 + GNLY + CCL5 + PF4 + IL17A + REN + VEGFA | 0,78 |
| GNLY + CCL5 + CXCL9 + PF4 + IL17A + REN + PROM1 | 0,78 |
| CXCL13 + GNLY + CCL5 + PF4 + IL17A + TSLP + REN | 0,78 |
| GNLY + CCL5 + CXCL9 + PF4 + IL17A + TSLP + REN | 0,78 |
| IFNG + CXCL13 + GNLY + PF4 + IL17A + REN + PROM1 | 0,78 |
| IFNG + CXCL13 + GNLY + ITGAE + PF4 + IL17A + REN | 0,78 |
| GZMH + IFNG + CXCL13 + GNLY + PF4 + IL17A + REN | 0,78 |
| GNLY + LAG3 + CCL5 + PF4 + IL17A + REN + PROM1 | 0,78 |
| IFNG + CXCL13 + GNLY + CXCL9 + PF4 + IL17A + REN | 0,78 |
| IFNG + CXCL13 + GNLY + PF4 + IL17A + REN + VEGFA | 0,78 |
| IFNG + CXCL13 + GNLY + PF4 + IL17A + REN + IHH | 0,78 |
| IFNG + CXCL13 + GNLY + PF4 + IL17A + TSLP + REN | 0,78 |
| GZMH + GNLY + CCL5 + CXCL9 + PF4 + IL17A + REN | 0,78 |
| IFNG + CXCL13 + GNLY + LAG3 + PF4 + IL17A + REN | 0,78 |
| GNLY + LAG3 + CCL5 + PF4 + IL17A + TSLP + REN | 0,78 |
| GNLY + LAG3 + CCL5 + PF4 + IL17A + REN + IHH | 0,78 |
| GNLY + CCL5 + PF4 + IL17A + REN + IHH + PROM1 | 0,78 |
| IFNG + GNLY + ITGAE + CCL5 + PF4 + REN + PROM1 | 0,78 |
| GNLY + LAG3 + CCL5 + PF4 + IL17A + REN + VEGFA | 0,78 |
| CXCL13 + GNLY + LAG3 + PF4 + IL17A + REN + IHH | 0,78 |
| GZMH + GNLY + ITGAE + PF4 + IL17A + TSLP + REN | 0,78 |
| IFNG + GNLY + ITGAE + CCL5 + PF4 + TSLP + REN | 0,78 |
| GZMH + CXCL13 + GNLY + PF4 + IL17A + REN + IHH | 0,78 |
| GZMH + GNLY + ITGAE + CXCL9 + PF4 + IL17A + REN | 0,78 |
| GZMH + CXCL13 + GNLY + LAG3 + PF4 + IL17A + REN | 0,78 |
| GZMH + IFNG + GNLY + ITGAE + CCL5 + PF4 + REN | 0,78 |
| GZMH + GNLY + LAG3 + CCL5 + PF4 + IL17A + REN | 0,78 |
| GNLY + CCL5 + PF4 + IL17A + TSLP + REN + IHH | 0,78 |
| GNLY + ITGAE + CCL5 + PF4 + TSLP + REN + PROM1 | 0,78 |
| GZMH + CXCL13 + GNLY + PF4 + IL17A + TSLP + REN | 0,78 |
| GZMH + CXCL13 + GNLY + ITGAE + PF4 + IL17A + REN | 0,77 |
| GZMH + GNLY + CXCL9 + PF4 + IL17A + TSLP + REN | 0,77 |
| GZMH + GNLY + ITGAE + PF4 + IL17A + REN + VEGFA | 0,77 |
| GZMH + CXCL13 + GNLY + PF4 + IL17A + REN + VEGFA | 0,77 |
| GZMH + GNLY + CXCL9 + PF4 + IL17A + REN + IHH | 0,77 |
| GZMH + CXCL13 + GNLY + PF4 + IL 17A + REN + PROM 1 | 0,77 |
| GZMH + GNLY + CXCL9 + PF4 + IL17A + REN + PROM1 | 0,77 |
| CXCL13 + GNLY + LAG3 + PF4 + IL17A + TSLP + REN | 0,77 |
| GZMH + GNLY + ITGAE + PF4 + IL17A + REN + PROM 1 | 0,77 |
| GNLY + CCL5 + PF4 + IL17A + TSLP + REN + PROM1 | 0,77 |
| GZMH + CXCL13 + GNLY + CXCL9 + PF4 + IL17A + REN | 0,77 |
| GZMH + IFNG + GNLY + CCL5 + PF4 + REN + PROM1 | 0,77 |
| IFNG + GNLY + LAG3 + CCL5 + CXCL9 + IL17A + REN | 0,77 |
| GZMH + GNLY + ITGAE + PF4 + IL17A + REN + IHH | 0,77 |
| GZMH + GNLY + LAG3 + ITGAE + PF4 + IL17A + REN | 0,77 |
| GZMH + GNLY + CXCL9 + PF4 + IL17A + REN + VEGFA | 0,77 |
| GNLY + ITGAE + CCL5 + CXCL9 + IL17A + REN + VEGFA | 0,77 |
| CXCL13 + GNLY + LAG3 + PF4 + IL17A + REN + VEGFA | 0,77 |
| GZMH + GNLY + CCL5 + PF4 + IL17A + REN + IHH | 0,77 |
| CXCL13 + GNLY + LAG3 + ITGAE + PF4 + IL17A + REN | 0,77 |
| CXCL13 + GNLY + LAG3 + PF4 + IL 17A + REN + PROM 1 | 0,77 |
| CXCL13 + GNLY + LAG3 + CXCL9 + PF4 + IL17A + REN | 0,77 |
| GNLY + CCL5 + PF4 + IL17A + REN + IHH + VEGFA | 0,77 |
| GNLY + CCL5 + PF4 + IL17A + REN + PROM1 + VEGFA | 0,77 |
| GZMH + GNLY + CCL5 + PF4 + IL17A + REN + PROM1 | 0,77 |
| GZMH + GNLY + PF4 + IL17A + REN + IHH + PROM 1 | 0,77 |
| GZMH + GNLY + LAG3 + CXCL9 + PF4 + IL17A + REN | 0,77 |
| GNLY + CXCL9 + PF4 + IL17A + REN + IHH + PROM1 | 0,77 |
| GNLY + LAG3 + CXCL9 + PF4 + IL17A + REN + IHH | 0,77 |
| GZMH + GNLY + PF4 + IL17A + TSLP + REN + IHH | 0,77 |
| GNLY + ITGAE + PF4 + IL17A + REN + IHH + PROM 1 | 0,77 |
| GNLY + PF4 + IL17A + REN + IHH + PROM1 + VEGFA | 0,77 |
| GNLY + PF4 + IL17A + TSLP + REN + IHH + PROM1 | 0,77 |
| IFNG + GNLY + CCL5 + PF4 + TSLP + REN + PROM1 | 0,77 |
| GNLY + LAG3 + PF4 + IL17A + REN + IHH + PROM 1 | 0,77 |
| IFNG + GNLY + LAG3 + ITGAE + CCL5 + PF4 + REN | 0,77 |
| CXCL13 + GNLY + ITGAE + CCL5 + IL17A + REN + PROM1 | 0,77 |
| GNLY + CXCL9 + PF4 + IL17A + REN + IHH + VEGFA | 0,77 |
| IFNG + GNLY + ITGAE + CCL5 + PF4 + REN + IHH | 0,77 |
| GZMH + IFNG + GNLY + LAG3 + CCL5 + IL17A + REN | 0,77 |
| CXCL13 + GNLY + CXCL9 + PF4 + IL17A + REN + IHH | 0,77 |
| CXCL13 + GNLY + PF4 + IL17A + REN + IHH + VEGFA | 0,77 |
| GNLY + ITGAE + CCL5 + CXCL9 + PF4 + REN + PROM1 | 0,77 |
| IFNG + GNLY + CCL5 + CXCL9 + IL17A + REN + IHH | 0,77 |
| GZMH + GNLY + PF4 + IL17A + TSLP + REN + PROM1 | 0,77 |
| IFNG + GNLY + LAG3 + CCL5 + PF4 + REN + PROM1 | 0,77 |
| CXCL13 + GNLY + ITGAE + CCL5 + IL17A + REN + IHH | 0,77 |
| CXCL13 + GNLY + CXCL9 + PF4 + IL17A + TSLP + REN | 0,77 |
| CXCL13 + GNLY + PF4 + IL17A + REN + IHH + PROM1 | 0,77 |
| CXCL13 + GNLY + CXCL9 + PF4 + IL17A + REN + PROM1 | 0,77 |
| CXCL13 + GNLY + ITGAE + PF4 + IL17A + REN + IHH | 0,77 |
| IFNG + GNLY + ITGAE + IL17A + TSLP + REN + IHH | 0,77 |
| GZMH + GNLY + CCL5 + PF4 + IL17A + TSLP + REN | 0,77 |
| CXCL13 + GNLY + CXCL9 + PF4 + IL17A + REN + VEGFA | 0,77 |
| GNLY + ITGAE + PF4 + IL17A + TSLP + REN + IHH | 0,77 |
| GZMH + IFNG + GNLY + ITGAE + IL17A + TSLP + REN | 0,77 |
| GNLY + CCL5 + PF4 + IL17A + TSLP + REN + VEGFA | 0,77 |
| IFNG + GNLY + CCL5 + PF4 + REN + PROM1 + VEGFA | 0,77 |
| IFNG + GNLY + ITGAE + CCL5 + PF4 + REN + VEGFA | 0,77 |
| GNLY + ITGAE + CXCL9 + PF4 + IL17A + REN + IHH | 0,77 |
| CXCL13 + GNLY + ITGAE + CXCL9 + PF4 + IL17A + REN | 0,77 |
| GNLY + PF4 + IL17A + TSLP + REN + IHH + VEGFA | 0,77 |
| GNLY + CXCL9 + PF4 + IL17A + TSLP + REN + IHH | 0,77 |
| IFNG + GNLY + LAG3 + CCL5 + IL17A + REN + PROM1 | 0,77 |
| GNLY + LAG3 + ITGAE + PF4 + IL17A + REN + IHH | 0,77 |
| GZMH + GNLY + LAG3 + PF4 + IL17A + TSLP + REN | 0,77 |
| GNLY + ITGAE + PF4 + IL17A + REN + IHH + VEGFA | 0,77 |
| GZMH + GNLY + PF4 + IL17A + REN + IHH + VEGFA | 0,77 |
| GNLY + LAG3 + PF4 + IL17A + TSLP + REN + IHH | 0,77 |
| CXCL13 + GNLY + PF4 + IL17A + TSLP + REN + IHH | 0,77 |
| GNLY + LAG3 + ITGAE + CCL5 + PF4 + REN + PROM1 | 0,77 |
| IFNG + GNLY + LAG3 + CCL5 + IL17A + REN + IHH | 0,77 |
| GZMH + GNLY + LAG3 + PF4 + IL17A + REN + PROM1 | 0,77 |
| GNLY + ITGAE + PF4 + IL17A + TSLP + REN + PROM1 | 0,77 |
| IFNG + GNLY + ITGAE + CCL5 + CXCL9 + PF4 + REN | 0,77 |
| IFNG + GNLY + ITGAE + IL17A + TSLP + REN + VEGFA | 0,77 |
| GNLY + ITGAE + CCL5 + PF4 + REN + IHH + PROM1 | 0,77 |
| GZMH + GNLY + ITGAE + CCL5 + PF4 + REN + PROM1 | 0,77 |
| GNLY + ITGAE + PF4 + IL17A + TSLP + REN + VEGFA | 0,77 |
| GZMH + GNLY + LAG3 + PF4 + IL17A + REN + IHH | 0,77 |
| IFNG + GNLY + LAG3 + ITGAE + IL17A + TSLP + REN | 0,77 |
| CXCL13 + GNLY + ITGAE + CCL5 + CXCL9 + IL17A + REN | 0,77 |
| GZMH + GNLY + PF4 + IL17A + TSLP + REN + VEGFA | 0,77 |
| GZMH + GNLY + CCL5 + PF4 + IL17A + REN + VEGFA | 0,77 |
| GNLY + LAG3 + CXCL9 + PF4 + IL17A + REN + VEGFA | 0,77 |
| GNLY + LAG3 + ITGAE + CXCL9 + PF4 + IL17A + REN | 0,77 |
| IFNG + GNLY + LAG3 + ITGAE + CXCL9 + IL17A + REN | 0,77 |
| GNLY + ITGAE + CCL5 + CXCL9 + PF4 + TSLP + REN | 0,77 |
| GNLY + CXCL9 + PF4 + IL17A + TSLP + REN + PROM1 | 0,77 |
| IFNG + GNLY + ITGAE + CXCL9 + IL17A + REN + PROM1 | 0,77 |
| GNLY + LAG3 + CXCL9 + PF4 + IL17A + REN + PROM1 | 0,77 |
| CXCL13 + GNLY + PF4 + IL17A + TSLP + REN + VEGFA | 0,77 |
| GNLY + ITGAE + CCL5 + PF4 + REN + PROM1 + VEGFA | 0,77 |
| CXCL13 + GNLY + PF4 + IL17A + TSLP + REN + PROM1 | 0,77 |
| IFNG + GNLY + ITGAE + CXCL9 + IL17A + REN + VEGFA | 0,77 |
| GNLY + ITGAE + PF4 + IL17A + REN + PROM1 + VEGFA | 0,77 |
| GNLY + LAG3 + CXCL9 + PF4 + IL17A + TSLP + REN | 0,77 |
| CXCL13 + GNLY + ITGAE + CCL5 + IL17A + REN + VEGFA | 0,77 |
| GNLY + LAG3 + ITGAE + PF4 + IL17A + REN + PROM 1 | 0,77 |
| GNLY + PF4 + IL17A + TSLP + REN + PROM1 + VEGFA | 0,77 |
| GZMH + IFNG + GNLY + ITGAE + CXCL9 + IL17A + REN | 0,77 |
| CXCL13 + GNLY + ITGAE + PF4 + IL17A + TSLP + REN | 0,77 |
| GNLY + CXCL9 + PF4 + IL17A + REN + PROM1 + VEGFA | 0,77 |
| IFNG + GNLY + LAG3 + CCL5 + IL17A + TSLP + REN | 0,77 |
| GZMH + IFNG + GNLY + CCL5 + PF4 + TSLP + REN | 0,77 |
| GNLY + LAG3 + ITGAE + PF4 + IL17A + TSLP + REN | 0,77 |
| IFNG + GNLY + CCL5 + PF4 + REN + IHH + PROM1 | 0,77 |
| GNLY + LAG3 + PF4 + IL17A + TSLP + REN + PROM1 | 0,77 |
| GZMH + IFNG + GNLY + ITGAE + IL17A + REN + IHH | 0,77 |
| GNLY + CXCL9 + PF4 + IL17A + TSLP + REN + VEGFA | 0,77 |
| GZMH + GNLY + PF4 + IL17A + REN + PROM1 + VEGFA | 0,77 |
| GNLY + LAG3 + ITGAE + PF4 + IL17A + REN + VEGFA | 0,77 |
| CXCL13 + GNLY + LAG3 + ITGAE + CCL5 + IL17A + REN | 0,77 |
| GNLY + ITGAE + CXCL9 + PF4 + IL17A + TSLP + REN | 0,77 |
| IFNG + GNLY + CCL5 + CXCL9 + IL17A + REN + VEGFA | 0,77 |
| IFNG + GNLY + LAG3 + CCL5 + IL17A + REN + VEGFA | 0,77 |
| CXCL13 + GNLY + ITGAE + PF4 + IL17A + REN + VEGFA | 0,77 |
| GNLY + ITGAE + CXCL9 + PF4 + IL17A + REN + PROM1 | 0,77 |
| GNLY + ITGAE + CXCL9 + PF4 + IL17A + REN + VEGFA | 0,77 |
| GZMH + IFNG + GNLY + ITGAE + IL17A + REN + VEGFA | 0,77 |
| IFNG + GNLY + ITGAE + IL17A + TSLP + REN + PROM1 | 0,77 |
| CXCL13 + GNLY + PF4 + IL 17A + REN + PROM1 + VEGFA | 0,77 |
| GZMH + GNLY + LAG3 + PF4 + IL17A + REN + VEGFA | 0,77 |
| IFNG + GNLY + CCL5 + CXCL9 + IL17A + TSLP + REN | 0,77 |
| GNLY + LAG3 + PF4 + IL17A + REN + IHH + VEGFA | 0,77 |
| GZMH + IFNG + GNLY + LAG3 + ITGAE + IL17A + REN | 0,77 |
| IFNG + GNLY + ITGAE + IL17A + REN + PROM1 + VEGFA | 0,77 |
| CXCL13 + GNLY + ITGAE + PF4 + IL 17A + REN + PROM 1 | 0,77 |
| GZMH + IFNG + GNLY + PF4 + TSLP + REN + PROM 1 | 0,77 |
| GNLY + LAG3 + PF4 + IL17A + REN + PROM1 + VEGFA | 0,77 |
| IFNG + GNLY + ITGAE + CXCL9 + IL17A + REN + IHH | 0,77 |
| IFNG + GNLY + ITGAE + IL17A + REN + IHH + VEGFA | 0,77 |
| IFNG + GNLY + LAG3 + ITGAE + IL17A + REN + VEGFA | 0,77 |
| IFNG + GNLY + LAG3 + ITGAE + IL17A + REN + PROM 1 | 0,77 |
| GZMH + IFNG + GNLY + LAG3 + CCL5 + PF4 + REN | 0,77 |
| IFNG + GNLY + ITGAE + PF4 + TSLP + REN + PROM1 | 0,77 |
| IFNG + GNLY + LAG3 + ITGAE + PF4 + REN + PROM1 | 0,77 |
| IFNG + GNLY + LAG3 + ITGAE + IL17A + REN + IHH | 0,77 |
| GZMH + IFNG + GNLY + ITGAE + IL17A + REN + PROM 1 | 0,77 |
| IFNG + GNLY + ITGAE + IL17A + REN + IHH + PROM 1 | 0,77 |
| GNLY + ITGAE + CCL5 + CXCL9 + IL17A + REN + PROM1 | 0,77 |
| GNLY + LAG3 + CCL5 + PF4 + TSLP + REN + PROM1 | 0,77 |
| IFNG + GNLY + PF4 + TSLP + REN + PROM1 + VEGFA | 0,77 |
| GNLY + CCL5 + CXCL9 + PF4 + TSLP + REN + PROM1 | 0,77 |
| GZMH + GNLY + ITGAE + CCL5 + PF4 + TSLP + REN | 0,77 |
| GZMH + CXCL13 + GNLY + ITGAE + CCL5 + IL17A + REN | 0,77 |
| IFNG + GNLY + LAG3 + PF4 + TSLP + REN + PROM1 | 0,77 |
| IFNG + GNLY + ITGAE + CXCL9 + IL17A + TSLP + REN | 0,77 |
| GNLY + LAG3 + PF4 + IL17A + TSLP + REN + VEGFA | 0,77 |
| IFNG + GNLY + CCL5 + PF4 + TSLP + REN + VEGFA | 0,77 |
| GNLY + ITGAE + CCL5 + PF4 + TSLP + REN + IHH | 0,77 |
| GNLY + LAG3 + ITGAE + CCL5 + IL17A + REN + PROM1 | 0,77 |
| GZMH + IFNG + GNLY + CCL5 + PF4 + REN + IHH | 0,77 |
| CXCL13 + GNLY + ITGAE + CCL5 + IL17A + TSLP + REN | 0,77 |
| GNLY + ITGAE + CCL5 + PF4 + TSLP + REN + VEGFA | 0,77 |
| GNLY + LAG3 + CCL5 + CXCL9 + PF4 + REN + PROM1 | 0,77 |
| GZMH + IFNG + GNLY + CXCL9 + IL17A + REN + IHH | 0,77 |
| GZMH + IFNG + GNLY + CCL5 + PF4 + REN + VEGFA | 0,77 |
| IFNG + GNLY + ITGAE + PF4 + REN + PROM1 + VEGFA | 0,77 |
| IFNG + GNLY + PF4 + TSLP + REN + IHH + PROM1 | 0,77 |
| GNLY + CCL5 + CXCL9 + PF4 + REN + IHH + PROM1 | 0,77 |
| IFNG + GNLY + LAG3 + PF4 + REN + PROM1 + VEGFA | 0,77 |
| IFNG + GNLY + LAG3 + CCL5 + PF4 + TSLP + REN | 0,77 |
| GZMH + IFNG + GNLY + CCL5 + CXCL9 + PF4 + REN | 0,77 |
| IFNG + CXCL13 + GNLY + LAG3 + CCL5 + IL17A + REN | 0,77 |
| GZMH + IFNG + GNLY + ITGAE + PF4 + REN + PROM1 | 0,77 |
| IFNG + GNLY + CXCL9 + IL17A + REN + IHH + VEGFA | 0,77 |
| IFNG + GNLY + LAG3 + ITGAE + PF4 + TSLP + REN | 0,77 |
| IFNG + GNLY + CCL5 + CXCL9 + PF4 + TSLP + REN | 0,77 |
| GNLY + ITGAE + CCL5 + CXCL9 + IL17A + TSLP + REN | 0,77 |
| GZMH + IFNG + GNLY + LAG3 + PF4 + REN + PROM1 | 0,77 |
| GZMH + IFNG + GNLY + CCL5 + CXCL9 + IL17A + REN | 0,77 |
| IFNG + GNLY + ITGAE + PF4 + REN + IHH + PROM1 | 0,77 |
| IFNG + GNLY + CCL5 + CXCL9 + PF4 + REN + PROM1 | 0,77 |
| GNLY + LAG3 + ITGAE + CCL5 + CXCL9 + IL17A + REN | 0,77 |
| IFNG + GNLY + PF4 + REN + IHH + PROM1 + VEGFA | 0,77 |
| GNLY + LAG3 + ITGAE + CCL5 + PF4 + TSLP + REN | 0,77 |
| GZMH + IFNG + GNLY + PF4 + REN + PROM1 + VEGFA | 0,77 |
| GZMH + IFNG + GNLY + ITGAE + PF4 + TSLP + REN | 0,77 |
| IFNG + GNLY + LAG3 + CXCL9 + IL17A + REN + IHH | 0,77 |
| IFNG + GNLY + CCL5 + PF4 + TSLP + REN + IHH | 0,77 |
| CXCL13 + GNLY + LAG3 + CCL5 + IL17A + REN + IHH | 0,77 |
| GNLY + CCL5 + PF4 + TSLP + REN + IHH + PROM1 | 0,77 |
| IFNG + GNLY + CXCL9 + IL17A + TSLP + REN + IHH | 0,77 |
| GZMH + IFNG + CXCL13 + GNLY + ITGAE + IL17A + REN | 0,77 |
| IFNG + GNLY + LAG3 + PF4 + REN + IHH + PROM1 | 0,77 |
| GNLY + LAG3 + ITGAE + CCL5 + IL17A + TSLP + REN | 0,77 |
| IFNG + GNLY + CCL5 + CXCL9 + IL17A + REN + PROM1 | 0,77 |
| GZMH + GNLY + LAG3 + CCL5 + PF4 + REN + PROM1 | 0,77 |
| GZMH + IFNG + GNLY + PF4 + REN + IHH + PROM1 | 0,77 |
| GZMH + IFNG + GNLY + CXCL9 + IL17A + REN + VEGFA | 0,77 |
| GZMH + IFNG + GNLY + PF4 + TSLP + REN + VEGFA | 0,77 |
| IFNG + CXCL13 + GNLY + ITGAE + IL17A + REN + PROM1 | 0,77 |
| IFNG + GNLY + ITGAE + PF4 + TSLP + REN + VEGFA | 0,77 |
| IFNG + GNLY + CXCL9 + IL17A + REN + IHH + PROM1 | 0,77 |
| IFNG + GNLY + CCL5 + CXCL9 + PF4 + REN + IHH | 0,77 |
| CXCL13 + GNLY + ITGAE + CCL5 + PF4 + IL17A + PROM1 | 0,77 |
| GNLY + LAG3 + CCL5 + PF4 + REN + IHH + PROM1 | 0,77 |
| IFNG + CXCL13 + GNLY + ITGAE + CXCL9 + IL17A + REN | 0,77 |
| GZMH + IFNG + GNLY + LAG3 + CXCL9 + IL17A + REN | 0,77 |
| GNLY + CCL5 + CXCL9 + PF4 + REN + PROM1 + VEGFA | 0,77 |
| IFNG + CXCL13 + GNLY + CCL5 + CXCL9 + IL17A + REN | 0,77 |
| IFNG + CXCL13 + GNLY + ITGAE + IL17A + TSLP + REN | 0,77 |
| IFNG + GNLY + LAG3 + CCL5 + CXCL9 + PF4 + REN | 0,77 |
| IFNG + GNLY + ITGAE + PF4 + TSLP + REN + IHH | 0,77 |
| IFNG + GNLY + LAG3 + CCL5 + PF4 + REN + VEGFA | 0,77 |
| IFNG + CXCL13 + GNLY + ITGAE + IL17A + REN + VEGFA | 0,77 |
| IFNG + GNLY + CXCL9 + PF4 + TSLP + REN + PROM1 | 0,77 |
| IFNG + GNLY + PF4 + TSLP + REN + IHH + VEGFA | 0,77 |
| GNLY + LAG3 + ITGAE + CCL5 + IL17A + REN + VEGFA | 0,77 |
| GZMH + IFNG + GNLY + PF4 + TSLP + REN + IHH | 0,77 |
| IFNG + CXCL13 + GNLY + ITGAE + IL17A + REN + IHH | 0,77 |
| IFNG + GNLY + LAG3 + CXCL9 + PF4 + REN + PROM1 | 0,77 |
| IFNG + GNLY + LAG3 + CCL5 + PF4 + REN + IHH | 0,77 |
| GNLY + LAG3 + CCL5 + PF4 + REN + PROM1 + VEGFA | 0,77 |
| IFNG + GNLY + LAG3 + PF4 + TSLP + REN + VEGFA | 0,77 |
| GNLY + ITGAE + CCL5 + CXCL9 + IL17A + REN + IHH | 0,77 |
| IFNG + GNLY + LAG3 + CXCL9 + IL17A + REN + VEGFA | 0,77 |
| GZMH + IFNG + GNLY + LAG3 + PF4 + TSLP + REN | 0,77 |
| IFNG + GNLY + CCL5 + CXCL9 + PF4 + REN + VEGFA | 0,77 |
| IFNG + GNLY + CXCL9 + PF4 + REN + PROM1 + VEGFA | 0,77 |
| IFNG + GNLY + ITGAE + CXCL9 + PF4 + REN + PROM1 | 0,77 |
| GNLY + CCL5 + PF4 + TSLP + REN + PROM1 + VEGFA | 0,77 |
| IFNG + CXCL13 + GNLY + LAG3 + ITGAE + IL17A + REN | 0,77 |
| IFNG + GNLY + CCL5 + IL17A + TSLP + REN + IHH | 0,77 |
| GNLY + ITGAE + CCL5 + CXCL9 + PF4 + REN + IHH | 0,77 |
| CXCL13 + GNLY + CCL5 + CXCL9 + IL17A + REN + IHH | 0,77 |
| GZMH + IFNG + GNLY + LAG3 + IL17A + TSLP + REN | 0,77 |
| IFNG + GNLY + CXCL9 + IL17A + TSLP + REN + VEGFA | 0,77 |
| IFNG + GNLY + CCL5 + PF4 + REN + IHH + VEGFA | 0,77 |
| GZMH + IFNG + GNLY + CXCL9 + IL17A + TSLP + REN | 0,77 |
| IFNG + GNLY + LAG3 + PF4 + TSLP + REN + IHH | 0,77 |
| CXCL13 + GNLY + ITGAE + CCL5 + PF4 + IL17A + IHH | 0,77 |
| GZMH + GNLY + CCL5 + CXCL9 + PF4 + REN + PROM1 | 0,77 |
| IFNG + GNLY + LAG3 + IL17A + TSLP + REN + VEGFA | 0,77 |
| IFNG + CXCL13 + GNLY + CCL5 + IL17A + REN + IHH | 0,77 |
| IFNG + GNLY + LAG3 + CXCL9 + IL17A + TSLP + REN | 0,77 |
| IFNG + GNLY + LAG3 + ITGAE + CXCL9 + PF4 + REN | 0,77 |
| CXCL13 + GNLY + CCL5 + IL17A + TSLP + REN + IHH | 0,77 |
| IFNG + GNLY + LAG3 + IL17A + REN + IHH + PROM 1 | 0,77 |
| GZMH + GNLY + ITGAE + PF4 + TSLP + REN + PROM1 | 0,77 |
| IFNG + GNLY + LAG3 + IL17A + REN + IHH + VEGFA | 0,77 |
| GNLY + LAG3 + CCL5 + CXCL9 + IL17A + REN + VEGFA | 0,77 |
| IFNG + GNLY + LAG3 + IL17A + TSLP + REN + IHH | 0,77 |
| GZMH + IFNG + GNLY + CXCL9 + IL17A + REN + PROM1 | 0,77 |
| IFNG + CXCL13 + GNLY + ITGAE + CCL5 + PF4 + IL17A | 0,77 |
| GZMH + IFNG + CXCL13 + GNLY + ITGAE + CCL5 + IL17A | 0,77 |
| IFNG + GNLY + LAG3 + CXCL9 + IL17A + REN + PROM1 | 0,77 |
| GZMH + GNLY + ITGAE + CCL5 + CXCL9 + IL17A + REN | 0,77 |
| GZMH + GNLY + CCL5 + PF4 + REN + IHH + PROM1 | 0,77 |
| GZMH + GNLY + CCL5 + PF4 + TSLP + REN + PROM1 | 0,77 |
| GNLY + CCL5 + CXCL9 + PF4 + TSLP + REN + IHH | 0,77 |
| GZMH + IFNG + GNLY + LAG3 + IL17A + REN + VEGFA | 0,77 |
| IFNG + GNLY + LAG3 + CXCL9 + PF4 + TSLP + REN | 0,77 |
| GZMH + IFNG + GNLY + LAG3 + IL17A + REN + IHH | 0,77 |
| IFNG + GNLY + LAG3 + IL17A + REN + PROM1 + VEGFA | 0,77 |
| CXCL13 + GNLY + LAG3 + ITGAE + CCL5 + PF4 + IL17A | 0,77 |
| GNLY + CCL5 + PF4 + REN + IHH + PROM1 + VEGFA | 0,77 |
| GZMH + CXCL13 + GNLY + CCL5 + IL17A + REN + IHH | 0,77 |
| IFNG + CXCL13 + GNLY + CXCL9 + IL17A + REN + IHH | 0,77 |
| IFNG + GNLY + LAG3 + ITGAE + PF4 + REN + VEGFA | 0,77 |
| IFNG + GNLY + CXCL9 + PF4 + REN + IHH + PROM1 | 0,77 |
| IFNG + GNLY + CXCL9 + IL17A + REN + PROM1 + VEGFA | 0,77 |
| IFNG + CXCL13 + GNLY + CXCL9 + IL17A + REN + PROM1 | 0,77 |
| IFNG + GNLY + IL17A + TSLP + REN + IHH + VEGFA | 0,77 |
| IFNG + GNLY + LAG3 + ITGAE + PF4 + REN + IHH | 0,77 |
| GZMH + CXCL13 + GNLY + ITGAE + CCL5 + PF4 + IL17A | 0,77 |
| GZMH + IFNG + CXCL13 + GNLY + CCL5 + IL17A + REN | 0,77 |
| IFNG + GNLY + ITGAE + CXCL9 + PF4 + TSLP + REN | 0,77 |
| IFNG + GNLY + LAG3 + IL17A + TSLP + REN + PROM1 | 0,77 |
| GZMH + IFNG + GNLY + IL17A + TSLP + REN + IHH | 0,77 |
| GNLY + LAG3 + ITGAE + CCL5 + IL17A + REN + IHH | 0,77 |
| CXCL13 + GNLY + ITGAE + CCL5 + PF4 + IL17A + VEGFA | 0,77 |
| GZMH + IFNG + GNLY + CXCL9 + PF4 + REN + PROM1 | 0,77 |
| GZMH + GNLY + ITGAE + CCL5 + CXCL9 + PF4 + REN | 0,77 |
| GNLY + LAG3 + CCL5 + CXCL9 + IL17A + REN + PROM1 | 0,77 |
| GZMH + IFNG + GNLY + LAG3 + IL17A + REN + PROM 1 | 0,77 |
| CXCL13 + GNLY + LAG3 + CCL5 + CXCL9 + IL17A + REN | 0,77 |
| GZMH + IFNG + GNLY + PF4 + REN + IHH + VEGFA | 0,77 |
| GZMH + IFNG + GNLY + LAG3 + ITGAE + PF4 + REN | 0,77 |
| GZMH + IFNG + GNLY + ITGAE + PF4 + REN + IHH | 0,77 |
| CXCL13 + GNLY + ITGAE + CCL5 + CXCL9 + PF4 + IL17A | 0,77 |
| GNLY + LAG3 + ITGAE + CCL5 + CXCL9 + PF4 + REN | 0,77 |
| GZMH + IFNG + GNLY + LAG3 + PF4 + REN + VEGFA | 0,77 |
| GZMH + GNLY + CCL5 + CXCL9 + PF4 + TSLP + REN | 0,77 |
| GZMH + IFNG + GNLY + ITGAE + PF4 + REN + VEGFA | 0,77 |
| GNLY + CCL5 + CXCL9 + PF4 + TSLP + REN + VEGFA | 0,76 |
| IFNG + CXCL13 + GNLY + CCL5 + IL17A + TSLP + REN | 0,76 |
| IFNG + GNLY + LAG3 + PF4 + REN + IHH + VEGFA | 0,76 |
| GZMH + IFNG + GNLY + CXCL9 + PF4 + TSLP + REN | 0,76 |
| GNLY + LAG3 + CCL5 + CXCL9 + PF4 + TSLP + REN | 0,76 |
| IFNG + GNLY + CXCL9 + IL17A + TSLP + REN + PROM1 | 0,76 |
| IFNG + GNLY + LAG3 + CXCL9 + PF4 + REN + VEGFA | 0,76 |
| IFNG + CXCL13 + GNLY + CCL5 + IL17A + REN + VEGFA | 0,76 |
| IFNG + GNLY + ITGAE + PF4 + REN + IHH + VEGFA | 0,76 |
| GZMH + IFNG + GNLY + LAG3 + PF4 + REN + IHH | 0,76 |
| GZMH + IFNG + CXCL13 + GNLY + IL17A + REN + IHH | 0,76 |
| GZMH + GNLY + LAG3 + ITGAE + CCL5 + IL17A + REN | 0,76 |
| GZMH + IFNG + GNLY + CCL5 + IL17A + REN + IHH | 0,76 |
| IFNG + CXCL13 + GNLY + ITGAE + CCL5 + IL17A + VEGFA | 0,76 |
| IFNG + GNLY + CXCL9 + PF4 + TSLP + REN + VEGFA | 0,76 |
| IFNG + GNLY + CCL5 + IL17A + REN + IHH + VEGFA | 0,76 |
| IFNG + GNLY + ITGAE + CCL5 + CXCL9 + IL17A + VEGFA | 0,76 |
| GNLY + ITGAE + CCL5 + IL17A + TSLP + REN + IHH | 0,76 |
| GZMH + GNLY + ITGAE + CCL5 + PF4 + REN + IHH | 0,76 |
| GZMH + GNLY + ITGAE + CCL5 + PF4 + REN + VEGFA | 0,76 |
| IFNG + GNLY + LAG3 + CXCL9 + PF4 + REN + IHH | 0,76 |
| GNLY + LAG3 + CCL5 + CXCL9 + IL17A + REN + IHH | 0,76 |
| GZMH + CXCL13 + GNLY + LAG3 + CCL5 + IL17A + REN | 0,76 |
| GNLY + CCL5 + CXCL9 + IL17A + REN + IHH + VEGFA | 0,76 |
| IFNG + GNLY + CXCL9 + PF4 + TSLP + REN + IHH | 0,76 |
| IFNG + CXCL13 + GNLY + IL17A + REN + IHH + PROM1 | 0,76 |
| IFNG + CXCL13 + GNLY + CXCL9 + IL17A + REN + VEGFA | 0,76 |
| GZMH + IFNG + CXCL13 + GNLY + CXCL9 + IL17A + REN | 0,76 |
| CXCL13 + GNLY + ITGAE + CCL5 + PF4 + IL17A + TSLP | 0,76 |
| CXCL13 + GNLY + CCL5 + IL17A + REN + IHH + PROM1 | 0,76 |
| IFNG + CXCL13 + GNLY + IL17A + TSLP + REN + IHH | 0,76 |
| IFNG + CXCL13 + GNLY + IL17A + REN + IHH + VEGFA | 0,76 |
| GNLY + ITGAE + CCL5 + IL17A + TSLP + REN + PROM1 | 0,76 |
| GZMH + IFNG + GNLY + LAG3 + CXCL9 + PF4 + REN | 0,76 |
| IFNG + GNLY + ITGAE + CCL5 + PF4 + IL17A + IHH | 0,76 |
| GZMH + IFNG + CXCL13 + GNLY + IL17A + REN + PROM1 | 0,76 |
| IFNG + CXCL13 + GNLY + IL 17A + REN + PROM1 + VEGFA | 0,76 |
| IFNG + CXCL13 + GNLY + IL17A + TSLP + REN + VEGFA | 0,76 |
| GZMH + GNLY + LAG3 + ITGAE + CCL5 + PF4 + REN | 0,76 |
| CXCL13 + GNLY + CCL5 + IL17A + REN + IHH + VEGFA | 0,76 |
| GNLY + LAG3 + ITGAE + CCL5 + PF4 + REN + VEGFA | 0,76 |
| IFNG + CXCL13 + GNLY + ITGAE + CCL5 + IL17A + PROM1 | 0,76 |
| GZMH + GNLY + CCL5 + PF4 + REN + PROM1 + VEGFA | 0,76 |
| GNLY + LAG3 + ITGAE + CCL5 + PF4 + REN + IHH | 0,76 |
| GNLY + ITGAE + CCL5 + PF4 + REN + IHH + VEGFA | 0,76 |
| GZMH + IFNG + GNLY + IL 17A + REN + IHH + PROM 1 | 0,76 |
| GZMH + IFNG + CXCL13 + GNLY + IL17A + REN + VEGFA | 0,76 |
| GZMH + IFNG + GNLY + IL17A + REN + IHH + VEGFA | 0,76 |
| IFNG + CXCL13 + GNLY + LAG3 + IL17A + REN + VEGFA | 0,76 |
| GZMH + IFNG + CXCL13 + GNLY + LAG3 + IL17A + REN | 0,76 |
| IFNG + CXCL13 + GNLY + LAG3 + IL17A + TSLP + REN | 0,76 |
| IFNG + GNLY + IL17A + REN + IHH + PROM1 + VEGFA | 0,76 |
| IFNG + CXCL13 + GNLY + LAG3 + IL 17A + REN + PROM 1 | 0,76 |
| GZMH + IFNG + CXCL13 + GNLY + CCL5 + PF4 + IL17A | 0,76 |
| GZMH + GNLY + CXCL9 + PF4 + TSLP + REN + PROM1 | 0,76 |
| GZMH + IFNG + CXCL13 + GNLY + IL17A + TSLP + REN | 0,76 |
| CXCL13 + GNLY + LAG3 + CCL5 + IL17A + TSLP + REN | 0,76 |
| IFNG + GNLY + ITGAE + CXCL9 + PF4 + REN + IHH | 0,76 |
| IFNG + GNLY + ITGAE + CXCL9 + PF4 + REN + VEGFA | 0,76 |
| IFNG + CXCL13 + GNLY + LAG3 + IL17A + REN + IHH | 0,76 |
| IFNG + CXCL13 + GNLY + IL17A + TSLP + REN + PROM1 | 0,76 |
| GZMH + IFNG + GNLY + IL17A + TSLP + REN + VEGFA | 0,76 |
| IFNG + CXCL13 + GNLY + ITGAE + CCL5 + CXCL9 + IL17A | 0,76 |
| IFNG + GNLY + IL17A + TSLP + REN + IHH + PROM1 | 0,76 |
| GZMH + GNLY + PF4 + TSLP + REN + IHH + PROM1 | 0,76 |
| GNLY + ITGAE + CCL5 + CXCL9 + PF4 + REN + VEGFA | 0,76 |
| IFNG + CXCL13 + GNLY + PF4 + TSLP + REN + PROM1 | 0,76 |
| GZMH + GNLY + PF4 + TSLP + REN + PROM1 + VEGFA | 0,76 |
| IFNG + CXCL13 + GNLY + LAG3 + ITGAE + CCL5 + IL17A | 0,76 |
| GZMH + CXCL13 + GNLY + CCL5 + PF4 + IL17A + PROM1 | 0,76 |
| CXCL13 + GNLY + LAG3 + CCL5 + IL17A + REN + VEGFA | 0,76 |
| GZMH + GNLY + LAG3 + CCL5 + CXCL9 + PF4 + REN | 0,76 |
| GZMH + IFNG + GNLY + CXCL9 + PF4 + REN + VEGFA | 0,76 |
| GNLY + LAG3 + CCL5 + CXCL9 + IL17A + TSLP + REN | 0,76 |
| GNLY + CCL5 + CXCL9 + IL17A + TSLP + REN + IHH | 0,76 |
| IFNG + GNLY + CXCL9 + PF4 + REN + IHH + VEGFA | 0,76 |
| IFNG + GNLY + IL17A + TSLP + REN + PROM1 + VEGFA | 0,76 |
| GZMH + IFNG + GNLY + CCL5 + IL17A + REN + VEGFA | 0,76 |
| IFNG + CXCL13 + GNLY + ITGAE + CCL5 + IL17A + TSLP | 0,76 |
| IFNG + CXCL13 + GNLY + ITGAE + PF4 + REN + PROM1 | 0,76 |
| GNLY + ITGAE + CCL5 + IL17A + REN + IHH + PROM1 | 0,76 |
| GNLY + LAG3 + CCL5 + CXCL9 + PF4 + REN + IHH | 0,76 |
| GZMH + IFNG + GNLY + ITGAE + CXCL9 + PF4 + REN | 0,76 |
| CXCL13 + GNLY + LAG3 + CCL5 + IL17A + REN + PROM1 | 0,76 |
| GNLY + LAG3 + CCL5 + CXCL9 + PF4 + REN + VEGFA | 0,76 |
| GZMH + IFNG + GNLY + CXCL9 + PF4 + REN + IHH | 0,76 |
| CXCL13 + GNLY + CCL5 + CXCL9 + PF4 + IL17A + IHH | 0,76 |
| IFNG + CXCL13 + GNLY + CCL5 + IL17A + REN + PROM1 | 0,76 |
| GZMH + CXCL13 + GNLY + CCL5 + CXCL9 + PF4 + IL17A | 0,76 |
| CXCL13 + GNLY + CCL5 + CXCL9 + PF4 + IL17A + PROM1 | 0,76 |
| GNLY + CCL5 + CXCL9 + PF4 + REN + IHH + VEGFA | 0,76 |
| IFNG + CXCL13 + GNLY + CXCL9 + IL17A + TSLP + REN | 0,76 |
| GZMH + GNLY + LAG3 + PF4 + TSLP + REN + PROM1 | 0,76 |
| CXCL13 + GNLY + ITGAE + CCL5 + PF4 + REN + PROM1 | 0,76 |
| GZMH + GNLY + ITGAE + PF4 + TSLP + REN + IHH | 0,76 |
| IFNG + CXCL13 + GNLY + CXCL9 + PF4 + REN + PROM1 | 0,76 |
| GNLY + ITGAE + PF4 + TSLP + REN + IHH + PROM1 | 0,76 |
| GNLY + ITGAE + CCL5 + IL17A + TSLP + REN + VEGFA | 0,76 |
| GZMH + GNLY + ITGAE + PF4 + TSLP + REN + VEGFA | 0,76 |
| GZMH + CXCL13 + GNLY + LAG3 + CCL5 + PF4 + IL17A | 0,76 |
| GZMH + CXCL13 + GNLY + CCL5 + PF4 + IL17A + VEGFA | 0,76 |
| GZMH + GNLY + ITGAE + PF4 + REN + IHH + PROM1 | 0,76 |
| IFNG + CXCL13 + GNLY + LAG3 + CXCL9 + IL17A + REN | 0,76 |
| GZMH + GNLY + CCL5 + CXCL9 + PF4 + REN + IHH | 0,76 |
| GNLY + ITGAE + PF4 + TSLP + REN + PROM1 + VEGFA | 0,76 |
| IFNG + GNLY + ITGAE + CCL5 + CXCL9 + IL17A + IHH | 0,76 |
| IFNG + GNLY + CCL5 + IL17A + REN + IHH + PROM1 | 0,76 |
| CXCL13 + GNLY + LAG3 + CCL5 + CXCL9 + PF4 + IL17A | 0,76 |
| IFNG + CXCL13 + GNLY + ITGAE + CCL5 + IL17A + IHH | 0,76 |
| GZMH + GNLY + LAG3 + ITGAE + PF4 + TSLP + REN | 0,76 |
| GZMH + IFNG + GNLY + IL17A + REN + PROM1 + VEGFA | 0,76 |
| GZMH + GNLY + LAG3 + ITGAE + PF4 + REN + PROM1 | 0,76 |
| GNLY + LAG3 + ITGAE + PF4 + TSLP + REN + PROM1 | 0,76 |
| IFNG + CXCL13 + GNLY + CCL5 + CXCL9 + PF4 + IL17A | 0,76 |
| IFNG + GNLY + CCL5 + IL17A + TSLP + REN + VEGFA | 0,76 |
| GZMH + GNLY + ITGAE + PF4 + REN + PROM1 + VEGFA | 0,76 |
| CXCL13 + GNLY + CCL5 + PF4 + IL17A + TSLP + PROM1 | 0,76 |
| GZMH + IFNG + GNLY + CCL5 + IL17A + TSLP + REN | 0,76 |
| GNLY + ITGAE + CXCL9 + PF4 + TSLP + REN + PROM1 | 0,76 |
| IFNG + CXCL13 + GNLY + CCL5 + PF4 + IL17A + IHH | 0,76 |
| GZMH + GNLY + ITGAE + CXCL9 + PF4 + REN + PROM1 | 0,76 |
| GZMH + GNLY + CCL5 + CXCL9 + PF4 + REN + VEGFA | 0,76 |
| GZMH + IFNG + GNLY + CCL5 + IL17A + REN + PROM1 | 0,76 |

| **Table 8: clusters of 8 genes:** | |
|---|---|
| **clusters of 8 genes** | **c_tau level** |
| IFNG + GNLY + ITGAE + CCL5 + PF4 + IL17A + REN + PROM1 | 0,8 |
| GZMH + IFNG + GNLY + ITGAE + CCL5 + PF4 + IL17A + REN | 0,8 |
| IFNG + GNLY + ITGAE + CCL5 + CXCL9 + PF4 + IL17A + REN | 0,8 |
| IFNG + GNLY + ITGAE + CCL5 + PF4 + IL17A + TSLP + REN | 0,79 |
| IFNG + GNLY + ITGAE + CCL5 + PF4 + IL17A + REN + VEGFA | 0,79 |
| IFNG + GNLY + LAG3 + ITGAE + CCL5 + PF4 + IL17A + REN | 0,79 |
| IFNG + GNLY + ITGAE + CCL5 + PF4 + IL17A + REN + IHH | 0,79 |
| IFNG + CXCL13 + GNLY + ITGAE + CCL5 + PF4 + IL17A + REN | 0,79 |
| GNLY + ITGAE + CCL5 + CXCL9 + PF4 + IL17A + REN + VEGFA | 0,79 |
| CXCL13 + GNLY + ITGAE + CCL5 + PF4 + IL17A + REN + PROM1 | 0,79 |
| IFNG + GNLY + LAG3 + CCL5 + PF4 + IL17A + REN + PROM1 | 0,79 |
| IFNG + GNLY + CCL5 + CXCL9 + PF4 + IL17A + REN + IHH | 0,79 |
| IFNG + GNLY + ITGAE + CCL5 + CXCL9 + IL17A + REN + VEGFA | 0,79 |
| IFNG + GNLY + LAG3 + CCL5 + CXCL9 + PF4 + IL17A + REN | 0,79 |
| GZMH + IFNG + GNLY + LAG3 + CCL5 + PF4 + IL17A + REN | 0,79 |
| GNLY + ITGAE + CCL5 + CXCL9 + PF4 + IL17A + REN + PROM1 | 0,79 |
| GZMH + IFNG + GNLY + CCL5 + PF4 + IL17A + REN + PROM1 | 0,79 |
| CXCL13 + GNLY + ITGAE + CCL5 + PF4 + IL17A + REN + IHH | 0,79 |
| GZMH + IFNG + GNLY + CCL5 + CXCL9 + PF4 + IL17A + REN | 0,79 |
| GZMH + IFNG + GNLY + LAG3 + ITGAE + CCL5 + IL17A + REN | 0,79 |
| GNLY + ITGAE + CCL5 + CXCL9 + PF4 + IL17A + TSLP + REN | 0,79 |
| GNLY + ITGAE + CCL5 + PF4 + IL17A + TSLP + REN + PROM1 | 0,79 |
| GNLY + LAG3 + ITGAE + CCL5 + PF4 + IL17A + REN + PROM1 | 0,79 |
| GNLY + ITGAE + CCL5 + PF4 + IL17A + REN + PROM1 + VEGFA | 0,79 |
| GZMH + IFNG + GNLY + CCL5 + PF4 + IL17A + REN + IHH | 0,79 |
| IFNG + GNLY + CCL5 + CXCL9 + PF4 + IL17A + REN + VEGFA | 0,79 |
| IFNG + GNLY + LAG3 + CCL5 + PF4 + IL17A + REN + IHH | 0,79 |
| IFNG + GNLY + LAG3 + ITGAE + CCL5 + IL17A + REN + PROM1 | 0,79 |
| IFNG + GNLY + LAG3 + ITGAE + CCL5 + IL17A + REN + VEGFA | 0,79 |
| IFNG + GNLY + LAG3 + CCL5 + PF4 + IL17A + TSLP + REN | 0,79 |
| GZMH + GNLY + ITGAE + CCL5 + CXCL9 + PF4 + IL17A + REN | 0,79 |
| CXCL13 + GNLY + ITGAE + CCL5 + CXCL9 + PF4 + IL17A + REN | 0,79 |
| IFNG + GNLY + LAG3 + ITGAE + CCL5 + IL17A + TSLP + REN | 0,79 |
| IFNG + GNLY + ITGAE + CCL5 + CXCL9 + IL17A + REN + IHH | 0,79 |
| IFNG + GNLY + LAG3 + ITGAE + CCL5 + CXCL9 + IL17A + REN | 0,79 |
| IFNG + GNLY + CCL5 + CXCL9 + PF4 + IL17A + TSLP + REN | 0,79 |
| GNLY + ITGAE + CCL5 + PF4 + IL17A + REN + IHH + PROM1 | 0,79 |
| IFNG + GNLY + ITGAE + CCL5 + CXCL9 + IL17A + TSLP + REN | 0,79 |
| CXCL13 + GNLY + ITGAE + CCL5 + PF4 + IL17A + REN + VEGFA | 0,79 |
| IFNG + GNLY + LAG3 + CCL5 + PF4 + IL17A + REN + VEGFA | 0,79 |
| GNLY + ITGAE + CCL5 + CXCL9 + PF4 + IL17A + REN + IHH | 0,79 |
| GZMH + GNLY + ITGAE + CCL5 + PF4 + IL17A + REN + PROM1 | 0,79 |
| GNLY + LAG3 + ITGAE + CCL5 + CXCL9 + PF4 + IL17A + REN | 0,79 |
| IFNG + GNLY + LAG3 + ITGAE + CCL5 + IL17A + REN + IHH | 0,79 |
| GZMH + IFNG + GNLY + ITGAE + CCL5 + CXCL9 + IL17A + REN | 0,79 |
| CXCL13 + GNLY + LAG3 + ITGAE + CCL5 + PF4 + IL17A + REN | 0,79 |
| CXCL13 + GNLY + ITGAE + CCL5 + PF4 + IL17A + TSLP + REN | 0,79 |
| IFNG + GNLY + ITGAE + CCL5 + CXCL9 + IL17A + REN + PROM1 | 0,79 |
| GZMH + IFNG + GNLY + CCL5 + PF4 + IL17A + REN + VEGFA | 0,79 |
| GZMH + IFNG + GNLY + CCL5 + PF4 + IL17A + TSLP + REN | 0,79 |
| GZMH + IFNG + GNLY + ITGAE + CCL5 + IL17A + REN + VEGFA | 0,79 |
| IFNG + GNLY + CCL5 + PF4 + IL17A + REN + IHH + PROM1 | 0,79 |
| GZMH + IFNG + GNLY + ITGAE + PF4 + IL17A + TSLP + REN | 0,78 |
| IFNG + GNLY + LAG3 + ITGAE + PF4 + IL17A + REN + PROM1 | 0,78 |
| IFNG + GNLY + LAG3 + ITGAE + CXCL9 + PF4 + IL17A + REN | 0,78 |
| IFNG + GNLY + CCL5 + PF4 + IL17A + TSLP + REN + IHH | 0,78 |
| IFNG + GNLY + CCL5 + PF4 + IL17A + TSLP + REN + PROM1 | 0,78 |
| GZMH + CXCL13 + GNLY + ITGAE + CCL5 + PF4 + IL17A + REN | 0,78 |
| GZMH + IFNG + GNLY + LAG3 + ITGAE + PF4 + IL17A + REN | 0,78 |
| IFNG + GNLY + CCL5 + PF4 + IL17A + REN + PROM1 + VEGFA | 0,78 |
| GZMH + IFNG + GNLY + ITGAE + CCL5 + IL17A + TSLP + REN | 0,78 |
| GZMH + IFNG + GNLY + ITGAE + CCL5 + IL17A + REN + PROM1 | 0,78 |
| CXCL13 + GNLY + CCL5 + CXCL9 + PF4 + IL17A + REN + IHH | 0,78 |
| GNLY + LAG3 + ITGAE + CCL5 + PF4 + IL17A + TSLP + REN | 0,78 |
| GZMH + CXCL13 + GNLY + CCL5 + PF4 + IL17A + REN + IHH | 0,78 |
| GZMH + IFNG + GNLY + ITGAE + PF4 + IL17A + REN + IHH | 0,78 |
| IFNG + GNLY + LAG3 + ITGAE + PF4 + IL17A + REN + IHH | 0,78 |
| IFNG + GNLY + LAG3 + ITGAE + PF4 + IL17A + TSLP + REN | 0,78 |
| IFNG + GNLY + CCL5 + CXCL9 + PF4 + IL17A + REN + PROM1 | 0,78 |
| IFNG + GNLY + ITGAE + PF4 + IL17A + TSLP + REN + VEGFA | 0,78 |
| IFNG + GNLY + LAG3 + ITGAE + PF4 + IL17A + REN + VEGFA | 0,78 |
| IFNG + GNLY + ITGAE + PF4 + IL17A + REN + IHH + PROM1 | 0,78 |
| GZMH + IFNG + GNLY + ITGAE + PF4 + IL17A + REN + VEGFA | 0,78 |
| IFNG + GNLY + CCL5 + PF4 + IL17A + REN + IHH + VEGFA | 0,78 |
| GZMH + IFNG + GNLY + ITGAE + PF4 + IL17A + REN + PROM1 | 0,78 |
| IFNG + GNLY + ITGAE + PF4 + IL17A + REN + PROM1 + VEGFA | 0,78 |
| IFNG + GNLY + ITGAE + PF4 + IL17A + REN + IHH + VEGFA | 0,78 |
| IFNG + GNLY + ITGAE + CCL5 + IL17A + TSLP + REN + PROM1 | 0,78 |
| IFNG + GNLY + ITGAE + PF4 + IL17A + TSLP + REN + PROM1 | 0,78 |
| GZMH + IFNG + GNLY + ITGAE + CCL5 + IL17A + REN + IHH | 0,78 |
| IFNG + GNLY + ITGAE + PF4 + IL17A + TSLP + REN + IHH | 0,78 |
| IFNG + GNLY + LAG3 + CXCL9 + PF4 + IL17A + REN + IHH | 0,78 |
| IFNG + GNLY + LAG3 + PF4 + IL17A + REN + IHH + PROM1 | 0,78 |
| IFNG + CXCL13 + GNLY + CCL5 + PF4 + IL17A + REN + IHH | 0,78 |
| GZMH + IFNG + GNLY + ITGAE + CXCL9 + PF4 + IL17A + REN | 0,78 |
| GNLY + LAG3 + ITGAE + CCL5 + PF4 + IL17A + REN + VEGFA | 0,78 |
| IFNG + GNLY + CCL5 + PF4 + IL17A + TSLP + REN + VEGFA | 0,78 |
| GNLY + LAG3 + CCL5 + CXCL9 + PF4 + IL17A + REN + VEGFA | 0,78 |
| IFNG + GNLY + ITGAE + CCL5 + IL17A + TSLP + REN + VEGFA | 0,78 |
| GZMH + IFNG + GNLY + PF4 + IL17A + REN + IHH + PROM1 | 0,78 |
| IFNG + GNLY + PF4 + IL17A + REN + IHH + PROM1 + VEGFA | 0,78 |
| IFNG + GNLY + ITGAE + CCL5 + IL17A + REN + PROM1 + VEGFA | 0,78 |
| IFNG + GNLY + ITGAE + CCL5 + IL17A + TSLP + REN + IHH | 0,78 |
| CXCL13 + GNLY + LAG3 + CCL5 + PF4 + IL17A + REN + IHH | 0,78 |
| GNLY + ITGAE + CCL5 + PF4 + IL17A + TSLP + REN + IHH | 0,78 |
| IFNG + GNLY + CXCL9 + PF4 + IL17A + REN + IHH + VEGFA | 0,78 |
| CXCL13 + GNLY + CCL5 + PF4 + IL17A + REN + IHH + PROM1 | 0,78 |
| IFNG + GNLY + ITGAE + CXCL9 + PF4 + IL17A + REN + VEGFA | 0,78 |
| GNLY + CCL5 + CXCL9 + PF4 + IL17A + REN + IHH + VEGFA | 0,78 |
| IFNG + CXCL13 + GNLY + CCL5 + CXCL9 + PF4 + IL17A + REN | 0,78 |
| GZMH + IFNG + GNLY + PF4 + IL17A + TSLP + REN + PROM1 | 0,78 |
| GZMH + IFNG + CXCL13 + GNLY + ITGAE + CCL5 + IL17A + REN | 0,78 |
| IFNG + GNLY + LAG3 + PF4 + IL17A + TSLP + REN + PROM1 | 0,78 |
| GZMH + IFNG + GNLY + LAG3 + PF4 + IL17A + REN + PROM1 | 0,78 |
| CXCL13 + GNLY + CCL5 + PF4 + IL17A + REN + IHH + VEGFA | 0,78 |
| IFNG + GNLY + PF4 + IL17A + TSLP + REN + IHH + PROM1 | 0,78 |
| GZMH + GNLY + ITGAE + CCL5 + PF4 + IL17A + TSLP + REN | 0,78 |
| IFNG + GNLY + LAG3 + PF4 + IL17A + REN + IHH + VEGFA | 0,78 |
| IFNG + GNLY + LAG3 + PF4 + IL17A + REN + PROM1 + VEGFA | 0,78 |
| CXCL13 + GNLY + CCL5 + PF4 + IL17A + TSLP + REN + IHH | 0,78 |
| GZMH + IFNG + GNLY + CXCL9 + PF4 + IL17A + REN + IHH | 0,78 |
| IFNG + GNLY + PF4 + IL17A + TSLP + REN + PROM1 + VEGFA | 0,78 |
| GZMH + IFNG + GNLY + LAG3 + PF4 + IL17A + REN + IHH | 0,78 |
| GZMH + IFNG + GNLY + CXCL9 + PF4 + IL17A + REN + VEGFA | 0,78 |
| GNLY + ITGAE + CCL5 + PF4 + IL17A + TSLP + REN + VEGFA | 0,78 |
| IFNG + CXCL13 + GNLY + ITGAE + CCL5 + IL17A + REN + PROM1 | 0,78 |
| GZMH + IFNG + CXCL13 + GNLY + CCL5 + PF4 + IL17A + REN | 0,78 |
| IFNG + GNLY + PF4 + IL17A + TSLP + REN + IHH + VEGFA | 0,78 |
| GZMH + IFNG + GNLY + PF4 + IL17A + REN + IHH + VEGFA | 0,78 |
| GZMH + IFNG + GNLY + LAG3 + PF4 + IL17A + TSLP + REN | 0,78 |
| IFNG + GNLY + ITGAE + CXCL9 + PF4 + IL17A + REN + PROM1 | 0,78 |
| IFNG + GNLY + LAG3 + PF4 + IL17A + TSLP + REN + IHH | 0,78 |
| GZMH + IFNG + GNLY + PF4 + IL17A + TSLP + REN + IHH | 0,78 |
| GZMH + IFNG + GNLY + PF4 + IL17A + TSLP + REN + VEGFA | 0,78 |
| IFNG + GNLY + ITGAE + CXCL9 + PF4 + IL17A + REN + IHH | 0,78 |
| GZMH + IFNG + GNLY + PF4 + IL17A + REN + PROM1 + VEGFA | 0,78 |
| IFNG + GNLY + LAG3 + CXCL9 + PF4 + IL17A + REN + PROM1 | 0,78 |
| IFNG + GNLY + LAG3 + CXCL9 + PF4 + IL17A + REN + VEGFA | 0,78 |
| GZMH + IFNG + GNLY + LAG3 + PF4 + IL17A + REN + VEGFA | 0,78 |
| IFNG + GNLY + ITGAE + CCL5 + IL17A + REN + IHH + VEGFA | 0,78 |
| IFNG + GNLY + LAG3 + PF4 + IL17A + TSLP + REN + VEGFA | 0,78 |
| CXCL13 + GNLY + LAG3 + CCL5 + CXCL9 + PF4 + IL17A + REN | 0,78 |
| GZMH + IFNG + GNLY + CXCL9 + PF4 + IL17A + REN + PROM1 | 0,78 |
| GNLY + CCL5 + CXCL9 + PF4 + IL17A + TSLP + REN + IHH | 0,78 |
| IFNG + GNLY + ITGAE + CCL5 + IL17A + REN + IHH + PROM1 | 0,78 |
| GNLY + CCL5 + CXCL9 + PF4 + IL17A + REN + IHH + PROM1 | 0,78 |
| GNLY + LAG3 + CCL5 + CXCL9 + PF4 + IL17A + REN + IHH | 0,78 |
| IFNG + CXCL13 + GNLY + ITGAE + CCL5 + CXCL9 + IL17A + REN | 0,78 |
| GZMH + GNLY + ITGAE + CCL5 + PF4 + IL17A + REN + VEGFA | 0,78 |
| GNLY + ITGAE + CCL5 + PF4 + IL17A + REN + IHH + VEGFA | 0,78 |
| IFNG + GNLY + CXCL9 + PF4 + IL17A + REN + IHH + PROM1 | 0,78 |
| GZMH + GNLY + LAG3 + ITGAE + CCL5 + PF4 + IL17A + REN | 0,78 |
| GNLY + LAG3 + CCL5 + CXCL9 + PF4 + IL17A + REN + PROM1 | 0,78 |
| IFNG + CXCL13 + GNLY + LAG3 + CCL5 + PF4 + IL17A + REN | 0,78 |
| IFNG + GNLY + CXCL9 + PF4 + IL17A + REN + PROM1 + VEGFA | 0,78 |
| CXCL13 + GNLY + CCL5 + CXCL9 + PF4 + IL17A + REN + VEGFA | 0,78 |
| IFNG + CXCL13 + GNLY + CCL5 + PF4 + IL17A + REN + PROM1 | 0,78 |
| GZMH + IFNG + GNLY + LAG3 + CXCL9 + PF4 + IL17A + REN | 0,78 |
| IFNG + CXCL13 + GNLY + LAG3 + ITGAE + CCL5 + IL17A + REN | 0,78 |
| CXCL13 + GNLY + CCL5 + CXCL9 + PF4 + IL17A + TSLP + REN | 0,78 |
| IFNG + CXCL13 + GNLY + ITGAE + CCL5 + IL17A + REN + VEGFA | 0,78 |
| IFNG + GNLY + LAG3 + CXCL9 + PF4 + IL17A + TSLP + REN | 0,78 |
| GNLY + LAG3 + ITGAE + CCL5 + PF4 + IL17A + REN + IHH | 0,78 |
| GZMH + GNLY + ITGAE + CCL5 + PF4 + IL17A + REN + IHH | 0,78 |
| GNLY + LAG3 + CCL5 + CXCL9 + PF4 + IL17A + TSLP + REN | 0,78 |
| IFNG + GNLY + CXCL9 + PF4 + IL17A + TSLP + REN + IHH | 0,78 |
| GZMH + CXCL13 + GNLY + CCL5 + CXCL9 + PF4 + IL17A + REN | 0,78 |
| CXCL13 + GNLY + LAG3 + CCL5 + PF4 + IL17A + REN + PROM1 | 0,78 |
| GZMH + IFNG + GNLY + CXCL9 + PF4 + IL17A + TSLP + REN | 0,78 |
| GZMH + CXCL13 + GNLY + CCL5 + PF4 + IL17A + REN + PROM1 | 0,78 |
| GZMH + GNLY + LAG3 + CCL5 + CXCL9 + PF4 + IL17A + REN | 0,78 |
| CXCL13 + GNLY + CCL5 + CXCL9 + PF4 + IL17A + REN + PROM1 | 0,78 |
| IFNG + GNLY + ITGAE + CXCL9 + PF4 + IL17A + TSLP + REN | 0,78 |
| GZMH + GNLY + CCL5 + CXCL9 + PF4 + IL17A + REN + IHH | 0,78 |
| CXCL13 + GNLY + CCL5 + PF4 + IL17A + REN + PROM1 + VEGFA | 0,78 |
| IFNG + CXCL13 + GNLY + ITGAE + CCL5 + IL17A + REN + IHH | 0,78 |
| GZMH + IFNG + CXCL13 + GNLY + ITGAE + PF4 + IL17A + REN | 0,78 |
| GNLY + LAG3 + CCL5 + PF4 + IL17A + REN + IHH + PROM1 | 0,78 |
| IFNG + CXCL13 + GNLY + ITGAE + CCL5 + IL17A + TSLP + REN | 0,78 |
| IFNG + GNLY + CXCL9 + PF4 + IL17A + TSLP + REN + PROM1 | 0,78 |
| IFNG + GNLY + CXCL9 + PF4 + IL17A + TSLP + REN + VEGFA | 0,78 |
| IFNG + CXCL13 + GNLY + CCL5 + PF4 + IL17A + REN + VEGFA | 0,78 |
| IFNG + CXCL13 + GNLY + CCL5 + PF4 + IL17A + TSLP + REN | 0,78 |
| GNLY + LAG3 + CCL5 + PF4 + IL17A + TSLP + REN + PROM1 | 0,78 |
| CXCL13 + GNLY + LAG3 + CCL5 + PF4 + IL17A + REN + VEGFA | 0,78 |
| GNLY + CCL5 + CXCL9 + PF4 + IL17A + TSLP + REN + PROM1 | 0,78 |
| CXCL13 + GNLY + LAG3 + CCL5 + PF4 + IL17A + TSLP + REN | 0,78 |
| GZMH + CXCL13 + GNLY + LAG3 + CCL5 + PF4 + IL17A + REN | 0,78 |
| IFNG + CXCL13 + GNLY + LAG3 + ITGAE + PF4 + IL17A + REN | 0,78 |
| CXCL13 + GNLY + CCL5 + PF4 + IL17A + TSLP + REN + PROM1 | 0,78 |
| GZMH + GNLY + CCL5 + CXCL9 + PF4 + IL17A + REN + VEGFA | 0,78 |
| GNLY + CCL5 + CXCL9 + PF4 + IL17A + TSLP + REN + VEGFA | 0,78 |
| GZMH + IFNG + GNLY + ITGAE + CCL5 + PF4 + REN + PROM1 | 0,78 |
| GZMH + IFNG + CXCL13 + GNLY + PF4 + IL17A + REN + PROM1 | 0,78 |
| GZMH + CXCL13 + GNLY + CCL5 + PF4 + IL17A + TSLP + REN | 0,78 |
| GZMH + IFNG + CXCL13 + GNLY + CXCL9 + PF4 + IL17A + REN | 0,78 |
| GNLY + CCL5 + CXCL9 + PF4 + IL17A + REN + PROM1 + VEGFA | 0,78 |
| CXCL13 + GNLY + CCL5 + PF4 + IL17A + TSLP + REN + VEGFA | 0,78 |
| GZMH + IFNG + CXCL 13 + GNLY + PF4 + IL17A + REN + IHH | 0,78 |
| IFNG + CXCL13 + GNLY + ITGAE + PF4 + IL17A + TSLP + REN | 0,78 |
| GZMH + CXCL13 + GNLY + CCL5 + PF4 + IL17A + REN + VEGFA | 0,78 |
| IFNG + CXCL13 + GNLY + ITGAE + PF4 + IL17A + REN + PROM1 | 0,78 |
| IFNG + CXCL13 + GNLY + CXCL9 + PF4 + IL17A + REN + PROM1 | 0,78 |
| IFNG + GNLY + ITGAE + CCL5 + PF4 + TSLP + REN + PROM1 | 0,78 |
| GZMH + IFNG + CXCL13 + GNLY + PF4 + IL17A + REN + VEGFA | 0,78 |
| IFNG + CXCL13 + GNLY + PF4 + IL17A + REN + PROM1 + VEGFA | 0,78 |
| IFNG + CXCL13 + GNLY + ITGAE + CXCL9 + PF4 + IL17A + REN | 0,78 |
| IFNG + CXCL13 + GNLY + ITGAE + PF4 + IL17A + REN + VEGFA | 0,78 |
| IFNG + CXCL13 + GNLY + LAG3 + PF4 + IL17A + REN + PROM1 | 0,78 |
| GZMH + GNLY + CCL5 + CXCL9 + PF4 + IL17A + TSLP + REN | 0,78 |
| IFNG + CXCL13 + GNLY + PF4 + IL17A + REN + IHH + PROM1 | 0,78 |
| GNLY + LAG3 + CCL5 + PF4 + IL17A + REN + PROM1 + VEGFA | 0,78 |
| GZMH + IFNG + CXCL13 + GNLY + PF4 + IL17A + TSLP + REN | 0,78 |
| IFNG + CXCL13 + GNLY + ITGAE + PF4 + IL17A + REN + IHH | 0,78 |
| IFNG + CXCL13 + GNLY + CXCL9 + PF4 + IL17A + REN + VEGFA | 0,78 |
| IFNG + CXCL 13 + GNLY + LAG3 + PF4 + IL17A + REN + IHH | 0,78 |
| IFNG + CXCL13 + GNLY + CXCL9 + PF4 + IL17A + REN + IHH | 0,78 |
| IFNG + CXCL13 + GNLY + PF4 + IL17A + TSLP + REN + PROM1 | 0,78 |
| IFNG + CXCL13 + GNLY + PF4 + IL17A + REN + IHH + VEGFA | 0,78 |
| GZMH + GNLY + CCL5 + CXCL9 + PF4 + IL17A + REN + PROM1 | 0,78 |
| GNLY + LAG3 + CCL5 + PF4 + IL17A + TSLP + REN + IHH | 0,78 |
| IFNG + CXCL13 + GNLY + PF4 + IL17A + TSLP + REN + IHH | 0,78 |
| IFNG + CXCL13 + GNLY + PF4 + IL17A + TSLP + REN + VEGFA | 0,78 |
| IFNG + CXCL13 + GNLY + CXCL9 + PF4 + IL17A + TSLP + REN | 0,78 |
| GNLY + CCL5 + PF4 + IL17A + TSLP + REN + IHH + PROM1 | 0,78 |
| GZMH + GNLY + LAG3 + CCL5 + PF4 + IL17A + REN + PROM1 | 0,78 |
| IFNG + CXCL13 + GNLY + LAG3 + CXCL9 + PF4 + IL17A + REN | 0,78 |
| GZMH + IFNG + CXCL13 + GNLY + LAG3 + PF4 + IL17A + REN | 0,78 |
| GNLY + LAG3 + CCL5 + PF4 + IL17A + REN + IHH + VEGFA | 0,78 |
| IFNG + CXCL13 + GNLY + LAG3 + PF4 + IL17A + REN + VEGFA | 0,78 |
| GNLY + LAG3 + CCL5 + PF4 + IL17A + TSLP + REN + VEGFA | 0,78 |
| GZMH + GNLY + CCL5 + PF4 + IL17A + REN + IHH + PROM1 | 0,78 |
| IFNG + CXCL13 + GNLY + LAG3 + PF4 + IL17A + TSLP + REN | 0,78 |
| GZMH + IFNG + GNLY + ITGAE + CCL5 + PF4 + TSLP + REN | 0,78 |
| GZMH + GNLY + LAG3 + CCL5 + PF4 + IL17A + TSLP + REN | 0,78 |
| IFNG + GNLY + ITGAE + CCL5 + PF4 + REN + PROM1 + VEGFA | 0,78 |
| GZMH + CXCL13 + GNLY + LAG3 + ITGAE + PF4 + IL17A + REN | 0,78 |
| GNLY + CCL5 + PF4 + IL17A + REN + IHH + PROM1 + VEGFA | 0,78 |
| IFNG + GNLY + ITGAE + CCL5 + PF4 + REN + IHH + PROM1 | 0,78 |
| GZMH + CXCL13 + GNLY + LAG3 + PF4 + IL17A + REN + IHH | 0,78 |
| GZMH + GNLY + ITGAE + PF4 + IL17A + TSLP + REN + IHH | 0,78 |
| GZMH + GNLY + ITGAE + PF4 + IL17A + TSLP + REN + PROM1 | 0,78 |
| GZMH + GNLY + ITGAE + CXCL9 + PF4 + IL17A + TSLP + REN | 0,78 |
| GZMH + GNLY + LAG3 + CCL5 + PF4 + IL17A + REN + IHH | 0,78 |
| GZMH + GNLY + CXCL9 + PF4 + IL17A + TSLP + REN + IHH | 0,78 |
| CXCL13 + GNLY + LAG3 + PF4 + IL17A + TSLP + REN + IHH | 0,78 |
| GZMH + IFNG + GNLY + LAG3 + CCL5 + CXCL9 + IL17A + REN | 0,78 |
| GZMH + GNLY + ITGAE + PF4 + IL17A + TSLP + REN + VEGFA | 0,78 |
| GZMH + CXCL13 + GNLY + PF4 + IL17A + TSLP + REN + IHH | 0,78 |
| GZMH + CXCL13 + GNLY + ITGAE + PF4 + IL17A + REN + IHH | 0,78 |
| CXCL13 + GNLY + LAG3 + PF4 + IL17A + REN + IHH + PROM1 | 0,78 |
| IFNG + GNLY + LAG3 + ITGAE + CCL5 + PF4 + REN + PROM1 | 0,78 |
| GZMH + GNLY + LAG3 + ITGAE + CXCL9 + PF4 + IL17A + REN | 0,78 |
| CXCL13 + GNLY + LAG3 + PF4 + IL17A + REN + IHH + VEGFA | 0,78 |
| GZMH + CXCL13 + GNLY + PF4 + IL17A + REN + IHH + PROM1 | 0,78 |
| GZMH + IFNG + GNLY + LAG3 + CCL5 + PF4 + REN + PROM1 | 0,78 |
| GZMH + GNLY + LAG3 + ITGAE + PF4 + IL17A + TSLP + REN | 0,78 |
| IFNG + GNLY + ITGAE + CCL5 + PF4 + TSLP + REN + IHH | 0,78 |
| GNLY + ITGAE + CCL5 + PF4 + TSLP + REN + IHH + PROM1 | 0,78 |
| CXCL13 + GNLY + LAG3 + ITGAE + PF4 + IL17A + REN + IHH | 0,78 |
| GZMH + CXCL13 + GNLY + LAG3 + PF4 + IL17A + TSLP + REN | 0,78 |
| GZMH + CXCL13 + GNLY + LAG3 + PF4 + IL17A + REN + VEGFA | 0,78 |
| GZMH + GNLY + ITGAE + CXCL9 + PF4 + IL17A + REN + VEGFA | 0,78 |
| GZMH + CXCL13 + GNLY + CXCL9 + PF4 + IL17A + REN + IHH | 0,78 |
| GZMH + CXCL13 + GNLY + LAG3 + CXCL9 + PF4 + IL17A + REN | 0,78 |
| GZMH + GNLY + ITGAE + CXCL9 + PF4 + IL17A + REN + IHH | 0,78 |
| GNLY + ITGAE + CCL5 + CXCL9 + IL17A + REN + PROM1 + VEGFA | 0,78 |
| GZMH + CXCL13 + GNLY + PF4 + IL17A + REN + IHH + VEGFA | 0,78 |
| GZMH + GNLY + CXCL9 + PF4 + IL17A + REN + IHH + VEGFA | 0,78 |
| GNLY + LAG3 + ITGAE + CCL5 + CXCL9 + IL17A + REN + VEGFA | 0,78 |
| GZMH + CXCL13 + GNLY + ITGAE + PF4 + IL17A + TSLP + REN | 0,78 |
| GZMH + GNLY + CXCL9 + PF4 + IL17A + REN + IHH + PROM1 | 0,78 |
| IFNG + GNLY + LAG3 + CCL5 + CXCL9 + IL17A + REN + VEGFA | 0,78 |
| GZMH + GNLY + ITGAE + PF4 + IL17A + REN + IHH + PROM1 | 0,78 |
| CXCL13 + GNLY + LAG3 + CXCL9 + PF4 + IL17A + REN + IHH | 0,78 |
| IFNG + GNLY + LAG3 + CCL5 + CXCL9 + IL17A + REN + IHH | 0,78 |
| IFNG + GNLY + LAG3 + CCL5 + CXCL9 + IL17A + TSLP + REN | 0,78 |
| GZMH + GNLY + LAG3 + CCL5 + PF4 + IL17A + REN + VEGFA | 0,78 |
| IFNG + GNLY + LAG3 + ITGAE + CCL5 + PF4 + TSLP + REN | 0,78 |
| GZMH + GNLY + CCL5 + PF4 + IL17A + TSLP + REN + IHH | 0,78 |
| GNLY + ITGAE + CCL5 + CXCL9 + PF4 + TSLP + REN + PROM1 | 0,78 |
| GZMH + IFNG + GNLY + LAG3 + ITGAE + CCL5 + PF4 + REN | 0,78 |
| GZMH + IFNG + GNLY + ITGAE + CCL5 + PF4 + REN + IHH | 0,78 |
| GZMH + GNLY + ITGAE + CXCL9 + PF4 + IL17A + REN + PROM1 | 0,78 |
| GZMH + CXCL13 + GNLY + PF4 + IL17A + TSLP + REN + VEGFA | 0,78 |
| GNLY + CCL5 + PF4 + IL17A + TSLP + REN + IHH + VEGFA | 0,78 |
| GZMH + IFNG + GNLY + CCL5 + PF4 + TSLP + REN + PROM1 | 0,78 |
| IFNG + GNLY + ITGAE + CCL5 + PF4 + TSLP + REN + VEGFA | 0,78 |
| GZMH + GNLY + CXCL9 + PF4 + IL17A + TSLP + REN + PROM1 | 0,78 |
| GZMH + GNLY + ITGAE + PF4 + IL17A + REN + IHH + VEGFA | 0,78 |
| GNLY + ITGAE + CCL5 + CXCL9 + IL17A + TSLP + REN + VEGFA | 0,78 |
| GZMH + GNLY + LAG3 + CXCL9 + PF4 + IL17A + REN + IHH | 0,77 |
| GZMH + CXCL13 + GNLY + PF4 + IL17A + TSLP + REN + PROM1 | 0,77 |
| GZMH + IFNG + GNLY + ITGAE + CCL5 + PF4 + REN + VEGFA | 0,77 |
| GZMH + CXCL13 + GNLY + CXCL9 + PF4 + IL17A + TSLP + REN | 0,77 |
| GZMH + CXCL13 + GNLY + ITGAE + CXCL9 + PF4 + IL17A + REN | 0,77 |
| GZMH + GNLY + CXCL9 + PF4 + IL17A + TSLP + REN + VEGFA | 0,77 |
| GZMH + CXCL13 + GNLY + ITGAE + PF4 + IL17A + REN + VEGFA | 0,77 |
| GNLY + ITGAE + CCL5 + PF4 + TSLP + REN + PROM1 + VEGFA | 0,77 |
| GZMH + GNLY + ITGAE + CCL5 + PF4 + TSLP + REN + PROM1 | 0,77 |
| GZMH + CXCL13 + GNLY + LAG3 + PF4 + IL17A + REN + PROM1 | 0,77 |
| IFNG + GNLY + LAG3 + CCL5 + CXCL9 + IL17A + REN + PROM1 | 0,77 |
| GZMH + GNLY + CCL5 + PF4 + IL17A + TSLP + REN + PROM1 | 0,77 |
| GZMH + CXCL13 + GNLY + ITGAE + PF4 + IL17A + REN + PROM1 | 0,77 |
| GZMH + IFNG + GNLY + LAG3 + CCL5 + IL17A + REN + PROM1 | 0,77 |
| GZMH + GNLY + LAG3 + CXCL9 + PF4 + IL17A + TSLP + REN | 0,77 |
| GNLY + ITGAE + CCL5 + CXCL9 + IL17A + REN + IHH + VEGFA | 0,77 |
| GZMH + IFNG + GNLY + CCL5 + PF4 + REN + PROM1 + VEGFA | 0,77 |
| GZMH + GNLY + LAG3 + ITGAE + PF4 + IL17A + REN + VEGFA | 0,77 |
| GZMH + IFNG + GNLY + CCL5 + PF4 + REN + IHH + PROM1 | 0,77 |
| IFNG + GNLY + LAG3 + CCL5 + PF4 + TSLP + REN + PROM1 | 0,77 |
| GZMH + GNLY + LAG3 + ITGAE + PF4 + IL17A + REN + IHH | 0,77 |
| GZMH + GNLY + CXCL9 + PF4 + IL17A + REN + PROM1 + VEGFA | 0,77 |
| GZMH + CXCL13 + GNLY + CXCL9 + PF4 + IL17A + REN + PROM1 | 0,77 |
| CXCL13 + GNLY + LAG3 + ITGAE + CCL5 + IL17A + REN + PROM1 | 0,77 |
| GZMH + CXCL13 + GNLY + CXCL9 + PF4 + IL17A + REN + VEGFA | 0,77 |
| GNLY + CCL5 + PF4 + IL17A + TSLP + REN + PROM1 + VEGFA | 0,77 |
| GZMH + GNLY + ITGAE + PF4 + IL17A + REN + PROM1 + VEGFA | 0,77 |
| CXCL13 + GNLY + LAG3 + PF4 + IL17A + TSLP + REN + VEGFA | 0,77 |
| CXCL13 + GNLY + LAG3 + ITGAE + PF4 + IL17A + TSLP + REN | 0,77 |
| GNLY + LAG3 + CXCL9 + PF4 + IL17A + REN + IHH + PROM1 | 0,77 |
| GZMH + GNLY + LAG3 + ITGAE + PF4 + IL17A + REN + PROM1 | 0,77 |
| GZMH + GNLY + PF4 + IL17A + TSLP + REN + IHH + PROM1 | 0,77 |
| GZMH + CXCL13 + GNLY + PF4 + IL17A + REN + PROM1 + VEGFA | 0,77 |
| CXCL13 + GNLY + LAG3 + PF4 + IL17A + TSLP + REN + PROM1 | 0,77 |
| CXCL13 + GNLY + ITGAE + CCL5 + CXCL9 + IL17A + REN + VEGFA | 0,77 |
| GZMH + GNLY + LAG3 + CXCL9 + PF4 + IL17A + REN + PROM1 | 0,77 |
| GZMH + GNLY + LAG3 + PF4 + IL17A + REN + IHH + PROM1 | 0,77 |
| GZMH + GNLY + CCL5 + PF4 + IL17A + REN + IHH + VEGFA | 0,77 |
| GNLY + ITGAE + PF4 + IL17A + REN + IHH + PROM1 + VEGFA | 0,77 |
| GNLY + LAG3 + ITGAE + CCL5 + PF4 + TSLP + REN + PROM1 | 0,77 |
| GZMH + GNLY + LAG3 + CXCL9 + PF4 + IL17A + REN + VEGFA | 0,77 |
| GNLY + LAG3 + CXCL9 + PF4 + IL17A + REN + IHH + VEGFA | 0,77 |
| GNLY + CXCL9 + PF4 + IL17A + REN + IHH + PROM1 + VEGFA | 0,77 |
| CXCL13 + GNLY + LAG3 + PF4 + IL17A + REN + PROM1 + VEGFA | 0,77 |
| CXCL13 + GNLY + LAG3 + ITGAE + PF4 + IL17A + REN + VEGFA | 0,77 |
| GZMH + GNLY + ITGAE + CCL5 + CXCL9 + IL17A + REN + VEGFA | 0,77 |
| IFNG + GNLY + CCL5 + CXCL9 + IL17A + REN + IHH + VEGFA | 0,77 |
| CXCL13 + GNLY + LAG3 + CXCL9 + PF4 + IL17A + TSLP + REN | 0,77 |
| CXCL13 + GNLY + LAG3 + CXCL9 + PF4 + IL17A + REN + VEGFA | 0,77 |
| GNLY + LAG3 + CXCL9 + PF4 + IL17A + TSLP + REN + IHH | 0,77 |
| GZMH + GNLY + LAG3 + PF4 + IL17A + TSLP + REN + IHH | 0,77 |
| GNLY + LAG3 + ITGAE + PF4 + IL17A + REN + IHH + PROM1 | 0,77 |
| GNLY + LAG3 + ITGAE + CXCL9 + PF4 + IL17A + REN + IHH | 0,77 |
| CXCL13 + GNLY + LAG3 + ITGAE + CXCL9 + PF4 + IL17A + REN | 0,77 |
| GZMH + IFNG + GNLY + LAG3 + CCL5 + IL17A + TSLP + REN | 0,77 |
| CXCL13 + GNLY + LAG3 + ITGAE + PF4 + IL17A + REN + PROM1 | 0,77 |
| GZMH + GNLY + CCL5 + PF4 + IL17A + REN + PROM1 + VEGFA | 0,77 |
| GNLY + CXCL9 + PF4 + IL17A + TSLP + REN + IHH + PROM1 | 0,77 |
| GNLY + LAG3 + ITGAE + CCL5 + CXCL9 + IL17A + REN + PROM1 | 0,77 |
| IFNG + GNLY + CCL5 + PF4 + TSLP + REN + PROM1 + VEGFA | 0,77 |
| CXCL13 + GNLY + LAG3 + ITGAE + CCL5 + IL17A + REN + IHH | 0,77 |
| CXCL13 + GNLY + ITGAE + CCL5 + IL17A + REN + IHH + PROM1 | 0,77 |
| GZMH + GNLY + LAG3 + PF4 + IL17A + TSLP + REN + PROM1 | 0,77 |
| GNLY + PF4 + IL17A + TSLP + REN + IHH + PROM1 + VEGFA | 0,77 |
| GNLY + ITGAE + CXCL9 + PF4 + IL17A + REN + IHH + PROM1 | 0,77 |
| GNLY + ITGAE + PF4 + IL17A + TSLP + REN + IHH + PROM1 | 0,77 |
| GZMH + CXCL13 + GNLY + ITGAE + CCL5 + IL17A + REN + PROM1 | 0,77 |
| GZMH + GNLY + PF4 + IL17A + TSLP + REN + IHH + VEGFA | 0,77 |
| GNLY + LAG3 + PF4 + IL17A + TSLP + REN + IHH + PROM1 | 0,77 |
| CXCL13 + GNLY + LAG3 + CXCL9 + PF4 + IL17A + REN + PROM1 | 0,77 |
| GZMH + GNLY + PF4 + IL17A + REN + IHH + PROM1 + VEGFA | 0,77 |
| GNLY + LAG3 + ITGAE + PF4 + IL17A + TSLP + REN + IHH | 0,77 |
| CXCL13 + GNLY + ITGAE + CCL5 + CXCL9 + IL17A + REN + PROM1 | 0,77 |
| GNLY + LAG3 + PF4 + IL17A + REN + IHH + PROM1 + VEGFA | 0,77 |
| GZMH + IFNG + GNLY + ITGAE + IL17A + TSLP + REN + IHH | 0,77 |
| GNLY + ITGAE + CXCL9 + PF4 + IL17A + REN + IHH + VEGFA | 0,77 |
| CXCL13 + GNLY + CXCL9 + PF4 + IL17A + TSLP + REN + PROM1 | 0,77 |
| GNLY + CXCL9 + PF4 + IL17A + TSLP + REN + IHH + VEGFA | 0,77 |
| CXCL13 + GNLY + LAG3 + ITGAE + CCL5 + IL17A + REN + VEGFA | 0,77 |
| GNLY + ITGAE + PF4 + IL17A + TSLP + REN + IHH + VEGFA | 0,77 |
| CXCL13 + GNLY + ITGAE + CCL5 + IL17A + TSLP + REN + PROM1 | 0,77 |
| CXCL13 + GNLY + ITGAE + CCL5 + IL17A + TSLP + REN + IHH | 0,77 |
| CXCL13 + GNLY + ITGAE + CXCL9 + PF4 + IL17A + REN + IHH | 0,77 |
| GZMH + IFNG + GNLY + ITGAE + CCL5 + CXCL9 + PF4 + REN | 0,77 |
| GZMH + IFNG + GNLY + LAG3 + CCL5 + IL17A + REN + IHH | 0,77 |
| CXCL13 + GNLY + CXCL9 + PF4 + IL17A + REN + IHH + VEGFA | 0,77 |
| GNLY + LAG3 + ITGAE + CCL5 + IL17A + REN + PROM1 + VEGFA | 0,77 |
| CXCL 13 + GNLY + ITGAE + PF4 + IL17A + REN + IHH + VEGFA | 0,77 |
| IFNG + GNLY + CCL5 + PF4 + TSLP + REN + IHH + PROM1 | 0,77 |
| CXCL13 + GNLY + LAG3 + ITGAE + CCL5 + CXCL9 + IL17A + REN | 0,77 |
| IFNG + GNLY + LAG3 + ITGAE + CCL5 + PF4 + REN + IHH | 0,77 |
| CXCL13 + GNLY + PF4 + IL17A + REN + IHH + PROM1 + VEGFA | 0,77 |
| GZMH + IFNG + GNLY + LAG3 + CCL5 + IL17A + REN + VEGFA | 0,77 |
| CXCL13 + GNLY + ITGAE + CCL5 + IL17A + REN + IHH + VEGFA | 0,77 |
| CXCL13 + GNLY + ITGAE + CCL5 + CXCL9 + IL17A + REN + IHH | 0,77 |
| CXCL13 + GNLY + ITGAE + CCL5 + IL17A + REN + PROM1 + VEGFA | 0,77 |
| CXCL13 + GNLY + CXCL9 + PF4 + IL17A + TSLP + REN + IHH | 0,77 |
| CXCL13 + GNLY + CXCL9 + PF4 + IL17A + REN + IHH + PROM1 | 0,77 |
| IFNG + GNLY + CCL5 + CXCL9 + IL17A + TSLP + REN + IHH | 0,77 |
| GZMH + IFNG + GNLY + CCL5 + CXCL9 + IL17A + REN + IHH | 0,77 |
| GNLY + ITGAE + CCL5 + CXCL9 + PF4 + REN + IHH + PROM1 | 0,77 |
| IFNG + GNLY + LAG3 + CCL5 + IL17A + TSLP + REN + PROM1 | 0,77 |
| CXCL13 + GNLY + CXCL9 + PF4 + IL17A + REN + PROM1 + VEGFA | 0,77 |
| IFNG + GNLY + LAG3 + CCL5 + IL17A + TSLP + REN + IHH | 0,77 |
| IFNG + GNLY + LAG3 + CCL5 + PF4 + REN + PROM1 + VEGFA | 0,77 |
| GZMH + IFNG + CXCL13 + GNLY + LAG3 + CCL5 + IL17A + REN | 0,77 |
| GNLY + LAG3 + ITGAE + CCL5 + IL17A + TSLP + REN + PROM1 | 0,77 |
| CXCL13 + GNLY + ITGAE + PF4 + IL17A + REN + IHH + PROM1 | 0,77 |
| IFNG + GNLY + ITGAE + CCL5 + CXCL9 + PF4 + TSLP + REN | 0,77 |
| CXCL13 + GNLY + ITGAE + CXCL9 + PF4 + IL17A + TSLP + REN | 0,77 |
| CXCL13 + GNLY + CXCL9 + PF4 + IL17A + TSLP + REN + VEGFA | 0,77 |
| CXCL13 + GNLY + PF4 + IL17A + TSLP + REN + IHH + VEGFA | 0,77 |
| GNLY + LAG3 + ITGAE + PF4 + IL17A + TSLP + REN + PROM1 | 0,77 |
| IFNG + GNLY + LAG3 + ITGAE + CCL5 + PF4 + REN + VEGFA | 0,77 |
| GZMH + IFNG + GNLY + LAG3 + ITGAE + IL17A + TSLP + REN | 0,77 |
| IFNG + GNLY + ITGAE + CCL5 + CXCL9 + PF4 + REN + PROM1 | 0,77 |
| CXCL13 + GNLY + PF4 + IL17A + TSLP + REN + IHH + PROM1 | 0,77 |
| IFNG + GNLY + ITGAE + CCL5 + PF4 + REN + IHH + VEGFA | 0,77 |
| GNLY + LAG3 + ITGAE + PF4 + IL17A + REN + IHH + VEGFA | 0,77 |
| GNLY + ITGAE + CXCL9 + PF4 + IL17A + TSLP + REN + IHH | 0,77 |
| GZMH + IFNG + GNLY + ITGAE + IL17A + TSLP + REN + VEGFA | 0,77 |
| CXCL13 + GNLY + ITGAE + PF4 + IL17A + TSLP + REN + IHH | 0,77 |
| IFNG + GNLY + LAG3 + ITGAE + IL17A + TSLP + REN + IHH | 0,77 |
| IFNG + GNLY + LAG3 + CCL5 + IL17A + REN + IHH + VEGFA | 0,77 |
| GZMH + GNLY + CCL5 + PF4 + IL17A + TSLP + REN + VEGFA | 0,77 |
| GZMH + CXCL13 + GNLY + ITGAE + CCL5 + IL17A + REN + IHH | 0,77 |
| IFNG + GNLY + LAG3 + CCL5 + PF4 + REN + IHH + PROM1 | 0,77 |
| IFNG + CXCL13 + GNLY + LAG3 + CCL5 + CXCL9 + IL17A + REN | 0,77 |
| GZMH + IFNG + GNLY + ITGAE + CXCL9 + IL17A + REN + IHH | 0,77 |
| CXCL13 + GNLY + ITGAE + CXCL9 + PF4 + IL17A + REN + VEGFA | 0,77 |
| GZMH + GNLY + PF4 + IL17A + TSLP + REN + PROM1 + VEGFA | 0,77 |
| CXCL13 + GNLY + ITGAE + CXCL9 + PF4 + IL17A + REN + PROM1 | 0,77 |
| GNLY + ITGAE + PF4 + IL17A + TSLP + REN + PROM1 + VEGFA | 0,77 |
| GNLY + LAG3 + ITGAE + CXCL9 + PF4 + IL17A + TSLP + REN | 0,77 |
| GZMH + GNLY + LAG3 + PF4 + IL17A + TSLP + REN + VEGFA | 0,77 |
| GZMH + GNLY + LAG3 + ITGAE + CCL5 + PF4 + REN + PROM1 | 0,77 |
| GNLY + LAG3 + ITGAE + CCL5 + CXCL9 + PF4 + REN + PROM1 | 0,77 |
| GNLY + LAG3 + ITGAE + CXCL9 + PF4 + IL17A + REN + VEGFA | 0,77 |
| IFNG + GNLY + ITGAE + IL17A + TSLP + REN + IHH + VEGFA | 0,77 |
| GNLY + LAG3 + PF4 + IL17A + TSLP + REN + IHH + VEGFA | 0,77 |
| IFNG + GNLY + LAG3 + CCL5 + IL17A + REN + PROM1 + VEGFA | 0,77 |
| GNLY + LAG3 + ITGAE + CCL5 + PF4 + REN + IHH + PROM1 | 0,77 |
| GNLY + LAG3 + ITGAE + PF4 + IL17A + TSLP + REN + VEGFA | 0,77 |
| GNLY + ITGAE + CCL5 + CXCL9 + IL17A + TSLP + REN + PROM1 | 0,77 |
| GNLY + LAG3 + CXCL9 + PF4 + IL17A + TSLP + REN + PROM1 | 0,77 |
| GNLY + ITGAE + CCL5 + CXCL9 + IL17A + TSLP + REN + IHH | 0,77 |
| GNLY + CXCL9 + PF4 + IL17A + TSLP + REN + PROM1 + VEGFA | 0,77 |
| GNLY + LAG3 + ITGAE + CCL5 + PF4 + REN + PROM1 + VEGFA | 0,77 |
| GNLY + ITGAE + CCL5 + PF4 + REN + IHH + PROM1 + VEGFA | 0,77 |
| GNLY + CCL5 + CXCL9 + PF4 + TSLP + REN + IHH + PROM1 | 0,77 |
| GZMH + CXCL13 + GNLY + ITGAE + CCL5 + IL17A + REN + VEGFA | 0,77 |
| GNLY + LAG3 + CXCL9 + PF4 + IL17A + REN + PROM1 + VEGFA | 0,77 |
| GNLY + LAG3 + ITGAE + CXCL9 + PF4 + IL17A + REN + PROM1 | 0,77 |
| GZMH + GNLY + LAG3 + PF4 + IL17A + REN + PROM1 + VEGFA | 0,77 |
| GZMH + GNLY + LAG3 + PF4 + IL17A + REN + IHH + VEGFA | 0,77 |
| GNLY + LAG3 + CXCL9 + PF4 + IL17A + TSLP + REN + VEGFA | 0,77 |
| IFNG + GNLY + LAG3 + ITGAE + CCL5 + CXCL9 + PF4 + REN | 0,77 |
| GNLY + LAG3 + ITGAE + PF4 + IL17A + REN + PROM1 + VEGFA | 0,77 |
| GZMH + IFNG + GNLY + ITGAE + CXCL9 + IL17A + REN + PROM1 | 0,77 |
| IFNG + GNLY + CCL5 + PF4 + REN + IHH + PROM1 + VEGFA | 0,77 |
| CXCL13 + GNLY + ITGAE + CCL5 + CXCL9 + IL17A + TSLP + REN | 0,77 |
| GZMH + GNLY + ITGAE + CCL5 + PF4 + REN + IHH + PROM1 | 0,77 |
| IFNG + GNLY + ITGAE + CCL5 + CXCL9 + PF4 + REN + IHH | 0,77 |
| GZMH + CXCL13 + GNLY + ITGAE + CCL5 + CXCL9 + IL17A + REN | 0,77 |
| GZMH + IFNG + GNLY + CCL5 + PF4 + TSLP + REN + VEGFA | 0,77 |
| CXCL13 + GNLY + ITGAE + CCL5 + IL17A + TSLP + REN + VEGFA | 0,77 |
| GNLY + ITGAE + CXCL9 + PF4 + IL17A + TSLP + REN + PROM1 | 0,77 |
| GNLY + LAG3 + ITGAE + CCL5 + CXCL9 + IL17A + TSLP + REN | 0,77 |
| IFNG + GNLY + LAG3 + ITGAE + IL17A + TSLP + REN + VEGFA | 0,77 |
| GZMH + IFNG + GNLY + ITGAE + CXCL9 + IL17A + REN + VEGFA | 0,77 |
| IFNG + GNLY + ITGAE + CXCL9 + IL17A + REN + PROM1 + VEGFA | 0,77 |
| GNLY + ITGAE + CXCL9 + PF4 + IL17A + REN + PROM1 + VEGFA | 0,77 |
| GNLY + ITGAE + CXCL9 + PF4 + IL17A + TSLP + REN + VEGFA | 0,77 |
| IFNG + GNLY + LAG3 + ITGAE + PF4 + TSLP + REN + PROM1 | 0,77 |
| CXCL13 + GNLY + ITGAE + PF4 + IL17A + TSLP + REN + VEGFA | 0,77 |
| GZMH + GNLY + ITGAE + CCL5 + PF4 + REN + PROM1 + VEGFA | 0,77 |
| GZMH + IFNG + GNLY + LAG3 + ITGAE + CXCL9 + IL17A + REN | 0,77 |
| IFNG + GNLY + ITGAE + CXCL9 + IL17A + REN + IHH + VEGFA | 0,77 |
| GNLY + ITGAE + CCL5 + CXCL9 + PF4 + TSLP + REN + IHH | 0,77 |
| CXCL13 + GNLY + PF4 + IL17A + TSLP + REN + PROM1 + VEGFA | 0,77 |
| GZMH + IFNG + GNLY + LAG3 + CCL5 + PF4 + TSLP + REN | 0,77 |
| GNLY + LAG3 + CCL5 + CXCL9 + IL17A + REN + IHH + VEGFA | 0,77 |
| GZMH + CXCL13 + GNLY + LAG3 + ITGAE + CCL5 + IL17A + REN | 0,77 |
| CXCL13 + GNLY + LAG3 + ITGAE + CCL5 + IL17A + TSLP + REN | 0,77 |
| GZMH + IFNG + GNLY + ITGAE + IL17A + REN + IHH + VEGFA | 0,77 |
| GZMH + IFNG + GNLY + CCL5 + CXCL9 + PF4 + REN + PROM1 | 0,77 |
| GZMH + IFNG + GNLY + LAG3 + ITGAE + IL17A + REN + IHH | 0,77 |
| IFNG + GNLY + LAG3 + ITGAE + CXCL9 + IL17A + REN + PROM1 | 0,77 |
| GNLY + LAG3 + CCL5 + CXCL9 + PF4 + TSLP + REN + PROM1 | 0,77 |
| GZMH + IFNG + GNLY + ITGAE + PF4 + TSLP + REN + PROM1 | 0,77 |
| GNLY + ITGAE + CCL5 + CXCL9 + IL17A + REN + IHH + PROM1 | 0,77 |
| GZMH + IFNG + GNLY + CCL5 + PF4 + TSLP + REN + IHH | 0,77 |
| IFNG + GNLY + LAG3 + ITGAE + CXCL9 + IL17A + REN + IHH | 0,77 |
| IFNG + GNLY + LAG3 + CCL5 + IL17A + REN + IHH + PROM1 | 0,77 |
| GZMH + IFNG + GNLY + ITGAE + IL17A + TSLP + REN + PROM1 | 0,77 |
| GNLY + LAG3 + PF4 + IL17A + TSLP + REN + PROM1 + VEGFA | 0,77 |
| GZMH + IFNG + GNLY + ITGAE + CXCL9 + IL17A + TSLP + REN | 0,77 |
| CXCL13 + GNLY + ITGAE + CCL5 + PF4 + IL17A + IHH + PROM1 | 0,77 |
| IFNG + GNLY + LAG3 + ITGAE + CXCL9 + IL17A + REN + VEGFA | 0,77 |
| IFNG + GNLY + CCL5 + CXCL9 + IL17A + TSLP + REN + VEGFA | 0,77 |
| GZMH + IFNG + GNLY + LAG3 + ITGAE + IL17A + REN + VEGFA | 0,77 |
| IFNG + GNLY + ITGAE + IL17A + TSLP + REN + PROM1 + VEGFA | 0,77 |
| IFNG + GNLY + LAG3 + ITGAE + CXCL9 + IL17A + TSLP + REN | 0,77 |
| GNLY + LAG3 + CCL5 + PF4 + TSLP + REN + IHH + PROM1 | 0,77 |
| CXCL13 + GNLY + ITGAE + PF4 + IL17A + TSLP + REN + PROM1 | 0,77 |
| GZMH + IFNG + GNLY + CCL5 + CXCL9 + IL17A + REN + VEGFA | 0,77 |
| IFNG + GNLY + ITGAE + CXCL9 + IL17A + TSLP + REN + PROM1 | 0,77 |
| GZMH + GNLY + ITGAE + CCL5 + CXCL9 + PF4 + REN + PROM1 | 0,77 |
| IFNG + GNLY + LAG3 + ITGAE + IL17A + REN + IHH + VEGFA | 0,77 |
| GZMH + GNLY + LAG3 + CCL5 + PF4 + TSLP + REN + PROM1 | 0,77 |
| IFNG + GNLY + ITGAE + IL17A + TSLP + REN + IHH + PROM1 | 0,77 |
| GNLY + LAG3 + ITGAE + CCL5 + IL17A + TSLP + REN + VEGFA | 0,77 |
| GZMH + GNLY + ITGAE + CCL5 + CXCL9 + PF4 + TSLP + REN | 0,77 |
| IFNG + GNLY + LAG3 + ITGAE + IL17A + TSLP + REN + PROM1 | 0,77 |
| IFNG + GNLY + ITGAE + IL17A + REN + IHH + PROM1 + VEGFA | 0,77 |
| GNLY + ITGAE + CCL5 + CXCL9 + PF4 + REN + PROM1 + VEGFA | 0,77 |
| CXCL13 + GNLY + ITGAE + PF4 + IL 17A + REN + PROM1 + VEGFA | 0,77 |
| IFNG + GNLY + ITGAE + PF4 + TSLP + REN + PROM1 + VEGFA | 0,77 |
| IFNG + GNLY + LAG3 + ITGAE + PF4 + REN + PROM1 + VEGFA | 0,77 |

**Table 9: clusters of 9 genes:**

| **clusters of 9 genes** | **c_tau level** |
|---|---|
| GZMH + IFNG + GNLY + ITGAE + CCL5 + PF4 + IL17A + REN + PROM1 | 0,8 |
| IFNG + GNLY + ITGAE + CCL5 + PF4 + IL17A + TSLP + REN + PROM1 | 0,8 |
| IFNG + GNLY + ITGAE + CCL5 + PF4 + IL17A + REN + PROM1 + VEGFA | 0,8 |
| GZMH + IFNG + GNLY + ITGAE + CCL5 + PF4 + IL17A + REN + VEGFA | 0,8 |
| IFNG + GNLY + ITGAE + CCL5 + PF4 + IL17A + REN + IHH + PROM1 | 0,8 |
| IFNG + GNLY + LAG3 + ITGAE + CCL5 + PF4 + IL17A + REN + PROM1 | 0,8 |
| GZMH + IFNG + GNLY + ITGAE + CCL5 + CXCL9 + PF4 + IL17A + REN | 0,8 |
| IFNG + GNLY + ITGAE + CCL5 + CXCL9 + PF4 + IL17A + REN + VEGFA | 0,8 |
| GZMH + IFNG + GNLY + ITGAE + CCL5 + PF4 + IL17A + REN + IHH | 0,8 |
| GZMH + IFNG + GNLY + ITGAE + CCL5 + PF4 + IL17A + TSLP + REN | 0,8 |
| IFNG + GNLY + LAG3 + ITGAE + CCL5 + PF4 + IL17A + REN + VEGFA | 0,8 |
| IFNG + GNLY + ITGAE + CCL5 + CXCL9 + PF4 + IL17A + REN + IHH | 0,8 |
| IFNG + GNLY + LAG3 + ITGAE + CCL5 + CXCL9 + PF4 + IL17A + REN | 0,8 |
| IFNG + GNLY + ITGAE + CCL5 + PF4 + IL17A + TSLP + REN + VEGFA | 0,79 |
| IFNG + GNLY + LAG3 + ITGAE + CCL5 + PF4 + IL17A + TSLP + REN | 0,79 |
| GZMH + IFNG + GNLY + LAG3 + ITGAE + CCL5 + PF4 + IL17A + REN | 0,79 |
| IFNG + GNLY + ITGAE + CCL5 + PF4 + IL17A + TSLP + REN + IHH | 0,79 |
| IFNG + GNLY + ITGAE + CCL5 + CXCL9 + PF4 + IL17A + REN + PROM1 | 0,79 |
| IFNG + GNLY + ITGAE + CCL5 + CXCL9 + PF4 + IL17A + TSLP + REN | 0,79 |
| IFNG + GNLY + ITGAE + CCL5 + PF4 + IL17A + REN + IHH + VEGFA | 0,79 |
| IFNG + GNLY + LAG3 + ITGAE + CCL5 + PF4 + IL17A + REN + IHH | 0,79 |
| IFNG + CXCL13 + GNLY + ITGAE + CCL5 + PF4 + IL17A + REN + PROM1 | 0,79 |
| IFNG + CXCL13 + GNLY + ITGAE + CCL5 + PF4 + IL17A + REN + IHH | 0,79 |
| IFNG + CXCL13 + GNLY + ITGAE + CCL5 + PF4 + IL17A + REN + VEGFA | 0,79 |
| IFNG + CXCL13 + GNLY + ITGAE + CCL5 + CXCL9 + PF4 + IL17A + REN | 0,79 |
| GZMH + IFNG + CXCL13 + GNLY + ITGAE + CCL5 + PF4 + IL17A + REN | 0,79 |
| IFNG + CXCL13 + GNLY + LAG3 + ITGAE + CCL5 + PF4 + IL17A + REN | 0,79 |
| IFNG + CXCL13 + GNLY + ITGAE + CCL5 + PF4 + IL17A + TSLP + REN | 0,79 |
| CXCL13 + GNLY + ITGAE + CCL5 + PF4 + IL17A + REN + IHH + PROM1 | 0,79 |
| GZMH + IFNG + GNLY + LAG3 + CCL5 + PF4 + IL17A + REN + PROM1 | 0,79 |
| CXCL13 + GNLY + LAG3 + ITGAE + CCL5 + PF4 + IL17A + REN + PROM1 | 0,79 |
| GNLY + ITGAE + CCL5 + CXCL9 + PF4 + IL17A + TSLP + REN + VEGFA | 0,79 |
| GZMH + CXCL13 + GNLY + ITGAE + CCL5 + PF4 + IL17A + REN + PROM1 | 0,79 |
| GZMH + IFNG + GNLY + CCL5 + CXCL9 + PF4 + IL17A + REN + IHH | 0,79 |
| GZMH + GNLY + ITGAE + CCL5 + CXCL9 + PF4 + IL17A + REN + VEGFA | 0,79 |
| GZMH + IFNG + GNLY + ITGAE + CCL5 + CXCL9 + IL17A + REN + VEGFA | 0,79 |
| GNLY + ITGAE + CCL5 + CXCL9 + PF4 + IL17A + REN + IHH + VEGFA | 0,79 |
| GZMH + IFNG + GNLY + LAG3 + CCL5 + CXCL9 + PF4 + IL17A + REN | 0,79 |
| GZMH + IFNG + GNLY + LAG3 + ITGAE + CCL5 + IL17A + REN + PROM1 | 0,79 |
| CXCL13 + GNLY + ITGAE + CCL5 + PF4 + IL17A + REN + PROM1 + VEGFA | 0,79 |
| GZMH + IFNG + GNLY + CCL5 + PF4 + IL17A + REN + IHH + PROM1 | 0,79 |
| CXCL13 + GNLY + ITGAE + CCL5 + PF4 + IL17A + TSLP + REN + PROM1 | 0,79 |
| IFNG + GNLY + LAG3 + CCL5 + CXCL9 + PF4 + IL17A + REN + IHH | 0,79 |
| GNLY + LAG3 + ITGAE + CCL5 + CXCL9 + PF4 + IL17A + REN + VEGFA | 0,79 |
| GNLY + ITGAE + CCL5 + CXCL9 + PF4 + IL17A + REN + PROM1 + VEGFA | 0,79 |
| GNLY + ITGAE + CCL5 + CXCL9 + PF4 + IL17A + TSLP + REN + PROM1 | 0,79 |
| IFNG + GNLY + LAG3 + CCL5 + CXCL9 + PF4 + IL17A + REN + VEGFA | 0,79 |
| GZMH + IFNG + GNLY + LAG3 + ITGAE + CCL5 + IL17A + REN + VEGFA | 0,79 |
| CXCL13 + GNLY + ITGAE + CCL5 + CXCL9 + PF4 + IL17A + REN + PROM1 | 0,79 |
| IFNG + GNLY + LAG3 + ITGAE + CCL5 + CXCL9 + IL17A + REN + VEGFA | 0,79 |
| IFNG + GNLY + LAG3 + CCL5 + PF4 + IL17A + REN + PROM1 + VEGFA | 0,79 |
| IFNG + GNLY + CCL5 + CXCL9 + PF4 + IL17A + REN + IHH + VEGFA | 0,79 |
| GZMH + IFNG + GNLY + LAG3 + CCL5 + PF4 + IL17A + TSLP + REN | 0,79 |
| GZMH + IFNG + GNLY + CCL5 + CXCL9 + PF4 + IL17A + REN + VEGFA | 0,79 |
| GZMH + IFNG + GNLY + LAG3 + CCL5 + PF4 + IL17A + REN + IHH | 0,79 |
| GNLY + ITGAE + CCL5 + CXCL9 + PF4 + IL17A + REN + IHH + PROM1 | 0,79 |
| IFNG + GNLY + LAG3 + CCL5 + PF4 + IL17A + TSLP + REN + PROM1 | 0,79 |
| IFNG + GNLY + LAG3 + CCL5 + PF4 + IL17A + REN + IHH + PROM1 | 0,79 |
| GNLY + LAG3 + ITGAE + CCL5 + PF4 + IL17A + TSLP + REN + PROM1 | 0,79 |
| IFNG + GNLY + CCL5 + CXCL9 + PF4 + IL17A + TSLP + REN + IHH | 0,79 |
| GNLY + ITGAE + CCL5 + PF4 + IL17A + TSLP + REN + IHH + PROM1 | 0,79 |
| GZMH + IFNG + GNLY + LAG3 + ITGAE + CCL5 + IL17A + REN + IHH | 0,79 |
| GNLY + LAG3 + ITGAE + CCL5 + PF4 + IL17A + REN + PROM1 + VEGFA | 0,79 |
| GNLY + ITGAE + CCL5 + PF4 + IL17A + TSLP + REN + PROM1 + VEGFA | 0,79 |
| GZMH + IFNG + GNLY + LAG3 + CCL5 + PF4 + IL17A + REN + VEGFA | 0,79 |
| IFNG + GNLY + LAG3 + ITGAE + CCL5 + IL17A + REN + IHH + VEGFA | 0,79 |
| CXCL13 + GNLY + ITGAE + CCL5 + PF4 + IL17A + REN + IHH + VEGFA | 0,79 |
| IFNG + GNLY + ITGAE + CCL5 + CXCL9 + IL17A + TSLP + REN + VEGFA | 0,79 |
| CXCL13 + GNLY + ITGAE + CCL5 + CXCL9 + PF4 + IL17A + REN + VEGFA | 0,79 |
| IFNG + GNLY + ITGAE + CCL5 + CXCL9 + IL17A + REN + PROM1 + VEGFA | 0,79 |
| GNLY + ITGAE + CCL5 + CXCL9 + PF4 + IL17A + TSLP + REN + IHH | 0,79 |
| IFNG + GNLY + LAG3 + ITGAE + CCL5 + IL17A + REN + PROM1 + VEGFA | 0,79 |
| IFNG + GNLY + ITGAE + CCL5 + CXCL9 + IL17A + REN + IHH + VEGFA | 0,79 |
| GZMH + IFNG + GNLY + CCL5 + PF4 + IL17A + TSLP + REN + PROM1 | 0,79 |
| GNLY + LAG3 + ITGAE + CCL5 + CXCL9 + PF4 + IL17A + REN + PROM1 | 0,79 |
| CXCL13 + GNLY + LAG3 + ITGAE + CCL5 + PF4 + IL17A + REN + IHH | 0,79 |
| CXCL13 + GNLY + ITGAE + CCL5 + CXCL9 + PF4 + IL17A + REN + IHH | 0,79 |
| GNLY + LAG3 + ITGAE + CCL5 + PF4 + IL17A + REN + IHH + PROM1 | 0,79 |
| GZMH + IFNG + GNLY + CCL5 + PF4 + IL17A + REN + IHH + VEGFA | 0,79 |
| GZMH + IFNG + GNLY + LAG3 + ITGAE + CCL5 + CXCL9 + IL17A + REN | 0,79 |
| GZMH + IFNG + GNLY + CCL5 + PF4 + IL17A + REN + PROM1 + VEGFA | 0,79 |
| IFNG + GNLY + LAG3 + CCL5 + PF4 + IL17A + TSLP + REN + IHH | 0,79 |
| GZMH + CXCL13 + GNLY + ITGAE + CCL5 + PF4 + IL17A + REN + IHH | 0,79 |
| IFNG + GNLY + LAG3 + CCL5 + CXCL9 + PF4 + IL17A + TSLP + REN | 0,79 |
| GZMH + GNLY + ITGAE + CCL5 + CXCL9 + PF4 + IL17A + TSLP + REN | 0,79 |
| GZMH + GNLY + ITGAE + CCL5 + PF4 + IL17A + TSLP + REN + PROM1 | 0,79 |
| IFNG + GNLY + LAG3 + ITGAE + CCL5 + IL17A + TSLP + REN + PROM1 | 0,79 |
| IFNG + GNLY + ITGAE + CCL5 + CXCL9 + IL17A + TSLP + REN + IHH | 0,79 |
| GZMH + GNLY + ITGAE + CCL5 + CXCL9 + PF4 + IL17A + REN + PROM1 | 0,79 |
| GZMH + GNLY + ITGAE + CCL5 + PF4 + IL17A + REN + PROM1 + VEGFA | 0,79 |
| GZMH + IFNG + GNLY + ITGAE + CCL5 + CXCL9 + IL17A + REN + IHH | 0,79 |
| IFNG + GNLY + LAG3 + ITGAE + CCL5 + CXCL9 + IL17A + REN + IHH | 0,79 |
| IFNG + GNLY + LAG3 + ITGAE + CCL5 + IL17A + TSLP + REN + VEGFA | 0,79 |
| GNLY + ITGAE + CCL5 + PF4 + IL17A + REN + IHH + PROM1 + VEGFA | 0,79 |
| GZMH + IFNG + GNLY + CCL5 + CXCL9 + PF4 + IL17A + TSLP + REN | 0,79 |
| GNLY + LAG3 + ITGAE + CCL5 + CXCL9 + PF4 + IL17A + TSLP + REN | 0,79 |
| IFNG + GNLY + LAG3 + CCL5 + PF4 + IL17A + REN + IHH + VEGFA | 0,79 |
| GZMH + IFNG + GNLY + CCL5 + PF4 + IL17A + TSLP + REN + IHH | 0,79 |
| IFNG + GNLY + CCL5 + CXCL9 + PF4 + IL17A + TSLP + REN + VEGFA | 0,79 |
| IFNG + GNLY + LAG3 + ITGAE + CCL5 + CXCL9 + IL17A + REN + PROM1 | 0,79 |
| GZMH + IFNG + GNLY + LAG3 + ITGAE + CCL5 + IL17A + TSLP + REN | 0,79 |
| IFNG + GNLY + LAG3 + ITGAE + CCL5 + CXCL9 + IL17A + TSLP + REN | 0,79 |
| CXCL13 + GNLY + LAG3 + ITGAE + CCL5 + PF4 + IL17A + REN + VEGFA | 0,79 |
| IFNG + GNLY + LAG3 + CCL5 + PF4 + IL17A + TSLP + REN + VEGFA | 0,79 |
| IFNG + GNLY + LAG3 + ITGAE + CCL5 + IL17A + TSLP + REN + IHH | 0,79 |
| CXCL13 + GNLY + ITGAE + CCL5 + PF4 + IL17A + TSLP + REN + IHH | 0,79 |
| CXCL13 + GNLY + LAG3 + ITGAE + CCL5 + CXCL9 + PF4 + IL17A + REN | 0,79 |
| GZMH + GNLY + LAG3 + ITGAE + CCL5 + PF4 + IL17A + REN + PROM1 | 0,79 |
| IFNG + GNLY + LAG3 + CCL5 + CXCL9 + PF4 + IL17A + REN + PROM1 | 0,79 |
| GZMH + GNLY + ITGAE + CCL5 + PF4 + IL17A + REN + IHH + PROM1 | 0,79 |
| GZMH + IFNG + GNLY + ITGAE + CCL5 + IL17A + REN + PROM1 + VEGFA | 0,79 |
| CXCL13 + GNLY + ITGAE + CCL5 + CXCL9 + PF4 + IL17A + TSLP + REN | 0,79 |
| GZMH + IFNG + GNLY + ITGAE + CCL5 + CXCL9 + IL17A + TSLP + REN | 0,79 |
| CXCL13 + GNLY + ITGAE + CCL5 + PF4 + IL17A + TSLP + REN + VEGFA | 0,79 |
| IFNG + GNLY + LAG3 + ITGAE + CCL5 + IL17A + REN + IHH + PROM1 | 0,79 |
| GZMH + GNLY + LAG3 + ITGAE + CCL5 + CXCL9 + PF4 + IL17A + REN | 0,79 |
| GZMH + IFNG + CXCL13 + GNLY + CCL5 + PF4 + IL17A + REN + IHH | 0,79 |
| IFNG + CXCL13 + GNLY + ITGAE + CCL5 + CXCL9 + IL17A + REN + VEGFA | 0,79 |
| GZMH + CXCL13 + GNLY + ITGAE + CCL5 + CXCL9 + PF4 + IL17A + REN | 0,79 |
| GZMH + IFNG + GNLY + ITGAE + CCL5 + CXCL9 + IL17A + REN + PROM1 | 0,79 |
| CXCL13 + GNLY + LAG3 + ITGAE + CCL5 + PF4 + IL17A + TSLP + REN | 0,79 |
| GZMH + GNLY + ITGAE + CCL5 + CXCL9 + PF4 + IL17A + REN + IHH | 0,79 |
| GZMH + IFNG + GNLY + CCL5 + PF4 + IL17A + TSLP + REN + VEGFA | 0,79 |
| GZMH + IFNG + GNLY + ITGAE + CCL5 + IL17A + TSLP + REN + PROM1 | 0,79 |
| GZMH + IFNG + GNLY + ITGAE + CCL5 + IL17A + REN + IHH + VEGFA | 0,79 |
| IFNG + CXCL13 + GNLY + CCL5 + CXCL9 + PF4 + IL17A + REN + IHH | 0,79 |
| GZMH + IFNG + GNLY + CCL5 + CXCL9 + PF4 + IL17A + REN + PROM1 | 0,79 |
| GZMH + IFNG + GNLY + LAG3 + ITGAE + PF4 + IL17A + TSLP + REN | 0,79 |
| GZMH + IFNG + GNLY + ITGAE + CCL5 + IL17A + TSLP + REN + VEGFA | 0,79 |
| GZMH + IFNG + CXCL13 + GNLY + ITGAE + CCL5 + IL17A + REN + PROM1 | 0,79 |
| GZMH + CXCL13 + GNLY + CCL5 + CXCL9 + PF4 + IL17A + REN + IHH | 0,79 |
| IFNG + GNLY + LAG3 + ITGAE + PF4 + IL17A + TSLP + REN + PROM1 | 0,79 |
| IFNG + GNLY + ITGAE + CCL5 + CXCL9 + IL17A + TSLP + REN + PROM1 | 0,79 |
| GZMH + IFNG + GNLY + ITGAE + CCL5 + IL17A + TSLP + REN + IHH | 0,79 |
| GZMH + IFNG + GNLY + LAG3 + ITGAE + PF4 + IL17A + REN + PROM1 | 0,79 |
| GNLY + LAG3 + ITGAE + CCL5 + CXCL9 + PF4 + IL17A + REN + IHH | 0,79 |
| IFNG + GNLY + CCL5 + PF4 + IL17A + REN + IHH + PROM1 + VEGFA | 0,79 |
| IFNG + GNLY + CCL5 + PF4 + IL17A + TSLP + REN + IHH + PROM1 | 0,79 |
| IFNG + GNLY + CCL5 + CXCL9 + PF4 + IL17A + REN + IHH + PROM1 | 0,79 |
| GZMH + IFNG + GNLY + ITGAE + PF4 + IL17A + TSLP + REN + VEGFA | 0,79 |
| GZMH + IFNG + GNLY + LAG3 + ITGAE + CXCL9 + PF4 + IL17A + REN | 0,79 |
| IFNG + GNLY + LAG3 + ITGAE + CXCL9 + PF4 + IL17A + REN + IHH | 0,79 |
| GZMH + CXCL13 + GNLY + ITGAE + CCL5 + PF4 + IL17A + REN + VEGFA | 0,79 |
| GZMH + CXCL13 + GNLY + LAG3 + CCL5 + PF4 + IL17A + REN + IHH | 0,79 |
| IFNG + GNLY + LAG3 + ITGAE + PF4 + IL17A + REN + IHH + PROM1 | 0,78 |
| GZMH + IFNG + GNLY + LAG3 + ITGAE + PF4 + IL17A + REN + VEGFA | 0,78 |
| GNLY + LAG3 + CCL5 + CXCL9 + PF4 + IL17A + REN + IHH + VEGFA | 0,78 |
| GZMH + CXCL13 + GNLY + CCL5 + PF4 + IL17A + REN + IHH + PROM1 | 0,78 |
| IFNG + GNLY + LAG3 + ITGAE + PF4 + IL17A + REN + PROM1 + VEGFA | 0,78 |
| GZMH + IFNG + GNLY + LAG3 + ITGAE + PF4 + IL17A + REN + IHH | 0,78 |
| CXCL13 + GNLY + LAG3 + CCL5 + CXCL9 + PF4 + IL17A + REN + IHH | 0,78 |
| IFNG + GNLY + CCL5 + PF4 + IL17A + TSLP + REN + IHH + VEGFA | 0,78 |
| IFNG + GNLY + ITGAE + CCL5 + CXCL9 + IL17A + REN + IHH + PROM1 | 0,78 |
| GZMH + IFNG + GNLY + ITGAE + PF4 + IL17A + TSLP + REN + PROM1 | 0,78 |
| GZMH + CXCL13 + GNLY + LAG3 + ITGAE + CCL5 + PF4 + IL17A + REN | 0,78 |
| GZMH + IFNG + GNLY + ITGAE + PF4 + IL17A + TSLP + REN + IHH | 0,78 |
| IFNG + GNLY + CCL5 + CXCL9 + PF4 + IL17A + REN + PROM1 + VEGFA | 0,78 |
| CXCL13 + GNLY + CCL5 + CXCL9 + PF4 + IL17A + REN + IHH + VEGFA | 0,78 |
| GZMH + CXCL13 + GNLY + ITGAE + CCL5 + PF4 + IL17A + TSLP + REN | 0,78 |
| IFNG + GNLY + LAG3 + ITGAE + PF4 + IL17A + TSLP + REN + VEGFA | 0,78 |
| GZMH + IFNG + CXCL13 + GNLY + ITGAE + CCL5 + IL17A + REN + VEGFA | 0,78 |
| IFNG + CXCL13 + GNLY + LAG3 + ITGAE + CCL5 + IL17A + REN + PROM1 | 0,78 |
| IFNG + GNLY + LAG3 + ITGAE + CXCL9 + PF4 + IL17A + REN + PROM1 | 0,78 |
| IFNG + GNLY + CCL5 + PF4 + IL17A + TSLP + REN + PROM1 + VEGFA | 0,78 |
| CXCL13 + GNLY + CCL5 + CXCL9 + PF4 + IL17A + TSLP + REN + IHH | 0,78 |
| GZMH + CXCL13 + GNLY + CCL5 + PF4 + IL17A + TSLP + REN + IHH | 0,78 |
| IFNG + GNLY + ITGAE + PF4 + IL17A + REN + IHH + PROM1 + VEGFA | 0,78 |
| GZMH + IFNG + GNLY + ITGAE + CCL5 + IL17A + REN + IHH + PROM1 | 0,78 |
| IFNG + GNLY + ITGAE + PF4 + IL17A + TSLP + REN + IHH + VEGFA | 0,78 |
| IFNG + GNLY + ITGAE + CCL5 + IL17A + TSLP + REN + PROM1 + VEGFA | 0,78 |
| GZMH + IFNG + GNLY + ITGAE + PF4 + IL17A + REN + IHH + VEGFA | 0,78 |
| IFNG + GNLY + LAG3 + ITGAE + CXCL9 + PF4 + IL17A + REN + VEGFA | 0,78 |
| IFNG + GNLY + LAG3 + ITGAE + PF4 + IL17A + TSLP + REN + IHH | 0,78 |
| GZMH + IFNG + CXCL13 + GNLY + LAG3 + CCL5 + PF4 + IL17A + REN | 0,78 |
| GZMH + IFNG + GNLY + ITGAE + PF4 + IL17A + REN + IHH + PROM1 | 0,78 |
| GZMH + IFNG + CXCL13 + GNLY + LAG3 + ITGAE + CCL5 + IL17A + REN | 0,78 |
| IFNG + GNLY + LAG3 + ITGAE + PF4 + IL17A + REN + IHH + VEGFA | 0,78 |
| GZMH + GNLY + LAG3 + ITGAE + CCL5 + PF4 + IL17A + TSLP + REN | 0,78 |
| GNLY + LAG3 + ITGAE + CCL5 + PF4 + IL17A + TSLP + REN + VEGFA | 0,78 |
| GNLY + LAG3 + ITGAE + CCL5 + PF4 + IL17A + TSLP + REN + IHH | 0,78 |
| IFNG + CXCL13 + GNLY + LAG3 + CCL5 + CXCL9 + PF4 + IL17A + REN | 0,78 |
| GZMH + IFNG + CXCL13 + GNLY + CCL5 + PF4 + IL17A + REN + PROM1 | 0,78 |
| GZMH + IFNG + GNLY + ITGAE + PF4 + IL17A + REN + PROM1 + VEGFA | 0,78 |
| IFNG + GNLY + ITGAE + PF4 + IL17A + TSLP + REN + PROM1 + VEGFA | 0,78 |
| GZMH + CXCL13 + GNLY + CCL5 + PF4 + IL17A + REN + IHH + VEGFA | 0,78 |
| IFNG + GNLY + LAG3 + CXCL9 + PF4 + IL17A + REN + IHH + VEGFA | 0,78 |
| CXCL13 + GNLY + LAG3 + CCL5 + PF4 + IL17A + REN + IHH + PROM1 | 0,78 |
| GZMH + IFNG + GNLY + ITGAE + CXCL9 + PF4 + IL17A + REN + VEGFA | 0,78 |
| IFNG + GNLY + ITGAE + CCL5 + IL17A + TSLP + REN + IHH + VEGFA | 0,78 |
| IFNG + CXCL13 + GNLY + LAG3 + CCL5 + PF4 + IL17A + REN + IHH | 0,78 |
| GNLY + LAG3 + CCL5 + CXCL9 + PF4 + IL17A + TSLP + REN + VEGFA | 0,78 |
| GZMH + IFNG + GNLY + ITGAE + CXCL9 + PF4 + IL17A + REN + IHH | 0,78 |
| GZMH + IFNG + CXCL13 + GNLY + CCL5 + CXCL9 + PF4 + IL17A + REN | 0,78 |
| IFNG + GNLY + CCL5 + CXCL9 + PF4 + IL17A + TSLP + REN + PROM1 | 0,78 |
| IFNG + CXCL13 + GNLY + CCL5 + PF4 + IL17A + REN + IHH + VEGFA | 0,78 |
| GNLY + LAG3 + CCL5 + CXCL9 + PF4 + IL17A + REN + IHH + PROM1 | 0,78 |
| CXCL13 + GNLY + CCL5 + CXCL9 + PF4 + IL17A + REN + IHH + PROM1 | 0,78 |
| IFNG + GNLY + ITGAE + CCL5 + IL17A + TSLP + REN + IHH + PROM1 | 0,78 |
| GNLY + ITGAE + CCL5 + PF4 + IL17A + TSLP + REN + IHH + VEGFA | 0,78 |
| IFNG + CXCL13 + GNLY + ITGAE + CCL5 + CXCL9 + IL17A + REN + PROM1 | 0,78 |
| GZMH + IFNG + GNLY + LAG3 + PF4 + IL17A + REN + IHH + PROM1 | 0,78 |
| GZMH + GNLY + LAG3 + CCL5 + CXCL9 + PF4 + IL17A + REN + VEGFA | 0,78 |
| IFNG + GNLY + LAG3 + ITGAE + CXCL9 + PF4 + IL17A + TSLP + REN | 0,78 |
| GNLY + LAG3 + CCL5 + CXCL9 + PF4 + IL17A + REN + PROM1 + VEGFA | 0,78 |
| IFNG + GNLY + ITGAE + PF4 + IL17A + TSLP + REN + IHH + PROM1 | 0,78 |
| IFNG + CXCL13 + GNLY + CCL5 + PF4 + IL17A + TSLP + REN + IHH | 0,78 |
| IFNG + CXCL13 + GNLY + CCL5 + CXCL9 + PF4 + IL17A + REN + VEGFA | 0,78 |
| GNLY + CCL5 + CXCL9 + PF4 + IL17A + TSLP + REN + IHH + VEGFA | 0,78 |
| IFNG + CXCL13 + GNLY + ITGAE + CCL5 + IL17A + REN + PROM1 + VEGFA | 0,78 |
| IFNG + GNLY + ITGAE + CXCL9 + PF4 + IL17A + REN + IHH + VEGFA | 0,78 |
| GZMH + GNLY + ITGAE + CCL5 + PF4 + IL17A + TSLP + REN + IHH | 0,78 |
| CXCL13 + GNLY + CCL5 + PF4 + IL17A + REN + IHH + PROM1 + VEGFA | 0,78 |
| GNLY + LAG3 + CCL5 + CXCL9 + PF4 + IL17A + TSLP + REN + IHH | 0,78 |
| GZMH + IFNG + GNLY + ITGAE + CXCL9 + PF4 + IL17A + REN + PROM1 | 0,78 |
| IFNG + GNLY + LAG3 + CXCL9 + PF4 + IL17A + REN + IHH + PROM1 | 0,78 |
| GZMH + IFNG + GNLY + CXCL9 + PF4 + IL17A + REN + IHH + VEGFA | 0,78 |
| CXCL13 + GNLY + LAG3 + CCL5 + PF4 + IL17A + REN + IHH + VEGFA | 0,78 |
| IFNG + GNLY + ITGAE + CCL5 + IL17A + REN + IHH + PROM1 + VEGFA | 0,78 |
| CXCL13 + GNLY + CCL5 + PF4 + IL17A + TSLP + REN + IHH + PROM1 | 0,78 |
| GZMH + IFNG + GNLY + LAG3 + CXCL9 + PF4 + IL17A + REN + IHH | 0,78 |
| GZMH + IFNG + CXCL13 + GNLY + CCL5 + PF4 + IL17A + TSLP + REN | 0,78 |
| GZMH + GNLY + CCL5 + CXCL9 + PF4 + IL17A + REN + IHH + VEGFA | 0,78 |
| CXCL13 + GNLY + LAG3 + CCL5 + PF4 + IL17A + TSLP + REN + IHH | 0,78 |
| IFNG + CXCL13 + GNLY + LAG3 + CCL5 + PF4 + IL17A + REN + PROM1 | 0,78 |
| GZMH + IFNG + CXCL13 + GNLY + ITGAE + CCL5 + IL17A + REN + IHH | 0,78 |
| GZMH + IFNG + GNLY + PF4 + IL17A + REN + IHH + PROM1 + VEGFA | 0,78 |
| GZMH + IFNG + GNLY + LAG3 + PF4 + IL17A + TSLP + REN + PROM1 | 0,78 |
| GZMH + IFNG + GNLY + PF4 + IL17A + TSLP + REN + IHH + PROM1 | 0,78 |
| IFNG + CXCL13 + GNLY + CCL5 + PF4 + IL17A + REN + IHH + PROM1 | 0,78 |
| GZMH + IFNG + GNLY + LAG3 + PF4 + IL17A + REN + IHH + VEGFA | 0,78 |
| IFNG + GNLY + LAG3 + PF4 + IL17A + TSLP + REN + IHH + PROM1 | 0,78 |
| GNLY + LAG3 + ITGAE + CCL5 + PF4 + IL17A + REN + IHH + VEGFA | 0,78 |
| GZMH + IFNG + CXCL13 + GNLY + ITGAE + CCL5 + IL17A + TSLP + REN | 0,78 |
| CXCL13 + GNLY + CCL5 + PF4 + IL17A + TSLP + REN + IHH + VEGFA | 0,78 |
| IFNG + GNLY + LAG3 + PF4 + IL17A + REN + IHH + PROM1 + VEGFA | 0,78 |
| IFNG + CXCL13 + GNLY + CCL5 + CXCL9 + PF4 + IL17A + TSLP + REN | 0,78 |
| IFNG + CXCL13 + GNLY + LAG3 + ITGAE + CCL5 + CXCL9 + IL17A + REN | 0,78 |
| IFNG + GNLY + ITGAE + CXCL9 + PF4 + IL17A + REN + PROM1 + VEGFA | 0,78 |
| CXCL13 + GNLY + LAG3 + CCL5 + CXCL9 + PF4 + IL17A + REN + VEGFA | 0,78 |
| GZMH + GNLY + ITGAE + CCL5 + PF4 + IL17A + TSLP + REN + VEGFA | 0,78 |
| IFNG + CXCL13 + GNLY + LAG3 + ITGAE + CCL5 + IL17A + REN + VEGFA | 0,78 |
| IFNG + GNLY + LAG3 + PF4 + IL17A + TSLP + REN + PROM1 + VEGFA | 0,78 |
| IFNG + CXCL13 + GNLY + ITGAE + CCL5 + IL17A + TSLP + REN + PROM1 | 0,78 |
| IFNG + GNLY + LAG3 + CXCL9 + PF4 + IL17A + TSLP + REN + IHH | 0,78 |
| GZMH + IFNG + GNLY + PF4 + IL17A + TSLP + REN + PROM1 + VEGFA | 0,78 |
| GZMH + IFNG + CXCL13 + GNLY + CCL5 + PF4 + IL17A + REN + VEGFA | 0,78 |
| GZMH + IFNG + GNLY + LAG3 + PF4 + IL17A + TSLP + REN + IHH | 0,78 |
| GZMH + IFNG + GNLY + PF4 + IL17A + TSLP + REN + IHH + VEGFA | 0,78 |
| GZMH + IFNG + GNLY + ITGAE + CXCL9 + PF4 + IL17A + TSLP + REN | 0,78 |
| IFNG + GNLY + PF4 + IL17A + TSLP + REN + IHH + PROM1 + VEGFA | 0,78 |
| IFNG + GNLY + LAG3 + PF4 + IL17A + TSLP + REN + IHH + VEGFA | 0,78 |
| GZMH + IFNG + GNLY + LAG3 + PF4 + IL17A + REN + PROM1 + VEGFA | 0,78 |
| GZMH + CXCL13 + GNLY + LAG3 + CCL5 + PF4 + IL17A + REN + PROM1 | 0,78 |
| GZMH + IFNG + GNLY + CXCL9 + PF4 + IL17A + REN + IHH + PROM1 | 0,78 |
| IFNG + CXCL13 + GNLY + ITGAE + CCL5 + CXCL9 + IL17A + REN + IHH | 0,78 |
| GNLY + CCL5 + CXCL9 + PF4 + IL17A + TSLP + REN + IHH + PROM1 | 0,78 |
| GZMH + IFNG + GNLY + ITGAE + CCL5 + PF4 + TSLP + REN + PROM1 | 0,78 |
| GNLY + LAG3 + CCL5 + PF4 + IL17A + TSLP + REN + IHH + PROM1 | 0,78 |
| CXCL13 + GNLY + LAG3 + CCL5 + CXCL9 + PF4 + IL17A + REN + PROM1 | 0,78 |
| GZMH + GNLY + ITGAE + CCL5 + PF4 + IL17A + REN + IHH + VEGFA | 0,78 |
| GZMH + IFNG + GNLY + LAG3 + PF4 + IL17A + TSLP + REN + VEGFA | 0,78 |
| IFNG + GNLY + ITGAE + CXCL9 + PF4 + IL17A + REN + IHH + PROM1 | 0,78 |
| GNLY + LAG3 + CCL5 + CXCL9 + PF4 + IL17A + TSLP + REN + PROM1 | 0,78 |
| IFNG + GNLY + LAG3 + CXCL9 + PF4 + IL17A + REN + PROM1 + VEGFA | 0,78 |
| GZMH + GNLY + LAG3 + CCL5 + CXCL9 + PF4 + IL17A + REN + PROM1 | 0,78 |
| IFNG + GNLY + CXCL9 + PF4 + IL17A + REN + IHH + PROM1 + VEGFA | 0,78 |
| IFNG + CXCL13 + GNLY + ITGAE + CCL5 + IL17A + REN + IHH + PROM1 | 0,78 |
| GZMH + IFNG + GNLY + CXCL9 + PF4 + IL17A + TSLP + REN + IHH | 0,78 |
| CXCL13 + GNLY + LAG3 + CCL5 + CXCL9 + PF4 + IL17A + TSLP + REN | 0,78 |
| IFNG + CXCL13 + GNLY + ITGAE + CCL5 + CXCL9 + IL17A + TSLP + REN | 0,78 |
| GZMH + IFNG + GNLY + CXCL9 + PF4 + IL17A + REN + PROM1 + VEGFA | 0,78 |
| GZMH + CXCL13 + GNLY + LAG3 + CCL5 + CXCL9 + PF4 + IL17A + REN | 0,78 |
| GZMH + CXCL13 + GNLY + CCL5 + CXCL9 + PF4 + IL17A + REN + PROM1 | 0,78 |
| IFNG + CXCL13 + GNLY + LAG3 + ITGAE + CCL5 + IL17A + TSLP + REN | 0,78 |
| GZMH + CXCL13 + GNLY + CCL5 + PF4 + IL17A + REN + PROM1 + VEGFA | 0,78 |
| GNLY + CCL5 + CXCL9 + PF4 + IL17A + REN + IHH + PROM1 + VEGFA | 0,78 |
| IFNG + GNLY + CXCL9 + PF4 + IL17A + TSLP + REN + IHH + VEGFA | 0,78 |
| GZMH + GNLY + LAG3 + CCL5 + CXCL9 + PF4 + IL17A + REN + IHH | 0,78 |
| GZMH + IFNG + GNLY + LAG3 + CXCL9 + PF4 + IL17A + REN + VEGFA | 0,78 |
| IFNG + CXCL13 + GNLY + CCL5 + PF4 + IL17A + TSLP + REN + PROM1 | 0,78 |
| IFNG + CXCL13 + GNLY + ITGAE + CCL5 + IL17A + REN + IHH + VEGFA | 0,78 |
| IFNG + CXCL13 + GNLY + ITGAE + CCL5 + IL17A + TSLP + REN + VEGFA | 0,78 |
| IFNG + CXCL13 + GNLY + LAG3 + CCL5 + PF4 + IL17A + TSLP + REN | 0,78 |
| GZMH + IFNG + CXCL13 + GNLY + ITGAE + CCL5 + CXCL9 + IL17A + REN | 0,78 |
| IFNG + CXCL13 + GNLY + LAG3 + CCL5 + PF4 + IL17A + REN + VEGFA | 0,78 |
| GZMH + GNLY + CCL5 + CXCL9 + PF4 + IL17A + TSLP + REN + IHH | 0,78 |
| CXCL13 + GNLY + CCL5 + CXCL9 + PF4 + IL17A + TSLP + REN + VEGFA | 0,78 |
| IFNG + CXCL13 + GNLY + LAG3 + ITGAE + CCL5 + IL17A + REN + IHH | 0,78 |
| GZMH + IFNG + GNLY + LAG3 + CXCL9 + PF4 + IL17A + REN + PROM1 | 0,78 |
| GZMH + CXCL13 + GNLY + CCL5 + CXCL9 + PF4 + IL17A + REN + VEGFA | 0,78 |
| GZMH + IFNG + GNLY + CXCL9 + PF4 + IL17A + TSLP + REN + VEGFA | 0,78 |
| IFNG + CXCL13 + GNLY + ITGAE + CCL5 + IL17A + TSLP + REN + IHH | 0,78 |
| IFNG + GNLY + ITGAE + CXCL9 + PF4 + IL17A + TSLP + REN + PROM1 | 0,78 |
| CXCL13 + GNLY + CCL5 + CXCL9 + PF4 + IL17A + REN + PROM1 + VEGFA | 0,78 |
| GZMH + CXCL13 + GNLY + CCL5 + CXCL9 + PF4 + IL17A + TSLP + REN | 0,78 |
| IFNG + GNLY + LAG3 + CXCL9 + PF4 + IL17A + TSLP + REN + VEGFA | 0,78 |
| GZMH + GNLY + CCL5 + CXCL9 + PF4 + IL17A + REN + IHH + PROM1 | 0,78 |
| GZMH + GNLY + LAG3 + CCL5 + CXCL9 + PF4 + IL17A + TSLP + REN | 0,78 |
| GZMH + IFNG + CXCL13 + GNLY + ITGAE + PF4 + IL17A + TSLP + REN | 0,78 |
| IFNG + GNLY + ITGAE + CXCL9 + PF4 + IL17A + TSLP + REN + IHH | 0,78 |
| IFNG + CXCL13 + GNLY + CCL5 + PF4 + IL17A + REN + PROM1 + VEGFA | 0,78 |
| CXCL13 + GNLY + LAG3 + CCL5 + PF4 + IL17A + TSLP + REN + PROM1 | 0,78 |
| CXCL13 + GNLY + LAG3 + CCL5 + PF4 + IL17A + REN + PROM1 + VEGFA | 0,78 |
| IFNG + CXCL13 + GNLY + CCL5 + CXCL9 + PF4 + IL17A + REN + PROM1 | 0,78 |
| GNLY + LAG3 + CCL5 + PF4 + IL17A + REN + IHH + PROM1 + VEGFA | 0,78 |
| CXCL13 + GNLY + CCL5 + CXCL9 + PF4 + IL17A + TSLP + REN + PROM1 | 0,78 |
| IFNG + CXCL13 + GNLY + LAG3 + ITGAE + PF4 + IL17A + REN + PROM1 | 0,78 |
| GZMH + IFNG + GNLY + LAG3 + CXCL9 + PF4 + IL17A + TSLP + REN | 0,78 |
| GZMH + IFNG + CXCL13 + GNLY + ITGAE + PF4 + IL17A + REN + PROM1 | 0,78 |
| CXCL13 + GNLY + CCL5 + PF4 + IL17A + TSLP + REN + PROM1 + VEGFA | 0,78 |
| GZMH + GNLY + LAG3 + ITGAE + CCL5 + PF4 + IL17A + REN + IHH | 0,78 |
| GZMH + CXCL13 + GNLY + CCL5 + PF4 + IL17A + TSLP + REN + PROM1 | 0,78 |
| GZMH + GNLY + LAG3 + CCL5 + PF4 + IL17A + REN + IHH + PROM1 | 0,78 |
| GZMH + IFNG + GNLY + CXCL9 + PF4 + IL17A + TSLP + REN + PROM1 | 0,78 |
| GZMH + GNLY + LAG3 + ITGAE + CCL5 + PF4 + IL17A + REN + VEGFA | 0,78 |
| IFNG + GNLY + LAG3 + CXCL9 + PF4 + IL17A + TSLP + REN + PROM1 | 0,78 |
| GZMH + IFNG + CXCL13 + GNLY + ITGAE + PF4 + IL17A + REN + VEGFA | 0,78 |
| IFNG + GNLY + CXCL9 + PF4 + IL17A + TSLP + REN + PROM1 + VEGFA | 0,78 |
| IFNG + GNLY + ITGAE + CXCL9 + PF4 + IL17A + TSLP + REN + VEGFA | 0,78 |
| IFNG + CXCL13 + GNLY + CCL5 + PF4 + IL17A + TSLP + REN + VEGFA | 0,78 |
| GZMH + IFNG + CXCL13 + GNLY + LAG3 + ITGAE + PF4 + IL17A + REN | 0,78 |
| GZMH + IFNG + CXCL13 + GNLY + ITGAE + CXCL9 + PF4 + IL17A + REN | 0,78 |
| GZMH + CXCL13 + GNLY + LAG3 + CCL5 + PF4 + IL17A + TSLP + REN | 0,78 |
| IFNG + CXCL13 + GNLY + LAG3 + ITGAE + PF4 + IL17A + REN + IHH | 0,78 |
| CXCL13 + GNLY + LAG3 + CCL5 + PF4 + IL17A + TSLP + REN + VEGFA | 0,78 |
| GNLY + LAG3 + CCL5 + PF4 + IL17A + TSLP + REN + PROM1 + VEGFA | 0,78 |
| GZMH + CXCL13 + GNLY + LAG3 + CCL5 + PF4 + IL17A + REN + VEGFA | 0,78 |
| IFNG + CXCL13 + GNLY + LAG3 + ITGAE + PF4 + IL17A + REN + VEGFA | 0,78 |
| IFNG + GNLY + ITGAE + CCL5 + PF4 + TSLP + REN + PROM1 + VEGFA | 0,78 |
| GZMH + IFNG + GNLY + ITGAE + CCL5 + PF4 + REN + IHH + PROM1 | 0,78 |
| GZMH + GNLY + CCL5 + CXCL9 + PF4 + IL17A + TSLP + REN + VEGFA | 0,78 |
| GNLY + CCL5 + CXCL9 + PF4 + IL17A + TSLP + REN + PROM1 + VEGFA | 0,78 |
| GZMH + GNLY + LAG3 + CCL5 + PF4 + IL17A + TSLP + REN + PROM1 | 0,78 |
| GZMH + IFNG + CXCL13 + GNLY + ITGAE + PF4 + IL17A + REN + IHH | 0,78 |
| GZMH + IFNG + CXCL13 + GNLY + CXCL9 + PF4 + IL17A + REN + IHH | 0,78 |
| GZMH + IFNG + CXCL13 + GNLY + CXCL9 + PF4 + IL17A + REN + PROM1 | 0,78 |
| IFNG + CXCL13 + GNLY + LAG3 + ITGAE + CXCL9 + PF4 + IL17A + REN | 0,78 |
| GZMH + IFNG + GNLY + ITGAE + CCL5 + PF4 + REN + PROM1 + VEGFA | 0,78 |
| GZMH + CXCL13 + GNLY + CCL5 + PF4 + IL17A + TSLP + REN + VEGFA | 0,78 |
| GNLY + LAG3 + CCL5 + PF4 + IL17A + TSLP + REN + IHH + VEGFA | 0,78 |
| GZMH + IFNG + CXCL13 + GNLY + PF4 + IL17A + REN + IHH + VEGFA | 0,78 |
| GZMH + IFNG + CXCL13 + GNLY + PF4 + IL17A + TSLP + REN + PROM1 | 0,78 |
| IFNG + CXCL13 + GNLY + ITGAE + PF4 + IL17A + REN + PROM1 + VEGFA | 0,78 |
| IFNG + CXCL13 + GNLY + ITGAE + CXCL9 + PF4 + IL17A + TSLP + REN | 0,78 |
| GZMH + IFNG + CXCL13 + GNLY + PF4 + IL17A + REN + PROM1 + VEGFA | 0,78 |
| GZMH + IFNG + GNLY + LAG3 + ITGAE + CCL5 + PF4 + REN + PROM1 | 0,78 |
| IFNG + CXCL13 + GNLY + ITGAE + CXCL9 + PF4 + IL17A + REN + PROM1 | 0,78 |
| IFNG + GNLY + CXCL9 + PF4 + IL17A + TSLP + REN + IHH + PROM1 | 0,78 |
| GZMH + IFNG + CXCL13 + GNLY + CXCL9 + PF4 + IL17A + REN + VEGFA | 0,78 |
| IFNG + CXCL13 + GNLY + ITGAE + PF4 + IL17A + TSLP + REN + VEGFA | 0,78 |
| GZMH + IFNG + CXCL13 + GNLY + LAG3 + PF4 + IL17A + REN + PROM1 | 0,78 |
| IFNG + CXCL13 + GNLY + LAG3 + ITGAE + PF4 + IL17A + TSLP + REN | 0,78 |
| IFNG + CXCL13 + GNLY + LAG3 + CXCL9 + PF4 + IL17A + REN + IHH | 0,78 |
| GZMH + IFNG + CXCL13 + GNLY + PF4 + IL17A + TSLP + REN + IHH | 0,78 |
| GZMH + GNLY + CCL5 + CXCL9 + PF4 + IL17A + REN + PROM1 + VEGFA | 0,78 |
| GZMH + IFNG + CXCL13 + GNLY + PF4 + IL17A + REN + IHH + PROM1 | 0,78 |
| GZMH + IFNG + CXCL13 + GNLY + PF4 + IL17A + TSLP + REN + VEGFA | 0,78 |
| IFNG + CXCL13 + GNLY + ITGAE + CXCL9 + PF4 + IL17A + REN + VEGFA | 0,78 |
| IFNG + CXCL13 + GNLY + ITGAE + PF4 + IL17A + TSLP + REN + PROM1 | 0,78 |
| GZMH + GNLY + CCL5 + PF4 + IL17A + TSLP + REN + IHH + PROM1 | 0,78 |
| IFNG + CXCL13 + GNLY + ITGAE + PF4 + IL17A + REN + IHH + PROM1 | 0,78 |
| GZMH + GNLY + CCL5 + CXCL9 + PF4 + IL17A + TSLP + REN + PROM1 | 0,78 |
| GZMH + IFNG + CXCL13 + GNLY + LAG3 + PF4 + IL17A + REN + IHH | 0,78 |
| IFNG + CXCL13 + GNLY + CXCL9 + PF4 + IL17A + REN + PROM1 + VEGFA | 0,78 |
| IFNG + GNLY + ITGAE + CCL5 + PF4 + TSLP + REN + IHH + PROM1 | 0,78 |
| GNLY + LAG3 + ITGAE + CCL5 + CXCL9 + IL17A + REN + PROM1 + VEGFA | 0,78 |
| IFNG + CXCL13 + GNLY + CXCL9 + PF4 + IL17A + REN + IHH + PROM1 | 0,78 |
| IFNG + CXCL13 + GNLY + LAG3 + PF4 + IL17A + TSLP + REN + PROM1 | 0,78 |
| IFNG + CXCL13 + GNLY + ITGAE + PF4 + IL17A + REN + IHH + VEGFA | 0,78 |
| IFNG + CXCL13 + GNLY + CXCL9 + PF4 + IL17A + REN + IHH + VEGFA | 0,78 |
| IFNG + CXCL13 + GNLY + ITGAE + CXCL9 + PF4 + IL17A + REN + IHH | 0,78 |
| IFNG + CXCL13 + GNLY + PF4 + IL17A + REN + IHH + PROM1 + VEGFA | 0,78 |
| GZMH + IFNG + CXCL13 + GNLY + CXCL9 + PF4 + IL17A + TSLP + REN | 0,78 |
| IFNG + CXCL13 + GNLY + CXCL9 + PF4 + IL17A + TSLP + REN + PROM1 | 0,78 |
| GZMH + IFNG + CXCL13 + GNLY + LAG3 + CXCL9 + PF4 + IL17A + REN | 0,78 |
| IFNG + GNLY + LAG3 + CCL5 + CXCL9 + IL17A + REN + IHH + VEGFA | 0,78 |
| GZMH + GNLY + LAG3 + CCL5 + PF4 + IL17A + TSLP + REN + IHH | 0,78 |
| IFNG + CXCL13 + GNLY + ITGAE + PF4 + IL17A + TSLP + REN + IHH | 0,78 |
| IFNG + CXCL13 + GNLY + LAG3 + PF4 + IL17A + REN + IHH + VEGFA | 0,78 |
| GZMH + IFNG + CXCL13 + GNLY + LAG3 + PF4 + IL17A + REN + VEGFA | 0,78 |
| IFNG + CXCL13 + GNLY + CXCL9 + PF4 + IL17A + TSLP + REN + IHH | 0,78 |
| IFNG + CXCL13 + GNLY + PF4 + IL17A + TSLP + REN + IHH + VEGFA | 0,78 |
| GZMH + GNLY + LAG3 + CCL5 + PF4 + IL17A + REN + PROM1 + VEGFA | 0,78 |
| GNLY + CCL5 + PF4 + IL17A + TSLP + REN + IHH + PROM1 + VEGFA | 0,78 |
| IFNG + CXCL13 + GNLY + CXCL9 + PF4 + IL17A + TSLP + REN + VEGFA | 0,78 |
| IFNG + CXCL13 + GNLY + PF4 + IL17A + TSLP + REN + PROM1 + VEGFA | 0,78 |
| IFNG + CXCL13 + GNLY + LAG3 + CXCL9 + PF4 + IL17A + REN + PROM1 | 0,78 |
| IFNG + CXCL13 + GNLY + PF4 + IL17A + TSLP + REN + IHH + PROM1 | 0,78 |
| IFNG + CXCL13 + GNLY + LAG3 + PF4 + IL17A + REN + IHH + PROM1 | 0,78 |
| IFNG + CXCL13 + GNLY + LAG3 + PF4 + IL17A + TSLP + REN + VEGFA | 0,78 |
| IFNG + CXCL13 + GNLY + LAG3 + CXCL9 + PF4 + IL17A + REN + VEGFA | 0,78 |
| IFNG + CXCL13 + GNLY + LAG3 + PF4 + IL17A + REN + PROM1 + VEGFA | 0,78 |
| IFNG + CXCL13 + GNLY + LAG3 + PF4 + IL17A + TSLP + REN + IHH | 0,78 |
| IFNG + CXCL13 + GNLY + LAG3 + CXCL9 + PF4 + IL17A + TSLP + REN | 0,78 |
| IFNG + GNLY + LAG3 + ITGAE + CCL5 + PF4 + TSLP + REN + PROM1 | 0,78 |
| GZMH + CXCL13 + GNLY + LAG3 + ITGAE + PF4 + IL17A + REN + IHH | 0,78 |
| GZMH + IFNG + CXCL13 + GNLY + LAG3 + PF4 + IL17A + TSLP + REN | 0,78 |
| GZMH + GNLY + LAG3 + CCL5 + PF4 + IL17A + REN + IHH + VEGFA | 0,78 |
| GZMH + GNLY + CCL5 + PF4 + IL17A + REN + IHH + PROM1 + VEGFA | 0,78 |
| GZMH + IFNG + GNLY + LAG3 + CCL5 + CXCL9 + IL17A + REN + VEGFA | 0,78 |
| GZMH + GNLY + ITGAE + CXCL9 + PF4 + IL17A + TSLP + REN + IHH | 0,78 |
| GZMH + IFNG + GNLY + ITGAE + CCL5 + PF4 + TSLP + REN + IHH | 0,78 |
| GZMH + GNLY + LAG3 + CCL5 + PF4 + IL17A + TSLP + REN + VEGFA | 0,78 |
| GNLY + LAG3 + ITGAE + CCL5 + CXCL9 + IL17A + TSLP + REN + VEGFA | 0,78 |
| GZMH + CXCL13 + GNLY + LAG3 + PF4 + IL17A + REN + IHH + VEGFA | 0,78 |
| GZMH + CXCL13 + GNLY + LAG3 + ITGAE + PF4 + IL17A + TSLP + REN | 0,78 |
| GZMH + CXCL13 + GNLY + LAG3 + ITGAE + PF4 + IL17A + REN + VEGFA | 0,78 |
| GZMH + IFNG + GNLY + LAG3 + CCL5 + CXCL9 + IL17A + REN + IHH | 0,78 |
| GZMH + GNLY + LAG3 + ITGAE + CXCL9 + PF4 + IL17A + TSLP + REN | 0,78 |
| GZMH + CXCL13 + GNLY + LAG3 + ITGAE + CXCL9 + PF4 + IL17A + REN | 0,78 |
| GZMH + CXCL13 + GNLY + LAG3 + PF4 + IL17A + TSLP + REN + IHH | 0,78 |
| GZMH + GNLY + CXCL9 + PF4 + IL17A + TSLP + REN + IHH + PROM1 | 0,78 |
| GZMH + GNLY + ITGAE + CXCL9 + PF4 + IL17A + REN + IHH + VEGFA | 0,78 |
| GZMH + GNLY + ITGAE + PF4 + IL17A + TSLP + REN + IHH + PROM1 | 0,78 |
| GZMH + GNLY + ITGAE + PF4 + IL17A + TSLP + REN + IHH + VEGFA | 0,78 |
| GZMH + IFNG + GNLY + ITGAE + CCL5 + PF4 + TSLP + REN + VEGFA | 0,78 |
| IFNG + GNLY + ITGAE + CCL5 + PF4 + REN + IHH + PROM1 + VEGFA | 0,78 |
| GZMH + GNLY + LAG3 + CXCL9 + PF4 + IL17A + TSLP + REN + IHH | 0,78 |
| GNLY + LAG3 + ITGAE + CCL5 + CXCL9 + IL17A + REN + IHH + VEGFA | 0,78 |
| GZMH + GNLY + LAG3 + ITGAE + CXCL9 + PF4 + IL17A + REN + IHH | 0,78 |
| GNLY + ITGAE + CCL5 + CXCL9 + IL17A + REN + IHH + PROM1 + VEGFA | 0,78 |
| GZMH + IFNG + GNLY + LAG3 + CCL5 + PF4 + TSLP + REN + PROM1 | 0,78 |
| GZMH + GNLY + LAG3 + ITGAE + PF4 + IL17A + TSLP + REN + IHH | 0,78 |
| GNLY + ITGAE + CCL5 + CXCL9 + IL17A + TSLP + REN + IHH + VEGFA | 0,78 |
| GZMH + GNLY + ITGAE + CXCL9 + PF4 + IL17A + REN + IHH + PROM1 | 0,78 |
| IFNG + GNLY + LAG3 + CCL5 + CXCL9 + IL17A + TSLP + REN + IHH | 0,78 |
| GZMH + GNLY + ITGAE + PF4 + IL17A + TSLP + REN + PROM1 + VEGFA | 0,78 |
| GZMH + GNLY + LAG3 + ITGAE + PF4 + IL17A + TSLP + REN + PROM1 | 0,78 |
| GZMH + CXCL13 + GNLY + ITGAE + PF4 + IL17A + TSLP + REN + IHH | 0,78 |
| GZMH + GNLY + ITGAE + CXCL9 + PF4 + IL17A + TSLP + REN + VEGFA | 0,78 |
| IFNG + GNLY + LAG3 + CCL5 + CXCL9 + IL17A + TSLP + REN + VEGFA | 0,78 |
| GZMH + GNLY + CXCL9 + PF4 + IL17A + TSLP + REN + IHH + VEGFA | 0,78 |
| GZMH + GNLY + ITGAE + CXCL9 + PF4 + IL17A + TSLP + REN + PROM1 | 0,78 |
| GZMH + CXCL13 + GNLY + LAG3 + ITGAE + PF4 + IL17A + REN + PROM1 | 0,78 |
| CXCL13 + GNLY + LAG3 + PF4 + IL17A + TSLP + REN + IHH + VEGFA | 0,78 |
| GZMH + IFNG + GNLY + LAG3 + CCL5 + CXCL9 + IL17A + TSLP + REN | 0,78 |
| GZMH + IFNG + GNLY + LAG3 + ITGAE + CCL5 + PF4 + TSLP + REN | 0,78 |
| GZMH + GNLY + CXCL9 + PF4 + IL17A + REN + IHH + PROM1 + VEGFA | 0,78 |
| GZMH + CXCL13 + GNLY + LAG3 + PF4 + IL17A + REN + IHH + PROM1 | 0,78 |
| CXCL13 + GNLY + LAG3 + ITGAE + PF4 + IL17A + TSLP + REN + IHH | 0,78 |
| GNLY + ITGAE + CCL5 + CXCL9 + IL17A + TSLP + REN + PROM1 + VEGFA | 0,78 |
| GZMH + GNLY + LAG3 + ITGAE + PF4 + IL17A + TSLP + REN + VEGFA | 0,78 |
| GZMH + CXCL13 + GNLY + LAG3 + CXCL9 + PF4 + IL17A + REN + IHH | 0,78 |
| GZMH + CXCL13 + GNLY + PF4 + IL17A + TSLP + REN + IHH + VEGFA | 0,78 |
| GZMH + CXCL13 + GNLY + CXCL9 + PF4 + IL17A + TSLP + REN + IHH | 0,78 |
| GZMH + CXCL13 + GNLY + PF4 + IL17A + TSLP + REN + IHH + PROM1 | 0,78 |
| GZMH + CXCL13 + GNLY + LAG3 + PF4 + IL17A + TSLP + REN + VEGFA | 0,78 |
| IFNG + GNLY + LAG3 + ITGAE + CCL5 + PF4 + REN + PROM1 + VEGFA | 0,78 |
| CXCL13 + GNLY + LAG3 + PF4 + IL17A + REN + IHH + PROM1 + VEGFA | 0,78 |
| GZMH + CXCL13 + GNLY + ITGAE + PF4 + IL17A + REN + IHH + PROM1 | 0,78 |
| GZMH + GNLY + LAG3 + ITGAE + CXCL9 + PF4 + IL17A + REN + VEGFA | 0,78 |
| GZMH + CXCL13 + GNLY + LAG3 + CXCL9 + PF4 + IL17A + TSLP + REN | 0,78 |
| CXCL13 + GNLY + LAG3 + PF4 + IL17A + TSLP + REN + IHH + PROM1 | 0,78 |
| IFNG + GNLY + LAG3 + ITGAE + CCL5 + PF4 + REN + IHH + PROM1 | 0,78 |
| GZMH + CXCL13 + GNLY + LAG3 + CXCL9 + PF4 + IL17A + REN + VEGFA | 0,78 |
| GZMH + IFNG + GNLY + LAG3 + CCL5 + PF4 + REN + PROM1 + VEGFA | 0,78 |
| CXCL13 + GNLY + LAG3 + CXCL9 + PF4 + IL17A + TSLP + REN + IHH | 0,78 |
| GZMH + CXCL13 + GNLY + ITGAE + CXCL9 + PF4 + IL17A + REN + IHH | 0,78 |
| GZMH + IFNG + GNLY + LAG3 + CCL5 + PF4 + REN + IHH + PROM1 | 0,78 |
| GZMH + GNLY + LAG3 + CXCL9 + PF4 + IL17A + REN + IHH + PROM1 | 0,78 |
| GZMH + GNLY + ITGAE + CXCL9 + PF4 + IL17A + REN + PROM1 + VEGFA | 0,78 |
| GZMH + CXCL13 + GNLY + PF4 + IL17A + REN + IHH + PROM1 + VEGFA | 0,78 |
| IFNG + GNLY + LAG3 + CCL5 + CXCL9 + IL17A + REN + PROM1 + VEGFA | 0,78 |
| GZMH + CXCL13 + GNLY + ITGAE + PF4 + IL17A + REN + IHH + VEGFA | 0,78 |
| GZMH + GNLY + LAG3 + CXCL9 + PF4 + IL17A + REN + IHH + VEGFA | 0,78 |
| GZMH + IFNG + GNLY + LAG3 + CCL5 + CXCL9 + IL17A + REN + PROM1 | 0,78 |
| CXCL13 + GNLY + LAG3 + ITGAE + PF4 + IL17A + REN + IHH + VEGFA | 0,78 |
| GZMH + CXCL13 + GNLY + CXCL9 + PF4 + IL17A + REN + IHH + VEGFA | 0,78 |
| GZMH + CXCL13 + GNLY + ITGAE + CXCL9 + PF4 + IL17A + TSLP + REN | 0,78 |
| CXCL13 + GNLY + LAG3 + CXCL9 + PF4 + IL17A + REN + IHH + VEGFA | 0,78 |
| GZMH + GNLY + ITGAE + PF4 + IL17A + REN + IHH + PROM1 + VEGFA | 0,78 |
| GZMH + GNLY + ITGAE + CCL5 + PF4 + TSLP + REN + IHH + PROM1 | 0,78 |
| GZMH + CXCL13 + GNLY + LAG3 + PF4 + IL17A + TSLP + REN + PROM1 | 0,78 |
| CXCL13 + GNLY + LAG3 + ITGAE + PF4 + IL17A + REN + IHH + PROM1 | 0,78 |
| CXCL13 + GNLY + LAG3 + ITGAE + CCL5 + CXCL9 + IL17A + REN + VEGFA | 0,78 |
| GZMH + GNLY + LAG3 + ITGAE + CXCL9 + PF4 + IL17A + REN + PROM1 | 0,78 |
| GNLY + ITGAE + CCL5 + PF4 + TSLP + REN + IHH + PROM1 + VEGFA | 0,78 |
| GZMH + GNLY + LAG3 + ITGAE + PF4 + IL17A + REN + IHH + PROM1 | 0,78 |
| GZMH + CXCL13 + GNLY + ITGAE + PF4 + IL17A + TSLP + REN + VEGFA | 0,78 |
| GZMH + GNLY + CCL5 + PF4 + IL17A + TSLP + REN + IHH + VEGFA | 0,78 |
| GZMH + CXCL13 + GNLY + CXCL9 + PF4 + IL17A + REN + IHH + PROM1 | 0,78 |
| CXCL13 + GNLY + ITGAE + CCL5 + CXCL9 + IL17A + REN + PROM1 + VEGFA | 0,78 |
| GZMH + CXCL13 + GNLY + LAG3 + PF4 + IL17A + REN + PROM1 + VEGFA | 0,78 |
| CXCL13 + GNLY + LAG3 + ITGAE + CXCL9 + PF4 + IL17A + REN + IHH | 0,78 |
| GZMH + CXCL13 + GNLY + ITGAE + PF4 + IL17A + TSLP + REN + PROM1 | 0,78 |
| GNLY + ITGAE + CCL5 + CXCL9 + PF4 + TSLP + REN + IHH + PROM1 | 0,78 |
| GZMH + IFNG + GNLY + CCL5 + PF4 + TSLP + REN + IHH + PROM1 | 0,78 |
| IFNG + GNLY + LAG3 + ITGAE + CCL5 + PF4 + TSLP + REN + IHH | 0,78 |
| GNLY + LAG3 + ITGAE + CCL5 + CXCL9 + IL17A + TSLP + REN + PROM1 | 0,78 |
| GZMH + GNLY + LAG3 + ITGAE + CCL5 + CXCL9 + IL17A + REN + VEGFA | 0,78 |
| GZMH + GNLY + CXCL9 + PF4 + IL17A + TSLP + REN + PROM1 + VEGFA | 0,78 |
| GZMH + IFNG + GNLY + CCL5 + PF4 + TSLP + REN + PROM1 + VEGFA | 0,78 |
| IFNG + GNLY + ITGAE + CCL5 + PF4 + TSLP + REN + IHH + VEGFA | 0,78 |
| GZMH + CXCL13 + GNLY + LAG3 + ITGAE + CCL5 + IL17A + REN + PROM1 | 0,78 |
| GZMH + CXCL13 + GNLY + CXCL9 + PF4 + IL17A + TSLP + REN + VEGFA | 0,78 |
| GZMH + GNLY + ITGAE + CCL5 + CXCL9 + IL17A + REN + PROM1 + VEGFA | 0,78 |
| GZMH + GNLY + LAG3 + CXCL9 + PF4 + IL17A + TSLP + REN + VEGFA | 0,78 |
| GZMH + GNLY + LAG3 + ITGAE + PF4 + IL17A + REN + IHH + VEGFA | 0,78 |
| GZMH + CXCL13 + GNLY + PF4 + IL17A + TSLP + REN + PROM1 + VEGFA | 0,78 |
| GZMH + CXCL13 + GNLY + CXCL9 + PF4 + IL17A + TSLP + REN + PROM1 | 0,78 |

| **Table 10: clusters of 10 genes:** | |
|---|---|
| **clusters of 10 genes** | **c_tau level** |
| GZMH + IFNG + GNLY + ITGAE + CCL5 + PF4 + IL17A + REN + PROM1 + VEGFA | 0,8 |
| GZMH + IFNG + GNLY + ITGAE + CCL5 + PF4 + IL17A + REN + IHH + PROM1 | 0,8 |
| GZMH + IFNG + GNLY + ITGAE + CCL5 + PF4 + IL17A + TSLP + REN + PROM1 | 0,8 |
| GZMH + IFNG + GNLY + LAG3 + ITGAE + CCL5 + PF4 + IL17A + REN + PROM1 | 0,8 |
| IFNG + GNLY + ITGAE + CCL5 + PF4 + IL17A + TSLP + REN + PROM1 + VEGFA | 0,8 |
| GZMH + IFNG + GNLY + ITGAE + CCL5 + PF4 + IL17A + REN + IHH + VEGFA | 0,8 |
| IFNG + GNLY + ITGAE + CCL5 + PF4 + IL17A + REN + IHH + PROM1 + VEGFA | 0,8 |
| IFNG + GNLY + ITGAE + CCL5 + PF4 + IL17A + TSLP + REN + IHH + PROM1 | 0,8 |
| IFNG + GNLY + LAG3 + ITGAE + CCL5 + PF4 + IL17A + REN + PROM1 + VEGFA | 0,8 |
| GZMH + IFNG + GNLY + ITGAE + CCL5 + CXCL9 + PF4 + IL17A + REN + VEGFA | 0,8 |
| GZMH + IFNG + GNLY + ITGAE + CCL5 + PF4 + IL17A + TSLP + REN + VEGFA | 0,8 |
| IFNG + GNLY + LAG3 + ITGAE + CCL5 + PF4 + IL17A + TSLP + REN + PROM1 | 0,8 |
| IFNG + GNLY + LAG3 + ITGAE + CCL5 + PF4 + IL17A + REN + IHH + PROM1 | 0,8 |
| GZMH + IFNG + GNLY + ITGAE + CCL5 + PF4 + IL17A + TSLP + REN + IHH | 0,8 |
| GZMH + IFNG + GNLY + ITGAE + CCL5 + CXCL9 + PF4 + IL17A + REN + IHH | 0,8 |
| GZMH + IFNG + GNLY + ITGAE + CCL5 + CXCL9 + PF4 + IL17A + REN + PROM1 | 0,8 |
| GZMH + IFNG + GNLY + LAG3 + ITGAE + CCL5 + PF4 + IL17A + REN + VEGFA | 0,8 |
| IFNG + GNLY + ITGAE + CCL5 + CXCL9 + PF4 + IL17A + REN + IHH + VEGFA | 0,8 |
| IFNG + GNLY + LAG3 + ITGAE + CCL5 + CXCL9 + PF4 + IL17A + REN + VEGFA | 0,8 |
| GZMH + IFNG + GNLY + LAG3 + ITGAE + CCL5 + CXCL9 + PF4 + IL17A + REN | 0,8 |
| IFNG + GNLY + LAG3 + ITGAE + CCL5 + PF4 + IL17A + REN + IHH + VEGFA | 0,8 |
| IFNG + GNLY + ITGAE + CCL5 + CXCL9 + PF4 + IL17A + TSLP + REN + VEGFA | 0,8 |
| IFNG + GNLY + ITGAE + CCL5 + PF4 + IL17A + TSLP + REN + IHH + VEGFA | 0,8 |
| IFNG + GNLY + LAG3 + ITGAE + CCL5 + PF4 + IL17A + TSLP + REN + VEGFA | 0,8 |
| GZMH + IFNG + GNLY + LAG3 + ITGAE + CCL5 + PF4 + IL17A + REN + IHH | 0,8 |
| IFNG + GNLY + LAG3 + ITGAE + CCL5 + CXCL9 + PF4 + IL17A + REN + IHH | 0,8 |
| GZMH + IFNG + GNLY + ITGAE + CCL5 + CXCL9 + PF4 + IL17A + TSLP + REN | 0,8 |
| GZMH + IFNG + GNLY + LAG3 + ITGAE + CCL5 + PF4 + IL17A + TSLP + REN | 0,8 |
| GZMH + IFNG + CXCL13 + GNLY + ITGAE + CCL5 + PF4 + IL17A + REN + PROM1 | 0,79 |
| IFNG + GNLY + ITGAE + CCL5 + CXCL9 + PF4 + IL17A + TSLP + REN + IHH | 0,79 |
| IFNG + GNLY + LAG3 + ITGAE + CCL5 + PF4 + IL17A + TSLP + REN + IHH | 0,79 |
| IFNG + GNLY + ITGAE + CCL5 + CXCL9 + PF4 + IL17A + REN + PROM1 + VEGFA | 0,79 |
| IFNG + GNLY + LAG3 + ITGAE + CCL5 + CXCL9 + PF4 + IL17A + TSLP + REN | 0,79 |
| IFNG + GNLY + ITGAE + CCL5 + CXCL9 + PF4 + IL17A + REN + IHH + PROM1 | 0,79 |
| IFNG + GNLY + ITGAE + CCL5 + CXCL9 + PF4 + IL17A + TSLP + REN + PROM1 | 0,79 |
| IFNG + CXCL13 + GNLY + ITGAE + CCL5 + PF4 + IL17A + REN + IHH + PROM1 | 0,79 |
| IFNG + CXCL13 + GNLY + ITGAE + CCL5 + PF4 + IL17A + REN + PROM1 + VEGFA | 0,79 |
| IFNG + GNLY + LAG3 + ITGAE + CCL5 + CXCL9 + PF4 + IL17A + REN + PROM1 | 0,79 |
| IFNG + CXCL13 + GNLY + LAG3 + ITGAE + CCL5 + PF4 + IL17A + REN + PROM1 | 0,79 |
| IFNG + CXCL13 + GNLY + ITGAE + CCL5 + PF4 + IL17A + TSLP + REN + PROM1 | 0,79 |
| IFNG + CXCL13 + GNLY + ITGAE + CCL5 + CXCL9 + PF4 + IL17A + REN + VEGFA | 0,79 |
| IFNG + CXCL13 + GNLY + ITGAE + CCL5 + PF4 + IL17A + REN + IHH + VEGFA | 0,79 |
| IFNG + CXCL13 + GNLY + ITGAE + CCL5 + CXCL9 + PF4 + IL17A + REN + PROM1 | 0,79 |
| GZMH + IFNG + CXCL13 + GNLY + ITGAE + CCL5 + PF4 + IL17A + REN + IHH | 0,79 |
| IFNG + CXCL13 + GNLY + LAG3 + ITGAE + CCL5 + PF4 + IL17A + REN + IHH | 0,79 |
| GZMH + IFNG + CXCL13 + GNLY + ITGAE + CCL5 + PF4 + IL17A + REN + VEGFA | 0,79 |
| IFNG + CXCL13 + GNLY + ITGAE + CCL5 + CXCL9 + PF4 + IL17A + REN + IHH | 0,79 |
| IFNG + CXCL13 + GNLY + LAG3 + ITGAE + CCL5 + PF4 + IL17A + REN + VEGFA | 0,79 |
| GZMH + IFNG + GNLY + LAG3 + CCL5 + PF4 + IL17A + REN + IHH + PROM1 | 0,79 |
| IFNG + CXCL13 + GNLY + ITGAE + CCL5 + PF4 + IL17A + TSLP + REN + VEGFA | 0,79 |
| IFNG + CXCL13 + GNLY + ITGAE + CCL5 + PF4 + IL17A + TSLP + REN + IHH | 0,79 |
| IFNG + CXCL13 + GNLY + LAG3 + ITGAE + CCL5 + CXCL9 + PF4 + IL17A + REN | 0,79 |
| GZMH + IFNG + CXCL13 + GNLY + ITGAE + CCL5 + CXCL9 + PF4 + IL17A + REN | 0,79 |
| GZMH + IFNG + CXCL13 + GNLY + ITGAE + CCL5 + PF4 + IL17A + TSLP + REN | 0,79 |
| GZMH + IFNG + GNLY + CCL5 + CXCL9 + PF4 + IL17A + REN + IHH + VEGFA | 0,79 |
| GZMH + IFNG + CXCL13 + GNLY + LAG3 + ITGAE + CCL5 + PF4 + IL17A + REN | 0,79 |
| GZMH + IFNG + GNLY + LAG3 + ITGAE + CCL5 + IL17A + REN + IHH + VEGFA | 0,79 |
| GZMH + IFNG + GNLY + LAG3 + CCL5 + PF4 + IL17A + REN + PROM1 + VEGFA | 0,79 |
| GZMH + IFNG + GNLY + LAG3 + CCL5 + CXCL9 + PF4 + IL17A + REN + IHH | 0,79 |
| GZMH + IFNG + GNLY + LAG3 + ITGAE + CCL5 + IL17A + REN + PROM1 + VEGFA | 0,79 |
| IFNG + CXCL13 + GNLY + LAG3 + ITGAE + CCL5 + PF4 + IL17A + TSLP + REN | 0,79 |
| IFNG + CXCL13 + GNLY + ITGAE + CCL5 + CXCL9 + PF4 + IL17A + TSLP + REN | 0,79 |
| CXCL13 + GNLY + LAG3 + ITGAE + CCL5 + PF4 + IL17A + REN + IHH + PROM1 | 0,79 |
| CXCL13 + GNLY + ITGAE + CCL5 + CXCL9 + PF4 + IL17A + REN + IHH + PROM1 | 0,79 |
| CXCL13 + GNLY + ITGAE + CCL5 + PF4 + IL17A + TSLP + REN + IHH + PROM1 | 0,79 |
| GZMH + CXCL13 + GNLY + ITGAE + CCL5 + PF4 + IL17A + REN + IHH + PROM1 | 0,79 |
| GNLY + ITGAE + CCL5 + CXCL9 + PF4 + IL17A + TSLP + REN + PROM1 + VEGFA | 0,79 |
| GZMH + IFNG + GNLY + LAG3 + ITGAE + CCL5 + CXCL9 + IL17A + REN + VEGFA | 0,79 |
| GZMH + IFNG + GNLY + LAG3 + CCL5 + CXCL9 + PF4 + IL17A + REN + VEGFA | 0,79 |
| IFNG + GNLY + LAG3 + CCL5 + CXCL9 + PF4 + IL17A + REN + IHH + VEGFA | 0,79 |
| GZMH + GNLY + ITGAE + CCL5 + CXCL9 + PF4 + IL17A + TSLP + REN + VEGFA | 0,79 |
| GNLY + ITGAE + CCL5 + CXCL9 + PF4 + IL17A + TSLP + REN + IHH + VEGFA | 0,79 |
| CXCL13 + GNLY + ITGAE + CCL5 + PF4 + IL17A + REN + IHH + PROM1 + VEGFA | 0,79 |
| GZMH + IFNG + GNLY + LAG3 + ITGAE + CCL5 + IL17A + REN + IHH + PROM1 | 0,79 |
| GZMH + CXCL13 + GNLY + ITGAE + CCL5 + PF4 + IL17A + TSLP + REN + PROM1 | 0,79 |
| GZMH + IFNG + GNLY + LAG3 + CCL5 + PF4 + IL17A + TSLP + REN + PROM1 | 0,79 |
| CXCL13 + GNLY + LAG3 + ITGAE + CCL5 + PF4 + IL17A + TSLP + REN + PROM1 | 0,79 |
| GZMH + IFNG + GNLY + ITGAE + CCL5 + CXCL9 + IL17A + REN + IHH + VEGFA | 0,79 |
| IFNG + GNLY + LAG3 + ITGAE + CCL5 + CXCL9 + IL17A + REN + PROM1 + VEGFA | 0,79 |
| GNLY + ITGAE + CCL5 + CXCL9 + PF4 + IL17A + TSLP + REN + IHH + PROM1 | 0,79 |
| GNLY + LAG3 + ITGAE + CCL5 + CXCL9 + PF4 + IL17A + TSLP + REN + VEGFA | 0,79 |
| GZMH + CXCL13 + GNLY + ITGAE + CCL5 + CXCL9 + PF4 + IL17A + REN + PROM1 | 0,79 |
| GNLY + ITGAE + CCL5 + CXCL9 + PF4 + IL17A + REN + IHH + PROM1 + VEGFA | 0,79 |
| GNLY + LAG3 + ITGAE + CCL5 + PF4 + IL17A + TSLP + REN + PROM1 + VEGFA | 0,79 |
| GZMH + GNLY + ITGAE + CCL5 + CXCL9 + PF4 + IL17A + REN + IHH + VEGFA | 0,79 |
| GZMH + IFNG + GNLY + ITGAE + CCL5 + CXCL9 + IL17A + TSLP + REN + VEGFA | 0,79 |
| CXCL13 + GNLY + LAG3 + ITGAE + CCL5 + PF4 + IL17A + REN + PROM1 + VEGFA | 0,79 |
| GZMH + IFNG + GNLY + LAG3 + ITGAE + CCL5 + IL17A + TSLP + REN + PROM1 | 0,79 |
| GZMH + IFNG + GNLY + ITGAE + CCL5 + CXCL9 + IL17A + REN + PROM1 + VEGFA | 0,79 |
| CXCL13 + GNLY + LAG3 + ITGAE + CCL5 + CXCL9 + PF4 + IL17A + REN + PROM1 | 0,79 |
| GZMH + IFNG + GNLY + CCL5 + CXCL9 + PF4 + IL17A + TSLP + REN + IHH | 0,79 |
| GNLY + LAG3 + ITGAE + CCL5 + PF4 + IL17A + TSLP + REN + IHH + PROM1 | 0,79 |
| CXCL13 + GNLY + ITGAE + CCL5 + CXCL9 + PF4 + IL17A + REN + PROM1 + VEGFA | 0,79 |
| CXCL13 + GNLY + ITGAE + CCL5 + PF4 + IL17A + TSLP + REN + PROM1 + VEGFA | 0,79 |
| GZMH + IFNG + GNLY + CCL5 + PF4 + IL17A + REN + IHH + PROM1 + VEGFA | 0,79 |
| GZMH + IFNG + GNLY + LAG3 + CCL5 + PF4 + IL17A + REN + IHH + VEGFA | 0,79 |
| GZMH + GNLY + ITGAE + CCL5 + CXCL9 + PF4 + IL17A + REN + PROM1 + VEGFA | 0,79 |
| GZMH + CXCL13 + GNLY + ITGAE + CCL5 + PF4 + IL17A + REN + PROM1 + VEGFA | 0,79 |
| GZMH + GNLY + LAG3 + ITGAE + CCL5 + CXCL9 + PF4 + IL17A + REN + VEGFA | 0,79 |
| GZMH + IFNG + GNLY + LAG3 + CCL5 + CXCL9 + PF4 + IL17A + TSLP + REN | 0,79 |
| GZMH + IFNG + GNLY + LAG3 + ITGAE + CCL5 + IL17A + TSLP + REN + IHH | 0,79 |
| GNLY + LAG3 + ITGAE + CCL5 + CXCL9 + PF4 + IL17A + REN + PROM1 + VEGFA | 0,79 |
| IFNG + GNLY + LAG3 + CCL5 + CXCL9 + PF4 + IL17A + TSLP + REN + IHH | 0,79 |
| GNLY + LAG3 + ITGAE + CCL5 + PF4 + IL17A + REN + IHH + PROM1 + VEGFA | 0,79 |
| GZMH + CXCL13 + GNLY + LAG3 + ITGAE + CCL5 + PF4 + IL17A + REN + PROM1 | 0,79 |
| IFNG + GNLY + LAG3 + ITGAE + CCL5 + CXCL9 + IL17A + REN + IHH + VEGFA | 0,79 |
| GNLY + ITGAE + CCL5 + PF4 + IL17A + TSLP + REN + IHH + PROM1 + VEGFA | 0,79 |
| GZMH + IFNG + GNLY + CCL5 + PF4 + IL17A + TSLP + REN + IHH + PROM1 | 0,79 |
| GNLY + LAG3 + ITGAE + CCL5 + CXCL9 + PF4 + IL17A + REN + IHH + VEGFA | 0,79 |
| IFNG + GNLY + CCL5 + CXCL9 + PF4 + IL17A + TSLP + REN + IHH + VEGFA | 0,79 |
| CXCL13 + GNLY + ITGAE + CCL5 + CXCL9 + PF4 + IL17A + REN + IHH + VEGFA | 0,79 |
| GZMH + IFNG + GNLY + LAG3 + ITGAE + CCL5 + CXCL9 + IL17A + REN + IHH | 0,79 |
| GZMH + GNLY + ITGAE + CCL5 + PF4 + IL17A + TSLP + REN + IHH + PROM1 | 0,79 |
| GZMH + IFNG + GNLY + LAG3 + CCL5 + PF4 + IL17A + TSLP + REN + IHH | 0,79 |
| GZMH + IFNG + GNLY + LAG3 + CCL5 + CXCL9 + PF4 + IL17A + REN + PROM1 | 0,79 |
| GZMH + GNLY + ITGAE + CCL5 + CXCL9 + PF4 + IL17A + TSLP + REN + PROM1 | 0,79 |
| GZMH + IFNG + GNLY + LAG3 + ITGAE + CCL5 + IL17A + TSLP + REN + VEGFA | 0,79 |
| CXCL13 + GNLY + ITGAE + CCL5 + CXCL9 + PF4 + IL17A + TSLP + REN + PROM1 | 0,79 |
| GNLY + LAG3 + ITGAE + CCL5 + CXCL9 + PF4 + IL17A + TSLP + REN + PROM1 | 0,79 |
| GZMH + IFNG + GNLY + CCL5 + CXCL9 + PF4 + IL17A + TSLP + REN + VEGFA | 0,79 |
| IFNG + GNLY + LAG3 + CCL5 + CXCL9 + PF4 + IL17A + TSLP + REN + VEGFA | 0,79 |
| GZMH + IFNG + GNLY + LAG3 + CCL5 + PF4 + IL17A + TSLP + REN + VEGFA | 0,79 |
| GZMH + GNLY + ITGAE + CCL5 + PF4 + IL17A + TSLP + REN + PROM1 + VEGFA | 0,79 |
| IFNG + GNLY + LAG3 + CCL5 + PF4 + IL17A + TSLP + REN + IHH + PROM1 | 0,79 |
| IFNG + GNLY + LAG3 + ITGAE + CCL5 + CXCL9 + IL17A + TSLP + REN + VEGFA | 0,79 |
| IFNG + GNLY + LAG3 + CCL5 + PF4 + IL17A + REN + IHH + PROM1 + VEGFA | 0,79 |
| IFNG + GNLY + LAG3 + ITGAE + CCL5 + IL17A + TSLP + REN + PROM1 + VEGFA | 0,79 |
| GZMH + GNLY + ITGAE + CCL5 + PF4 + IL17A + REN + IHH + PROM1 + VEGFA | 0,79 |
| GZMH + IFNG + GNLY + ITGAE + CCL5 + CXCL9 + IL17A + TSLP + REN + IHH | 0,79 |
| CXCL13 + GNLY + ITGAE + CCL5 + PF4 + IL17A + TSLP + REN + IHH + VEGFA | 0,79 |
| CXCL13 + GNLY + LAG3 + ITGAE + CCL5 + PF4 + IL17A + REN + IHH + VEGFA | 0,79 |
| IFNG + GNLY + LAG3 + CCL5 + PF4 + IL17A + TSLP + REN + PROM1 + VEGFA | 0,79 |
| IFNG + GNLY + ITGAE + CCL5 + CXCL9 + IL17A + REN + IHH + PROM1 + VEGFA | 0,79 |
| IFNG + GNLY + LAG3 + CCL5 + PF4 + IL17A + TSLP + REN + IHH + VEGFA | 0,79 |
| CXCL13 + GNLY + LAG3 + ITGAE + CCL5 + CXCL9 + PF4 + IL17A + REN + VEGFA | 0,79 |
| CXCL13 + GNLY + ITGAE + CCL5 + CXCL9 + PF4 + IL17A + TSLP + REN + VEGFA | 0,79 |
| GZMH + GNLY + LAG3 + ITGAE + CCL5 + PF4 + IL17A + TSLP + REN + PROM1 | 0,79 |
| CXCL13 + GNLY + LAG3 + ITGAE + CCL5 + CXCL9 + PF4 + IL17A + REN + IHH | 0,79 |
| GZMH + CXCL13 + GNLY + ITGAE + CCL5 + CXCL9 + PF4 + IL17A + REN + IHH | 0,79 |
| GNLY + LAG3 + ITGAE + CCL5 + CXCL9 + PF4 + IL17A + REN + IHH + PROM1 | 0,79 |
| GZMH + IFNG + GNLY + LAG3 + ITGAE + CCL5 + CXCL9 + IL17A + REN + PROM1 | 0,79 |
| IFNG + GNLY + LAG3 + ITGAE + CCL5 + IL17A + REN + IHH + PROM1 + VEGFA | 0,79 |
| GZMH + IFNG + GNLY + CCL5 + PF4 + IL17A + TSLP + REN + IHH + VEGFA | 0,79 |
| GZMH + GNLY + LAG3 + ITGAE + CCL5 + PF4 + IL17A + REN + PROM1 + VEGFA | 0,79 |
| IFNG + GNLY + LAG3 + ITGAE + CCL5 + CXCL9 + IL17A + TSLP + REN + IHH | 0,79 |
| GZMH + IFNG + GNLY + LAG3 + ITGAE + CCL5 + CXCL9 + IL17A + TSLP + REN | 0,79 |
| IFNG + GNLY + ITGAE + CCL5 + CXCL9 + IL17A + TSLP + REN + PROM1 + VEGFA | 0,79 |
| GZMH + GNLY + LAG3 + ITGAE + CCL5 + CXCL9 + PF4 + IL17A + REN + PROM1 | 0,79 |
| CXCL13 + GNLY + ITGAE + CCL5 + CXCL9 + PF4 + IL17A + TSLP + REN + IHH | 0,79 |
| IFNG + GNLY + ITGAE + CCL5 + CXCL9 + IL17A + TSLP + REN + IHH + VEGFA | 0,79 |
| GZMH + CXCL13 + GNLY + LAG3 + ITGAE + CCL5 + PF4 + IL17A + REN + IHH | 0,79 |
| GZMH + GNLY + ITGAE + CCL5 + CXCL9 + PF4 + IL17A + REN + IHH + PROM1 | 0,79 |
| GZMH + IFNG + CXCL13 + GNLY + LAG3 + ITGAE + CCL5 + IL17A + REN + PROM1 | 0,79 |
| GZMH + IFNG + GNLY + CCL5 + PF4 + IL17A + TSLP + REN + PROM1 + VEGFA | 0,79 |
| GZMH + GNLY + LAG3 + ITGAE + CCL5 + PF4 + IL17A + REN + IHH + PROM1 | 0,79 |
| GZMH + CXCL13 + GNLY + ITGAE + CCL5 + PF4 + IL17A + REN + IHH + VEGFA | 0,79 |
| GZMH + CXCL13 + GNLY + ITGAE + CCL5 + PF4 + IL17A + TSLP + REN + IHH | 0,79 |
| IFNG + GNLY + LAG3 + ITGAE + CCL5 + IL17A + TSLP + REN + IHH + PROM1 | 0,79 |
| GZMH + GNLY + ITGAE + CCL5 + CXCL9 + PF4 + IL17A + TSLP + REN + IHH | 0,79 |
| GZMH + GNLY + LAG3 + ITGAE + CCL5 + CXCL9 + PF4 + IL17A + TSLP + REN | 0,79 |
| IFNG + CXCL13 + GNLY + LAG3 + ITGAE + CCL5 + CXCL9 + IL17A + REN + VEGFA | 0,79 |
| GZMH + IFNG + GNLY + CCL5 + CXCL9 + PF4 + IL17A + REN + IHH + PROM1 | 0,79 |
| IFNG + GNLY + LAG3 + CCL5 + CXCL9 + PF4 + IL17A + REN + PROM1 + VEGFA | 0,79 |
| GZMH + IFNG + GNLY + ITGAE + CCL5 + IL17A + TSLP + REN + IHH + VEGFA | 0,79 |
| GZMH + CXCL13 + GNLY + ITGAE + CCL5 + CXCL9 + PF4 + IL17A + REN + VEGFA | 0,79 |
| GZMH + IFNG + CXCL13 + GNLY + CCL5 + CXCL9 + PF4 + IL17A + REN + IHH | 0,79 |
| GZMH + IFNG + GNLY + CCL5 + CXCL9 + PF4 + IL17A + REN + PROM1 + VEGFA | 0,79 |
| CXCL13 + GNLY + LAG3 + ITGAE + CCL5 + PF4 + IL17A + TSLP + REN + IHH | 0,79 |
| GZMH + IFNG + GNLY + ITGAE + CCL5 + IL17A + TSLP + REN + PROM1 + VEGFA | 0,79 |
| IFNG + GNLY + LAG3 + ITGAE + CCL5 + CXCL9 + IL17A + TSLP + REN + PROM1 | 0,79 |
| GNLY + LAG3 + ITGAE + CCL5 + CXCL9 + PF4 + IL17A + TSLP + REN + IHH | 0,79 |
| GZMH + IFNG + GNLY + ITGAE + CCL5 + IL17A + REN + IHH + PROM1 + VEGFA | 0,79 |
| IFNG + GNLY + LAG3 + ITGAE + CCL5 + IL17A + TSLP + REN + IHH + VEGFA | 0,79 |
| CXCL13 + GNLY + LAG3 + ITGAE + CCL5 + CXCL9 + PF4 + IL17A + TSLP + REN | 0,79 |
| GZMH + IFNG + CXCL13 + GNLY + ITGAE + CCL5 + CXCL9 + IL17A + REN + VEGFA | 0,79 |
| IFNG + GNLY + LAG3 + CCL5 + CXCL9 + PF4 + IL17A + REN + IHH + PROM1 | 0,79 |
| GZMH + IFNG + CXCL13 + GNLY + LAG3 + CCL5 + PF4 + IL17A + REN + IHH | 0,79 |
| GZMH + IFNG + CXCL13 + GNLY + ITGAE + CCL5 + IL17A + REN + PROM1 + VEGFA | 0,79 |
| IFNG + GNLY + LAG3 + CCL5 + CXCL9 + PF4 + IL17A + TSLP + REN + PROM1 | 0,79 |
| GZMH + IFNG + CXCL13 + GNLY + CCL5 + PF4 + IL17A + TSLP + REN + IHH | 0,79 |
| IFNG + GNLY + CCL5 + CXCL9 + PF4 + IL17A + REN + IHH + PROM1 + VEGFA | 0,79 |
| CXCL13 + GNLY + LAG3 + ITGAE + CCL5 + PF4 + IL17A + TSLP + REN + VEGFA | 0,79 |
| GZMH + IFNG + GNLY + ITGAE + CCL5 + CXCL9 + IL17A + REN + IHH + PROM1 | 0,79 |
| GZMH + IFNG + GNLY + LAG3 + ITGAE + PF4 + IL17A + TSLP + REN + PROM1 | 0,79 |
| GZMH + CXCL13 + GNLY + LAG3 + ITGAE + CCL5 + CXCL9 + PF4 + IL17A + REN | 0,79 |
| IFNG + GNLY + LAG3 + ITGAE + CCL5 + CXCL9 + IL17A + REN + IHH + PROM1 | 0,79 |
| GZMH + CXCL13 + GNLY + ITGAE + CCL5 + CXCL9 + PF4 + IL17A + TSLP + REN | 0,79 |
| GZMH + IFNG + GNLY + ITGAE + CCL5 + IL17A + TSLP + REN + IHH + PROM1 | 0,79 |
| GZMH + IFNG + CXCL13 + GNLY + CCL5 + PF4 + IL17A + REN + IHH + VEGFA | 0,79 |
| IFNG + CXCL13 + GNLY + ITGAE + CCL5 + CXCL9 + IL17A + REN + IHH + VEGFA | 0,79 |
| GZMH + IFNG + CXCL13 + GNLY + LAG3 + CCL5 + PF4 + IL17A + REN + PROM1 | 0,79 |
| IFNG + CXCL13 + GNLY + ITGAE + CCL5 + CXCL9 + IL17A + TSLP + REN + VEGFA | 0,79 |
| GNLY + LAG3 + CCL5 + CXCL9 + PF4 + IL17A + REN + IHH + PROM1 + VEGFA | 0,79 |
| IFNG + CXCL13 + GNLY + LAG3 + CCL5 + CXCL9 + PF4 + IL17A + REN + IHH | 0,79 |
| GZMH + CXCL13 + GNLY + LAG3 + CCL5 + PF4 + IL17A + REN + IHH + PROM1 | 0,79 |
| GNLY + LAG3 + CCL5 + CXCL9 + PF4 + IL17A + TSLP + REN + IHH + VEGFA | 0,79 |
| GZMH + IFNG + GNLY + LAG3 + ITGAE + PF4 + IL17A + REN + PROM1 + VEGFA | 0,79 |
| GZMH + IFNG + CXCL13 + GNLY + LAG3 + ITGAE + CCL5 + IL17A + REN + VEGFA | 0,79 |
| GZMH + CXCL13 + GNLY + LAG3 + CCL5 + CXCL9 + PF4 + IL17A + REN + IHH | 0,79 |
| GZMH + IFNG + CXCL13 + GNLY + CCL5 + PF4 + IL17A + REN + IHH + PROM1 | 0,79 |
| IFNG + CXCL13 + GNLY + CCL5 + CXCL9 + PF4 + IL17A + REN + IHH + VEGFA | 0,79 |
| GZMH + IFNG + CXCL13 + GNLY + ITGAE + CCL5 + IL17A + REN + IHH + PROM1 | 0,79 |
| IFNG + GNLY + LAG3 + ITGAE + PF4 + IL17A + TSLP + REN + PROM1 + VEGFA | 0,79 |
| GZMH + GNLY + LAG3 + CCL5 + CXCL9 + PF4 + IL17A + REN + IHH + VEGFA | 0,79 |
| GZMH + IFNG + GNLY + LAG3 + ITGAE + PF4 + IL17A + TSLP + REN + VEGFA | 0,79 |
| GZMH + CXCL13 + GNLY + CCL5 + CXCL9 + PF4 + IL17A + REN + IHH + PROM1 | 0,79 |
| GZMH + IFNG + GNLY + ITGAE + CCL5 + CXCL9 + IL17A + TSLP + REN + PROM1 | 0,79 |
| GZMH + IFNG + GNLY + LAG3 + ITGAE + CXCL9 + PF4 + IL17A + REN + IHH | 0,79 |
| GZMH + CXCL13 + GNLY + CCL5 + CXCL9 + PF4 + IL17A + TSLP + REN + IHH | 0,79 |
| IFNG + GNLY + LAG3 + ITGAE + CXCL9 + PF4 + IL17A + REN + IHH + VEGFA | 0,79 |
| GZMH + IFNG + GNLY + CCL5 + CXCL9 + PF4 + IL17A + TSLP + REN + PROM1 | 0,79 |
| GZMH + IFNG + CXCL13 + GNLY + LAG3 + CCL5 + CXCL9 + PF4 + IL17A + REN | 0,79 |
| CXCL13 + GNLY + LAG3 + CCL5 + CXCL9 + PF4 + IL17A + REN + IHH + VEGFA | 0,79 |
| GZMH + IFNG + GNLY + LAG3 + ITGAE + PF4 + IL17A + REN + IHH + PROM1 | 0,79 |
| GZMH + IFNG + GNLY + ITGAE + PF4 + IL17A + TSLP + REN + IHH + VEGFA | 0,79 |
| GZMH + CXCL13 + GNLY + CCL5 + CXCL9 + PF4 + IL17A + REN + IHH + VEGFA | 0,79 |
| GZMH + GNLY + LAG3 + ITGAE + CCL5 + CXCL9 + PF4 + IL17A + REN + IHH | 0,79 |
| GZMH + CXCL13 + GNLY + ITGAE + CCL5 + PF4 + IL17A + TSLP + REN + VEGFA | 0,79 |
| GZMH + IFNG + CXCL13 + GNLY + ITGAE + CCL5 + IL17A + TSLP + REN + PROM1 | 0,79 |
| IFNG + GNLY + LAG3 + ITGAE + PF4 + IL17A + REN + IHH + PROM1 + VEGFA | 0,79 |
| GZMH + IFNG + GNLY + LAG3 + ITGAE + PF4 + IL17A + TSLP + REN + IHH | 0,79 |
| GZMH + CXCL13 + GNLY + LAG3 + CCL5 + PF4 + IL17A + TSLP + REN + IHH | 0,78 |
| IFNG + CXCL13 + GNLY + LAG3 + ITGAE + CCL5 + CXCL9 + IL17A + REN + PROM1 | 0,78 |
| GZMH + CXCL13 + GNLY + CCL5 + PF4 + IL17A + REN + IHH + PROM1 + VEGFA | 0,78 |
| IFNG + GNLY + CCL5 + PF4 + IL17A + TSLP + REN + IHH + PROM1 + VEGFA | 0,78 |
| IFNG + GNLY + LAG3 + ITGAE + CXCL9 + PF4 + IL17A + TSLP + REN + IHH | 0,78 |
| IFNG + GNLY + LAG3 + ITGAE + PF4 + IL17A + TSLP + REN + IHH + PROM1 | 0,78 |
| GZMH + IFNG + CXCL13 + GNLY + ITGAE + CCL5 + IL17A + REN + IHH + VEGFA | 0,78 |
| IFNG + CXCL13 + GNLY + LAG3 + ITGAE + CCL5 + IL17A + REN + PROM1 + VEGFA | 0,78 |
| IFNG + GNLY + LAG3 + ITGAE + CXCL9 + PF4 + IL17A + REN + IHH + PROM1 | 0,78 |
| IFNG + CXCL13 + GNLY + ITGAE + CCL5 + CXCL9 + IL17A + REN + PROM1 + VEGFA | 0,78 |
| GZMH + CXCL13 + GNLY + CCL5 + PF4 + IL17A + TSLP + REN + IHH + PROM1 | 0,78 |
| IFNG + CXCL13 + GNLY + LAG3 + ITGAE + CCL5 + IL17A + TSLP + REN + PROM1 | 0,78 |
| IFNG + CXCL13 + GNLY + CCL5 + CXCL9 + PF4 + IL17A + TSLP + REN + IHH | 0,78 |
| IFNG + GNLY + CCL5 + CXCL9 + PF4 + IL17A + TSLP + REN + IHH + PROM1 | 0,78 |
| IFNG + GNLY + LAG3 + ITGAE + CXCL9 + PF4 + IL17A + REN + PROM1 + VEGFA | 0,78 |
| CXCL13 + GNLY + LAG3 + CCL5 + CXCL9 + PF4 + IL17A + TSLP + REN + IHH | 0,78 |
| GZMH + CXCL13 + GNLY + LAG3 + ITGAE + CCL5 + PF4 + IL17A + REN + VEGFA | 0,78 |
| CXCL13 + GNLY + CCL5 + CXCL9 + PF4 + IL17A + TSLP + REN + IHH + VEGFA | 0,78 |
| GZMH + CXCL13 + GNLY + LAG3 + ITGAE + CCL5 + PF4 + IL17A + TSLP + REN | 0,78 |
| GZMH + IFNG + GNLY + LAG3 + ITGAE + CXCL9 + PF4 + IL17A + REN + VEGFA | 0,78 |
| GZMH + IFNG + GNLY + LAG3 + ITGAE + PF4 + IL17A + REN + IHH + VEGFA | 0,78 |
| GZMH + IFNG + CXCL13 + GNLY + ITGAE + CCL5 + CXCL9 + IL17A + REN + PROM1 | 0,78 |
| IFNG + CXCL13 + GNLY + LAG3 + CCL5 + CXCL9 + PF4 + IL17A + REN + VEGFA | 0,78 |
| GNLY + LAG3 + ITGAE + CCL5 + PF4 + IL17A + TSLP + REN + IHH + VEGFA | 0,78 |
| GZMH + IFNG + GNLY + ITGAE + PF4 + IL17A + TSLP + REN + PROM1 + VEGFA | 0,78 |
| CXCL13 + GNLY + LAG3 + CCL5 + CXCL9 + PF4 + IL17A + REN + IHH + PROM1 | 0,78 |
| GZMH + GNLY + ITGAE + CCL5 + PF4 + IL17A + TSLP + REN + IHH + VEGFA | 0,78 |
| GZMH + IFNG + CXCL13 + GNLY + LAG3 + ITGAE + CCL5 + IL17A + TSLP + REN | 0,78 |
| GZMH + CXCL13 + GNLY + LAG3 + CCL5 + PF4 + IL17A + REN + IHH + VEGFA | 0,78 |
| GNLY + LAG3 + CCL5 + CXCL9 + PF4 + IL17A + TSLP + REN + IHH + PROM1 | 0,78 |
| IFNG + GNLY + CCL5 + CXCL9 + PF4 + IL17A + TSLP + REN + PROM1 + VEGFA | 0,78 |
| GZMH + IFNG + GNLY + ITGAE + CXCL9 + PF4 + IL17A + REN + IHH + VEGFA | 0,78 |
| IFNG + GNLY + ITGAE + CCL5 + CXCL9 + IL17A + TSLP + REN + IHH + PROM1 | 0,78 |
| GZMH + IFNG + CXCL13 + GNLY + LAG3 + CCL5 + PF4 + IL17A + TSLP + REN | 0,78 |
| GZMH + IFNG + CXCL13 + GNLY + LAG3 + CCL5 + PF4 + IL17A + REN + VEGFA | 0,78 |
| IFNG + GNLY + ITGAE + CCL5 + IL17A + TSLP + REN + IHH + PROM1 + VEGFA | 0,78 |
| GZMH + IFNG + GNLY + ITGAE + PF4 + IL17A + TSLP + REN + IHH + PROM1 | 0,78 |
| GZMH + IFNG + CXCL13 + GNLY + CCL5 + PF4 + IL17A + REN + PROM1 + VEGFA | 0,78 |
| IFNG + GNLY + LAG3 + ITGAE + PF4 + IL17A + TSLP + REN + IHH + VEGFA | 0,78 |
| GZMH + IFNG + GNLY + ITGAE + PF4 + IL17A + REN + IHH + PROM1 + VEGFA | 0,78 |
| GZMH + IFNG + CXCL13 + GNLY + CCL5 + CXCL9 + PF4 + IL17A + REN + VEGFA | 0,78 |
| GZMH + IFNG + CXCL13 + GNLY + LAG3 + ITGAE + CCL5 + IL17A + REN + IHH | 0,78 |
| GZMH + IFNG + CXCL13 + GNLY + CCL5 + CXCL9 + PF4 + IL17A + REN + PROM1 | 0,78 |
| GZMH + GNLY + LAG3 + ITGAE + CCL5 + PF4 + IL17A + TSLP + REN + IHH | 0,78 |
| GZMH + IFNG + CXCL13 + GNLY + CCL5 + PF4 + IL17A + TSLP + REN + PROM1 | 0,78 |
| GZMH + CXCL13 + GNLY + CCL5 + PF4 + IL17A + TSLP + REN + IHH + VEGFA | 0,78 |
| GZMH + IFNG + CXCL13 + GNLY + CCL5 + CXCL9 + PF4 + IL17A + TSLP + REN | 0,78 |
| CXCL13 + GNLY + LAG3 + CCL5 + PF4 + IL17A + TSLP + REN + IHH + PROM1 | 0,78 |
| CXCL13 + GNLY + LAG3 + CCL5 + PF4 + IL17A + REN + IHH + PROM1 + VEGFA | 0,78 |
| IFNG + CXCL13 + GNLY + LAG3 + CCL5 + PF4 + IL17A + REN + IHH + VEGFA | 0,78 |
| IFNG + GNLY + LAG3 + ITGAE + CXCL9 + PF4 + IL17A + TSLP + REN + PROM1 | 0,78 |
| IFNG + CXCL13 + GNLY + LAG3 + CCL5 + PF4 + IL17A + TSLP + REN + IHH | 0,78 |
| GZMH + IFNG + CXCL13 + GNLY + LAG3 + ITGAE + CCL5 + CXCL9 + IL17A + REN | 0,78 |
| IFNG + CXCL13 + GNLY + LAG3 + CCL5 + CXCL9 + PF4 + IL17A + TSLP + REN | 0,78 |
| GZMH + GNLY + LAG3 + CCL5 + CXCL9 + PF4 + IL17A + REN + IHH + PROM1 | 0,78 |
| GZMH + GNLY + LAG3 + CCL5 + CXCL9 + PF4 + IL17A + TSLP + REN + VEGFA | 0,78 |
| GZMH + IFNG + CXCL13 + GNLY + ITGAE + CCL5 + IL17A + TSLP + REN + VEGFA | 0,78 |
| IFNG + GNLY + ITGAE + PF4 + IL17A + TSLP + REN + IHH + PROM1 + VEGFA | 0,78 |
| GZMH + IFNG + GNLY + LAG3 + ITGAE + CXCL9 + PF4 + IL17A + TSLP + REN | 0,78 |
| GNLY + LAG3 + CCL5 + CXCL9 + PF4 + IL17A + TSLP + REN + PROM1 + VEGFA | 0,78 |
| GZMH + GNLY + LAG3 + CCL5 + CXCL9 + PF4 + IL17A + REN + PROM1 + VEGFA | 0,78 |
| IFNG + CXCL13 + GNLY + LAG3 + CCL5 + PF4 + IL17A + REN + IHH + PROM1 | 0,78 |
| CXCL13 + GNLY + CCL5 + CXCL9 + PF4 + IL17A + TSLP + REN + IHH + PROM1 | 0,78 |
| GZMH + CXCL13 + GNLY + LAG3 + CCL5 + CXCL9 + PF4 + IL17A + REN + PROM1 | 0,78 |
| CXCL13 + GNLY + CCL5 + PF4 + IL17A + TSLP + REN + IHH + PROM1 + VEGFA | 0,78 |
| GZMH + IFNG + GNLY + ITGAE + CXCL9 + PF4 + IL17A + TSLP + REN + IHH | 0,78 |
| IFNG + CXCL13 + GNLY + CCL5 + PF4 + IL17A + TSLP + REN + IHH + VEGFA | 0,78 |
| IFNG + CXCL13 + GNLY + ITGAE + CCL5 + CXCL9 + IL17A + TSLP + REN + PROM1 | 0,78 |
| CXCL13 + GNLY + CCL5 + CXCL9 + PF4 + IL17A + REN + IHH + PROM1 + VEGFA | 0,78 |
| IFNG + CXCL13 + GNLY + LAG3 + ITGAE + CCL5 + IL17A + REN + IHH + PROM1 | 0,78 |
| GZMH + IFNG + GNLY + LAG3 + CXCL9 + PF4 + IL17A + REN + IHH + VEGFA | 0,78 |
| IFNG + GNLY + LAG3 + CXCL9 + PF4 + IL17A + REN + IHH + PROM1 + VEGFA | 0,78 |
| GZMH + IFNG + GNLY + ITGAE + CXCL9 + PF4 + IL17A + REN + IHH + PROM1 | 0,78 |
| GZMH + GNLY + CCL5 + CXCL9 + PF4 + IL17A + TSLP + REN + IHH + VEGFA | 0,78 |
| IFNG + CXCL13 + GNLY + LAG3 + ITGAE + CCL5 + IL17A + TSLP + REN + VEGFA | 0,78 |
| IFNG + CXCL13 + GNLY + ITGAE + CCL5 + IL17A + TSLP + REN + PROM1 + VEGFA | 0,78 |
| GZMH + IFNG + GNLY + ITGAE + CXCL9 + PF4 + IL17A + REN + PROM1 + VEGFA | 0,78 |
| GZMH + IFNG + GNLY + LAG3 + ITGAE + CXCL9 + PF4 + IL17A + REN + PROM1 | 0,78 |
| IFNG + CXCL13 + GNLY + LAG3 + CCL5 + PF4 + IL17A + TSLP + REN + PROM1 | 0,78 |
| IFNG + CXCL13 + GNLY + CCL5 + CXCL9 + PF4 + IL17A + REN + IHH + PROM1 | 0,78 |
| GZMH + GNLY + LAG3 + CCL5 + CXCL9 + PF4 + IL17A + TSLP + REN + IHH | 0,78 |
| GZMH + IFNG + CXCL13 + GNLY + ITGAE + CCL5 + CXCL9 + IL17A + REN + IHH | 0,78 |
| IFNG + CXCL13 + GNLY + LAG3 + CCL5 + CXCL9 + PF4 + IL17A + REN + PROM1 | 0,78 |
| IFNG + CXCL13 + GNLY + CCL5 + CXCL9 + PF4 + IL17A + TSLP + REN + VEGFA | 0,78 |
| IFNG + CXCL13 + GNLY + LAG3 + ITGAE + CCL5 + CXCL9 + IL17A + TSLP + REN | 0,78 |
| GZMH + IFNG + CXCL13 + GNLY + CCL5 + PF4 + IL17A + TSLP + REN + VEGFA | 0,78 |
| GZMH + IFNG + GNLY + LAG3 + PF4 + IL17A + REN + IHH + PROM1 + VEGFA | 0,78 |
| CXCL13 + GNLY + LAG3 + CCL5 + PF4 + IL17A + TSLP + REN + IHH + VEGFA | 0,78 |
| IFNG + GNLY + LAG3 + ITGAE + CXCL9 + PF4 + IL17A + TSLP + REN + VEGFA | 0,78 |
| GZMH + CXCL13 + GNLY + LAG3 + CCL5 + PF4 + IL17A + REN + PROM1 + VEGFA | 0,78 |
| IFNG + GNLY + LAG3 + CXCL9 + PF4 + IL17A + TSLP + REN + IHH + VEGFA | 0,78 |
| GZMH + IFNG + GNLY + LAG3 + CXCL9 + PF4 + IL17A + TSLP + REN + IHH | 0,78 |
| GZMH + IFNG + GNLY + ITGAE + CCL5 + PF4 + TSLP + REN + IHH + PROM1 | 0,78 |
| IFNG + CXCL13 + GNLY + LAG3 + ITGAE + CCL5 + CXCL9 + IL17A + REN + IHH | 0,78 |
| GZMH + IFNG + GNLY + LAG3 + PF4 + IL17A + TSLP + REN + IHH + PROM1 | 0,78 |
| CXCL13 + GNLY + LAG3 + CCL5 + CXCL9 + PF4 + IL17A + TSLP + REN + VEGFA | 0,78 |
| GZMH + IFNG + CXCL13 + GNLY + ITGAE + CCL5 + IL17A + TSLP + REN + IHH | 0,78 |
| IFNG + CXCL13 + GNLY + ITGAE + CCL5 + CXCL9 + IL17A + TSLP + REN + IHH | 0,78 |
| GZMH + IFNG + GNLY + CXCL9 + PF4 + IL17A + TSLP + REN + IHH + VEGFA | 0,78 |
| IFNG + CXCL13 + GNLY + CCL5 + PF4 + IL17A + REN + IHH + PROM1 + VEGFA | 0,78 |
| GZMH + IFNG + GNLY + CXCL9 + PF4 + IL17A + REN + IHH + PROM1 + VEGFA | 0,78 |
| GZMH + CXCL13 + GNLY + LAG3 + CCL5 + CXCL9 + PF4 + IL17A + REN + VEGFA | 0,78 |
| IFNG + CXCL13 + GNLY + LAG3 + ITGAE + CCL5 + IL17A + REN + IHH + VEGFA | 0,78 |
| GZMH + CXCL13 + GNLY + LAG3 + CCL5 + PF4 + IL17A + TSLP + REN + PROM1 | 0,78 |
| IFNG + CXCL13 + GNLY + LAG3 + ITGAE + CCL5 + IL17A + TSLP + REN + IHH | 0,78 |
| GZMH + IFNG + GNLY + LAG3 + PF4 + IL17A + TSLP + REN + PROM1 + VEGFA | 0,78 |
| GZMH + IFNG + GNLY + LAG3 + CXCL9 + PF4 + IL17A + REN + IHH + PROM1 | 0,78 |
| GZMH + GNLY + LAG3 + ITGAE + CCL5 + PF4 + IL17A + TSLP + REN + VEGFA | 0,78 |
| GZMH + IFNG + GNLY + PF4 + IL17A + TSLP + REN + IHH + PROM1 + VEGFA | 0,78 |
| GNLY + LAG3 + CCL5 + PF4 + IL17A + TSLP + REN + IHH + PROM1 + VEGFA | 0,78 |
| IFNG + GNLY + LAG3 + PF4 + IL17A + TSLP + REN + IHH + PROM1 + VEGFA | 0,78 |
| GZMH + IFNG + GNLY + ITGAE + CXCL9 + PF4 + IL17A + TSLP + REN + PROM1 | 0,78 |
| GZMH + IFNG + GNLY + LAG3 + PF4 + IL17A + TSLP + REN + IHH + VEGFA | 0,78 |
| IFNG + CXCL13 + GNLY + CCL5 + PF4 + IL17A + TSLP + REN + IHH + PROM1 | 0,78 |
| IFNG + CXCL13 + GNLY + ITGAE + CCL5 + IL17A + TSLP + REN + IHH + VEGFA | 0,78 |
| GZMH + IFNG + CXCL13 + GNLY + LAG3 + ITGAE + PF4 + IL17A + REN + PROM1 | 0,78 |
| GZMH + GNLY + LAG3 + CCL5 + CXCL9 + PF4 + IL17A + TSLP + REN + PROM1 | 0,78 |
| GZMH + IFNG + GNLY + ITGAE + CXCL9 + PF4 + IL17A + TSLP + REN + VEGFA | 0,78 |
| IFNG + GNLY + ITGAE + CXCL9 + PF4 + IL17A + REN + IHH + PROM1 + VEGFA | 0,78 |
| GZMH + GNLY + CCL5 + CXCL9 + PF4 + IL17A + REN + IHH + PROM1 + VEGFA | 0,78 |
| CXCL13 + GNLY + LAG3 + CCL5 + CXCL9 + PF4 + IL17A + REN + PROM1 + VEGFA | 0,78 |
| CXCL13 + GNLY + LAG3 + CCL5 + CXCL9 + PF4 + IL17A + TSLP + REN + PROM1 | 0,78 |
| GNLY + CCL5 + CXCL9 + PF4 + IL17A + TSLP + REN + IHH + PROM1 + VEGFA | 0,78 |
| GZMH + CXCL13 + GNLY + CCL5 + CXCL9 + PF4 + IL17A + REN + PROM1 + VEGFA | 0,78 |
| GZMH + CXCL13 + GNLY + LAG3 + CCL5 + CXCL9 + PF4 + IL17A + TSLP + REN | 0,78 |
| IFNG + CXCL13 + GNLY + LAG3 + CCL5 + PF4 + IL17A + REN + PROM1 + VEGFA | 0,78 |
| GZMH + GNLY + LAG3 + CCL5 + PF4 + IL17A + TSLP + REN + IHH + PROM1 | 0,78 |
| GZMH + IFNG + CXCL13 + GNLY + ITGAE + CCL5 + CXCL9 + IL17A + TSLP + REN | 0,78 |
| IFNG + CXCL13 + GNLY + ITGAE + CCL5 + IL17A + REN + IHH + PROM1 + VEGFA | 0,78 |
| GZMH + CXCL13 + GNLY + CCL5 + CXCL9 + PF4 + IL17A + TSLP + REN + PROM1 | 0,78 |
| GZMH + GNLY + CCL5 + CXCL9 + PF4 + IL17A + TSLP + REN + IHH + PROM1 | 0,78 |
| IFNG + CXCL13 + GNLY + LAG3 + CCL5 + PF4 + IL17A + TSLP + REN + VEGFA | 0,78 |
| GZMH + CXCL13 + GNLY + CCL5 + CXCL9 + PF4 + IL17A + TSLP + REN + VEGFA | 0,78 |
| GZMH + CXCL13 + GNLY + CCL5 + PF4 + IL17A + TSLP + REN + PROM1 + VEGFA | 0,78 |
| GZMH + IFNG + GNLY + ITGAE + CCL5 + PF4 + TSLP + REN + PROM1 + VEGFA | 0,78 |
| IFNG + GNLY + ITGAE + CXCL9 + PF4 + IL17A + TSLP + REN + PROM1 + VEGFA | 0,78 |
| IFNG + GNLY + ITGAE + CXCL9 + PF4 + IL17A + TSLP + REN + IHH + VEGFA | 0,78 |
| GZMH + IFNG + GNLY + LAG3 + CXCL9 + PF4 + IL17A + REN + PROM1 + VEGFA | 0,78 |
| GZMH + IFNG + CXCL13 + GNLY + LAG3 + ITGAE + CXCL9 + PF4 + IL17A + REN | 0,78 |
| CXCL13 + GNLY + LAG3 + CCL5 + PF4 + IL17A + TSLP + REN + PROM1 + VEGFA | 0,78 |
| CXCL13 + GNLY + CCL5 + CXCL9 + PF4 + IL17A + TSLP + REN + PROM1 + VEGFA | 0,78 |
| GZMH + GNLY + LAG3 + CCL5 + PF4 + IL17A + REN + IHH + PROM1 + VEGFA | 0,78 |
| GZMH + IFNG + CXCL13 + GNLY + ITGAE + CXCL9 + PF4 + IL17A + REN + PROM1 | 0,78 |
| IFNG + GNLY + LAG3 + CXCL9 + PF4 + IL17A + TSLP + REN + IHH + PROM1 | 0,78 |
| GZMH + IFNG + GNLY + LAG3 + CXCL9 + PF4 + IL17A + TSLP + REN + VEGFA | 0,78 |
| IFNG + CXCL13 + GNLY + ITGAE + CCL5 + IL17A + TSLP + REN + IHH + PROM1 | 0,78 |
| IFNG + CXCL13 + GNLY + ITGAE + CCL5 + CXCL9 + IL17A + REN + IHH + PROM1 | 0,78 |
| IFNG + CXCL13 + GNLY + LAG3 + ITGAE + PF4 + IL17A + REN + PROM1 + VEGFA | 0,78 |
| GZMH + IFNG + CXCL13 + GNLY + ITGAE + PF4 + IL17A + TSLP + REN + VEGFA | 0,78 |
| GZMH + GNLY + LAG3 + ITGAE + CCL5 + PF4 + IL17A + REN + IHH + VEGFA | 0,78 |
| IFNG + CXCL13 + GNLY + LAG3 + ITGAE + PF4 + IL17A + TSLP + REN + PROM1 | 0,78 |
| IFNG + CXCL13 + GNLY + CCL5 + PF4 + IL17A + TSLP + REN + PROM1 + VEGFA | 0,78 |
| IFNG + CXCL13 + GNLY + CCL5 + CXCL9 + PF4 + IL17A + REN + PROM1 + VEGFA | 0,78 |
| IFNG + CXCL13 + GNLY + CCL5 + CXCL9 + PF4 + IL17A + TSLP + REN + PROM1 | 0,78 |
| GZMH + IFNG + CXCL13 + GNLY + ITGAE + PF4 + IL17A + TSLP + REN + PROM1 | 0,78 |
| GZMH + IFNG + CXCL13 + GNLY + LAG3 + ITGAE + PF4 + IL17A + REN + IHH | 0,78 |
| GZMH + IFNG + GNLY + CXCL9 + PF4 + IL17A + TSLP + REN + PROM1 + VEGFA | 0,78 |
| GZMH + IFNG + CXCL13 + GNLY + LAG3 + ITGAE + PF4 + IL17A + REN + VEGFA | 0,78 |
| IFNG + CXCL13 + GNLY + LAG3 + ITGAE + CXCL9 + PF4 + IL17A + REN + PROM1 | 0,78 |
| GZMH + IFNG + GNLY + CXCL9 + PF4 + IL17A + TSLP + REN + IHH + PROM1 | 0,78 |
| IFNG + GNLY + LAG3 + CXCL9 + PF4 + IL17A + TSLP + REN + PROM1 + VEGFA | 0,78 |
| GZMH + IFNG + CXCL13 + GNLY + ITGAE + PF4 + IL17A + REN + IHH + PROM1 | 0,78 |
| IFNG + GNLY + ITGAE + CXCL9 + PF4 + IL17A + TSLP + REN + IHH + PROM1 | 0,78 |
| IFNG + CXCL13 + GNLY + LAG3 + ITGAE + CXCL9 + PF4 + IL17A + REN + IHH | 0,78 |
| GZMH + IFNG + GNLY + LAG3 + ITGAE + CCL5 + PF4 + TSLP + REN + PROM1 | 0,78 |
| GZMH + IFNG + CXCL13 + GNLY + ITGAE + CXCL9 + PF4 + IL17A + REN + VEGFA | 0,78 |
| GZMH + IFNG + CXCL13 + GNLY + ITGAE + PF4 + IL17A + REN + PROM1 + VEGFA | 0,78 |
| GZMH + IFNG + CXCL13 + GNLY + ITGAE + CXCL9 + PF4 + IL17A + TSLP + REN | 0,78 |
| GZMH + IFNG + CXCL13 + GNLY + LAG3 + ITGAE + PF4 + IL17A + TSLP + REN | 0,78 |
| GZMH + IFNG + CXCL13 + GNLY + ITGAE + CXCL9 + PF4 + IL17A + REN + IHH | 0,78 |
| GZMH + CXCL13 + GNLY + LAG3 + CCL5 + PF4 + IL17A + TSLP + REN + VEGFA | 0,78 |
| GZMH + IFNG + GNLY + LAG3 + CXCL9 + PF4 + IL17A + TSLP + REN + PROM1 | 0,78 |
| IFNG + CXCL13 + GNLY + LAG3 + ITGAE + PF4 + IL17A + REN + IHH + VEGFA | 0,78 |
| GZMH + IFNG + CXCL13 + GNLY + ITGAE + PF4 + IL17A + TSLP + REN + IHH | 0,78 |
| GZMH + GNLY + LAG3 + CCL5 + PF4 + IL17A + TSLP + REN + PROM1 + VEGFA | 0,78 |
| GZMH + IFNG + GNLY + ITGAE + CCL5 + PF4 + REN + IHH + PROM1 + VEGFA | 0,78 |
| GZMH + IFNG + GNLY + LAG3 + ITGAE + CCL5 + PF4 + REN + IHH + PROM1 | 0,78 |
| GZMH + IFNG + GNLY + LAG3 + CCL5 + CXCL9 + IL17A + REN + IHH + VEGFA | 0,78 |
| GZMH + IFNG + CXCL13 + GNLY + ITGAE + PF4 + IL17A + REN + IHH + VEGFA | 0,78 |
| IFNG + GNLY + CXCL9 + PF4 + IL17A + TSLP + REN + IHH + PROM1 + VEGFA | 0,78 |
| GZMH + IFNG + CXCL13 + GNLY + CXCL9 + PF4 + IL17A + REN + IHH + VEGFA | 0,78 |
| IFNG + CXCL13 + GNLY + LAG3 + ITGAE + CXCL9 + PF4 + IL17A + REN + VEGFA | 0,78 |
| IFNG + CXCL13 + GNLY + LAG3 + ITGAE + PF4 + IL17A + TSLP + REN + VEGFA | 0,78 |
| IFNG + CXCL13 + GNLY + LAG3 + ITGAE + PF4 + IL17A + TSLP + REN + IHH | 0,78 |
| GZMH + GNLY + LAG3 + ITGAE + CCL5 + CXCL9 + IL17A + REN + PROM1 + VEGFA | 0,78 |
| IFNG + CXCL13 + GNLY + ITGAE + CXCL9 + PF4 + IL17A + REN + IHH + PROM1 | 0,78 |
| GZMH + IFNG + CXCL13 + GNLY + PF4 + IL17A + REN + IHH + PROM1 + VEGFA | 0,78 |
| IFNG + CXCL13 + GNLY + ITGAE + CXCL9 + PF4 + IL17A + REN + PROM1 + VEGFA | 0,78 |
| GNLY + LAG3 + ITGAE + CCL5 + CXCL9 + IL17A + TSLP + REN + IHH + VEGFA | 0,78 |
| GZMH + IFNG + CXCL13 + GNLY + CXCL9 + PF4 + IL17A + TSLP + REN + PROM1 | 0,78 |
| IFNG + CXCL13 + GNLY + ITGAE + CXCL9 + PF4 + IL17A + TSLP + REN + VEGFA | 0,78 |
| IFNG + CXCL13 + GNLY + LAG3 + ITGAE + PF4 + IL17A + REN + IHH + PROM1 | 0,78 |
| GZMH + IFNG + CXCL13 + GNLY + CXCL9 + PF4 + IL17A + TSLP + REN + IHH | 0,78 |
| GZMH + IFNG + CXCL13 + GNLY + CXCL9 + PF4 + IL17A + REN + IHH + PROM1 | 0,78 |
| IFNG + CXCL13 + GNLY + LAG3 + ITGAE + CXCL9 + PF4 + IL17A + TSLP + REN | 0,78 |
| IFNG + CXCL13 + GNLY + CXCL9 + PF4 + IL17A + REN + IHH + PROM1 + VEGFA | 0,78 |
| GZMH + GNLY + CCL5 + CXCL9 + PF4 + IL17A + TSLP + REN + PROM1 + VEGFA | 0,78 |
| GZMH + IFNG + CXCL13 + GNLY + PF4 + IL17A + TSLP + REN + IHH + VEGFA | 0,78 |
| GZMH + GNLY + CCL5 + PF4 + IL17A + TSLP + REN + IHH + PROM1 + VEGFA | 0,78 |
| GZMH + GNLY + LAG3 + CCL5 + PF4 + IL17A + TSLP + REN + IHH + VEGFA | 0,78 |
| GZMH + IFNG + CXCL13 + GNLY + LAG3 + CXCL9 + PF4 + IL17A + REN + VEGFA | 0,78 |
| IFNG + CXCL13 + GNLY + LAG3 + CXCL9 + PF4 + IL17A + REN + IHH + VEGFA | 0,78 |
| GZMH + IFNG + CXCL13 + GNLY + CXCL9 + PF4 + IL17A + REN + PROM1 + VEGFA | 0,78 |
| GZMH + IFNG + GNLY + LAG3 + ITGAE + CCL5 + PF4 + REN + PROM1 + VEGFA | 0,78 |
| IFNG + GNLY + ITGAE + CCL5 + PF4 + TSLP + REN + IHH + PROM1 + VEGFA | 0,78 |
| IFNG + CXCL13 + GNLY + ITGAE + CXCL9 + PF4 + IL17A + TSLP + REN + PROM1 | 0,78 |
| GZMH + IFNG + CXCL13 + GNLY + CXCL9 + PF4 + IL17A + TSLP + REN + VEGFA | 0,78 |
| GZMH + IFNG + CXCL13 + GNLY + PF4 + IL17A + TSLP + REN + IHH + PROM1 | 0,78 |
| IFNG + CXCL13 + GNLY + ITGAE + PF4 + IL17A + REN + IHH + PROM1 + VEGFA | 0,78 |
| IFNG + CXCL13 + GNLY + ITGAE + CXCL9 + PF4 + IL17A + REN + IHH + VEGFA | 0,78 |
| GZMH + IFNG + CXCL13 + GNLY + PF4 + IL17A + TSLP + REN + PROM1 + VEGFA | 0,78 |
| GZMH + IFNG + CXCL13 + GNLY + LAG3 + PF4 + IL17A + REN + IHH + VEGFA | 0,78 |
| GZMH + IFNG + CXCL13 + GNLY + LAG3 + CXCL9 + PF4 + IL17A + TSLP + REN | 0,78 |
| IFNG + CXCL13 + GNLY + ITGAE + PF4 + IL17A + TSLP + REN + IHH + VEGFA | 0,78 |
| GNLY + LAG3 + ITGAE + CCL5 + CXCL9 + IL17A + REN + IHH + PROM1 + VEGFA | 0,78 |
| IFNG + GNLY + LAG3 + CCL5 + CXCL9 + IL17A + TSLP + REN + IHH + VEGFA | 0,78 |
| IFNG + GNLY + LAG3 + ITGAE + CCL5 + PF4 + TSLP + REN + IHH + PROM1 | 0,78 |
| IFNG + CXCL13 + GNLY + ITGAE + PF4 + IL17A + TSLP + REN + PROM1 + VEGFA | 0,78 |
| GZMH + IFNG + CXCL13 + GNLY + LAG3 + PF4 + IL17A + TSLP + REN + PROM1 | 0,78 |
| GNLY + LAG3 + ITGAE + CCL5 + CXCL9 + IL17A + TSLP + REN + PROM1 + VEGFA | 0,78 |
| GZMH + IFNG + CXCL13 + GNLY + LAG3 + CXCL9 + PF4 + IL17A + REN + IHH | 0,78 |
| IFNG + CXCL13 + GNLY + ITGAE + CXCL9 + PF4 + IL17A + TSLP + REN + IHH | 0,78 |
| GZMH + IFNG + CXCL13 + GNLY + LAG3 + PF4 + IL17A + REN + IHH + PROM1 | 0,78 |
| IFNG + GNLY + LAG3 + ITGAE + CCL5 + PF4 + TSLP + REN + PROM1 + VEGFA | 0,78 |
| IFNG + CXCL13 + GNLY + CXCL9 + PF4 + IL17A + TSLP + REN + IHH + VEGFA | 0,78 |
| GZMH + IFNG + GNLY + LAG3 + CCL5 + CXCL9 + IL17A + REN + PROM1 + VEGFA | 0,78 |
| GZMH + GNLY + LAG3 + ITGAE + CXCL9 + PF4 + IL17A + TSLP + REN + IHH | 0,78 |
| GZMH + IFNG + CXCL13 + GNLY + LAG3 + PF4 + IL17A + TSLP + REN + IHH | 0,78 |
| IFNG + CXCL13 + GNLY + LAG3 + CXCL9 + PF4 + IL17A + REN + IHH + PROM1 | 0,78 |
| GZMH + IFNG + CXCL13 + GNLY + LAG3 + PF4 + IL17A + TSLP + REN + VEGFA | 0,78 |
| IFNG + CXCL13 + GNLY + ITGAE + PF4 + IL17A + TSLP + REN + IHH + PROM1 | 0,78 |
| GZMH + IFNG + GNLY + LAG3 + CCL5 + CXCL9 + IL17A + TSLP + REN + VEGFA | 0,78 |
| IFNG + CXCL13 + GNLY + LAG3 + PF4 + IL17A + REN + IHH + PROM1 + VEGFA | 0,78 |
| CXCL13 + GNLY + LAG3 + ITGAE + CCL5 + CXCL9 + IL17A + REN + PROM1 + VEGFA | 0,78 |
| IFNG + CXCL13 + GNLY + CXCL9 + PF4 + IL17A + TSLP + REN + PROM1 + VEGFA | 0,78 |
| IFNG + CXCL13 + GNLY + CXCL9 + PF4 + IL17A + TSLP + REN + IHH + PROM1 | 0,78 |
| IFNG + CXCL13 + GNLY + LAG3 + PF4 + IL17A + TSLP + REN + PROM1 + VEGFA | 0,78 |
| GZMH + IFNG + GNLY + LAG3 + CCL5 + CXCL9 + IL17A + TSLP + REN + IHH | 0,78 |
| IFNG + CXCL13 + GNLY + LAG3 + CXCL9 + PF4 + IL17A + REN + PROM1 + VEGFA | 0,78 |
| GZMH + IFNG + CXCL13 + GNLY + LAG3 + PF4 + IL17A + REN + PROM1 + VEGFA | 0,78 |
| IFNG + CXCL13 + GNLY + PF4 + IL17A + TSLP + REN + IHH + PROM1 + VEGFA | 0,78 |
| IFNG + CXCL13 + GNLY + LAG3 + CXCL9 + PF4 + IL17A + TSLP + REN + VEGFA | 0,78 |
| GZMH + CXCL13 + GNLY + LAG3 + ITGAE + PF4 + IL17A + TSLP + REN + IHH | 0,78 |
| GNLY + ITGAE + CCL5 + CXCL9 + IL17A + TSLP + REN + IHH + PROM1 + VEGFA | 0,78 |
| IFNG + CXCL13 + GNLY + LAG3 + CXCL9 + PF4 + IL17A + TSLP + REN + IHH | 0,78 |
| GZMH + GNLY + ITGAE + CXCL9 + PF4 + IL17A + TSLP + REN + IHH + PROM1 | 0,78 |
| GZMH + IFNG + CXCL13 + GNLY + LAG3 + CXCL9 + PF4 + IL17A + REN + PROM1 | 0,78 |
| IFNG + CXCL13 + GNLY + LAG3 + PF4 + IL17A + TSLP + REN + IHH + PROM1 | 0,78 |
| GZMH + CXCL 13 + GNLY + LAG3 + ITGAE + PF4 + IL17A + REN + IHH + VEGFA | 0,78 |
| GZMH + GNLY + LAG3 + ITGAE + CXCL9 + PF4 + IL17A + REN + IHH + VEGFA | 0,78 |
| GZMH + CXCL13 + GNLY + LAG3 + ITGAE + CXCL9 + PF4 + IL17A + REN + IHH | 0,78 |
| IFNG + CXCL13 + GNLY + LAG3 + CXCL9 + PF4 + IL17A + TSLP + REN + PROM1 | 0,78 |
| GZMH + CXCL13 + GNLY + LAG3 + ITGAE + PF4 + IL17A + TSLP + REN + VEGFA | 0,78 |
| GZMH + GNLY + CXCL9 + PF4 + IL17A + TSLP + REN + IHH + PROM1 + VEGFA | 0,78 |
| IFNG + CXCL13 + GNLY + LAG3 + PF4 + IL17A + TSLP + REN + IHH + VEGFA | 0,78 |
| GZMH + CXCL13 + GNLY + LAG3 + PF4 + IL17A + TSLP + REN + IHH + VEGFA | 0,78 |
| GZMH + GNLY + ITGAE + CXCL9 + PF4 + IL17A + TSLP + REN + IHH + VEGFA | 0,78 |
| GZMH + CXCL13 + GNLY + LAG3 + CXCL9 + PF4 + IL17A + TSLP + REN + IHH | 0,78 |
| GZMH + CXCL13 + GNLY + LAG3 + ITGAE + CXCL9 + PF4 + IL17A + TSLP + REN | 0,78 |
| GZMH + GNLY + ITGAE + CXCL9 + PF4 + IL17A + REN + IHH + PROM1 + VEGFA | 0,78 |
| GZMH + CXCL13 + GNLY + LAG3 + ITGAE + CXCL9 + PF4 + IL17A + REN + VEGFA | 0,78 |
| GZMH + CXCL13 + GNLY + LAG3 + CXCL9 + PF4 + IL17A + REN + IHH + VEGFA | 0,78 |
| GZMH + CXCL13 + GNLY + LAG3 + ITGAE + PF4 + IL17A + TSLP + REN + PROM1 | 0,78 |
| GZMH + CXCL13 + GNLY + LAG3 + ITGAE + PF4 + IL17A + REN + IHH + PROM1 | 0,78 |
| GZMH + GNLY + LAG3 + ITGAE + CXCL9 + PF4 + IL17A + REN + IHH + PROM1 | 0,78 |
| GZMH + GNLY + ITGAE + PF4 + IL17A + TSLP + REN + IHH + PROM1 + VEGFA | 0,78 |
| GZMH + GNLY + LAG3 + ITGAE + CXCL9 + PF4 + IL17A + TSLP + REN + PROM1 | 0,78 |
| GZMH + GNLY + LAG3 + ITGAE + CXCL9 + PF4 + IL17A + TSLP + REN + VEGFA | 0,78 |
| GZMH + IFNG + GNLY + ITGAE + CCL5 + PF4 + TSLP + REN + IHH + VEGFA | 0,78 |
| GZMH + CXCL13 + GNLY + LAG3 + ITGAE + PF4 + IL17A + REN + PROM1 + VEGFA | 0,78 |
| GZMH + GNLY + LAG3 + ITGAE + CCL5 + CXCL9 + IL17A + TSLP + REN + VEGFA | 0,78 |
| CXCL13 + GNLY + LAG3 + ITGAE + CCL5 + CXCL9 + IL17A + REN + IHH + VEGFA | 0,78 |
| GZMH + GNLY + LAG3 + ITGAE + PF4 + IL17A + TSLP + REN + IHH + PROM1 | 0,78 |
| GZMH + GNLY + LAG3 + CXCL9 + PF4 + IL17A + TSLP + REN + IHH + PROM1 | 0,78 |
| GZMH + GNLY + ITGAE + CXCL9 + PF4 + IL17A + TSLP + REN + PROM1 + VEGFA | 0,78 |
| GZMH + GNLY + LAG3 + ITGAE + PF4 + IL17A + TSLP + REN + IHH + VEGFA | 0,78 |
| GZMH + CXCL13 + GNLY + LAG3 + PF4 + IL17A + REN + IHH + PROM1 + VEGFA | 0,78 |
| GZMH + GNLY + LAG3 + CXCL9 + PF4 + IL17A + TSLP + REN + IHH + VEGFA | 0,78 |
| GZMH + CXCL13 + GNLY + LAG3 + PF4 + IL17A + TSLP + REN + IHH + PROM1 | 0,78 |

| **Table 11: clusters of 11 genes:** | |
|---|---|
| **clusters of 11 genes** | **c_tau level** |
| GZMH + IFNG + GNLY + ITGAE + CCL5 + PF4 + IL17A + REN + IHH + PROM1 + VEGFA | 0,8 |
| GZMH + IFNG + GNLY + ITGAE + CCL5 + PF4 + IL17A + TSLP + REN + PROM1 + VEGFA | 0,8 |
| GZMH + IFNG + GNLY + ITGAE + CCL5 + PF4 + IL17A + TSLP + REN + IHH + PROM1 | 0,8 |
| GZMH + IFNG + GNLY + LAG3 + ITGAE + CCL5 + PF4 + IL17A + REN + PROM1 + VEGFA | 0,8 |
| GZMH + IFNG + GNLY + LAG3 + ITGAE + CCL5 + PF4 + IL17A + REN + IHH + PROM1 | 0,8 |
| GZMH + IFNG + GNLY + ITGAE + CCL5 + PF4 + IL17A + TSLP + REN + IHH + VEGFA | 0,8 |
| IFNG + GNLY + ITGAE + CCL5 + PF4 + IL17A + TSLP + REN + IHH + PROM1 + VEGFA | 0,8 |
| GZMH + IFNG + GNLY + ITGAE + CCL5 + CXCL9 + PF4 + IL17A + REN + IHH + VEGFA | 0,8 |
| IFNG + GNLY + LAG3 + ITGAE + CCL5 + PF4 + IL17A + REN + IHH + PROM1 + VEGFA | 0,8 |
| GZMH + IFNG + GNLY + LAG3 + ITGAE + CCL5 + PF4 + IL17A + TSLP + REN + PROM1 | 0,8 |
| IFNG + GNLY + LAG3 + ITGAE + CCL5 + PF4 + IL17A + TSLP + REN + PROM1 + VEGFA | 0,8 |
| GZMH + IFNG + GNLY + ITGAE + CCL5 + CXCL9 + PF4 + IL17A + TSLP + REN + VEGFA | 0,8 |
| GZMH + IFNG + GNLY + ITGAE + CCL5 + CXCL9 + PF4 + IL17A + REN + PROM1 + VEGFA | 0,8 |
| IFNG + GNLY + LAG3 + ITGAE + CCL5 + PF4 + IL17A + TSLP + REN + IHH + PROM1 | 0,8 |
| GZMH + IFNG + GNLY + LAG3 + ITGAE + CCL5 + CXCL9 + PF4 + IL17A + REN + VEGFA | 0,8 |
| GZMH + IFNG + GNLY + LAG3 + ITGAE + CCL5 + PF4 + IL17A + REN + IHH + VEGFA | 0,8 |
| GZMH + IFNG + GNLY + LAG3 + ITGAE + CCL5 + CXCL9 + PF4 + IL17A + REN + PROM1 | 0,8 |
| GZMH + IFNG + GNLY + LAG3 + ITGAE + CCL5 + PF4 + IL17A + TSLP + REN + VEGFA | 0,8 |
| GZMH + IFNG + GNLY + ITGAE + CCL5 + CXCL9 + PF4 + IL17A + REN + IHH + PROM1 | 0,8 |
| GZMH + IFNG + GNLY + LAG3 + ITGAE + CCL5 + CXCL9 + PF4 + IL17A + REN + IHH | 0,8 |
| IFNG + GNLY + LAG3 + ITGAE + CCL5 + CXCL9 + PF4 + IL17A + REN + IHH + VEGFA | 0,8 |
| GZMH + IFNG + GNLY + LAG3 + ITGAE + CCL5 + PF4 + IL17A + TSLP + REN + IHH | 0,8 |
| GZMH + IFNG + GNLY + ITGAE + CCL5 + CXCL9 + PF4 + IL17A + TSLP + REN + IHH | 0,8 |
| GZMH + IFNG + GNLY + ITGAE + CCL5 + CXCL9 + PF4 + IL17A + TSLP + REN + PROM1 | 0,8 |
| IFNG + GNLY + ITGAE + CCL5 + CXCL9 + PF4 + IL17A + TSLP + REN + IHH + VEGFA | 0,8 |
| GZMH + IFNG + CXCL13 + GNLY + ITGAE + CCL5 + PF4 + IL17A + REN + IHH + PROM1 | 0,8 |
| GZMH + IFNG + GNLY + LAG3 + ITGAE + CCL5 + CXCL9 + PF4 + IL17A + TSLP + REN | 0,8 |
| IFNG + GNLY + LAG3 + ITGAE + CCL5 + CXCL9 + PF4 + IL17A + TSLP + REN + VEGFA | 0,8 |
| IFNG + GNLY + LAG3 + ITGAE + CCL5 + PF4 + IL17A + TSLP + REN + IHH + VEGFA | 0,8 |
| GZMH + IFNG + CXCL13 + GNLY + ITGAE + CCL5 + PF4 + IL17A + REN + PROM1 + VEGFA | 0,8 |
| GZMH + IFNG + CXCL13 + GNLY + LAG3 + ITGAE + CCL5 + PF4 + IL17A + REN + PROM1 | 0,79 |
| GZMH + IFNG + CXCL13 + GNLY + ITGAE + CCL5 + PF4 + IL17A + TSLP + REN + PROM1 | 0,79 |
| GZMH + IFNG + CXCL13 + GNLY + ITGAE + CCL5 + CXCL9 + PF4 + IL17A + REN + PROM1 | 0,79 |
| IFNG + GNLY + LAG3 + ITGAE + CCL5 + CXCL9 + PF4 + IL17A + TSLP + REN + IHH | 0,79 |
| IFNG + GNLY + ITGAE + CCL5 + CXCL9 + PF4 + IL17A + REN + IHH + PROM1 + VEGFA | 0,79 |
| IFNG + GNLY + ITGAE + CCL5 + CXCL9 + PF4 + IL17A + TSLP + REN + PROM1 + VEGFA | 0,79 |
| IFNG + GNLY + LAG3 + ITGAE + CCL5 + CXCL9 + PF4 + IL17A + REN + PROM1 + VEGFA | 0,79 |
| IFNG + CXCL13 + GNLY + LAG3 + ITGAE + CCL5 + PF4 + IL17A + REN + IHH + PROM1 | 0,79 |
| IFNG + CXCL13 + GNLY + LAG3 + ITGAE + CCL5 + PF4 + IL17A + REN + PROM1 + VEGFA | 0,79 |
| IFNG + GNLY + ITGAE + CCL5 + CXCL9 + PF4 + IL17A + TSLP + REN + IHH + PROM1 | 0,79 |
| IFNG + CXCL13 + GNLY + ITGAE + CCL5 + PF4 + IL17A + TSLP + REN + IHH + PROM1 | 0,79 |
| IFNG + CXCL13 + GNLY + ITGAE + CCL5 + PF4 + IL17A + TSLP + REN + PROM1 + VEGFA | 0,79 |
| IFNG + GNLY + LAG3 + ITGAE + CCL5 + CXCL9 + PF4 + IL17A + TSLP + REN + PROM1 | 0,79 |
| IFNG + GNLY + LAG3 + ITGAE + CCL5 + CXCL9 + PF4 + IL17A + REN + IHH + PROM1 | 0,79 |
| IFNG + CXCL13 + GNLY + ITGAE + CCL5 + PF4 + IL17A + REN + IHH + PROM1 + VEGFA | 0,79 |
| IFNG + CXCL13 + GNLY + LAG3 + ITGAE + CCL5 + PF4 + IL17A + TSLP + REN + PROM1 | 0,79 |
| IFNG + CXCL13 + GNLY + ITGAE + CCL5 + CXCL9 + PF4 + IL17A + REN + IHH + VEGFA | 0,79 |
| GZMH + IFNG + CXCL13 + GNLY + ITGAE + CCL5 + CXCL9 + PF4 + IL17A + REN + VEGFA | 0,79 |
| IFNG + CXCL13 + GNLY + LAG3 + ITGAE + CCL5 + CXCL9 + PF4 + IL17A + REN + VEGFA | 0,79 |
| GZMH + IFNG + CXCL13 + GNLY + ITGAE + CCL5 + PF4 + IL17A + REN + IHH + VEGFA | 0,79 |
| IFNG + CXCL13 + GNLY + ITGAE + CCL5 + CXCL9 + PF4 + IL17A + REN + PROM1 + VEGFA | 0,79 |
| IFNG + CXCL13 + GNLY + ITGAE + CCL5 + CXCL9 + PF4 + IL17A + TSLP + REN + VEGFA | 0,79 |
| IFNG + CXCL13 + GNLY + ITGAE + CCL5 + CXCL9 + PF4 + IL17A + REN + IHH + PROM1 | 0,79 |
| IFNG + CXCL13 + GNLY + LAG3 + ITGAE + CCL5 + PF4 + IL17A + REN + IHH + VEGFA | 0,79 |
| GZMH + IFNG + CXCL13 + GNLY + ITGAE + CCL5 + CXCL9 + PF4 + IL17A + REN + IHH | 0,79 |
| IFNG + CXCL13 + GNLY + ITGAE + CCL5 + PF4 + IL17A + TSLP + REN + IHH + VEGFA | 0,79 |
| IFNG + CXCL13 + GNLY + ITGAE + CCL5 + CXCL9 + PF4 + IL17A + TSLP + REN + PROM1 | 0,79 |
| IFNG + CXCL13 + GNLY + LAG3 + ITGAE + CCL5 + CXCL9 + PF4 + IL17A + REN + PROM1 | 0,79 |
| GZMH + IFNG + CXCL13 + GNLY + LAG3 + ITGAE + CCL5 + PF4 + IL17A + REN + IHH | 0,79 |
| GZMH + IFNG + CXCL13 + GNLY + ITGAE + CCL5 + PF4 + IL17A + TSLP + REN + VEGFA | 0,79 |
| GZMH + IFNG + GNLY + LAG3 + CCL5 + PF4 + IL17A + REN + IHH + PROM1 + VEGFA | 0,79 |
| GZMH + IFNG + CXCL13 + GNLY + ITGAE + CCL5 + PF4 + IL17A + TSLP + REN + IHH | 0,79 |
| GZMH + IFNG + GNLY + LAG3 + CCL5 + CXCL9 + PF4 + IL17A + REN + IHH + VEGFA | 0,79 |
| IFNG + CXCL13 + GNLY + LAG3 + ITGAE + CCL5 + PF4 + IL17A + TSLP + REN + IHH | 0,79 |
| IFNG + CXCL13 + GNLY + LAG3 + ITGAE + CCL5 + CXCL9 + PF4 + IL17A + REN + IHH | 0,79 |
| GZMH + IFNG + GNLY + LAG3 + ITGAE + CCL5 + CXCL9 + IL17A + REN + PROM1 + VEGFA | 0,79 |
| GZMH + IFNG + CXCL13 + GNLY + LAG3 + ITGAE + CCL5 + PF4 + IL17A + REN + VEGFA | 0,79 |
| IFNG + CXCL13 + GNLY + LAG3 + ITGAE + CCL5 + PF4 + IL17A + TSLP + REN + VEGFA | 0,79 |
| IFNG + CXCL13 + GNLY + ITGAE + CCL5 + CXCL9 + PF4 + IL17A + TSLP + REN + IHH | 0,79 |
| GZMH + IFNG + CXCL13 + GNLY + LAG3 + ITGAE + CCL5 + CXCL9 + PF4 + IL17A + REN | 0,79 |
| GZMH + IFNG + CXCL13 + GNLY + ITGAE + CCL5 + CXCL9 + PF4 + IL17A + TSLP + REN | 0,79 |
| GZMH + IFNG + GNLY + LAG3 + ITGAE + CCL5 + IL17A + REN + IHH + PROM1 + VEGFA | 0,79 |
| GZMH + IFNG + CXCL13 + GNLY + LAG3 + ITGAE + CCL5 + PF4 + IL17A + TSLP + REN | 0,79 |
| GZMH + IFNG + GNLY + LAG3 + CCL5 + PF4 + IL17A + TSLP + REN + IHH + PROM1 | 0,79 |
| GZMH + IFNG + GNLY + LAG3 + ITGAE + CCL5 + IL17A + TSLP + REN + IHH + VEGFA | 0,79 |
| GZMH + CXCL13 + GNLY + ITGAE + CCL5 + PF4 + IL17A + REN + IHH + PROM1 + VEGFA | 0,79 |
| IFNG + CXCL13 + GNLY + LAG3 + ITGAE + CCL5 + CXCL9 + PF4 + IL17A + TSLP + REN | 0,79 |
| GZMH + IFNG + GNLY + LAG3 + ITGAE + CCL5 + IL17A + TSLP + REN + PROM1 + VEGFA | 0,79 |
| GZMH + CXCL13 + GNLY + ITGAE + CCL5 + PF4 + IL17A + TSLP + REN + IHH + PROM1 | 0,79 |
| GNLY + LAG3 + ITGAE + CCL5 + PF4 + IL17A + TSLP + REN + IHH + PROM1 + VEGFA | 0,79 |
| GNLY + ITGAE + CCL5 + CXCL9 + PF4 + IL17A + TSLP + REN + IHH + PROM1 + VEGFA | 0,79 |
| GZMH + IFNG + GNLY + LAG3 + CCL5 + CXCL9 + PF4 + IL17A + TSLP + REN + IHH | 0,79 |
| CXCL13 + GNLY + LAG3 + ITGAE + CCL5 + CXCL9 + PF4 + IL17A + REN + IHH + PROM1 | 0,79 |
| GZMH + IFNG + GNLY + CCL5 + CXCL9 + PF4 + IL17A + TSLP + REN + IHH + VEGFA | 0,79 |
| GZMH + CXCL13 + GNLY + ITGAE + CCL5 + CXCL9 + PF4 + IL17A + REN + IHH + PROM1 | 0,79 |
| GZMH + IFNG + GNLY + LAG3 + ITGAE + CCL5 + CXCL9 + IL17A + TSLP + REN + VEGFA | 0,79 |
| CXCL13 + GNLY + ITGAE + CCL5 + PF4 + IL17A + TSLP + REN + IHH + PROM1 + VEGFA | 0,79 |
| CXCL13 + GNLY + ITGAE + CCL5 + CXCL9 + PF4 + IL17A + REN + IHH + PROM1 + VEGFA | 0,79 |
| GZMH + IFNG + GNLY + LAG3 + CCL5 + CXCL9 + PF4 + IL17A + TSLP + REN + VEGFA | 0,79 |
| GZMH + IFNG + GNLY + LAG3 + CCL5 + PF4 + IL17A + TSLP + REN + PROM1 + VEGFA | 0,79 |
| CXCL13 + GNLY + LAG3 + ITGAE + CCL5 + PF4 + IL17A + TSLP + REN + IHH + PROM1 | 0,79 |
| GZMH + CXCL13 + GNLY + LAG3 + ITGAE + CCL5 + PF4 + IL17A + REN + IHH + PROM1 | 0,79 |
| GZMH + IFNG + GNLY + ITGAE + CCL5 + CXCL9 + IL17A + TSLP + REN + IHH + VEGFA | 0,79 |
| CXCL13 + GNLY + ITGAE + CCL5 + CXCL9 + PF4 + IL17A + TSLP + REN + IHH + PROM1 | 0,79 |
| GZMH + IFNG + GNLY + LAG3 + ITGAE + CCL5 + CXCL9 + IL17A + REN + IHH + VEGFA | 0,79 |
| GZMH + GNLY + ITGAE + CCL5 + CXCL9 + PF4 + IL17A + TSLP + REN + PROM1 + VEGFA | 0,79 |
| GZMH + GNLY + ITGAE + CCL5 + CXCL9 + PF4 + IL17A + TSLP + REN + IHH + VEGFA | 0,79 |
| CXCL13 + GNLY + LAG3 + ITGAE + CCL5 + PF4 + IL17A + REN + IHH + PROM1 + VEGFA | 0,79 |
| CXCL13 + GNLY + LAG3 + ITGAE + CCL5 + CXCL9 + PF4 + IL17A + REN + PROM1 + VEGFA | 0,79 |
| GZMH + IFNG + GNLY + LAG3 + CCL5 + CXCL9 + PF4 + IL17A + REN + PROM1 + VEGFA | 0,79 |
| GZMH + IFNG + GNLY + ITGAE + CCL5 + CXCL9 + IL17A + REN + IHH + PROM1 + VEGFA | 0,79 |
| GZMH + CXCL13 + GNLY + LAG3 + ITGAE + CCL5 + PF4 + IL17A + TSLP + REN + PROM1 | 0,79 |
| GZMH + IFNG + GNLY + LAG3 + CCL5 + PF4 + IL17A + TSLP + REN + IHH + VEGFA | 0,79 |
| IFNG + GNLY + LAG3 + CCL5 + CXCL9 + PF4 + IL17A + TSLP + REN + IHH + VEGFA | 0,79 |
| GZMH + GNLY + ITGAE + CCL5 + CXCL9 + PF4 + IL17A + REN + IHH + PROM1 + VEGFA | 0,79 |
| GZMH + CXCL13 + GNLY + ITGAE + CCL5 + CXCL9 + PF4 + IL17A + REN + PROM1 + VEGFA | 0,79 |
| GZMH + CXCL13 + GNLY + ITGAE + CCL5 + CXCL9 + PF4 + IL17A + TSLP + REN + PROM1 | 0,79 |
| GZMH + CXCL13 + GNLY + LAG3 + ITGAE + CCL5 + PF4 + IL17A + REN + PROM1 + VEGFA | 0,79 |
| GNLY + LAG3 + ITGAE + CCL5 + CXCL9 + PF4 + IL17A + TSLP + REN + IHH + VEGFA | 0,79 |
| GZMH + IFNG + GNLY + LAG3 + ITGAE + CCL5 + CXCL9 + IL17A + TSLP + REN + IHH | 0,79 |
| GZMH + CXCL13 + GNLY + ITGAE + CCL5 + PF4 + IL17A + TSLP + REN + PROM1 + VEGFA | 0,79 |
| CXCL13 + GNLY + LAG3 + ITGAE + CCL5 + PF4 + IL17A + TSLP + REN + PROM1 + VEGFA | 0,79 |
| GZMH + GNLY + ITGAE + CCL5 + PF4 + IL17A + TSLP + REN + IHH + PROM1 + VEGFA | 0,79 |
| CXCL13 + GNLY + ITGAE + CCL5 + CXCL9 + PF4 + IL17A + TSLP + REN + PROM1 + VEGFA | 0,79 |
| GZMH + GNLY + LAG3 + ITGAE + CCL5 + CXCL9 + PF4 + IL17A + TSLP + REN + VEGFA | 0,79 |
| GZMH + GNLY + ITGAE + CCL5 + CXCL9 + PF4 + IL17A + TSLP + REN + IHH + PROM1 | 0,79 |
| GNLY + LAG3 + ITGAE + CCL5 + CXCL9 + PF4 + IL17A + TSLP + REN + PROM1 + VEGFA | 0,79 |
| GZMH + GNLY + LAG3 + ITGAE + CCL5 + PF4 + IL17A + TSLP + REN + IHH + PROM1 | 0,79 |
| GNLY + LAG3 + ITGAE + CCL5 + CXCL9 + PF4 + IL17A + REN + IHH + PROM1 + VEGFA | 0,79 |
| GNLY + LAG3 + ITGAE + CCL5 + CXCL9 + PF4 + IL17A + TSLP + REN + IHH + PROM1 | 0,79 |
| IFNG + GNLY + LAG3 + ITGAE + CCL5 + CXCL9 + IL17A + TSLP + REN + PROM1 + VEGFA | 0,79 |
| CXCL13 + GNLY + LAG3 + ITGAE + CCL5 + CXCL9 + PF4 + IL17A + TSLP + REN + PROM1 | 0,79 |
| GZMH + IFNG + GNLY + LAG3 + CCL5 + CXCL9 + PF4 + IL17A + REN + IHH + PROM1 | 0,79 |
| IFNG + GNLY + LAG3 + ITGAE + CCL5 + CXCL9 + IL17A + REN + IHH + PROM1 + VEGFA | 0,79 |
| IFNG + GNLY + LAG3 + ITGAE + CCL5 + CXCL9 + IL17A + TSLP + REN + IHH + VEGFA | 0,79 |
| GZMH + GNLY + LAG3 + ITGAE + CCL5 + CXCL9 + PF4 + IL17A + REN + PROM1 + VEGFA | 0,79 |
| GZMH + IFNG + GNLY + LAG3 + ITGAE + CCL5 + IL17A + TSLP + REN + IHH + PROM1 | 0,79 |
| GZMH + GNLY + LAG3 + ITGAE + CCL5 + CXCL9 + PF4 + IL17A + TSLP + REN + PROM1 | 0,79 |
| GZMH + IFNG + GNLY + LAG3 + ITGAE + CCL5 + CXCL9 + IL17A + REN + IHH + PROM1 | 0,79 |
| GZMH + IFNG + GNLY + CCL5 + PF4 + IL17A + TSLP + REN + IHH + PROM1 + VEGFA | 0,79 |
| CXCL13 + GNLY + ITGAE + CCL5 + CXCL9 + PF4 + IL17A + TSLP + REN + IHH + VEGFA | 0,79 |
| GZMH + CXCL13 + GNLY + ITGAE + CCL5 + CXCL9 + PF4 + IL17A + REN + IHH + VEGFA | 0,79 |
| GZMH + IFNG + GNLY + ITGAE + CCL5 + CXCL9 + IL17A + TSLP + REN + PROM1 + VEGFA | 0,79 |
| IFNG + GNLY + LAG3 + ITGAE + CCL5 + IL17A + TSLP + REN + IHH + PROM1 + VEGFA | 0,79 |
| CXCL13 + GNLY + LAG3 + ITGAE + CCL5 + CXCL9 + PF4 + IL17A + REN + IHH + VEGFA | 0,79 |
| GZMH + GNLY + LAG3 + ITGAE + CCL5 + CXCL9 + PF4 + IL17A + REN + IHH + VEGFA | 0,79 |
| GZMH + CXCL13 + GNLY + LAG3 + ITGAE + CCL5 + CXCL9 + PF4 + IL17A + REN + PROM1 | 0,79 |
| GZMH + IFNG + CXCL13 + GNLY + LAG3 + ITGAE + CCL5 + IL17A + REN + PROM1 + VEGFA | 0,79 |
| GZMH + IFNG + GNLY + LAG3 + ITGAE + CCL5 + CXCL9 + IL17A + TSLP + REN + PROM1 | 0,79 |
| GZMH + GNLY + LAG3 + ITGAE + CCL5 + PF4 + IL17A + TSLP + REN + PROM1 + VEGFA | 0,79 |
| IFNG + GNLY + LAG3 + CCL5 + PF4 + IL17A + TSLP + REN + IHH + PROM1 + VEGFA | 0,79 |
| GZMH + IFNG + GNLY + LAG3 + CCL5 + CXCL9 + PF4 + IL17A + TSLP + REN + PROM1 | 0,79 |
| GZMH + IFNG + GNLY + CCL5 + CXCL9 + PF4 + IL17A + REN + IHH + PROM1 + VEGFA | 0,79 |
| CXCL13 + GNLY + LAG3 + ITGAE + CCL5 + PF4 + IL17A + TSLP + REN + IHH + VEGFA | 0,79 |
| IFNG + GNLY + LAG3 + CCL5 + CXCL9 + PF4 + IL17A + REN + IHH + PROM1 + VEGFA | 0,79 |
| GZMH + IFNG + CXCL13 + GNLY + LAG3 + ITGAE + CCL5 + CXCL9 + IL17A + REN + VEGFA | 0,79 |
| GZMH + GNLY + LAG3 + ITGAE + CCL5 + PF4 + IL17A + REN + IHH + PROM1 + VEGFA | 0,79 |
| GZMH + IFNG + GNLY + ITGAE + CCL5 + IL17A + TSLP + REN + IHH + PROM1 + VEGFA | 0,79 |
| CXCL13 + GNLY + LAG3 + ITGAE + CCL5 + CXCL9 + PF4 + IL17A + TSLP + REN + VEGFA | 0,79 |
| GZMH + CXCL13 + GNLY + LAG3 + ITGAE + CCL5 + CXCL9 + PF4 + IL17A + REN + IHH | 0,79 |
| GZMH + GNLY + LAG3 + ITGAE + CCL5 + CXCL9 + PF4 + IL17A + REN + IHH + PROM1 | 0,79 |
| CXCL13 + GNLY + LAG3 + ITGAE + CCL5 + CXCL9 + PF4 + IL17A + TSLP + REN + IHH | 0,79 |
| GZMH + CXCL13 + GNLY + ITGAE + CCL5 + CXCL9 + PF4 + IL17A + TSLP + REN + IHH | 0,79 |
| GZMH + IFNG + CXCL13 + GNLY + LAG3 + ITGAE + CCL5 + CXCL9 + IL17A + REN + PROM1 | 0,79 |
| GZMH + CXCL13 + GNLY + ITGAE + CCL5 + PF4 + IL17A + TSLP + REN + IHH + VEGFA | 0,79 |
| GZMH + CXCL13 + GNLY + LAG3 + ITGAE + CCL5 + PF4 + IL17A + TSLP + REN + IHH | 0,79 |
| GZMH + IFNG + CXCL13 + GNLY + LAG3 + CCL5 + CXCL9 + PF4 + IL17A + REN + IHH | 0,79 |
| GZMH + IFNG + CXCL13 + GNLY + LAG3 + ITGAE + CCL5 + IL17A + TSLP + REN + PROM1 | 0,79 |
| GZMH + CXCL13 + GNLY + ITGAE + CCL5 + CXCL9 + PF4 + IL17A + TSLP + REN + VEGFA | 0,79 |
| GZMH + CXCL13 + GNLY + LAG3 + ITGAE + CCL5 + PF4 + IL17A + REN + IHH + VEGFA | 0,79 |
| IFNG + CXCL13 + GNLY + LAG3 + ITGAE + CCL5 + CXCL9 + IL17A + TSLP + REN + VEGFA | 0,79 |
| GZMH + IFNG + CXCL13 + GNLY + LAG3 + ITGAE + CCL5 + IL17A + REN + IHH + PROM1 | 0,79 |
| IFNG + CXCL13 + GNLY + LAG3 + ITGAE + CCL5 + CXCL9 + IL17A + REN + PROM1 + VEGFA | 0,79 |
| IFNG + CXCL13 + GNLY + LAG3 + ITGAE + CCL5 + CXCL9 + IL17A + REN + IHH + VEGFA | 0,79 |
| GZMH + IFNG + CXCL13 + GNLY + ITGAE + CCL5 + CXCL9 + IL17A + REN + IHH + VEGFA | 0,79 |
| IFNG + GNLY + ITGAE + CCL5 + CXCL9 + IL17A + TSLP + REN + IHH + PROM1 + VEGFA | 0,79 |
| GZMH + CXCL13 + GNLY + LAG3 + ITGAE + CCL5 + CXCL9 + PF4 + IL17A + REN + VEGFA | 0,79 |
| GZMH + IFNG + CXCL13 + GNLY + CCL5 + CXCL9 + PF4 + IL17A + REN + IHH + VEGFA | 0,79 |
| GZMH + IFNG + CXCL13 + GNLY + CCL5 + CXCL9 + PF4 + IL17A + TSLP + REN + IHH | 0,79 |
| IFNG + GNLY + LAG3 + CCL5 + CXCL9 + PF4 + IL17A + TSLP + REN + PROM1 + VEGFA | 0,79 |
| GZMH + GNLY + LAG3 + ITGAE + CCL5 + CXCL9 + PF4 + IL17A + TSLP + REN + IHH | 0,79 |
| GZMH + IFNG + CXCL13 + GNLY + LAG3 + CCL5 + PF4 + IL17A + REN + IHH + VEGFA | 0,79 |
| GZMH + IFNG + CXCL13 + GNLY + ITGAE + CCL5 + CXCL9 + IL17A + TSLP + REN + VEGFA | 0,79 |
| GZMH + IFNG + CXCL13 + GNLY + LAG3 + CCL5 + PF4 + IL17A + TSLP + REN + IHH | 0,79 |
| GZMH + IFNG + CXCL13 + GNLY + LAG3 + CCL5 + CXCL9 + PF4 + IL17A + REN + VEGFA | 0,79 |
| GZMH + CXCL13 + GNLY + LAG3 + CCL5 + CXCL9 + PF4 + IL17A + REN + IHH + PROM1 | 0,79 |
| GZMH + IFNG + CXCL13 + GNLY + LAG3 + CCL5 + PF4 + IL17A + REN + PROM1 + VEGFA | 0,79 |
| GZMH + IFNG + GNLY + CCL5 + CXCL9 + PF4 + IL17A + TSLP + REN + IHH + PROM1 | 0,79 |
| GZMH + IFNG + CXCL13 + GNLY + ITGAE + CCL5 + IL17A + TSLP + REN + PROM1 + VEGFA | 0,79 |
| IFNG + GNLY + LAG3 + CCL5 + CXCL9 + PF4 + IL17A + TSLP + REN + IHH + PROM1 | 0,79 |
| IFNG + CXCL13 + GNLY + LAG3 + CCL5 + CXCL9 + PF4 + IL17A + REN + IHH + VEGFA | 0,79 |
| GZMH + IFNG + GNLY + CCL5 + CXCL9 + PF4 + IL17A + TSLP + REN + PROM1 + VEGFA | 0,79 |
| GZMH + IFNG + CXCL13 + GNLY + ITGAE + CCL5 + CXCL9 + IL17A + REN + PROM1 + VEGFA | 0,79 |
| GZMH + IFNG + CXCL13 + GNLY + ITGAE + CCL5 + IL17A + REN + IHH + PROM1 + VEGFA | 0,79 |
| IFNG + GNLY + LAG3 + ITGAE + CCL5 + CXCL9 + IL17A + TSLP + REN + IHH + PROM1 | 0,79 |
| GZMH + GNLY + LAG3 + CCL5 + CXCL9 + PF4 + IL17A + REN + IHH + PROM1 + VEGFA | 0,79 |
| GZMH + IFNG + CXCL13 + GNLY + LAG3 + CCL5 + PF4 + IL17A + REN + IHH + PROM1 | 0,79 |
| GZMH + IFNG + CXCL13 + GNLY + CCL5 + PF4 + IL17A + TSLP + REN + IHH + VEGFA | 0,79 |
| GZMH + IFNG + CXCL13 + GNLY + LAG3 + CCL5 + PF4 + IL17A + TSLP + REN + PROM1 | 0,79 |
| GZMH + GNLY + LAG3 + CCL5 + CXCL9 + PF4 + IL17A + TSLP + REN + IHH + VEGFA | 0,79 |
| GZMH + CXCL13 + GNLY + LAG3 + ITGAE + CCL5 + CXCL9 + PF4 + IL17A + TSLP + REN | 0,79 |
| GNLY + LAG3 + CCL5 + CXCL9 + PF4 + IL17A + TSLP + REN + IHH + PROM1 + VEGFA | 0,79 |
| GZMH + CXCL13 + GNLY + LAG3 + CCL5 + PF4 + IL17A + REN + IHH + PROM1 + VEGFA | 0,79 |
| GZMH + IFNG + GNLY + LAG3 + ITGAE + PF4 + IL17A + TSLP + REN + PROM1 + VEGFA | 0,79 |
| GZMH + IFNG + CXCL13 + GNLY + LAG3 + ITGAE + CCL5 + IL17A + REN + IHH + VEGFA | 0,79 |
| GZMH + IFNG + CXCL13 + GNLY + CCL5 + CXCL9 + PF4 + IL17A + REN + IHH + PROM1 | 0,79 |
| GZMH + CXCL13 + GNLY + LAG3 + CCL5 + PF4 + IL17A + TSLP + REN + IHH + PROM1 | 0,79 |
| GZMH + IFNG + GNLY + ITGAE + CCL5 + CXCL9 + IL17A + TSLP + REN + IHH + PROM1 | 0,79 |
| GZMH + IFNG + CXCL13 + GNLY + CCL5 + PF4 + IL17A + REN + IHH + PROM1 + VEGFA | 0,79 |
| GZMH + CXCL13 + GNLY + LAG3 + CCL5 + CXCL9 + PF4 + IL17A + REN + IHH + VEGFA | 0,79 |
| IFNG + CXCL13 + GNLY + ITGAE + CCL5 + CXCL9 + IL17A + TSLP + REN + IHH + VEGFA | 0,79 |
| GZMH + CXCL13 + GNLY + LAG3 + CCL5 + CXCL9 + PF4 + IL17A + TSLP + REN + IHH | 0,79 |
| GZMH + IFNG + CXCL13 + GNLY + CCL5 + PF4 + IL17A + TSLP + REN + IHH + PROM1 | 0,79 |
| GZMH + IFNG + CXCL13 + GNLY + LAG3 + CCL5 + CXCL9 + PF4 + IL17A + TSLP + REN | 0,79 |
| GZMH + IFNG + CXCL13 + GNLY + LAG3 + CCL5 + CXCL9 + PF4 + IL17A + REN + PROM1 | 0,79 |
| IFNG + CXCL13 + GNLY + LAG3 + ITGAE + CCL5 + IL17A + TSLP + REN + PROM1 + VEGFA | 0,79 |
| GZMH + CXCL13 + GNLY + CCL5 + CXCL9 + PF4 + IL17A + REN + IHH + PROM1 + VEGFA | 0,79 |
| GZMH + CXCL13 + GNLY + CCL5 + CXCL9 + PF4 + IL17A + TSLP + REN + IHH + PROM1 | 0,79 |
| GZMH + IFNG + GNLY + LAG3 + ITGAE + PF4 + IL17A + TSLP + REN + IHH + PROM1 | 0,79 |
| GZMH + CXCL13 + GNLY + CCL5 + CXCL9 + PF4 + IL17A + TSLP + REN + IHH + VEGFA | 0,79 |
| IFNG + CXCL13 + GNLY + ITGAE + CCL5 + CXCL9 + IL17A + TSLP + REN + PROM1 + VEGFA | 0,79 |
| IFNG + GNLY + LAG3 + ITGAE + CXCL9 + PF4 + IL17A + REN + IHH + PROM1 + VEGFA | 0,79 |
| GZMH + IFNG + GNLY + LAG3 + ITGAE + CXCL9 + PF4 + IL17A + REN + IHH + VEGFA | 0,79 |
| GZMH + IFNG + GNLY + LAG3 + ITGAE + PF4 + IL17A + REN + IHH + PROM1 + VEGFA | 0,79 |
| CXCL13 + GNLY + LAG3 + CCL5 + CXCL9 + PF4 + IL17A + TSLP + REN + IHH + VEGFA | 0,79 |
| IFNG + GNLY + CCL5 + CXCL9 + PF4 + IL17A + TSLP + REN + IHH + PROM1 + VEGFA | 0,79 |
| IFNG + CXCL13 + GNLY + LAG3 + CCL5 + CXCL9 + PF4 + IL17A + TSLP + REN + IHH | 0,78 |
| GZMH + IFNG + GNLY + LAG3 + ITGAE + PF4 + IL17A + TSLP + REN + IHH + VEGFA | 0,78 |
| IFNG + CXCL13 + GNLY + LAG3 + ITGAE + CCL5 + CXCL9 + IL17A + TSLP + REN + PROM1 | 0,78 |
| GZMH + CXCL13 + GNLY + CCL5 + PF4 + IL17A + TSLP + REN + IHH + PROM1 + VEGFA | 0,78 |
| IFNG + CXCL13 + GNLY + CCL5 + CXCL9 + PF4 + IL17A + TSLP + REN + IHH + VEGFA | 0,78 |
| GZMH + IFNG + CXCL13 + GNLY + ITGAE + CCL5 + IL17A + TSLP + REN + IHH + PROM1 | 0,78 |
| CXCL13 + GNLY + LAG3 + CCL5 + CXCL9 + PF4 + IL17A + REN + IHH + PROM1 + VEGFA | 0,78 |
| GZMH + IFNG + CXCL13 + GNLY + LAG3 + ITGAE + CCL5 + IL17A + TSLP + REN + VEGFA | 0,78 |
| IFNG + GNLY + LAG3 + ITGAE + PF4 + IL17A + TSLP + REN + IHH + PROM1 + VEGFA | 0,78 |
| GZMH + CXCL13 + GNLY + LAG3 + ITGAE + CCL5 + PF4 + IL17A + TSLP + REN + VEGFA | 0,78 |
| GZMH + IFNG + GNLY + LAG3 + ITGAE + CXCL9 + PF4 + IL17A + TSLP + REN + IHH | 0,78 |
| GZMH + IFNG + CXCL13 + GNLY + CCL5 + CXCL9 + PF4 + IL17A + TSLP + REN + VEGFA | 0,78 |
| CXCL13 + GNLY + LAG3 + CCL5 + CXCL9 + PF4 + IL17A + TSLP + REN + IHH + PROM1 | 0,78 |
| IFNG + GNLY + LAG3 + ITGAE + CXCL9 + PF4 + IL17A + TSLP + REN + IHH + VEGFA | 0,78 |
| GZMH + IFNG + CXCL13 + GNLY + ITGAE + CCL5 + CXCL9 + IL17A + REN + IHH + PROM1 | 0,78 |
| GZMH + GNLY + LAG3 + CCL5 + CXCL9 + PF4 + IL17A + TSLP + REN + IHH + PROM1 | 0,78 |
| GZMH + CXCL13 + GNLY + LAG3 + CCL5 + PF4 + IL17A + TSLP + REN + IHH + VEGFA | 0,78 |
| IFNG + CXCL13 + GNLY + LAG3 + CCL5 + CXCL9 + PF4 + IL17A + TSLP + REN + VEGFA | 0,78 |
| IFNG + CXCL13 + GNLY + LAG3 + ITGAE + CCL5 + IL17A + REN + IHH + PROM1 + VEGFA | 0,78 |
| GZMH + IFNG + CXCL13 + GNLY + ITGAE + CCL5 + IL17A + TSLP + REN + IHH + VEGFA | 0,78 |
| GZMH + IFNG + CXCL13 + GNLY + LAG3 + CCL5 + PF4 + IL17A + TSLP + REN + VEGFA | 0,78 |
| GZMH + IFNG + CXCL13 + GNLY + ITGAE + CCL5 + CXCL9 + IL17A + TSLP + REN + PROM1 | 0,78 |
| GZMH + IFNG + GNLY + ITGAE + PF4 + IL17A + TSLP + REN + IHH + PROM1 + VEGFA | 0,78 |
| GZMH + IFNG + CXCL13 + GNLY + CCL5 + PF4 + IL17A + TSLP + REN + PROM1 + VEGFA | 0,78 |
| IFNG + CXCL13 + GNLY + ITGAE + CCL5 + CXCL9 + IL17A + REN + IHH + PROM1 + VEGFA | 0,78 |
| GZMH + IFNG + GNLY + LAG3 + ITGAE + CXCL9 + PF4 + IL17A + TSLP + REN + VEGFA | 0,78 |
| IFNG + CXCL13 + GNLY + LAG3 + CCL5 + CXCL9 + PF4 + IL17A + REN + IHH + PROM1 | 0,78 |
| GZMH + IFNG + CXCL13 + GNLY + LAG3 + ITGAE + CCL5 + IL17A + TSLP + REN + IHH | 0,78 |
| GZMH + IFNG + CXCL13 + GNLY + CCL5 + CXCL9 + PF4 + IL17A + REN + PROM1 + VEGFA | 0,78 |
| GZMH + IFNG + CXCL13 + GNLY + CCL5 + CXCL9 + PF4 + IL17A + TSLP + REN + PROM1 | 0,78 |
| IFNG + GNLY + LAG3 + ITGAE + CXCL9 + PF4 + IL17A + TSLP + REN + PROM1 + VEGFA | 0,78 |
| GZMH + GNLY + LAG3 + ITGAE + CCL5 + PF4 + IL17A + TSLP + REN + IHH + VEGFA | 0,78 |
| IFNG + CXCL13 + GNLY + LAG3 + ITGAE + CCL5 + IL17A + TSLP + REN + IHH + VEGFA | 0,78 |
| IFNG + CXCL13 + GNLY + LAG3 + CCL5 + PF4 + IL17A + TSLP + REN + IHH + VEGFA | 0,78 |
| GZMH + GNLY + LAG3 + CCL5 + CXCL9 + PF4 + IL17A + TSLP + REN + PROM1 + VEGFA | 0,78 |
| CXCL13 + GNLY + LAG3 + CCL5 + PF4 + IL17A + TSLP + REN + IHH + PROM1 + VEGFA | 0,78 |
| GZMH + IFNG + GNLY + ITGAE + CXCL9 + PF4 + IL17A + REN + IHH + PROM1 + VEGFA | 0,78 |
| GZMH + IFNG + GNLY + LAG3 + ITGAE + CXCL9 + PF4 + IL17A + REN + IHH + PROM1 | 0,78 |
| GZMH + IFNG + CXCL13 + GNLY + LAG3 + ITGAE + CCL5 + CXCL9 + IL17A + REN + IHH | 0,78 |
| GZMH + IFNG + GNLY + LAG3 + ITGAE + CXCL9 + PF4 + IL17A + REN + PROM1 + VEGFA | 0,78 |
| GZMH + CXCL13 + GNLY + LAG3 + CCL5 + CXCL9 + PF4 + IL17A + TSLP + REN + PROM1 | 0,78 |
| GZMH + CXCL13 + GNLY + LAG3 + CCL5 + CXCL9 + PF4 + IL17A + REN + PROM1 + VEGFA | 0,78 |
| CXCL13 + GNLY + CCL5 + CXCL9 + PF4 + IL17A + TSLP + REN + IHH + PROM1 + VEGFA | 0,78 |
| IFNG + CXCL13 + GNLY + LAG3 + ITGAE + CCL5 + CXCL9 + IL17A + TSLP + REN + IHH | 0,78 |
| GZMH + IFNG + CXCL13 + GNLY + LAG3 + ITGAE + CCL5 + CXCL9 + IL17A + TSLP + REN | 0,78 |
| GZMH + IFNG + CXCL13 + GNLY + ITGAE + CCL5 + CXCL9 + IL17A + TSLP + REN + IHH | 0,78 |
| GZMH + IFNG + GNLY + ITGAE + CXCL9 + PF4 + IL17A + TSLP + REN + IHH + VEGFA | 0,78 |
| GZMH + IFNG + GNLY + LAG3 + CXCL9 + PF4 + IL17A + TSLP + REN + IHH + VEGFA | 0,78 |
| IFNG + CXCL13 + GNLY + CCL5 + CXCL9 + PF4 + IL17A + REN + IHH + PROM1 + VEGFA | 0,78 |
| GZMH + CXCL13 + GNLY + LAG3 + CCL5 + PF4 + IL17A + TSLP + REN + PROM1 + VEGFA | 0,78 |
| IFNG + CXCL13 + GNLY + LAG3 + ITGAE + CCL5 + IL17A + TSLP + REN + IHH + PROM1 | 0,78 |
| IFNG + GNLY + LAG3 + ITGAE + CXCL9 + PF4 + IL17A + TSLP + REN + IHH + PROM1 | 0,78 |
| IFNG + CXCL13 + GNLY + LAG3 + CCL5 + CXCL9 + PF4 + IL17A + TSLP + REN + PROM1 | 0,78 |
| IFNG + CXCL13 + GNLY + LAG3 + CCL5 + CXCL9 + PF4 + IL17A + REN + PROM1 + VEGFA | 0,78 |
| IFNG + CXCL13 + GNLY + LAG3 + CCL5 + PF4 + IL17A + TSLP + REN + IHH + PROM1 | 0,78 |
| IFNG + CXCL13 + GNLY + LAG3 + CCL5 + PF4 + IL17A + TSLP + REN + PROM1 + VEGFA | 0,78 |
| GZMH + GNLY + LAG3 + CCL5 + PF4 + IL17A + TSLP + REN + IHH + PROM1 + VEGFA | 0,78 |
| GZMH + IFNG + CXCL13 + GNLY + LAG3 + ITGAE + PF4 + IL17A + TSLP + REN + PROM1 | 0,78 |
| IFNG + CXCL13 + GNLY + CCL5 + CXCL9 + PF4 + IL17A + TSLP + REN + IHH + PROM1 | 0,78 |
| IFNG + CXCL13 + GNLY + LAG3 + CCL5 + PF4 + IL17A + REN + IHH + PROM1 + VEGFA | 0,78 |
| GZMH + CXCL13 + GNLY + LAG3 + CCL5 + CXCL9 + PF4 + IL17A + TSLP + REN + VEGFA | 0,78 |
| GZMH + IFNG + GNLY + LAG3 + CXCL9 + PF4 + IL17A + REN + IHH + PROM1 + VEGFA | 0,78 |
| GZMH + IFNG + GNLY + LAG3 + ITGAE + CXCL9 + PF4 + IL17A + TSLP + REN + PROM1 | 0,78 |
| GZMH + IFNG + GNLY + ITGAE + CCL5 + PF4 + TSLP + REN + IHH + PROM1 + VEGFA | 0,78 |
| GZMH + IFNG + GNLY + LAG3 + PF4 + IL17A + TSLP + REN + IHH + PROM1 + VEGFA | 0,78 |
| IFNG + CXCL13 + GNLY + LAG3 + ITGAE + CCL5 + CXCL9 + IL17A + REN + IHH + PROM1 | 0,78 |
| GZMH + GNLY + CCL5 + CXCL9 + PF4 + IL17A + TSLP + REN + IHH + PROM1 + VEGFA | 0,78 |
| GZMH + IFNG + GNLY + ITGAE + CXCL9 + PF4 + IL17A + TSLP + REN + PROM1 + VEGFA | 0,78 |
| GZMH + CXCL13 + GNLY + CCL5 + CXCL9 + PF4 + IL17A + TSLP + REN + PROM1 + VEGFA | 0,78 |
| CXCL13 + GNLY + LAG3 + CCL5 + CXCL9 + PF4 + IL17A + TSLP + REN + PROM1 + VEGFA | 0,78 |
| IFNG + CXCL13 + GNLY + CCL5 + PF4 + IL17A + TSLP + REN + IHH + PROM1 + VEGFA | 0,78 |
| GZMH + IFNG + CXCL13 + GNLY + LAG3 + ITGAE + PF4 + IL17A + REN + PROM1 + VEGFA | 0,78 |
| GZMH + IFNG + GNLY + ITGAE + CXCL9 + PF4 + IL17A + TSLP + REN + IHH + PROM1 | 0,78 |
| IFNG + CXCL13 + GNLY + ITGAE + CCL5 + IL17A + TSLP + REN + IHH + PROM1 + VEGFA | 0,78 |
| IFNG + CXCL13 + GNLY + ITGAE + CCL5 + CXCL9 + IL17A + TSLP + REN + IHH + PROM1 | 0,78 |
| GZMH + IFNG + CXCL13 + GNLY + LAG3 + ITGAE + CXCL9 + PF4 + IL17A + REN + VEGFA | 0,78 |
| IFNG + GNLY + LAG3 + CXCL9 + PF4 + IL17A + TSLP + REN + IHH + PROM1 + VEGFA | 0,78 |
| GZMH + IFNG + GNLY + LAG3 + ITGAE + CCL5 + PF4 + TSLP + REN + IHH + PROM1 | 0,78 |
| IFNG + CXCL13 + GNLY + LAG3 + ITGAE + CXCL9 + PF4 + IL17A + REN + PROM1 + VEGFA | 0,78 |
| GZMH + IFNG + CXCL13 + GNLY + LAG3 + ITGAE + PF4 + IL17A + REN + IHH + VEGFA | 0,78 |
| GZMH + IFNG + CXCL13 + GNLY + LAG3 + ITGAE + PF4 + IL17A + TSLP + REN + VEGFA | 0,78 |
| GZMH + IFNG + GNLY + LAG3 + CXCL9 + PF4 + IL17A + TSLP + REN + IHH + PROM1 | 0,78 |
| IFNG + CXCL13 + GNLY + LAG3 + ITGAE + PF4 + IL17A + TSLP + REN + PROM1 + VEGFA | 0,78 |
| GZMH + IFNG + CXCL13 + GNLY + LAG3 + ITGAE + CXCL9 + PF4 + IL17A + TSLP + REN | 0,78 |
| GZMH + IFNG + GNLY + CXCL9 + PF4 + IL17A + TSLP + REN + IHH + PROM1 + VEGFA | 0,78 |
| IFNG + CXCL13 + GNLY + CCL5 + CXCL9 + PF4 + IL17A + TSLP + REN + PROM1 + VEGFA | 0,78 |
| GZMH + IFNG + CXCL13 + GNLY + LAG3 + ITGAE + CXCL9 + PF4 + IL17A + REN + IHH | 0,78 |
| GZMH + IFNG + CXCL13 + GNLY + ITGAE + CXCL9 + PF4 + IL17A + REN + PROM1 + VEGFA | 0,78 |
| GZMH + IFNG + CXCL13 + GNLY + ITGAE + CXCL9 + PF4 + IL17A + TSLP + REN + PROM1 | 0,78 |
| IFNG + GNLY + ITGAE + CXCL9 + PF4 + IL17A + TSLP + REN + IHH + PROM1 + VEGFA | 0,78 |
| IFNG + CXCL13 + GNLY + LAG3 + ITGAE + CXCL9 + PF4 + IL17A + REN + IHH + VEGFA | 0,78 |
| IFNG + CXCL13 + GNLY + LAG3 + ITGAE + PF4 + IL17A + TSLP + REN + IHH + PROM1 | 0,78 |
| GZMH + IFNG + CXCL13 + GNLY + ITGAE + PF4 + IL17A + REN + IHH + PROM1 + VEGFA | 0,78 |
| GZMH + IFNG + CXCL13 + GNLY + LAG3 + ITGAE + PF4 + IL17A + TSLP + REN + IHH | 0,78 |
| GZMH + IFNG + CXCL13 + GNLY + LAG3 + ITGAE + PF4 + IL17A + REN + IHH + PROM1 | 0,78 |
| GZMH + IFNG + CXCL13 + GNLY + ITGAE + CXCL9 + PF4 + IL17A + TSLP + REN + VEGFA | 0,78 |
| IFNG + CXCL13 + GNLY + LAG3 + ITGAE + PF4 + IL17A + REN + IHH + PROM1 + VEGFA | 0,78 |
| IFNG + CXCL13 + GNLY + LAG3 + ITGAE + CXCL9 + PF4 + IL17A + TSLP + REN + PROM1 | 0,78 |
| GZMH + IFNG + CXCL13 + GNLY + ITGAE + CXCL9 + PF4 + IL17A + REN + IHH + VEGFA | 0,78 |
| GZMH + IFNG + CXCL13 + GNLY + ITGAE + CXCL9 + PF4 + IL17A + REN + IHH + PROM1 | 0,78 |
| GZMH + IFNG + CXCL13 + GNLY + ITGAE + PF4 + IL17A + TSLP + REN + PROM1 + VEGFA | 0,78 |
| GZMH + IFNG + CXCL13 + GNLY + ITGAE + PF4 + IL17A + TSLP + REN + IHH + PROM1 | 0,78 |
| GZMH + IFNG + CXCL13 + GNLY + LAG3 + ITGAE + CXCL9 + PF4 + IL17A + REN + PROM1 | 0,78 |
| GZMH + IFNG + GNLY + LAG3 + ITGAE + CCL5 + PF4 + TSLP + REN + PROM1 + VEGFA | 0,78 |
| GZMH + IFNG + CXCL13 + GNLY + ITGAE + PF4 + IL17A + TSLP + REN + IHH + VEGFA | 0,78 |
| GZMH + IFNG + GNLY + LAG3 + CXCL9 + PF4 + IL17A + TSLP + REN + PROM1 + VEGFA | 0,78 |
| GZMH + IFNG + CXCL13 + GNLY + ITGAE + CXCL9 + PF4 + IL17A + TSLP + REN + IHH | 0,78 |
| GZMH + IFNG + GNLY + LAG3 + CCL5 + CXCL9 + IL17A + TSLP + REN + IHH + VEGFA | 0,78 |
| GNLY + LAG3 + ITGAE + CCL5 + CXCL9 + IL17A + TSLP + REN + IHH + PROM1 + VEGFA | 0,78 |
| GZMH + GNLY + LAG3 + ITGAE + CCL5 + CXCL9 + IL17A + TSLP + REN + PROM1 + VEGFA | 0,78 |
| IFNG + CXCL13 + GNLY + LAG3 + ITGAE + CXCL9 + PF4 + IL17A + TSLP + REN + IHH | 0,78 |
| GZMH + IFNG + GNLY + LAG3 + ITGAE + CCL5 + PF4 + REN + IHH + PROM1 + VEGFA | 0,78 |
| IFNG + CXCL13 + GNLY + LAG3 + ITGAE + CXCL9 + PF4 + IL17A + REN + IHH + PROM1 | 0,78 |
| IFNG + CXCL13 + GNLY + LAG3 + ITGAE + PF4 + IL17A + TSLP + REN + IHH + VEGFA | 0,78 |
| IFNG + CXCL13 + GNLY + ITGAE + CXCL9 + PF4 + IL17A + REN + IHH + PROM1 + VEGFA | 0,78 |
| GZMH + IFNG + CXCL13 + GNLY + CXCL9 + PF4 + IL17A + TSLP + REN + PROM1 + VEGFA | 0,78 |
| GZMH + IFNG + CXCL13 + GNLY + CXCL9 + PF4 + IL17A + TSLP + REN + IHH + VEGFA | 0,78 |
| IFNG + CXCL13 + GNLY + LAG3 + ITGAE + CXCL9 + PF4 + IL17A + TSLP + REN + VEGFA | 0,78 |
| GZMH + GNLY + LAG3 + ITGAE + CCL5 + CXCL9 + IL17A + TSLP + REN + IHH + VEGFA | 0,78 |
| CXCL13 + GNLY + LAG3 + ITGAE + CCL5 + CXCL9 + IL17A + REN + IHH + PROM1 + VEGFA | 0,78 |
| GZMH + IFNG + CXCL13 + GNLY + CXCL9 + PF4 + IL17A + REN + IHH + PROM1 + VEGFA | 0,78 |
| GZMH + GNLY + LAG3 + ITGAE + CCL5 + CXCL9 + IL17A + REN + IHH + PROM1 + VEGFA | 0,78 |
| CXCL13 + GNLY + LAG3 + ITGAE + CCL5 + CXCL9 + IL17A + TSLP + REN + PROM1 + VEGFA | 0,78 |
| GZMH + IFNG + CXCL13 + GNLY + PF4 + IL17A + TSLP + REN + IHH + PROM1 + VEGFA | 0,78 |
| IFNG + GNLY + LAG3 + ITGAE + CCL5 + PF4 + TSLP + REN + IHH + PROM1 + VEGFA | 0,78 |
| IFNG + CXCL13 + GNLY + ITGAE + CXCL9 + PF4 + IL17A + TSLP + REN + PROM1 + VEGFA | 0,78 |
| GZMH + IFNG + CXCL13 + GNLY + CXCL9 + PF4 + IL17A + TSLP + REN + IHH + PROM1 | 0,78 |
| IFNG + CXCL13 + GNLY + ITGAE + CXCL9 + PF4 + IL17A + TSLP + REN + IHH + VEGFA | 0,78 |
| GZMH + IFNG + CXCL13 + GNLY + LAG3 + CXCL9 + PF4 + IL17A + TSLP + REN + VEGFA | 0,78 |
| GZMH + IFNG + CXCL13 + GNLY + LAG3 + CXCL9 + PF4 + IL17A + REN + IHH + PROM1 | 0,78 |
| GZMH + IFNG + GNLY + LAG3 + CCL5 + CXCL9 + IL17A + TSLP + REN + PROM1 + VEGFA | 0,78 |
| GZMH + GNLY + ITGAE + CXCL9 + PF4 + IL17A + TSLP + REN + IHH + PROM1 + VEGFA | 0,78 |
| GZMH + GNLY + LAG3 + ITGAE + CXCL9 + PF4 + IL17A + TSLP + REN + IHH + VEGFA | 0,78 |
| GZMH + IFNG + CXCL13 + GNLY + LAG3 + PF4 + IL17A + REN + IHH + PROM1 + VEGFA | 0,78 |
| IFNG + CXCL13 + GNLY + LAG3 + CXCL9 + PF4 + IL17A + REN + IHH + PROM1 + VEGFA | 0,78 |
| GZMH + IFNG + CXCL13 + GNLY + LAG3 + CXCL9 + PF4 + IL17A + REN + IHH + VEGFA | 0,78 |
| IFNG + CXCL13 + GNLY + ITGAE + CXCL9 + PF4 + IL17A + TSLP + REN + IHH + PROM1 | 0,78 |
| GZMH + GNLY + LAG3 + ITGAE + CXCL9 + PF4 + IL17A + TSLP + REN + IHH + PROM1 | 0,78 |
| GZMH + IFNG + CXCL13 + GNLY + LAG3 + PF4 + IL17A + TSLP + REN + IHH + PROM1 | 0,78 |
| GZMH + CXCL13 + GNLY + LAG3 + ITGAE + CXCL9 + PF4 + IL17A + TSLP + REN + IHH | 0,78 |
| IFNG + CXCL13 + GNLY + ITGAE + PF4 + IL17A + TSLP + REN + IHH + PROM1 + VEGFA | 0,78 |
| IFNG + CXCL13 + GNLY + LAG3 + CXCL9 + PF4 + IL17A + TSLP + REN + PROM1 + VEGFA | 0,78 |
| GZMH + CXCL13 + GNLY + LAG3 + ITGAE + PF4 + IL17A + TSLP + REN + IHH + VEGFA | 0,78 |
| IFNG + CXCL13 + GNLY + CXCL9 + PF4 + IL17A + TSLP + REN + IHH + PROM1 + VEGFA | 0,78 |
| GZMH + IFNG + GNLY + LAG3 + CCL5 + CXCL9 + IL17A + REN + IHH + PROM1 + VEGFA | 0,78 |
| GZMH + IFNG + CXCL13 + GNLY + LAG3 + PF4 + IL17A + TSLP + REN + IHH + VEGFA | 0,78 |
| GZMH + IFNG + CXCL13 + GNLY + LAG3 + CXCL9 + PF4 + IL17A + TSLP + REN + PROM1 | 0,78 |
| GZMH + IFNG + CXCL13 + GNLY + LAG3 + CXCL9 + PF4 + IL17A + TSLP + REN + IHH | 0,78 |
| IFNG + CXCL13 + GNLY + LAG3 + CXCL9 + PF4 + IL17A + TSLP + REN + IHH + PROM1 | 0,78 |
| GZMH + IFNG + CXCL13 + GNLY + LAG3 + PF4 + IL17A + TSLP + REN + PROM1 + VEGFA | 0,78 |
| GZMH + GNLY + LAG3 + ITGAE + CXCL9 + PF4 + IL17A + REN + IHH + PROM1 + VEGFA | 0,78 |
| GZMH + CXCL13 + GNLY + LAG3 + ITGAE + CCL5 + CXCL9 + IL17A + REN + PROM1 + VEGFA | 0,78 |
| GZMH + CXCL13 + GNLY + LAG3 + ITGAE + CXCL9 + PF4 + IL17A + TSLP + REN + VEGFA | 0,78 |
| GZMH + CXCL13 + GNLY + LAG3 + ITGAE + PF4 + IL17A + TSLP + REN + PROM1 + VEGFA | 0,78 |
| IFNG + CXCL13 + GNLY + LAG3 + CXCL9 + PF4 + IL17A + TSLP + REN + IHH + VEGFA | 0,78 |
| GZMH + CXCL13 + GNLY + LAG3 + ITGAE + CXCL9 + PF4 + IL17A + REN + IHH + VEGFA | 0,78 |
| GZMH + CXCL13 + GNLY + LAG3 + CXCL9 + PF4 + IL17A + TSLP + REN + IHH + VEGFA | 0,78 |
| GZMH + GNLY + ITGAE + CCL5 + CXCL9 + IL17A + TSLP + REN + IHH + PROM1 + VEGFA | 0,78 |
| GZMH + CXCL13 + GNLY + LAG3 + ITGAE + PF4 + IL17A + TSLP + REN + IHH + PROM1 | 0,78 |
| GZMH + GNLY + LAG3 + CXCL9 + PF4 + IL17A + TSLP + REN + IHH + PROM1 + VEGFA | 0,78 |
| GZMH + CXCL13 + GNLY + LAG3 + ITGAE + CXCL9 + PF4 + IL17A + TSLP + REN + PROM1 | 0,78 |
| GZMH + IFNG + CXCL13 + GNLY + LAG3 + CXCL9 + PF4 + IL17A + REN + PROM1 + VEGFA | 0,78 |
| IFNG + CXCL13 + GNLY + LAG3 + PF4 + IL17A + TSLP + REN + IHH + PROM1 + VEGFA | 0,78 |
| CXCL13 + GNLY + LAG3 + ITGAE + CCL5 + CXCL9 + IL17A + TSLP + REN + IHH + VEGFA | 0,78 |
| GZMH + GNLY + LAG3 + ITGAE + CXCL9 + PF4 + IL17A + TSLP + REN + PROM1 + VEGFA | 0,78 |
| GZMH + CXCL13 + GNLY + LAG3 + ITGAE + PF4 + IL17A + REN + IHH + PROM1 + VEGFA | 0,78 |
| GZMH + GNLY + LAG3 + ITGAE + PF4 + IL17A + TSLP + REN + IHH + PROM1 + VEGFA | 0,78 |
| GZMH + CXCL13 + GNLY + LAG3 + ITGAE + CXCL9 + PF4 + IL17A + REN + IHH + PROM1 | 0,78 |
| GZMH + CXCL13 + GNLY + LAG3 + ITGAE + CXCL9 + PF4 + IL17A + REN + PROM1 + VEGFA | 0,78 |
| GZMH + CXCL13 + GNLY + LAG3 + PF4 + IL17A + TSLP + REN + IHH + PROM1 + VEGFA | 0,78 |
| GZMH + CXCL13 + GNLY + LAG3 + ITGAE + CCL5 + IL17A + TSLP + REN + IHH + PROM1 | 0,78 |
| CXCL13 + GNLY + LAG3 + ITGAE + CCL5 + IL17A + TSLP + REN + IHH + PROM1 + VEGFA | 0,78 |
| GZMH + CXCL13 + GNLY + LAG3 + CXCL9 + PF4 + IL17A + TSLP + REN + IHH + PROM1 | 0,78 |
| GZMH + CXCL13 + GNLY + LAG3 + CXCL9 + PF4 + IL17A + REN + IHH + PROM1 + VEGFA | 0,78 |
| GZMH + CXCL13 + GNLY + ITGAE + CXCL9 + PF4 + IL17A + TSLP + REN + IHH + VEGFA | 0,78 |
| GZMH + CXCL13 + GNLY + LAG3 + ITGAE + CCL5 + CXCL9 + IL17A + REN + IHH + VEGFA | 0,78 |
| CXCL13 + GNLY + ITGAE + CCL5 + CXCL9 + IL17A + TSLP + REN + IHH + PROM1 + VEGFA | 0,78 |
| GZMH + CXCL13 + GNLY + ITGAE + PF4 + IL17A + TSLP + REN + IHH + PROM1 + VEGFA | 0,78 |
| GZMH + CXCL13 + GNLY + ITGAE + CXCL9 + PF4 + IL17A + TSLP + REN + IHH + PROM1 | 0,78 |
| GZMH + IFNG + GNLY + LAG3 + CCL5 + PF4 + TSLP + REN + IHH + PROM1 + VEGFA | 0,78 |
| GZMH + CXCL13 + GNLY + LAG3 + CXCL9 + PF4 + IL17A + TSLP + REN + PROM1 + VEGFA | 0,78 |
| CXCL13 + GNLY + LAG3 + ITGAE + CCL5 + CXCL9 + IL17A + TSLP + REN + IHH + PROM1 | 0,78 |
| GZMH + CXCL13 + GNLY + ITGAE + CCL5 + CXCL9 + IL17A + TSLP + REN + IHH + VEGFA | 0,78 |
| GZMH + CXCL13 + GNLY + LAG3 + ITGAE + CCL5 + IL17A + REN + IHH + PROM1 + VEGFA | 0,78 |
| GZMH + CXCL13 + GNLY + ITGAE + CXCL9 + PF4 + IL17A + TSLP + REN + PROM1 + VEGFA | 0,78 |
| CXCL13 + GNLY + LAG3 + ITGAE + CXCL9 + PF4 + IL17A + TSLP + REN + IHH + VEGFA | 0,78 |
| GZMH + CXCL13 + GNLY + ITGAE + CCL5 + CXCL9 + IL17A + REN + IHH + PROM1 + VEGFA | 0,78 |
| GZMH + CXCL13 + GNLY + LAG3 + ITGAE + CCL5 + CXCL9 + IL17A + TSLP + REN + VEGFA | 0,78 |
| CXCL13 + GNLY + LAG3 + CXCL9 + PF4 + IL17A + TSLP + REN + IHH + PROM1 + VEGFA | 0,78 |
| GZMH + CXCL13 + GNLY + LAG3 + ITGAE + CCL5 + CXCL9 + IL17A + REN + IHH + PROM1 | 0,78 |
| GZMH + CXCL13 + GNLY + LAG3 + ITGAE + CCL5 + IL17A + TSLP + REN + IHH + VEGFA | 0,78 |
| GZMH + CXCL13 + GNLY + CXCL9 + PF4 + IL17A + TSLP + REN + IHH + PROM1 + VEGFA | 0,78 |
| CXCL13 + GNLY + LAG3 + ITGAE + CXCL9 + PF4 + IL17A + REN + IHH + PROM1 + VEGFA | 0,78 |
| GZMH + CXCL13 + GNLY + LAG3 + ITGAE + CCL5 + CXCL9 + IL17A + TSLP + REN + PROM1 | 0,78 |
| GZMH + CXCL13 + GNLY + ITGAE + CCL5 + CXCL9 + IL17A + TSLP + REN + PROM1 + VEGFA | 0,78 |
| GZMH + CXCL13 + GNLY + LAG3 + ITGAE + CCL5 + IL17A + TSLP + REN + PROM1 + VEGFA | 0,78 |
| CXCL13 + GNLY + LAG3 + ITGAE + PF4 + IL17A + TSLP + REN + IHH + PROM1 + VEGFA | 0,78 |
| IFNG + GNLY + LAG3 + CCL5 + CXCL9 + IL17A + TSLP + REN + IHH + PROM1 + VEGFA | 0,78 |
| GZMH + IFNG + GNLY + LAG3 + ITGAE + CCL5 + PF4 + TSLP + REN + IHH + VEGFA | 0,78 |
| GZMH + GNLY + LAG3 + ITGAE + CCL5 + IL17A + TSLP + REN + IHH + PROM1 + VEGFA | 0,78 |
| GNLY + LAG3 + ITGAE + CXCL9 + PF4 + IL17A + TSLP + REN + IHH + PROM1 + VEGFA | 0,78 |
| GZMH + IFNG + GNLY + LAG3 + CCL5 + CXCL9 + IL17A + TSLP + REN + IHH + PROM1 | 0,78 |
| GZMH + CXCL13 + GNLY + ITGAE + CXCL9 + PF4 + IL17A + REN + IHH + PROM1 + VEGFA | 0,78 |
| GZMH + GNLY + LAG3 + ITGAE + CCL5 + CXCL9 + IL17A + TSLP + REN + IHH + PROM1 | 0,78 |
| CXCL13 + GNLY + LAG3 + ITGAE + CXCL9 + PF4 + IL17A + TSLP + REN + IHH + PROM1 | 0,78 |
| GZMH + IFNG + GNLY + ITGAE + CCL5 + CXCL9 + PF4 + TSLP + REN + IHH + PROM1 | 0,78 |
| GZMH + IFNG + CXCL13 + GNLY + LAG3 + CCL5 + CXCL9 + IL17A + REN + IHH + VEGFA | 0,78 |
| GZMH + CXCL13 + GNLY + ITGAE + CCL5 + CXCL9 + IL17A + TSLP + REN + IHH + PROM1 | 0,78 |
| GZMH + IFNG + CXCL13 + GNLY + LAG3 + CCL5 + CXCL9 + IL17A + TSLP + REN + VEGFA | 0,78 |
| GZMH + CXCL13 + GNLY + ITGAE + CCL5 + IL17A + TSLP + REN + IHH + PROM1 + VEGFA | 0,77 |
| CXCL13 + GNLY + LAG3 + ITGAE + CXCL9 + PF4 + IL17A + TSLP + REN + PROM1 + VEGFA | 0,77 |
| GZMH + IFNG + GNLY + LAG3 + ITGAE + CCL5 + CXCL9 + PF4 + REN + IHH + PROM1 | 0,77 |
| GZMH + IFNG + CXCL13 + GNLY + LAG3 + CCL5 + CXCL9 + IL17A + REN + PROM1 + VEGFA | 0,77 |
| GZMH + GNLY + LAG3 + ITGAE + CCL5 + PF4 + TSLP + REN + IHH + PROM1 + VEGFA | 0,77 |
| GZMH + CXCL13 + GNLY + LAG3 + ITGAE + CCL5 + CXCL9 + IL17A + TSLP + REN + IHH | 0,77 |
| GZMH + IFNG + GNLY + LAG3 + ITGAE + CCL5 + CXCL9 + PF4 + TSLP + REN + PROM1 | 0,77 |
| IFNG + CXCL13 + GNLY + LAG3 + CCL5 + CXCL9 + IL17A + TSLP + REN + IHH + VEGFA | 0,77 |
| GZMH + IFNG + CXCL13 + GNLY + LAG3 + CCL5 + CXCL9 + IL17A + TSLP + REN + IHH | 0,77 |
| GZMH + IFNG + CXCL13 + GNLY + LAG3 + CCL5 + CXCL9 + IL17A + TSLP + REN + PROM1 | 0,77 |
| GZMH + IFNG + CXCL13 + GNLY + LAG3 + ITGAE + CCL5 + PF4 + IL17A + IHH + PROM1 | 0,77 |
| GZMH + IFNG + CXCL13 + GNLY + LAG3 + CCL5 + IL17A + TSLP + REN + PROM1 + VEGFA | 0,77 |
| GZMH + IFNG + CXCL13 + GNLY + LAG3 + CCL5 + CXCL9 + IL17A + REN + IHH + PROM1 | 0,77 |
| GZMH + IFNG + CXCL13 + GNLY + ITGAE + CCL5 + CXCL9 + PF4 + IL17A + IHH + PROM1 | 0,77 |
| GZMH + GNLY + LAG3 + ITGAE + CCL5 + CXCL9 + PF4 + TSLP + REN + IHH + PROM1 | 0,77 |
| GZMH + IFNG + CXCL13 + GNLY + LAG3 + CCL5 + IL17A + TSLP + REN + IHH + VEGFA | 0,77 |
| GZMH + IFNG + GNLY + LAG3 + ITGAE + CCL5 + CXCL9 + PF4 + TSLP + REN + IHH | 0,77 |
| GZMH + IFNG + GNLY + ITGAE + CCL5 + CXCL9 + PF4 + TSLP + REN + PROM1 + VEGFA | 0,77 |
| GZMH + IFNG + CXCL13 + GNLY + LAG3 + CCL5 + IL17A + TSLP + REN + IHH + PROM1 | 0,77 |
| GZMH + IFNG + GNLY + LAG3 + CCL5 + CXCL9 + PF4 + REN + IHH + PROM1 + VEGFA | 0,77 |
| GZMH + GNLY + LAG3 + CCL5 + CXCL9 + IL17A + TSLP + REN + IHH + PROM1 + VEGFA | 0,77 |
| GZMH + IFNG + CXCL13 + GNLY + ITGAE + CCL5 + PF4 + IL17A + IHH + PROM1 + VEGFA | 0,77 |
| GZMH + IFNG + CXCL13 + GNLY + ITGAE + CCL5 + PF4 + IL17A + TSLP + IHH + PROM1 | 0,77 |
| GZMH + IFNG + GNLY + LAG3 + CCL5 + IL17A + TSLP + REN + IHH + PROM1 + VEGFA | 0,77 |
| IFNG + GNLY + LAG3 + ITGAE + CCL5 + CXCL9 + PF4 + TSLP + REN + IHH + PROM1 | 0,77 |
| GZMH + IFNG + CXCL13 + GNLY + LAG3 + ITGAE + CCL5 + PF4 + IL17A + PROM1 + VEGFA | 0,77 |
| GZMH + GNLY + ITGAE + CCL5 + CXCL9 + PF4 + TSLP + REN + IHH + PROM1 + VEGFA | 0,77 |
| GZMH + IFNG + GNLY + LAG3 + ITGAE + CXCL9 + IL17A + TSLP + REN + IHH + VEGFA | 0,77 |
| GZMH + IFNG + GNLY + ITGAE + CCL5 + CXCL9 + PF4 + REN + IHH + PROM1 + VEGFA | 0,77 |
| IFNG + CXCL13 + GNLY + LAG3 + CCL5 + CXCL9 + IL17A + REN + IHH + PROM1 + VEGFA | 0,77 |
| GZMH + IFNG + CXCL13 + GNLY + LAG3 + ITGAE + CCL5 + CXCL9 + PF4 + IL17A + PROM1 | 0,77 |
| IFNG + CXCL13 + GNLY + ITGAE + CCL5 + CXCL9 + PF4 + IL17A + IHH + PROM1 + VEGFA | 0,77 |
| IFNG + CXCL13 + GNLY + LAG3 + CCL5 + CXCL9 + IL17A + TSLP + REN + PROM1 + VEGFA | 0,77 |
| GZMH + IFNG + CXCL13 + GNLY + LAG3 + ITGAE + CCL5 + PF4 + IL17A + TSLP + PROM1 | 0,77 |
| GZMH + IFNG + CXCL13 + GNLY + LAG3 + CCL5 + IL17A + REN + IHH + PROM1 + VEGFA | 0,77 |
| GZMH + IFNG + GNLY + LAG3 + CCL5 + CXCL9 + PF4 + TSLP + REN + PROM1 + VEGFA | 0,77 |
| GZMH + IFNG + GNLY + LAG3 + ITGAE + CCL5 + CXCL9 + PF4 + REN + PROM1 + VEGFA | 0,77 |
| CXCL13 + GNLY + ITGAE + CXCL9 + PF4 + IL17A + TSLP + REN + IHH + PROM1 + VEGFA | 0,77 |
| GZMH + IFNG + GNLY + LAG3 + CCL5 + CXCL9 + PF4 + TSLP + REN + IHH + PROM1 | 0,77 |
| GZMH + IFNG + GNLY + LAG3 + ITGAE + CCL5 + CXCL9 + PF4 + IL17A + IHH + VEGFA | 0,77 |
| GZMH + IFNG + GNLY + ITGAE + CCL5 + CXCL9 + PF4 + IL17A + IHH + PROM1 + VEGFA | 0,77 |
| GNLY + LAG3 + ITGAE + CCL5 + CXCL9 + PF4 + TSLP + REN + IHH + PROM1 + VEGFA | 0,77 |
| GZMH + GNLY + LAG3 + ITGAE + CCL5 + CXCL9 + PF4 + TSLP + REN + PROM1 + VEGFA | 0,77 |
| IFNG + CXCL13 + GNLY + LAG3 + ITGAE + CCL5 + PF4 + IL17A + IHH + PROM1 + VEGFA | 0,77 |
| GZMH + GNLY + LAG3 + CCL5 + CXCL9 + PF4 + TSLP + REN + IHH + PROM1 + VEGFA | 0,77 |
| GZMH + CXCL13 + GNLY + LAG3 + ITGAE + CCL5 + PF4 + IL17A + TSLP + IHH + PROM1 | 0,77 |
| GZMH + CXCL13 + GNLY + LAG3 + ITGAE + CCL5 + CXCL9 + PF4 + IL17A + IHH + PROM1 | 0,77 |
| IFNG + CXCL13 + GNLY + LAG3 + ITGAE + CCL5 + CXCL9 + PF4 + IL17A + IHH + PROM1 | 0,77 |
| GZMH + CXCL13 + GNLY + LAG3 + CCL5 + CXCL9 + IL17A + TSLP + REN + IHH + VEGFA | 0,77 |
| GZMH + IFNG + GNLY + LAG3 + ITGAE + CXCL9 + IL17A + REN + IHH + PROM1 + VEGFA | 0,77 |
| GZMH + IFNG + CXCL13 + GNLY + LAG3 + ITGAE + CCL5 + PF4 + TSLP + REN + PROM1 | 0,77 |
| GZMH + IFNG + CXCL13 + GNLY + ITGAE + CCL5 + PF4 + IL17A + TSLP + PROM1 + VEGFA | 0,77 |
| IFNG + CXCL13 + GNLY + LAG3 + ITGAE + CCL5 + PF4 + IL17A + TSLP + IHH + PROM1 | 0,77 |
| GZMH + CXCL13 + GNLY + ITGAE + CCL5 + CXCL9 + PF4 + IL17A + TSLP + IHH + PROM1 | 0,77 |
| GZMH + IFNG + GNLY + CCL5 + CXCL9 + IL17A + TSLP + REN + IHH + PROM1 + VEGFA | 0,77 |
| CXCL13 + GNLY + LAG3 + CCL5 + CXCL9 + IL17A + TSLP + REN + IHH + PROM1 + VEGFA | 0,77 |
| IFNG + GNLY + ITGAE + CCL5 + CXCL9 + PF4 + TSLP + REN + IHH + PROM1 + VEGFA | 0,77 |
| GZMH + IFNG + CXCL13 + GNLY + ITGAE + CCL5 + PF4 + TSLP + REN + PROM1 + VEGFA | 0,77 |
| GZMH + IFNG + GNLY + LAG3 + ITGAE + IL17A + TSLP + REN + IHH + PROM1 + VEGFA | 0,77 |
| GZMH + IFNG + GNLY + ITGAE + CXCL9 + IL17A + TSLP + REN + IHH + PROM1 + VEGFA | 0,77 |
| IFNG + CXCL13 + GNLY + LAG3 + CCL5 + CXCL9 + IL17A + TSLP + REN + IHH + PROM1 | 0,77 |
| GZMH + IFNG + CXCL13 + GNLY + ITGAE + CCL5 + CXCL9 + PF4 + IL17A + TSLP + PROM1 | 0,77 |
| GZMH + IFNG + GNLY + ITGAE + CCL5 + CXCL9 + PF4 + IL17A + TSLP + IHH + VEGFA | 0,77 |
| IFNG + CXCL13 + GNLY + ITGAE + CCL5 + PF4 + IL17A + TSLP + IHH + PROM1 + VEGFA | 0,77 |
| GZMH + CXCL13 + GNLY + LAG3 + CCL5 + CXCL9 + IL17A + REN + IHH + PROM1 + VEGFA | 0,77 |
| GZMH + CXCL13 + GNLY + LAG3 + CCL5 + CXCL9 + IL17A + TSLP + REN + IHH + PROM1 | 0,77 |
| GZMH + CXCL13 + GNLY + LAG3 + ITGAE + CCL5 + PF4 + IL17A + IHH + PROM1 + VEGFA | 0,77 |
| IFNG + CXCL13 + GNLY + ITGAE + CCL5 + CXCL9 + PF4 + IL17A + TSLP + IHH + PROM1 | 0,77 |
| GZMH + IFNG + GNLY + CCL5 + CXCL9 + PF4 + TSLP + REN + IHH + PROM1 + VEGFA | 0,77 |
| IFNG + GNLY + LAG3 + ITGAE + CCL5 + CXCL9 + PF4 + TSLP + REN + PROM1 + VEGFA | 0,77 |
| GZMH + IFNG + CXCL13 + GNLY + ITGAE + CXCL9 + IL17A + TSLP + REN + IHH + VEGFA | 0,77 |
| GZMH + IFNG + GNLY + LAG3 + ITGAE + CXCL9 + IL17A + TSLP + REN + IHH + PROM1 | 0,77 |
| GZMH + IFNG + GNLY + LAG3 + ITGAE + CXCL9 + IL17A + TSLP + REN + PROM1 + VEGFA | 0,77 |
| CXCL13 + GNLY + LAG3 + ITGAE + CCL5 + CXCL9 + PF4 + IL17A + TSLP + IHH + PROM1 | 0,77 |
| GZMH + CXCL13 + GNLY + LAG3 + CCL5 + IL17A + TSLP + REN + IHH + PROM1 + VEGFA | 0,77 |

| **Table 12: clusters of 12 genes:** | |
|---|---|
| **clusters of 12 genes** | **c_tau level** |
| GZMH + IFNG + GNLY + ITGAE + CCL5 + PF4 + IL17A + TSLP + REN + IHH + PROM1 + VEGFA | 0,8 |
| GZMH + IFNG + GNLY + LAG3 + ITGAE + CCL5 + PF4 + IL17A + REN + IHH + PROM1 + VEGFA | 0,8 |
| GZMH + IFNG + GNLY + LAG3 + ITGAE + CCL5 + PF4 + IL17A + TSLP + REN + IHH + PROM 1 | 0,8 |
| GZMH + IFNG + GNLY + LAG3 + ITGAE + CCL5 + PF4 + IL17A + TSLP + REN + PROM1 + VEGFA | 0,8 |
| IFNG + GNLY + LAG3 + ITGAE + CCL5 + PF4 + IL17A + TSLP + REN + IHH + PROM1 + VEGFA | 0,8 |
| GZMH + IFNG + GNLY + ITGAE + CCL5 + CXCL9 + PF4 + IL17A + TSLP + REN + IHH + VEGFA | 0,8 |
| GZMH + IFNG + GNLY + ITGAE + CCL5 + CXCL9 + PF4 + IL17A + REN + IHH + PROM1 + VEGFA | 0,8 |
| GZMH + IFNG + GNLY + LAG3 + ITGAE + CCL5 + PF4 + IL17A + TSLP + REN + IHH + VEGFA | 0,8 |
| GZMH + IFNG + GNLY + LAG3 + ITGAE + CCL5 + CXCL9 + PF4 + IL17A + REN + IHH + VEGFA | 0,8 |
| GZMH + IFNG + GNLY + ITGAE + CCL5 + CXCL9 + PF4 + IL17A + TSLP + REN + PROM1 + VEGFA | 0,8 |
| GZMH + IFNG + GNLY + LAG3 + ITGAE + CCL5 + CXCL9 + PF4 + IL17A + REN + IHH + PROM 1 | 0,8 |
| GZMH + IFNG + GNLY + LAG3 + ITGAE + CCL5 + CXCL9 + PF4 + IL17A + TSLP + REN + VEGFA | 0,8 |
| GZMH + IFNG + GNLY + LAG3 + ITGAE + CCL5 + CXCL9 + PF4 + IL17A + REN + PROM1 + VEGFA | 0,8 |
| GZMH + IFNG + GNLY + LAG3 + ITGAE + CCL5 + CXCL9 + PF4 + IL17A + TSLP + REN + PROM 1 | 0,8 |
| GZMH + IFNG + GNLY + ITGAE + CCL5 + CXCL9 + PF4 + IL17A + TSLP + REN + IHH + PROM 1 | 0,8 |
| GZMH + IFNG + CXCL13 + GNLY + ITGAE + CCL5 + PF4 + IL17A + REN + IHH + PROM1 + VEGFA | 0,8 |
| GZMH + IFNG + GNLY + LAG3 + ITGAE + CCL5 + CXCL9 + PF4 + IL17A + TSLP + REN + IHH | 0,8 |
| IFNG + GNLY + LAG3 + ITGAE + CCL5 + CXCL9 + PF4 + IL17A + TSLP + REN + IHH + VEGFA | 0,8 |
| GZMH + IFNG + CXCL13 + GNLY + LAG3 + ITGAE + CCL5 + PF4 + IL17A + REN + IHH + PROM1 | 0,8 |
| GZMH + IFNG + CXCL13 + GNLY + ITGAE + CCL5 + PF4 + IL17A + TSLP + REN + IHH + PROM 1 | 0,8 |
| GZMH + IFNG + CXCL13 + GNLY + ITGAE + CCL5 + PF4 + IL17A + TSLP + REN + PROM1 + VEGFA | 0,8 |
| GZMH + IFNG + CXCL13 + GNLY + LAG3 + ITGAE + CCL5 + PF4 + IL17A + REN + PROM1 + VEGFA | 0,8 |
| GZMH + IFNG + CXCL13 + GNLY + LAG3 + ITGAE + CCL5 + PF4 + IL17A + TSLP + REN + PROM1 | 0,79 |
| GZMH + IFNG + CXCL13 + GNLY + LAG3 + ITGAE + CCL5 + CXCL9 + PF4 + IL17A + REN + PROM1 | 0,79 |
| GZMH + IFNG + CXCL13 + GNLY + ITGAE + CCL5 + CXCL9 + PF4 + IL17A + REN + IHH + PROM1 | 0,79 |
| GZMH + IFNG + CXCL13 + GNLY + ITGAE + CCL5 + CXCL9 + PF4 + IL17A + TSLP + REN + PROM1 | 0,79 |
| IFNG + GNLY + ITGAE + CCL5 + CXCL9 + PF4 + IL17A + TSLP + REN + IHH + PROM1 + VEGFA | 0,79 |
| IFNG + GNLY + LAG3 + ITGAE + CCL5 + CXCL9 + PF4 + IL17A + REN + IHH + PROM1 + VEGFA | 0,79 |
| GZMH + IFNG + CXCL13 + GNLY + ITGAE + CCL5 + CXCL9 + PF4 + IL17A + REN + IHH + VEGFA | 0,79 |
| GZMH + IFNG + CXCL13 + GNLY + ITGAE + CCL5 + CXCL9 + PF4 + IL17A + REN + PROM1 + VEGFA | 0,79 |
| IFNG + CXCL13 + GNLY + LAG3 + ITGAE + CCL5 + PF4 + IL17A + TSLP + REN + IHH + PROM1 | 0,79 |
| IFNG + GNLY + LAG3 + ITGAE + CCL5 + CXCL9 + PF4 + IL17A + TSLP + REN + PROM1 + VEGFA | 0,79 |
| IFNG + CXCL13 + GNLY + ITGAE + CCL5 + PF4 + IL17A + TSLP + REN + IHH + PROM1 + VEGFA | 0,79 |
| IFNG + CXCL13 + GNLY + LAG3 + ITGAE + CCL5 + PF4 + IL17A + TSLP + REN + PROM1 + VEGFA | 0,79 |
| IFNG + CXCL13 + GNLY + LAG3 + ITGAE + CCL5 + PF4 + IL17A + REN + IHH + PROM1 + VEGFA | 0,79 |
| GZMH + IFNG + CXCL13 + GNLY + ITGAE + CCL5 + CXCL9 + PF4 + IL17A + TSLP + REN + VEGFA | 0,79 |
| IFNG + CXCL13 + GNLY + LAG3 + ITGAE + CCL5 + CXCL9 + PF4 + IL17A + REN + IHH + VEGFA | 0,79 |
| IFNG + GNLY + LAG3 + ITGAE + CCL5 + CXCL9 + PF4 + IL17A + TSLP + REN + IHH + PROM1 | 0,79 |
| IFNG + CXCL13 + GNLY + ITGAE + CCL5 + CXCL9 + PF4 + IL17A + TSLP + REN + IHH + VEGFA | 0,79 |
| GZMH + IFNG + CXCL13 + GNLY + ITGAE + CCL5 + PF4 + IL17A + TSLP + REN + IHH + VEGFA | 0,79 |
| IFNG + CXCL13 + GNLY + LAG3 + ITGAE + CCL5 + CXCL9 + PF4 + IL17A + REN + PROM1 + VEGFA | 0,79 |
| GZMH + IFNG + CXCL13 + GNLY + LAG3 + ITGAE + CCL5 + CXCL9 + PF4 + IL17A + REN + VEGFA | 0,79 |
| GZMH + IFNG + CXCL13 + GNLY + LAG3 + ITGAE + CCL5 + PF4 + IL17A + REN + IHH + VEGFA | 0,79 |
| IFNG + CXCL13 + GNLY + LAG3 + ITGAE + CCL5 + CXCL9 + PF4 + IL17A + TSLP + REN + VEGFA | 0,79 |
| IFNG + CXCL13 + GNLY + ITGAE + CCL5 + CXCL9 + PF4 + IL17A + TSLP + REN + PROM1 + VEGFA | 0,79 |
| IFNG + CXCL13 + GNLY + ITGAE + CCL5 + CXCL9 + PF4 + IL17A + REN + IHH + PROM1 + VEGFA | 0,79 |
| GZMH + IFNG + CXCL13 + GNLY + LAG3 + ITGAE + CCL5 + CXCL9 + PF4 + IL17A + REN + IHH | 0,79 |
| IFNG + CXCL13 + GNLY + LAG3 + ITGAE + CCL5 + CXCL9 + PF4 + IL17A + REN + IHH + PROM1 | 0,79 |
| IFNG + CXCL13 + GNLY + LAG3 + ITGAE + CCL5 + PF4 + IL17A + TSLP + REN + IHH + VEGFA | 0,79 |
| GZMH + IFNG + CXCL13 + GNLY + ITGAE + CCL5 + CXCL9 + PF4 + IL17A + TSLP + REN + IHH | 0,79 |
| IFNG + CXCL13 + GNLY + LAG3 + ITGAE + CCL5 + CXCL9 + PF4 + IL17A + TSLP + REN + PROM1 | 0,79 |
| IFNG + CXCL13 + GNLY + ITGAE + CCL5 + CXCL9 + PF4 + IL17A + TSLP + REN + IHH + PROM1 | 0,79 |
| GZMH + IFNG + CXCL13 + GNLY + LAG3 + ITGAE + CCL5 + PF4 + IL17A + TSLP + REN + VEGFA | 0,79 |
| GZMH + IFNG + CXCL13 + GNLY + LAG3 + ITGAE + CCL5 + PF4 + IL17A + TSLP + REN + IHH | 0,79 |
| GZMH + IFNG + GNLY + LAG3 + CCL5 + PF4 + IL17A + TSLP + REN + IHH + PROM1 + VEGFA | 0,79 |
| GZMH + IFNG + GNLY + LAG3 + CCL5 + CXCL9 + PF4 + IL17A + TSLP + REN + IHH + VEGFA | 0,79 |
| IFNG + CXCL13 + GNLY + LAG3 + ITGAE + CCL5 + CXCL9 + PF4 + IL17A + TSLP + REN + IHH | 0,79 |
| GZMH + IFNG + GNLY + LAG3 + ITGAE + CCL5 + CXCL9 + IL17A + TSLP + REN + IHH + VEGFA | 0,79 |
| GZMH + IFNG + CXCL13 + GNLY + LAG3 + ITGAE + CCL5 + CXCL9 + PF4 + IL17A + TSLP + REN | 0,79 |
| GZMH + IFNG + GNLY + LAG3 + ITGAE + CCL5 + CXCL9 + IL17A + REN + IHH + PROM 1 + VEGFA | 0,79 |
| GZMH + CXCL13 + GNLY + ITGAE + CCL5 + PF4 + IL17A + TSLP + REN + IHH + PROM1 + VEGFA | 0,79 |
| GZMH + CXCL13 + GNLY + ITGAE + CCL5 + CXCL9 + PF4 + IL17A + REN + IHH + PROM1 + VEGFA | 0,79 |
| GZMH + CXCL13 + GNLY + ITGAE + CCL5 + CXCL9 + PF4 + IL17A + TSLP + REN + IHH + PROM1 | 0,79 |
| GZMH + IFNG + GNLY + LAG3 + ITGAE + CCL5 + IL17A + TSLP + REN + IHH + PROM1 + VEGFA | 0,79 |
| GZMH + GNLY + ITGAE + CCL5 + CXCL9 + PF4 + IL17A + TSLP + REN + IHH + PROM1 + VEGFA | 0,79 |
| CXCL13 + GNLY + LAG3 + ITGAE + CCL5 + PF4 + IL17A + TSLP + REN + IHH + PROM1 + VEGFA | 0,79 |
| GZMH + IFNG + GNLY + LAG3 + ITGAE + CCL5 + CXCL9 + IL17A + TSLP + REN + PROM1 + VEGFA | 0,79 |
| CXCL13 + GNLY + ITGAE + CCL5 + CXCL9 + PF4 + IL17A + TSLP + REN + IHH + PROM1 + VEGFA | 0,79 |
| GZMH + CXCL13 + GNLY + LAG3 + ITGAE + CCL5 + CXCL9 + PF4 + IL17A + REN + IHH + PROM1 | 0,79 |
| CXCL13 + GNLY + LAG3 + ITGAE + CCL5 + CXCL9 + PF4 + IL17A + TSLP + REN + IHH + PROM1 | 0,79 |
| GZMH + CXCL13 + GNLY + LAG3 + ITGAE + CCL5 + PF4 + IL17A + REN + IHH + PROM1 + VEGFA | 0,79 |
| GZMH + CXCL13 + GNLY + LAG3 + ITGAE + CCL5 + PF4 + IL17A + TSLP + REN + IHH + PROM1 | 0,79 |
| CXCL13 + GNLY + LAG3 + ITGAE + CCL5 + CXCL9 + PF4 + IL17A + REN + IHH + PROM1 + VEGFA | 0,79 |
| GZMH + IFNG + GNLY + LAG3 + CCL5 + CXCL9 + PF4 + IL17A + REN + IHH + PROM1 + VEGFA | 0,79 |
| GNLY + LAG3 + ITGAE + CCL5 + CXCL9 + PF4 + IL17A + TSLP + REN + IHH + PROM1 + VEGFA | 0,79 |
| GZMH + GNLY + LAG3 + ITGAE + CCL5 + PF4 + IL17A + TSLP + REN + IHH + PROM1 + VEGFA | 0,79 |
| GZMH + IFNG + GNLY + LAG3 + CCL5 + CXCL9 + PF4 + IL17A + TSLP + REN + PROM1 + VEGFA | 0,79 |
| CXCL13 + GNLY + LAG3 + ITGAE + CCL5 + CXCL9 + PF4 + IL17A + TSLP + REN + PROM1 + VEGFA | 0,79 |
| GZMH + CXCL13 + GNLY + ITGAE + CCL5 + CXCL9 + PF4 + IL17A + TSLP + REN + PROM1 + VEGFA | 0,79 |
| GZMH + IFNG + CXCL13 + GNLY + LAG3 + ITGAE + CCL5 + CXCL9 + IL17A + REN + PROM1 + VEGFA | 0,79 |
| GZMH + CXCL13 + GNLY + LAG3 + ITGAE + CCL5 + PF4 + IL17A + TSLP + REN + PROM1 + VEGFA | 0,79 |
| GZMH + GNLY + LAG3 + ITGAE + CCL5 + CXCL9 + PF4 + IL17A + REN + IHH + PROM1 + VEGFA | 0,79 |
| GZMH + GNLY + LAG3 + ITGAE + CCL5 + CXCL9 + PF4 + IL17A + TSLP + REN + IHH + VEGFA | 0,79 |
| GZMH + GNLY + LAG3 + ITGAE + CCL5 + CXCL9 + PF4 + IL17A + TSLP + REN + PROM1 + VEGFA | 0,79 |
| GZMH + CXCL13 + GNLY + LAG3 + ITGAE + CCL5 + CXCL9 + PF4 + IL17A + TSLP + REN + PROM1 | 0,79 |
| .GZMH + CXCL13 + GNLY + LAG3 + ITGAE + CCL5 + CXCL9 + PF4 + IL17A + REN + PROM1 + VEGFA | 0,79 |
| GZMH + GNLY + LAG3 + ITGAE + CCL5 + CXCL9 + PF4 + IL17A + TSLP + REN + IHH + PROM1 | 0,79 |
| GZMH + IFNG + GNLY + ITGAE + CCL5 + CXCL9 + IL17A + TSLP + REN + IHH + PROM1 + VEGFA | 0,79 |
| GZMH + CXCL13 + GNLY + LAG3 + ITGAE + CCL5 + CXCL9 + PF4 + IL17A + REN + IHH + VEGFA | 0,79 |
| CXCL13 + GNLY + LAG3 + ITGAE + CCL5 + CXCL9 + PF4 + IL17A + TSLP + REN + IHH + VEGFA | 0,79 |
| GZMH + CXCL13 + GNLY + ITGAE + CCL5 + CXCL9 + PF4 + IL17A + TSLP + REN + IHH + VEGFA | 0,79 |
| GZMH + IFNG + GNLY + LAG3 + CCL5 + CXCL9 + PF4 + IL17A + TSLP + REN + IHH + PROM1 | 0,79 |
| GZMH + IFNG + CXCL13 + GNLY + LAG3 + ITGAE + CCL5 + IL17A + TSLP + REN + PROM1 + VEGFA | 0,79 |
| GZMH + IFNG + CXCL13 + GNLY + LAG3 + ITGAE + CCL5 + CXCL9 + IL17A + TSLP + REN + VEGFA | 0,79 |
| GZMH + IFNG + GNLY + LAG3 + ITGAE + CCL5 + CXCL9 + IL17A + TSLP + REN + IHH + PROM1 | 0,79 |
| GZMH + IFNG + CXCL13 + GNLY + LAG3 + CCL5 + CXCL9 + PF4 + IL17A + REN + IHH + VEGFA | 0,79 |
| IFNG + GNLY + LAG3 + ITGAE + CCL5 + CXCL9 + IL17A + TSLP + REN + IHH + PROM1 + VEGFA | 0,79 |
| GZMH + IFNG + CXCL13 + GNLY + LAG3 + ITGAE + CCL5 + CXCL9 + IL17A + REN + IHH + VEGFA | 0,79 |
| GZMH + CXCL13 + GNLY + LAG3 + ITGAE + CCL5 + CXCL9 + PF4 + IL17A + TSLP + REN + IHH | 0,79 |
| GZMH + IFNG + GNLY + CCL5 + CXCL9 + PF4 + IL17A + TSLP + REN + IHH + PROM1 + VEGFA | 0,79 |
| IFNG + CXCL13 + GNLY + LAG3 + ITGAE + CCL5 + CXCL9 + IL17A + TSLP + REN + PROM1 + VEGFA | 0,79 |
| GZMH + IFNG + CXCL13 + GNLY + LAG3 + ITGAE + CCL5 + CXCL9 + IL17A + TSLP + REN + PROM1 | 0,79 |
| GZMH + IFNG + CXCL13 + GNLY + LAG3 + CCL5 + CXCL9 + PF4 + IL17A + TSLP + REN + IHH | 0,79 |
| IFNG + GNLY + LAG3 + CCL5 + CXCL9 + PF4 + IL17A + TSLP + REN + IHH + PROM1 + VEGFA | 0,79 |
| GZMH + CXCL13 + GNLY + LAG3 + ITGAE + CCL5 + PF4 + IL17A + TSLP + REN + IHH + VEGFA | 0,79 |
| GZMH + IFNG + CXCL13 + GNLY + LAG3 + ITGAE + CCL5 + IL17A + TSLP + REN + IHH + PROM1 | 0,79 |
| GZMH + IFNG + CXCL13 + GNLY + LAG3 + ITGAE + CCL5 + IL17A + REN + IHH + PROM1 + VEGFA | 0,79 |
| IFNG + CXCL13 + GNLY + LAG3 + ITGAE + CCL5 + CXCL9 + IL17A + TSLP + REN + IHH + VEGFA | 0,79 |
| GZMH + CXCL13 + GNLY + LAG3 + ITGAE + CCL5 + CXCL9 + PF4 + IL17A + TSLP + REN + VEGFA | 0,79 |
| GZMH + IFNG + CXCL13 + GNLY + CCL5 + CXCL9 + PF4 + IL17A + TSLP + REN + IHH + VEGFA | 0,79 |
| GZMH + IFNG + CXCL13 + GNLY + LAG3 + CCL5 + PF4 + IL17A + TSLP + REN + IHH + VEGFA | 0,79 |
| GZMH + IFNG + CXCL13 + GNLY + ITGAE + CCL5 + CXCL9 + IL17A + TSLP + REN + IHH + VEGFA | 0,79 |
| GZMH + IFNG + CXCL13 + GNLY + LAG3 + CCL5 + CXCL9 + PF4 + IL17A + TSLP + REN + VEGFA | 0,79 |
| GZMH + CXCL13 + GNLY + LAG3 + CCL5 + CXCL9 + PF4 + IL17A + REN + IHH + PROM1 + VEGFA | 0,79 |
| IFNG + CXCL13 + GNLY + LAG3 + ITGAE + CCL5 + CXCL9 + IL17A + REN + IHH + PROM1 + VEGFA | 0,79 |
| GZMH + IFNG + CXCL13 + GNLY + LAG3 + CCL5 + CXCL9 + PF4 + IL17A + REN + IHH + PROM1 | 0,79 |
| GZMH + IFNG + CXCL13 + GNLY + LAG3 + CCL5 + PF4 + IL17A + TSLP + REN + PROM1 + VEGFA | 0,79 |
| GZMH + GNLY + LAG3 + CCL5 + CXCL9 + PF4 + IL17A + TSLP + REN + IHH + PROM1 + VEGFA | 0,79 |
| GZMH + CXCL13 + GNLY + LAG3 + CCL5 + CXCL9 + PF4 + IL17A + TSLP + REN + IHH + PROM1 | 0,79 |
| GZMH + IFNG + CXCL13 + GNLY + LAG3 + CCL5 + PF4 + IL17A + REN + IHH + PROM1 + VEGFA | 0,79 |
| GZMH + IFNG + CXCL13 + GNLY + LAG3 + ITGAE + CCL5 + CXCL9 + IL17A + REN + IHH + PROM1 | 0,79 |
| GZMH + IFNG + CXCL13 + GNLY + LAG3 + CCL5 + PF4 + IL17A + TSLP + REN + IHH + PROM1 | 0,79 |
| GZMH + IFNG + CXCL13 + GNLY + CCL5 + CXCL9 + PF4 + IL17A + REN + IHH + PROM1 + VEGFA | 0,79 |
| GZMH + CXCL13 + GNLY + LAG3 + CCL5 + PF4 + IL17A + TSLP + REN + IHH + PROM1 + VEGFA | 0,79 |
| GZMH + IFNG + GNLY + LAG3 + ITGAE + PF4 + IL17A + TSLP + REN + IHH + PROM1 + VEGFA | 0,79 |
| GZMH + CXCL13 + GNLY + LAG3 + CCL5 + CXCL9 + PF4 + IL17A + TSLP + REN + IHH + VEGFA | 0,79 |
| IFNG + CXCL13 + GNLY + LAG3 + CCL5 + CXCL9 + PF4 + IL17A + TSLP + REN + IHH + VEGFA | 0,79 |
| GZMH + IFNG + CXCL13 + GNLY + CCL5 + CXCL9 + PF4 + IL17A + TSLP + REN + IHH + PROM1 | 0,79 |
| GZMH + IFNG + CXCL13 + GNLY + CCL5 + PF4 + IL17A + TSLP + REN + IHH + PROM1 + VEGFA | 0,79 |
| GZMH + IFNG + CXCL13 + GNLY + LAG3 + CCL5 + CXCL9 + PF4 + IL17A + REN + PROM1 + VEGFA | 0,79 |
| GZMH + IFNG + CXCL13 + GNLY + LAG3 + CCL5 + CXCL9 + PF4 + IL17A + TSLP + REN + PROM1 | 0,79 |
| GZMH + IFNG + CXCL13 + GNLY + ITGAE + CCL5 + IL17A + TSLP + REN + IHH + PROM1 + VEGFA | 0,79 |
| GZMH + CXCL13 + GNLY + CCL5 + CXCL9 + PF4 + IL17A + TSLP + REN + IHH + PROM1 + VEGFA | 0,79 |
| GZMH + IFNG + CXCL13 + GNLY + ITGAE + CCL5 + CXCL9 + IL17A + TSLP + REN + PROM1 + VEGFA | 0,79 |
| GZMH + IFNG + CXCL13 + GNLY + LAG3 + ITGAE + CCL5 + IL17A + TSLP + REN + IHH + VEGFA | 0,79 |
| GZMH + IFNG + CXCL13 + GNLY + ITGAE + CCL5 + CXCL9 + IL17A + REN + IHH + PROM1 + VEGFA | 0,78 |
| GZMH + IFNG + GNLY + LAG3 + ITGAE + CXCL9 + PF4 + IL17A + TSLP + REN + IHH + VEGFA | 0,78 |
| CXCL13 + GNLY + LAG3 + CCL5 + CXCL9 + PF4 + IL17A + TSLP + REN + IHH + PROM1 + VEGFA | 0,78 |
| GZMH + IFNG + CXCL13 + GNLY + LAG3 + ITGAE + CCL5 + CXCL9 + IL17A + TSLP + REN + IHH | 0,78 |
| GZMH + IFNG + GNLY + LAG3 + ITGAE + CXCL9 + PF4 + IL17A + REN + IHH + PROM1 + VEGFA | 0,78 |
| GZMH + IFNG + CXCL13 + GNLY + CCL5 + CXCL9 + PF4 + IL17A + TSLP + REN + PROM1 + VEGFA | 0,78 |
| IFNG + CXCL13 + GNLY + LAG3 + CCL5 + CXCL9 + PF4 + IL17A + REN + IHH + PROM1 + VEGFA | 0,78 |
| IFNG + CXCL13 + GNLY + ITGAE + CCL5 + CXCL9 + IL17A + TSLP + REN + IHH + PROM1 + VEGFA | 0,78 |
| IFNG + CXCL13 + GNLY + LAG3 + ITGAE + CCL5 + IL17A + TSLP + REN + IHH + PROM1 + VEGFA | 0,78 |
| GZMH + CXCL13 + GNLY + LAG3 + CCL5 + CXCL9 + PF4 + IL17A + TSLP + REN + PROM1 + VEGFA | 0,78 |
| IFNG + CXCL13 + GNLY + LAG3 + CCL5 + CXCL9 + PF4 + IL17A + TSLP + REN + IHH + PROM1 | 0,78 |
| GZMH + IFNG + CXCL13 + GNLY + ITGAE + CCL5 + CXCL9 + IL17A + TSLP + REN + IHH + PROM1 | 0,78 |
| IFNG + CXCL13 + GNLY + LAG3 + CCL5 + CXCL9 + PF4 + IL17A + TSLP + REN + PROM1 + VEGFA | 0,78 |
| IFNG + GNLY + LAG3 + ITGAE + CXCL9 + PF4 + IL17A + TSLP + REN + IHH + PROM1 + VEGFA | 0,78 |
| IFNG + CXCL13 + GNLY + LAG3 + ITGAE + CCL5 + CXCL9 + IL17A + TSLP + REN + IHH + PROM1 | 0,78 |
| IFNG + CXCL13 + GNLY + CCL5 + CXCL9 + PF4 + IL17A + TSLP + REN + IHH + PROM1 + VEGFA | 0,78 |
| IFNG + CXCL13 + GNLY + LAG3 + CCL5 + PF4 + IL17A + TSLP + REN + IHH + PROM1 + VEGFA | 0,78 |
| GZMH + IFNG + GNLY + LAG3 + ITGAE + CXCL9 + PF4 + IL17A + TSLP + REN + PROM1 + VEGFA | 0,78 |
| GZMH + IFNG + GNLY + LAG3 + ITGAE + CXCL9 + PF4 + IL17A + TSLP + REN + IHH + PROM1 | 0,78 |
| GZMH + IFNG + GNLY + ITGAE + CXCL9 + PF4 + IL17A + TSLP + REN + IHH + PROM1 + VEGFA | 0,78 |
| GZMH + IFNG + CXCL13 + GNLY + LAG3 + ITGAE + PF4 + IL17A + TSLP + REN + PROM1 + VEGFA | 0,78 |
| GZMH + IFNG + CXCL13 + GNLY + LAG3 + ITGAE + CXCL9 + PF4 + IL17A + REN + PROM1 + VEGFA | 0,78 |
| GZMH + IFNG + CXCL13 + GNLY + LAG3 + ITGAE + PF4 + IL17A + TSLP + REN + IHH + PROM1 | 0,78 |
| GZMH + IFNG + CXCL13 + GNLY + LAG3 + ITGAE + PF4 + IL17A + REN + IHH + PROM1 + VEGFA | 0,78 |
| GZMH + IFNG + GNLY + LAG3 + CXCL9 + PF4 + IL17A + TSLP + REN + IHH + PROM1 + VEGFA | 0,78 |
| GZMH + IFNG + CXCL13 + GNLY + LAG3 + ITGAE + CXCL9 + PF4 + IL17A + TSLP + REN + PROM1 | 0,78 |
| GZMH + IFNG + CXCL13 + GNLY + LAG3 + ITGAE + CXCL9 + PF4 + IL17A + TSLP + REN + VEGFA | 0,78 |
| IFNG + CXCL13 + GNLY + LAG3 + ITGAE + CXCL9 + PF4 + IL17A + TSLP + REN + PROM1 + VEGFA | 0,78 |
| GZMH + IFNG + CXCL13 + GNLY + LAG3 + ITGAE + CXCL9 + PF4 + IL17A + TSLP + REN + IHH | 0,78 |
| GZMH + IFNG + CXCL13 + GNLY + LAG3 + ITGAE + CXCL9 + PF4 + IL17A + REN + IHH + VEGFA | 0,78 |
| GZMH + IFNG + GNLY + LAG3 + ITGAE + CCL5 + PF4 + TSLP + REN + IHH + PROM1 + VEGFA | 0,78 |
| GZMH + IFNG + CXCL13 + GNLY + LAG3 + ITGAE + CXCL9 + PF4 + IL17A + REN + IHH + PROM1 | 0,78 |
| GZMH + IFNG + CXCL13 + GNLY + LAG3 + ITGAE + PF4 + IL17A + TSLP + REN + IHH + VEGFA | 0,78 |
| IFNG + CXCL13 + GNLY + LAG3 + ITGAE + PF4 + IL17A + TSLP + REN + IHH + PROM1 + VEGFA | 0,78 |
| GZMH + IFNG + CXCL13 + GNLY + ITGAE + CXCL9 + PF4 + IL17A + TSLP + REN + PROM1 + VEGFA | 0,78 |
| GZMH + IFNG + CXCL13 + GNLY + ITGAE + CXCL9 + PF4 + IL17A + TSLP + REN + IHH + VEGFA | 0,78 |
| GZMH + IFNG + CXCL13 + GNLY + ITGAE + CXCL9 + PF4 + IL17A + REN + IHH + PROM1 + VEGFA | 0,78 |
| IFNG + CXCL13 + GNLY + LAG3 + ITGAE + CXCL9 + PF4 + IL17A + REN + IHH + PROM1 + VEGFA | 0,78 |
| GZMH + IFNG + CXCL13 + GNLY + ITGAE + PF4 + IL17A + TSLP + REN + IHH + PROM1 + VEGFA | 0,78 |
| GZMH + IFNG + CXCL13 + GNLY + ITGAE + CXCL9 + PF4 + IL17A + TSLP + REN + IHH + PROM1 | 0,78 |
| IFNG + CXCL13 + GNLY + LAG3 + ITGAE + CXCL9 + PF4 + IL17A + TSLP + REN + IHH + PROM1 | 0,78 |
| GZMH + GNLY + LAG3 + ITGAE + CCL5 + CXCL9 + IL17A + TSLP + REN + IHH + PROM1 + VEGFA | 0,78 |
| IFNG + CXCL13 + GNLY + LAG3 + ITGAE + CXCL9 + PF4 + IL17A + TSLP + REN + IHH + VEGFA | 0,78 |
| CXCL13 + GNLY + LAG3 + ITGAE + CCL5 + CXCL9 + IL17A + TSLP + REN + IHH + PROM1 + VEGFA | 0,78 |
| GZMH + GNLY + LAG3 + ITGAE + CXCL9 + PF4 + IL17A + TSLP + REN + IHH + PROM1 + VEGFA | 0,78 |
| GZMH + CXCL13 + GNLY + LAG3 + ITGAE + CCL5 + CXCL9 + IL17A + TSLP + REN + PROM1 + VEGFA | 0,78 |
| GZMH + CXCL13 + GNLY + LAG3 + ITGAE + CCL5 + CXCL9 + IL17A + REN + IHH + PROM1 + VEGFA | 0,78 |
| GZMH + IFNG + CXCL13 + GNLY + CXCL9 + PF4 + IL17A + TSLP + REN + IHH + PROM1 + VEGFA | 0,78 |
| GZMH + IFNG + CXCL13 + GNLY + LAG3 + CXCL9 + PF4 + IL17A + REN + IHH + PROM1 + VEGFA | 0,78 |
| IFNG + CXCL13 + GNLY + ITGAE + CXCL9 + PF4 + IL17A + TSLP + REN + IHH + PROM1 + VEGFA | 0,78 |
| GZMH + CXCL13 + GNLY + LAG3 + ITGAE + CXCL9 + PF4 + IL17A + TSLP + REN + IHH + VEGFA | 0,78 |
| GZMH + IFNG + GNLY + LAG3 + CCL5 + CXCL9 + IL17A + TSLP + REN + IHH + PROM1 + VEGFA | 0,78 |
| GZMH + IFNG + CXCL13 + GNLY + LAG3 + CXCL9 + PF4 + IL17A + TSLP + REN + IHH + PROM1 | 0,78 |
| GZMH + CXCL13 + GNLY + LAG3 + ITGAE + CXCL9 + PF4 + IL17A + TSLP + REN + PROM1 + VEGFA | 0,78 |
| GZMH + IFNG + CXCL13 + GNLY + LAG3 + CXCL9 + PF4 + IL17A + TSLP + REN + IHH + VEGFA | 0,78 |
| GZMH + IFNG + CXCL13 + GNLY + LAG3 + PF4 + IL17A + TSLP + REN + IHH + PROM1 + VEGFA | 0,78 |
| IFNG + CXCL13 + GNLY + LAG3 + CXCL9 + PF4 + IL17A + TSLP + REN + IHH + PROM1 + VEGFA | 0,78 |
| GZMH + CXCL13 + GNLY + LAG3 + ITGAE + CXCL9 + PF4 + IL17A + TSLP + REN + IHH + PROM1 | 0,78 |
| GZMH + CXCL13 + GNLY + LAG3 + ITGAE + PF4 + IL17A + TSLP + REN + IHH + PROM1 + VEGFA | 0,78 |
| GZMH + CXCL13 + GNLY + LAG3 + ITGAE + CCL5 + CXCL9 + IL17A + TSLP + REN + IHH + VEGFA | 0,78 |
| GZMH + CXCL13 + GNLY + LAG3 + CXCL9 + PF4 + IL17A + TSLP + REN + IHH + PROM1 + VEGFA | 0,78 |
| GZMH + IFNG + CXCL13 + GNLY + LAG3 + CXCL9 + PF4 + IL17A + TSLP + REN + PROM1 + VEGFA | 0,78 |
| GZMH + CXCL13 + GNLY + LAG3 + ITGAE + CXCL9 + PF4 + IL17A + REN + IHH + PROM1 + VEGFA | 0,78 |
| GZMH + CXCL13 + GNLY + LAG3 + ITGAE + CCL5 + IL17A + TSLP + REN + IHH + PROM1 + VEGFA | 0,78 |
| GZMH + CXCL13 + GNLY + ITGAE + CCL5 + CXCL9 + IL17A + TSLP + REN + IHH + PROM1 + VEGFA | 0,78 |
| GZMH + CXCL13 + GNLY + LAG3 + ITGAE + CCL5 + CXCL9 + IL17A + TSLP + REN + IHH + PROM1 | 0,78 |
| GZMH + CXCL13 + GNLY + ITGAE + CXCL9 + PF4 + IL17A + TSLP + REN + IHH + PROM1 + VEGFA | 0,78 |
| CXCL13 + GNLY + LAG3 + ITGAE + CXCL9 + PF4 + IL17A + TSLP + REN + IHH + PROM1 + VEGFA | 0,78 |
| GZMH + IFNG + CXCL13 + GNLY + LAG3 + CCL5 + CXCL9 + IL17A + TSLP + REN + IHH + VEGFA | 0,78 |
| GZMH + IFNG + GNLY + LAG3 + ITGAE + CCL5 + CXCL9 + PF4 + TSLP + REN + IHH + PROM1 | 0,78 |
| GZMH + IFNG + CXCL13 + GNLY + LAG3 + CCL5 + CXCL9 + IL17A + TSLP + REN + PROM1 + VEGFA | 0,77 |
| GZMH + IFNG + CXCL13 + GNLY + LAG3 + ITGAE + CCL5 + CXCL9 + PF4 + IL17A + IHH + PROM1 | 0,77 |
| GZMH + IFNG + CXCL13 + GNLY + LAG3 + CCL5 + CXCL9 + IL17A + REN + IHH + PROM1 + VEGFA | 0,77 |
| GZMH + IFNG + GNLY + ITGAE + CCL5 + CXCL9 + PF4 + TSLP + REN + IHH + PROM1 + VEGFA | 0,77 |
| GZMH + IFNG + CXCL13 + GNLY + LAG3 + ITGAE + CCL5 + PF4 + IL17A + TSLP + IHH + PROM1 | 0,77 |
| GZMH + IFNG + CXCL13 + GNLY + LAG3 + ITGAE + CCL5 + PF4 + IL17A + IHH + PROM1 + VEGFA | 0,77 |
| GZMH + IFNG + CXCL13 + GNLY + LAG3 + CCL5 + CXCL9 + IL17A + TSLP + REN + IHH + PROM1 | 0,77 |
| GZMH + IFNG + CXCL13 + GNLY + ITGAE + CCL5 + CXCL9 + PF4 + IL17A + IHH + PROM1 + VEGFA | 0,77 |
| GZMH + IFNG + CXCL13 + GNLY + ITGAE + CCL5 + CXCL9 + PF4 + IL17A + TSLP + IHH + PROM1 | 0,77 |
| GZMH + IFNG + GNLY + LAG3 + ITGAE + CCL5 + CXCL9 + PF4 + TSLP + REN + PROM1 + VEGFA | 0,77 |
| GZMH + IFNG + GNLY + LAG3 + ITGAE + CCL5 + CXCL9 + PF4 + REN + IHH + PROM1 + VEGFA | 0,77 |
| GZMH + IFNG + CXCL13 + GNLY + LAG3 + ITGAE + CCL5 + PF4 + IL17A + TSLP + PROM1 + VEGFA | 0,77 |
| GZMH + IFNG + CXCL13 + GNLY + ITGAE + CCL5 + PF4 + IL17A + TSLP + IHH + PROM1 + VEGFA | 0,77 |
| GZMH + GNLY + LAG3 + ITGAE + CCL5 + CXCL9 + PF4 + TSLP + REN + IHH + PROM1 + VEGFA | 0,77 |
| GZMH + IFNG + GNLY + LAG3 + CCL5 + CXCL9 + PF4 + TSLP + REN + IHH + PROM1 + VEGFA | 0,77 |
| IFNG + CXCL13 + GNLY + LAG3 + ITGAE + CCL5 + CXCL9 + PF4 + IL17A + IHH + PROM1 + VEGFA | 0,77 |
| IFNG + CXCL13 + GNLY + LAG3 + CCL5 + CXCL9 + IL17A + TSLP + REN + IHH + PROM1 + VEGFA | 0,77 |
| GZMH + IFNG + CXCL13 + GNLY + LAG3 + ITGAE + CCL5 + CXCL9 + PF4 + IL17A + TSLP + PROM1 | 0,77 |
| GZMH + IFNG + CXCL13 + GNLY + LAG3 + ITGAE + CCL5 + PF4 + TSLP + REN + PROM1 + VEGFA | 0,77 |
| GZMH + IFNG + CXCL13 + GNLY + LAG3 + ITGAE + CCL5 + CXCL9 + PF4 + IL17A + PRO1 + VEGFA | 0,77 |
| IFNG + CXCL13 + GNLY + LAG3 + ITGAE + CCL5 + PF4 + IL17A + TSLP + IHH + PROM1 + VEGFA | 0,77 |
| GZMH + CXCL13 + GNLY + LAG3 + ITGAE + CCL5 + CXCL9 + PF4 + IL17A + TSLP + IHH + PROM1 | 0,77 |
| GZMH + CXCL13 + GNLY + LAG3 + CCL5 + CXCL9 + IL17A + TSLP + REN + IHH + PROM1 + VEGFA | 0,77 |
| GZMH + IFNG + CXCL13 + GNLY + LAG3 + CCL5 + IL17A + TSLP + REN + IHH + PROM1 + VEGFA | 0,77 |
| GZMH + IFNG + GNLY + LAG3 + ITGAE + CCL5 + CXCL9 + PF4 + IL17A + IHH + PROM1 + VEGFA | 0,77 |
| GZMH + IFNG + CXCL13 + GNLY + LAG3 + ITGAE + CCL5 + PF4 + TSLP + REN + IHH + PROM1 | 0,77 |
| GZMH + IFNG + CXCL13 + GNLY + LAG3 + ITGAE + CCL5 + CXCL9 + PF4 + TSLP + REN + PROM1 | 0,77 |
| GZMH + IFNG + GNLY + LAG3 + ITGAE + CXCL9 + IL17A + TSLP + REN + IHH + PROM1 + VEGFA | 0,77 |
| IFNG + GNLY + LAG3 + ITGAE + CCL5 + CXCL9 + PF4 + TSLP + REN + IHH + PROM1 + VEGFA | 0,77 |
| IFNG + CXCL13 + GNLY + LAG3 + ITGAE + CCL5 + CXCL9 + PF4 + IL17A + TSLP + IHH + PROM1 | 0,77 |
| IFNG + CXCL13 + GNLY + ITGAE + CCL5 + CXCL9 + PF4 + IL17A + TSLP + IHH + PROM1 + VEGFA | 0,77 |
| GZMH + IFNG + CXCL13 + GNLY + ITGAE + CCL5 + CXCL9 + PF4 + IL17A + TSLP + PROM1 + VEGFA | 0,77 |
| GZMH + CXCL13 + GNLY + ITGAE + CCL5 + CXCL9 + PF4 + IL17A + TSLP + IHH + PROM1 + VEGFA | 0,77 |
| GZMH + CXCL13 + GNLY + LAG3 + ITGAE + CCL5 + CXCL9 + PF4 + IL17A + IHH + PROM1 + VEGFA | 0,77 |
| GZMH + IFNG + CXCL13 + GNLY + ITGAE + CCL5 + PF4 + TSLP + REN + IHH + PROM1 + VEGFA | 0,77 |
| GZMH + IFNG + GNLY + LAG3 + ITGAE + CCL5 + CXCL9 + PF4 + IL17A + TSLP + IHH + VEGFA | 0,77 |
| GZMH + CXCL13 + GNLY + LAG3 + ITGAE + CCL5 + PF4 + IL17A + TSLP + IHH + PROM1 + VEGFA | 0,77 |
| GZMH + IFNG + CXCL13 + GNLY + ITGAE + CCL5 + CXCL9 + PF4 + TSLP + REN + IHH + PROM1 | 0,77 |
| GZMH + IFNG + CXCL13 + GNLY + LAG3 + ITGAE + CCL5 + CXCL9 + PF4 + IL17A + IHH + VEGFA | 0,77 |
| GZMH + IFNG + GNLY + LAG3 + ITGAE + CCL5 + CXCL9 + PF4 + TSLP + REN + IHH + VEGFA | 0,77 |
| CXCL13 + GNLY + LAG3 + ITGAE + CCL5 + CXCL9 + PF4 + IL17A + TSLP + IHH + PROM1 + VEGFA | 0,77 |
| GZMH + IFNG + CXCL13 + GNLY + ITGAE + CCL5 + CXCL9 + PF4 + TSLP + REN + PROM1 + VEGFA | 0,77 |
| GZMH + IFNG + GNLY + ITGAE + CCL5 + CXCL9 + PF4 + IL17A + TSLP + IHH + PROM1 + VEGFA | 0,77 |
| GZMH + IFNG + CXCL13 + GNLY + LAG3 + ITGAE + CXCL9 + IL17A + TSLP + REN + IHH + VEGFA | 0,77 |
| GZMH + IFNG + CXCL13 + GNLY + LAG3 + ITGAE + CCL5 + CXCL9 + PF4 + IL17A + TSLP + IHH | 0,77 |
| GZMH + CXCL13 + GNLY + LAG3 + ITGAE + CCL5 + CXCL9 + PF4 + IL17A + TSLP + PROM1 + VEGFA | 0,77 |
| GZMH + IFNG + CXCL13 + GNLY + LAG3 + ITGAE + CCL5 + CXCL9 + PF4 + REN + IHH + PROM1 | 0,77 |
| IFNG + CXCL13 + GNLY + LAG3 + ITGAE + CCL5 + CXCL9 + PF4 + TSLP + REN + PROM1 + VEGFA | 0,77 |
| GZMH + IFNG + CXCL13 + GNLY + LAG3 + ITGAE + CCL5 + PF4 + IL17A + TSLP + IHH + VEGFA | 0,77 |
| GZMH + IFNG + CXCL13 + GNLY + LAG3 + ITGAE + CXCL9 + IL17A + TSLP + REN + PROM1 + VEGFA | 0,77 |
| GZMH + IFNG + CXCL13 + GNLY + ITGAE + CXCL9 + IL17A + TSLP + REN + IHH + PROM1 + VEGFA | 0,77 |
| IFNG + CXCL13 + GNLY + LAG3 + ITGAE + CCL5 + CXCL9 + PF4 + TSLP + REN + IHH + PROM1 | 0,77 |
| GZMH + IFNG + CXCL13 + GNLY + ITGAE + CCL5 + CXCL9 + PF4 + IL17A + TSLP + IHH + VEGFA | 0,77 |
| IFNG + CXCL13 + GNLY + LAG3 + ITGAE + CCL5 + PF4 + TSLP + REN + IHH + PROM1 + VEGFA | 0,77 |
| GZMH + IFNG + CXCL13 + GNLY + LAG3 + ITGAE + CCL5 + CXCL9 + PF4 + REN + PROM1 + VEGFA | 0,77 |
| IFNG + CXCL13 + GNLY + LAG3 + ITGAE + CCL5 + CXCL9 + PF4 + IL17A + TSLP + IHH + VEGFA | 0,77 |
| IFNG + CXCL13 + GNLY + ITGAE + CCL5 + CXCL9 + PF4 + TSLP + REN + IHH + PROM1 + VEGFA | 0,77 |
| GZMH + IFNG + CXCL13 + GNLY + LAG3 + ITGAE + CCL5 + CXCL9 + IL17A + IHH + PROM1 + VEGFA | 0,77 |
| GZMH + IFNG + CXCL13 + GNLY + LAG3 + ITGAE + CXCL9 + IL17A + REN + IHH + PROM1 + VEGFA | 0,77 |
| GZMH + IFNG + CXCL13 + GNLY + LAG3 + ITGAE + CXCL9 + IL17A + TSLP + REN + IHH + PROM1 | 0,77 |
| IFNG + GNLY + LAG3 + ITGAE + CCL5 + CXCL9 + PF4 + IL17A + TSLP + IHH + PROM1 + VEGFA | 0,77 |
| GZMH + CXCL13 + GNLY + LAG3 + ITGAE + CCL5 + CXCL9 + PF4 + TSLP + REN + IHH + PROM1 | 0,77 |
| GZMH + IFNG + CXCL13 + GNLY + LAG3 + ITGAE + CCL5 + CXCL9 + IL17A + TSLP + PROM1 + VEGFA | 0,77 |
| GZMH + IFNG + CXCL13 + GNLY + LAG3 + ITGAE + IL17A + TSLP + REN + IHH + PROM1 + VEGFA | 0,77 |
| GZMH + CXCL13 + GNLY + LAG3 + ITGAE + CCL5 + CXCL9 + PF4 + TSLP + REN + PROM1 + VEGFA | 0,77 |
| GZMH + IFNG + CXCL13 + GNLY + ITGAE + CCL5 + CXCL9 + PF4 + REN + IHH + PROM1 + VEGFA | 0,77 |
| GZMH + IFNG + CXCL13 + GNLY + LAG3 + ITGAE + CCL5 + PF4 + REN + IHH + PROM1 + VEGFA | 0,77 |
| GZMH + IFNG + CXCL13 + GNLY + LAG3 + ITGAE + CCL5 + CXCL9 + PF4 + TSLP + REN + VEGFA | 0,77 |
| GZMH + IFNG + CXCL13 + GNLY + LAG3 + ITGAE + CCL5 + CXCL9 + PF4 + IL17A + TSLP + VEGFA | 0,77 |
| GZMH + IFNG + CXCL13 + GNLY + CCL5 + CXCL9 + IL17A + TSLP + REN + IHH + PROM1 + VEGFA | 0,77 |
| CXCL13 + GNLY + LAG3 + ITGAE + CCL5 + CXCL9 + PF4 + TSLP + REN + IHH + PROM1 + VEGFA | 0,77 |
| GZMH + CXCL13 + GNLY + LAG3 + ITGAE + CCL5 + PF4 + TSLP + REN + IHH + PROM1 + VEGFA | 0,77 |
| IFNG + CXCL13 + GNLY + LAG3 + ITGAE + CCL5 + CXCL9 + PF4 + TSLP + REN + IHH + VEGFA | 0,77 |
| GZMH + IFNG + CXCL13 + GNLY + LAG3 + ITGAE + CCL5 + CXCL9 + PF4 + TSLP + REN + IHH | 0,77 |
| GZMH + CXCL13 + GNLY + ITGAE + CCL5 + CXCL9 + PF4 + TSLP + REN + IHH + PROM1 + VEGFA | 0,77 |
| GZMH + CXCL13 + GNLY + LAG3 + ITGAE + CCL5 + CXCL9 + PF4 + IL17A + TSLP + IHH + VEGFA | 0,77 |
| GZMH + IFNG + CXCL13 + GNLY + LAG3 + CCL5 + CXCL9 + PF4 + TSLP + REN + PROM1 + VEGFA | 0,77 |
| GZMH + IFNG + CXCL13 + GNLY + ITGAE + CCL5 + CXCL9 + PF4 + TSLP + REN + IHH + VEGFA | 0,77 |
| GZMH + IFNG + GNLY + LAG3 + ITGAE + CCL5 + CXCL9 + IL17A + TSLP + IHH + PROM1 + VEGFA | 0,77 |
| GZMH + IFNG + GNLY + LAG3 + ITGAE + CXCL9 + PF4 + TSLP + REN + IHH + PROM 1 + VEGFA | 0,77 |
| IFNG + CXCL13 + GNLY + LAG3 + ITGAE + CXCL9 + IL17A + TSLP + REN + IHH + PROM1 + VEGFA | 0,77 |
| GZMH + IFNG + GNLY + LAG3 + ITGAE + CCL5 + CXCL9 + PF4 + IL17A + TSLP + IHH + PROM1 | 0,77 |
| IFNG + CXCL13 + GNLY + LAG3 + ITGAE + CCL5 + CXCL9 + PF4 + IL17A + TSLP + PROM1 + VEGFA | 0,77 |
| GZMH + IFNG + CXCL13 + GNLY + LAG3 + ITGAE + CCL5 + CXCL9 + IL17A + TSLP + IHH + PROM1 | 0,77 |
| GZMH + IFNG + CXCL13 + GNLY + LAG3 + ITGAE + CXCL9 + PF4 + TSLP + REN + PROM1 + VEGFA | 0,77 |
| IFNG + CXCL13 + GNLY + LAG3 + ITGAE + CCL5 + CXCL9 + PF4 + REN + IHH + PROM1 + VEGFA | 0,77 |
| GZMH + IFNG + CXCL13 + GNLY + LAG3 + CCL5 + CXCL9 + PF4 + TSLP + REN + IHH + PROM1 | 0,77 |
| GZMH + IFNG + CXCL13 + GNLY + ITGAE + CCL5 + CXCL9 + IL17A + TSLP + IHH + PROM1 + VEGFA | 0,77 |
| GZMH + IFNG + GNLY + LAG3 + ITGAE + CCL5 + PF4 + IL17A + TSLP + IHH + PROM1 + VEGFA | 0,77 |
| GZMH + IFNG + CXCL13 + GNLY + LAG3 + CCL5 + CXCL9 + PF4 + REN + IHH + PROM1 + VEGFA | 0,77 |
| GZMH + IFNG + CXCL13 + GNLY + LAG3 + ITGAE + CCL5 + CXCL9 + PF4 + REN + IHH + VEGFA | 0,77 |
| GZMH + CXCL13 + GNLY + LAG3 + CCL5 + CXCL9 + PF4 + TSLP + REN + IHH + PROM1 + VEGFA | 0,77 |
| GZMH + CXCL13 + GNLY + LAG3 + ITGAE + CCL5 + CXCL9 + PF4 + REN + IHH + PROM1 + VEGFA | 0,76 |
| GZMH + IFNG + CXCL13 + GNLY + LAG3 + ITGAE + CCL5 + CXCL9 + IL17A + TSLP + IHH + VEGFA | 0,76 |
| GZMH + IFNG + CXCL13 + GNLY + LAG3 + CCL5 + PF4 + TSLP + REN + IHH + PROM1 + VEGFA | 0,76 |
| GZMH + IFNG + CXCL13 + GNLY + LAG3 + ITGAE + CCL5 + IL17A + TSLP + IHH + PROM1 + VEGFA | 0,76 |
| GZMH + IFNG + CXCL13 + GNLY + ITGAE + CXCL9 + PF4 + TSLP + REN + IHH + PROM1 + VEGFA | 0,76 |
| GZMH + IFNG + CXCL13 + GNLY + LAG3 + ITGAE + CCL5 + PF4 + TSLP + REN + IHH + VEGFA | 0,76 |
| IFNG + CXCL13 + GNLY + LAG3 + ITGAE + CCL5 + CXCL9 + IL17A + TSLP + IHH + PROM1 + VEGFA | 0,76 |
| GZMH + IFNG + CXCL13 + GNLY + LAG3 + CCL5 + CXCL9 + PF4 + IL17A + IHH + PROM1 + VEGFA | 0,76 |
| GZMH + IFNG + CXCL13 + GNLY + LAG3 + ITGAE + CXCL9 + PF4 + TSLP + REN + IHH + PROM1 | 0,76 |
| GZMH + IFNG + CXCL13 + GNLY + LAG3 + CCL5 + PF4 + IL17A + TSLP + IHH + PROM1 + VEGFA | 0,76 |
| GZMH + IFNG + CXCL13 + GNLY + LAG3 + CXCL9 + IL17A + TSLP + REN + IHH + PROM1 + VEGFA | 0,76 |
| GZMH + IFNG + CXCL13 + GNLY + LAG3 + CCL5 + CXCL9 + PF4 + IL17A + TSLP + IHH + VEGFA | 0,76 |
| IFNG + CXCL13 + GNLY + LAG3 + CCL5 + CXCL9 + PF4 + TSLP + REN + IHH + PROM1 + VEGFA | 0,76 |
| GZMH + IFNG + CXCL13 + GNLY + CCL5 + CXCL9 + PF4 + TSLP + REN + IHH + PROM1 + VEGFA | 0,76 |
| GZMH + IFNG + CXCL13 + GNLY + LAG3 + ITGAE + CXCL9 + PF4 + TSLP + REN + IHH + VEGFA | 0,76 |
| GZMH + IFNG + GNLY + LAG3 + ITGAE + CCL5 + CXCL9 + PF4 + IL17A + TSLP + PROM1 + VEGFA | 0,76 |
| IFNG + CXCL13 + GNLY + LAG3 + ITGAE + CXCL9 + PF4 + TSLP + REN + IHH + PROM1 + VEGFA | 0,76 |
| GZMH + IFNG + CXCL13 + GNLY + LAG3 + CCL5 + CXCL9 + PF4 + TSLP + REN + IHH + VEGFA | 0,76 |
| GZMH + IFNG + CXCL13 + GNLY + LAG3 + CCL5 + CXCL9 + PF4 + IL17A + TSLP + PROM1 + VEGFA | 0,76 |
| GZMH + CXCL13 + GNLY + LAG3 + CCL5 + CXCL9 + PF4 + IL17A + TSLP + IHH + PROM1 + VEGFA | 0,76 |
| GZMH + IFNG + CXCL13 + GNLY + LAG3 + CCL5 + CXCL9 + PF4 + IL17A + TSLP + IHH + PROM1 | 0,76 |
| GZMH + IFNG + CXCL13 + GNLY + LAG3 + ITGAE + PF4 + TSLP + REN + IHH + PROM1 + VEGFA | 0,76 |
| GZMH + IFNG + CXCL13 + GNLY + CCL5 + CXCL9 + PF4 + IL17A + TSLP + IHH + PROM1 + VEGFA | 0,76 |
| GZMH + CXCL13 + GNLY + LAG3 + ITGAE + CCL5 + CXCL9 + PF4 + TSLP + REN + IHH + VEGFA | 0,76 |
| IFNG + CXCL13 + GNLY + LAG3 + CCL5 + CXCL9 + PF4 + IL17A + TSLP + IHH + PROM1 + VEGFA | 0,76 |
| GZMH + IFNG + CXCL13 + GNLY + LAG3 + ITGAE + CXCL9 + PF4 + REN + IHH + PROM1 + VEGFA | 0,76 |
| GZMH + CXCL13 + GNLY + LAG3 + ITGAE + CXCL9 + PF4 + TSLP + REN + IHH + PROM1 + VEGFA | 0,76 |
| GZMH + IFNG + CXCL13 + GNLY + LAG3 + CXCL9 + PF4 + TSLP + REN + IHH + PROM1 + VEGFA | 0,76 |
| GZMH + CXCL13 + GNLY + LAG3 + ITGAE + CCL5 + CXCL9 + IL17A + TSLP + IHH + PROM1 + VEGFA | 0,76 |
| GZMH + CXCL13 + GNLY + LAG3 + ITGAE + CXCL9 + IL17A + TSLP + REN + IHH + PROM1 + VEGFA | 0,76 |
| GZMH + IFNG + CXCL13 + GNLY + LAG3 + ITGAE + CCL5 + CXCL9 + TSLP + REN + PROM1 + VEGFA | 0,76 |
| GZMH + IFNG + GNLY + LAG3 + CCL5 + CXCL9 + PF4 + IL17A + TSLP + IHH + PROM1 + VEGFA | 0,76 |
| GZMH + IFNG + CXCL13 + GNLY + LAG3 + ITGAE + CXCL9 + PF4 + IL17A + IHH + PROM1 + VEGFA | 0,76 |
| IFNG + CXCL13 + GNLY + LAG3 + ITGAE + CCL5 + CXCL9 + TSLP + REN + IHH + PROM1 + VEGFA | 0,76 |
| GZMH + IFNG + CXCL13 + GNLY + LAG3 + ITGAE + CCL5 + CXCL9 + TSLP + REN + IHH + PROM1 | 0,76 |
| GZMH + IFNG + CXCL13 + GNLY + LAG3 + ITGAE + PF4 + IL17A + TSLP + IHH + PROM1 + VEGFA | 0,76 |
| GZMH + IFNG + CXCL13 + GNLY + LAG3 + ITGAE + CXCL9 + PF4 + IL17A + TSLP + IHH + VEGFA | 0,76 |
| GZMH + GNLY + LAG3 + ITGAE + CCL5 + CXCL9 + PF4 + IL17A + TSLP + IHH + PROM1 + VEGFA | 0,76 |
| GZMH + IFNG + CXCL13 + GNLY + LAG3 + ITGAE + CXCL9 + PF4 + IL17A + TSLP + PROM1 + VEGFA | 0,76 |
| IFNG + CXCL13 + GNLY + LAG3 + ITGAE + CXCL9 + PF4 + IL17A + TSLP + IHH + PROM1 + VEGFA | 0,76 |
| GZMH + IFNG + CXCL13 + GNLY + LAG3 + ITGAE + CXCL9 + PF4 + IL17A + TSLP + IHH + PROM1 | 0,76 |
| GZMH + IFNG + GNLY + LAG3 + ITGAE + CXCL9 + PF4 + IL17A + TSLP + IHH + PROM1 + VEGFA | 0,76 |
| GZMH + IFNG + CXCL13 + GNLY + LAG3 + CCL5 + CXCL9 + IL17A + TSLP + IHH + PROM1 + VEGFA | 0,76 |
| GZMH + IFNG + CXCL13 + GNLY + LAG3 + ITGAE + CCL5 + CXCL9 + TSLP + REN + IHH + VEGFA | 0,76 |
| GZMH + IFNG + GNLY + LAG3 + ITGAE + CCL5 + CXCL9 + TSLP + REN + IHH + PROM1 + VEGFA | 0,76 |
| GZMH + IFNG + CXCL13 + GNLY + LAG3 + ITGAE + CCL5 + TSLP + REN + IHH + PROM1 + VEGFA | 0,75 |
| GZMH + CXCL13 + GNLY + LAG3 + ITGAE + CXCL9 + PF4 + IL17A + TSLP + IHH + PROM1 + VEGFA | 0,75 |
| GZMH + IFNG + CXCL13 + GNLY + LAG3 + CXCL9 + PF4 + IL17A + TSLP + IHH + PROM1 + VEGFA | 0,75 |
| GZMH + IFNG + CXCL13 + GNLY + ITGAE + CXCL9 + PF4 + IL17A + TSLP + IHH + PROM1 + VEGFA | 0,75 |
| GZMH + IFNG + CXCL13 + GNLY + ITGAE + CCL5 + CXCL9 + TSLP + REN + IHH + PROM1 + VEGFA | 0,75 |
| GZMH + IFNG + GNLY + LAG3 + ITGAE + CCL5 + CXCL9 + PF4 + TSLP + IHH + PROM1 + VEGFA | 0,75 |
| GZMH + IFNG + CXCL13 + GNLY + LAG3 + ITGAE + CCL5 + CXCL9 + REN + IHH + PROM1 + VEGFA | 0,75 |
| GZMH + IFNG + LAG3 + CCL5 + CXCL9 + PF4 + IL17A + TSLP + REN + IHH + PROM1 + VEGFA | 0,75 |
| GZMH + LAG3 + ITGAE + CCL5 + CXCL9 + PF4 + IL17A + TSLP + REN + IHH + PROM1 + VEGFA | 0,75 |
| IFNG + CXCL13 + LAG3 + CCL5 + CXCL9 + PF4 + IL17A + TSLP + REN + IHH + PROM1 + VEGFA | 0,75 |
| GZMH + CXCL13 + LAG3 + CCL5 + CXCL9 + PF4 + IL17A + TSLP + REN + IHH + PROM1 + VEGFA | 0,75 |
| CXCL13 + LAG3 + ITGAE + CCL5 + CXCL9 + PF4 + IL17A + TSLP + REN + IHH + PROM1 + VEGFA | 0,75 |
| GZMH + IFNG + CXCL13 + GNLY + LAG3 + CCL5 + CXCL9 + TSLP + REN + IHH + PROM1 + VEGFA | 0,75 |
| GZMH + IFNG + CXCL13 + GNLY + LAG3 + ITGAE + CXCL9 + IL17A + TSLP + IHH + PROM1 + VEGFA | 0,75 |
| IFNG + LAG3 + ITGAE + CCL5 + CXCL9 + PF4 + IL17A + TSLP + REN + IHH + PROM1 + VEGFA | 0,75 |
| IFNG + CXCL13 + LAG3 + ITGAE + CCL5 + CXCL9 + PF4 + IL17A + REN + IHH + PROM1 + VEGFA | 0,75 |
| GZMH + IFNG + CXCL13 + GNLY + LAG3 + ITGAE + CXCL9 + TSLP + REN + IHH + PROM1 + VEGFA | 0,75 |
| GZMH + IFNG + LAG3 + ITGAE + CXCL9 + PF4 + IL17A + TSLP + REN + IHH + PROM1 + VEGFA | 0,75 |
| GZMH + IFNG + CXCL13 + LAG3 + CCL5 + CXCL9 + PF4 + IL17A + TSLP + REN + PROM1 + VEGFA | 0,75 |
| GZMH + IFNG + CXCL13 + LAG3 + CCL5 + CXCL9 + PF4 + IL17A + REN + IHH + PROM1 + VEGFA | 0,75 |
| IFNG + CXCL13 + GNLY + LAG3 + ITGAE + CCL5 + CXCL9 + PF4 + TSLP + IHH + PROM1 + VEGFA | 0,75 |
| GZMH + IFNG + CXCL13 + GNLY + LAG3 + ITGAE + CCL5 + CXCL9 + PF4 + TSLP + IHH + PROM1 | 0,75 |
| GZMH + IFNG + CXCL13 + GNLY + ITGAE + CCL5 + CXCL9 + PF4 + TSLP + IHH + PROM1 + VEGFA | 0,75 |
| GZMH + CXCL13 + LAG3 + ITGAE + CXCL9 + PF4 + IL17A + TSLP + REN + IHH + PROM1 + VEGFA | 0,75 |
| GZMH + IFNG + CXCL13 + LAG3 + CCL5 + CXCL9 + PF4 + IL17A + TSLP + REN + IHH + VEGFA | 0,74 |
| GZMH + CXCL13 + GNLY + LAG3 + ITGAE + CCL5 + CXCL9 + PF4 + TSLP + IHH + PROM1 + VEGFA | 0,74 |
| IFNG + CXCL13 + LAG3 + ITGAE + CCL5 + CXCL9 + PF4 + IL17A + TSLP + REN + IHH + VEGFA | 0,74 |
| GZMH + IFNG + LAG3 + ITGAE + CCL5 + CXCL9 + PF4 + IL17A + REN + IHH + PROM1 + VEGFA | 0,74 |
| GZMH + CXCL13 + LAG3 + ITGAE + CCL5 + CXCL9 + PF4 + IL17A + TSLP + REN + IHH + VEGFA | 0,74 |
| GZMH + IFNG + CXCL13 + GNLY + LAG3 + ITGAE + CCL5 + PF4 + TSLP + IHH + PROM1 + VEGFA | 0,74 |
| GZMH + CXCL13 + GNLY + LAG3 + ITGAE + CCL5 + CXCL9 + TSLP + REN + IHH + PROM1 + VEGFA | 0,74 |
| GZMH + IFNG + CXCL13 + GNLY + LAG3 + ITGAE + CCL5 + CXCL9 + PF4 + TSLP + PROM1 + VEGFA | 0,74 |
| GZMH + CXCL13 + LAG3 + ITGAE + CCL5 + CXCL9 + PF4 + IL17A + REN + IHH + PROM1 + VEGFA | 0,74 |
| GZMH + CXCL13 + LAG3 + ITGAE + CCL5 + CXCL9 + PF4 + IL17A + TSLP + REN + PROM1 + VEGFA | 0,74 |
| GZMH + IFNG + CXCL13 + LAG3 + CCL5 + CXCL9 + PF4 + IL17A + TSLP + REN + IHH + PROM1 | 0,74 |
| GZMH + IFNG + CXCL13 + LAG3 + ITGAE + CXCL9 + PF4 + IL17A + TSLP + REN + IHH + VEGFA | 0,74 |
| GZMH + IFNG + CXCL13 + GNLY + LAG3 + ITGAE + CCL5 + CXCL9 + PF4 + TSLP + IHH + VEGFA | 0,74 |
| GZMH + IFNG + CXCL13 + GNLY + LAG3 + ITGAE + CCL5 + CXCL9 + PF4 + IHH + PROM1 + VEGFA | 0,74 |
| GZMH + IFNG + CXCL13 + LAG3 + ITGAE + CXCL9 + PF4 + IL17A + TSLP + REN + PROM1 + VEGFA | 0,74 |
| GZMH + IFNG + CXCL13 + ITGAE + CXCL9 + PF4 + IL17A + TSLP + REN + IHH + PROM1 + VEGFA | 0,74 |
| IFNG + CXCL13 + LAG3 + ITGAE + CCL5 + CXCL9 + PF4 + IL17A + TSLP + REN + PROM1 + VEGFA | 0,74 |
| GZMH + IFNG + CXCL13 + LAG3 + ITGAE + CXCL9 + PF4 + IL17A + REN + IHH + PROM1 + VEGFA | 0,74 |
| GZMH + IFNG + LAG3 + ITGAE + CCL5 + CXCL9 + PF4 + IL17A + TSLP + REN + IHH + VEGFA | 0,74 |
| GZMH + CXCL13 + ITGAE + CCL5 + CXCL9 + PF4 + IL17A + TSLP + REN + IHH + PROM1 + VEGFA | 0,74 |
| GZMH + IFNG + CXCL13 + LAG3 + CXCL9 + PF4 + IL17A + TSLP + REN + IHH + PROM1 + VEGFA | 0,74 |
| IFNG + CXCL13 + ITGAE + CCL5 + CXCL9 + PF4 + IL17A + TSLP + REN + IHH + PROM1 + VEGFA | 0,74 |
| GZMH + CXCL13 + LAG3 + ITGAE + CCL5 + CXCL9 + PF4 + IL17A + TSLP + REN + IHH + PROM1 | 0,74 |
| GZMH + IFNG + CXCL13 + GNLY + LAG3 + CCL5 + CXCL9 + PF4 + TSLP + IHH + PROM1 + VEGFA | 0,74 |
| GZMH + IFNG + LAG3 + ITGAE + CCL5 + CXCL9 + PF4 + IL17A + TSLP + REN + PROM1 + VEGFA | 0,74 |
| GZMH + IFNG + ITGAE + CCL5 + CXCL9 + PF4 + IL17A + TSLP + REN + IHH + PROM1 + VEGFA | 0,74 |
| GZMH + IFNG + CXCL13 + LAG3 + ITGAE + CCL5 + CXCL9 + PF4 + IL17A + REN + IHH + VEGFA | 0,74 |
| GZMH + IFNG + CXCL13 + CCL5 + CXCL9 + PF4 + IL17A + TSLP + REN + IHH + PROM1 + VEGFA | 0,74 |
| GZMH + IFNG + LAG3 + ITGAE + CCL5 + CXCL9 + PF4 + IL17A + TSLP + REN + IHH + PROM1 | 0,74 |
| IFNG + CXCL13 + LAG3 + ITGAE + CCL5 + CXCL9 + PF4 + IL17A + TSLP + REN + IHH + PROM1 | 0,74 |
| IFNG + CXCL13 + LAG3 + ITGAE + CXCL9 + PF4 + IL17A + TSLP + REN + IHH + PROM1 + VEGFA | 0,74 |
| GZMH + IFNG + CXCL13 + LAG3 + ITGAE + CCL5 + CXCL9 + PF4 + IL17A + REN + PROM1 + VEGFA | 0,74 |
| GZMH + IFNG + CXCL13 + LAG3 + ITGAE + CXCL9 + PF4 + IL17A + TSLP + REN + IHH + PROM1 | 0,74 |
| GZMH + IFNG + CXCL13 + LAG3 + CCL5 + PF4 + IL17A + TSLP + REN + IHH + PROM1 + VEGFA | 0,74 |
| GZMH + IFNG + CXCL13 + LAG3 + ITGAE + PF4 + IL17A + TSLP + REN + IHH + PROM1 + VEGFA | 0,74 |
| IFNG + CXCL13 + LAG3 + ITGAE + CCL5 + PF4 + IL17A + TSLP + REN + IHH + PROM1 + VEGFA | 0,74 |
| GZMH + IFNG + LAG3 + ITGAE + CCL5 + PF4 + IL17A + TSLP + REN + IHH + PROM1 + VEGFA | 0,74 |
| GZMH + IFNG + CXCL13 + LAG3 + ITGAE + CCL5 + CXCL9 + PF4 + IL17A + TSLP + REN + IHH | 0,74 |
| GZMH + IFNG + CXCL13 + LAG3 + ITGAE + CCL5 + CXCL9 + PF4 + IL17A + TSLP + REN + VEGFA | 0,74 |
| GZMH + IFNG + CXCL13 + LAG3 + ITGAE + CCL5 + CXCL9 + PF4 + IL17A + TSLP + REN + PROM1 | 0,74 |
| GZMH + IFNG + CXCL13 + GNLY + LAG3 + ITGAE + CXCL9 + PF4 + TSLP + IHH + PROM1 + VEGFA | 0,74 |
| GZMH + IFNG + CXCL13 + LAG3 + ITGAE + CCL5 + CXCL9 + PF4 + IL17A + REN + IHH + PROM1 | 0,74 |
| GZMH + IFNG + CXCL13 + ITGAE + CCL5 + CXCL9 + PF4 + IL17A + TSLP + REN + IHH + VEGFA | 0,74 |
| GZMH + CXCL13 + LAG3 + ITGAE + CCL5 + CXCL9 + IL17A + TSLP + REN + IHH + PROM1 + VEGFA | 0,74 |
| GZMH + IFNG + CXCL13 + GNLY + LAG3 + ITGAE + CCL5 + CXCL9 + TSLP + IHH + PROM1 + VEGFA | 0,74 |
| GZMH + IFNG + CXCL13 + ITGAE + CCL5 + CXCL9 + PF4 + IL17A + TSLP + REN + PROM1 + VEGFA | 0,74 |
| GZMH + IFNG + CXCL13 + ITGAE + CCL5 + CXCL9 + PF4 + IL17A + REN + IHH + PROM1 + VEGFA | 0,74 |
| GZMH + IFNG + CXCL13 + ITGAE + CCL5 + CXCL9 + PF4 + IL17A + TSLP + REN + IHH + PROM1 | 0,74 |
| GZMH + CXCL13 + LAG3 + ITGAE + CCL5 + PF4 + IL17A + TSLP + REN + IHH + PROM1 + VEGFA | 0,74 |
| IFNG + CXCL13 + LAG3 + ITGAE + CCL5 + CXCL9 + IL17A + TSLP + REN + IHH + PROM1 + VEGFA | 0,74 |
| GZMH + IFNG + CXCL13 + LAG3 + ITGAE + CXCL9 + PF4 + TSLP + REN + IHH + PROM1 + VEGFA | 0,74 |
| GZMH + IFNG + CXCL13 + LAG3 + ITGAE + CCL5 + PF4 + IL17A + REN + IHH + PROM1 + VEGFA | 0,73 |
| IFNG + CXCL13 + LAG3 + ITGAE + CCL5 + CXCL9 + PF4 + IL17A + TSLP + IHH + PROM1 + VEGFA | 0,73 |
| GZMH + IFNG + CXCL13 + LAG3 + ITGAE + CCL5 + PF4 + IL17A + TSLP + REN + IHH + VEGFA | 0,73 |
| GZMH + IFNG + CXCL13 + LAG3 + ITGAE + CCL5 + PF4 + IL17A + TSLP + REN + IHH + PROM1 | 0,73 |
| GZMH + IFNG + CXCL13 + LAG3 + ITGAE + CCL5 + CXCL9 + IL17A + REN + IHH + PROM1 + VEGFA | 0,73 |
| GZMH + IFNG + CXCL13 + LAG3 + CCL5 + CXCL9 + PF4 + TSLP + REN + IHH + PROM1 + VEGFA | 0,73 |
| GZMH + CXCL13 + LAG3 + ITGAE + CCL5 + CXCL9 + PF4 + IL17A + TSLP + IHH + PROM1 + VEGFA | 0,73 |
| GZMH + CXCL13 + LAG3 + ITGAE + CCL5 + CXCL9 + PF4 + TSLP + REN + IHH + PROM1 + VEGFA | 0,73 |
| GZMH + IFNG + LAG3 + ITGAE + CCL5 + CXCL9 + IL17A + TSLP + REN + IHH + PROM1 + VEGFA | 0,73 |
| GZMH + IFNG + CXCL13 + LAG3 + ITGAE + CCL5 + PF4 + IL17A + TSLP + REN + PROM1 + VEGFA | 0,73 |
| GZMH + IFNG + CXCL13 + LAG3 + CCL5 + CXCL9 + IL17A + TSLP + REN + IHH + PROM1 + VEGFA | 0,73 |
| IFNG + CXCL13 + LAG3 + ITGAE + CCL5 + CXCL9 + PF4 + TSLP + REN + IHH + PROM1 + VEGFA | 0,73 |
| GZMH + IFNG + CXCL13 + ITGAE + CCL5 + PF4 + IL17A + TSLP + REN + IHH + PROM1 + VEGFA | 0,73 |
| GZMH + IFNG + LAG3 + ITGAE + CCL5 + CXCL9 + PF4 + TSLP + REN + IHH + PROM1 + VEGFA | 0,73 |
| GZMH + IFNG + CXCL13 + LAG3 + ITGAE + CCL5 + CXCL9 + IL17A + TSLP + REN + IHH + VEGFA | 0,73 |
| GZMH + IFNG + CXCL13 + LAG3 + ITGAE + CCL5 + CXCL9 + IL17A + TSLP + REN + PROM1 + VEGFA | 0,73 |
| GZMH + IFNG + CXCL13 + LAG3 + CCL5 + CXCL9 + PF4 + IL17A + TSLP + IHH + PROM1 + VEGFA | 0,73 |
| GZMH + IFNG + CXCL13 + LAG3 + ITGAE + CXCL9 + IL17A + TSLP + REN + IHH + PROM1 + VEGFA | 0,73 |
| GZMH + IFNG + CXCL13 + LAG3 + ITGAE + CCL5 + CXCL9 + PF4 + IL17A + IHH + PROM1 + VEGFA | 0,73 |
| GZMH + IFNG + CXCL13 + ITGAE + CCL5 + CXCL9 + PF4 + IL17A + TSLP + IHH + PROM1 + VEGFA | 0,73 |
| GZMH + IFNG + LAG3 + ITGAE + CCL5 + CXCL9 + PF4 + IL17A + TSLP + IHH + PROM1 + VEGFA | 0,73 |
| GZMH + IFNG + CXCL13 + LAG3 + ITGAE + CCL5 + CXCL9 + PF4 + TSLP + REN + IHH + PROM1 | 0,73 |
| GZMH + IFNG + CXCL13 + LAG3 + ITGAE + CCL5 + CXCL9 + PF4 + IL17A + TSLP + IHH + PROM1 | 0,73 |
| GZMH + IFNG + CXCL13 + LAG3 + ITGAE + CCL5 + CXCL9 + PF4 + IL17A + TSLP + IHH + VEGFA | 0,73 |
| GZMH + IFNG + CXCL13 + LAG3 + ITGAE + CCL5 + CXCL9 + PF4 + IL17A + TSLP + PROM1 + VEGFA | 0,73 |
| GZMH + IFNG + CXCL13 + LAG3 + ITGAE + CCL5 + CXCL9 + IL17A + TSLP + REN + IHH + PROM1 | 0,73 |
| GZMH + IFNG + CXCL13 + LAG3 + ITGAE + CCL5 + CXCL9 + PF4 + TSLP + REN + PROM1 + VEGFA | 0,73 |
| GZMH + IFNG + CXCL13 + LAG3 + ITGAE + CCL5 + CXCL9 + PF4 + REN + IHH + PROM1 + VEGFA | 0,73 |
| GZMH + IFNG + CXCL13 + LAG3 + ITGAE + CCL5 + CXCL9 + PF4 + TSLP + REN + IHH + VEGFA | 0,73 |
| GZMH + IFNG + CXCL13 + ITGAE + CCL5 + CXCL9 + PF4 + TSLP + REN + IHH + PROM 1 + VEGFA | 0,73 |
| GZMH + IFNG + CXCL13 + LAG3 + ITGAE + CXCL9 + PF4 + IL17A + TSLP + IHH + PROM1 + VEGFA | 0,72 |
| GZMH + IFNG + CXCL13 + ITGAE + CCL5 + CXCL9 + IL17A + TSLP + REN + IHH + PROM1 + VEGFA | 0,72 |
| GZMH + IFNG + CXCL13 + LAG3 + ITGAE + CCL5 + PF4 + IL17A + TSLP + IHH + PROM1 + VEGFA | 0,72 |
| GZMH + IFNG + CXCL13 + LAG3 + ITGAE + CCL5 + IL17A + TSLP + REN + IHH + PROM1 + VEGFA | 0,72 |
| GZMH + IFNG + CXCL13 + LAG3 + ITGAE + CCL5 + PF4 + TSLP + REN + IHH + PROM1 + VEGFA | 0,72 |
| GZMH + IFNG + CXCL13 + LAG3 + ITGAE + CCL5 + CXCL9 + IL17A + TSLP + IHH + PROM1 + VEGFA | 0,72 |
| GZMH + IFNG + CXCL13 + LAG3 + ITGAE + CCL5 + CXCL9 + TSLP + REN + IHH + PROM1 + VEGFA | 0,72 |
| GZMH + IFNG + CXCL13 + LAG3 + ITGAE + CCL5 + CXCL9 + PF4 + TSLP + IHH + PROM1 + VEGFA | 0,71 |

| **Table 13: clusters of 13 genes:** | |
|---|---|
| **clusters of 13 genes** | **c_tau level** |
| GZMH + IFNG + GNLY + LAG3 + ITGAE + CCL5 + PF4 + IL17A + TSLP + REN + IHH + PROM1 + VEGFA | 0,8 |
| GZMH + IFNG + GNLY + ITGAE + CCL5 + CXCL9 + PF4 + IL17A + TSLP + REN + IHH + PROM1 + VEGFA | 0,8 |
| GZMH + IFNG + GNLY + LAG3 + ITGAE + CCL5 + CXCL9 + PF4 + IL17A + TSLP + REN + IHH + VEGFA | 0,8 |
| GZMH + IFNG + GNLY + LAG3 + ITGAE + CCL5 + CXCL9 + PF4 + IL17A + REN + IHH + PROM1 + VEGFA | 0,8 |
| GZMH + IFNG + GNLY + LAG3 + ITGAE + CCL5 + CXCL9 + PF4 + IL17A + TSLP + REN + PROM1 + VEGFA | 0,8 |
| GZMH + IFNG + GNLY + LAG3 + ITGAE + CCL5 + CXCL9 + PF4 + IL17A + TSLP + REN + IHH + PROM1 | 0,8 |
| GZMH + IFNG + CXCL13 + GNLY + ITGAE + CCL5 + PF4 + IL17A + TSLP + REN + IHH + PROM1 + VEGFA | 0,8 |
| GZMH + IFNG + CXCL13 + GNLY + LAG3 + ITGAE + CCL5 + PF4 + IL17A + REN + IHH + PROM1 + VEGFA | 0,8 |
| GZMH + IFNG + CXCL13 + GNLY + LAG3 + ITGAE + CCL5 + PF4 + IL17A + TSLP + REN + IHH + PROM1 | 0,8 |
| GZMH + IFNG + CXCL13 + GNLY + LAG3 + ITGAE + CCL5 + PF4 + IL17A + TSLP + REN + PROM1 + VEGFA | 0,79 |
| GZMH + IFNG + CXCL13 + GNLY + LAG3 + ITGAE + CCL5 + CXCL9 + PF4 + IL17A + REN + PROM1 + VEGFA | 0,79 |
| GZMH + IFNG + CXCL13 + GNLY + LAG3 + ITGAE + CCL5 + CXCL9 + PF4 + IL17A + REN + IHH + PROM1 | 0,79 |
| GZMH + IFNG + CXCL13 + GNLY + ITGAE + CCL5 + CXCL9 + PF4 + IL17A + REN + IHH + PROM1 + VEGFA | 0,79 |
| GZMH + IFNG + CXCL13 + GNLY + ITGAE + CCL5 + CXCL9 + PF4 + IL17A + TSLP + REN + IHH + VEGFA | 0,79 |
| GZMH + IFNG + CXCL13 + GNLY + LAG3 + ITGAE + CCL5 + CXCL9 + PF4 + IL17A + TSLP + REN + PROM1 | 0,79 |
| GZMH + IFNG + CXCL13 + GNLY + ITGAE + CCL5 + CXCL9 + PF4 + IL17A + TSLP + REN + PROM1 + VEGFA | 0,79 |
| GZMH + IFNG + CXCL13 + GNLY + ITGAE + CCL5 + CXCL9 + PF4 + IL17A + TSLP + REN + IHH + PROM1 | 0,79 |
| GZMH + IFNG + CXCL13 + GNLY + LAG3 + ITGAE + CCL5 + CXCL9 + PF4 + IL17A + REN + IHH + VEGFA | 0,79 |
| IFNG + GNLY + LAG3 + ITGAE + CCL5 + CXCL9 + PF4 + IL17A + TSLP + REN + IHH + PROM1 + VEGFA | 0,79 |
| IFNG + CXCL13 + GNLY + LAG3 + ITGAE + CCL5 + PF4 + IL17A + TSLP + REN + IHH + PROM1 + VEGFA | 0,79 |
| IFNG + CXCL13 + GNLY + LAG3 + ITGAE + CCL5 + CXCL9 + PF4 + IL17A + TSLP + REN + IHH + VEGFA | 0,79 |
| GZMH + IFNG + CXCL13 + GNLY + LAG3 + ITGAE + CCL5 + CXCL9 + PF4 + IL17A + TSLP + REN + VEGFA | 0,79 |
| GZMH + IFNG + CXCL13 + GNLY + LAG3 + ITGAE + CCL5 + PF4 + IL17A + TSLP + REN + IHH + VEGFA | 0,79 |
| IFNG + CXCL13 + GNLY + LAG3 + ITGAE + CCL5 + CXCL9 + PF4 + IL17A + TSLP + REN + PROM1 + VEGFA | 0,79 |
| IFNG + CXCL13 + GNLY + LAG3 + ITGAE + CCL5 + CXCL9 + PF4 + IL17A + REN + IHH + PROM1 + VEGFA | 0,79 |
| IFNG + CXCL13 + GNLY + ITGAE + CCL5 + CXCL9 + PF4 + IL17A + TSLP + REN + IHH + PROM1 + VEGFA | 0,79 |
| GZMH + IFNG + CXCL13 + GNLY + LAG3 + ITGAE + CCL5 + CXCL9 + PF4 + IL17A + TSLP + REN + IHH | 0,79 |
| IFNG + CXCL13 + GNLY + LAG3 + ITGAE + CCL5 + CXCL9 + PF4 + IL17A + TSLP + REN + IHH + PROM1 | 0,79 |
| GZMH + CXCL13 + GNLY + ITGAE + CCL5 + CXCL9 + PF4 + IL17A + TSLP + REN + IHH + PROM1 + VEGFA | 0,79 |
| CXCL13 + GNLY + LAG3 + ITGAE + CCL5 + CXCL9 + PF4 + IL17A + TSLP + REN + IHH + PROM1 + VEGFA | 0,79 |
| GZMH + CXCL13 + GNLY + LAG3 + ITGAE + CCL5 + CXCL9 + PF4 + IL17A + REN + IHH + PROM1 + VEGFA | 0,79 |
| GZMH + CXCL13 + GNLY + LAG3 + ITGAE + CCL5 + CXCL9 + PF4 + IL17A + TSLP + REN + IHH + PROM1 | 0,79 |
| GZMH + GNLY + LAG3 + ITGAE + CCL5 + CXCL9 + PF4 + IL17A + TSLP + REN + IHH + PROM1 + VEGFA | 0,79 |
| GZMH + IFNG + GNLY + LAG3 + ITGAE + CCL5 + CXCL9 + IL17A + TSLP + REN + IHH + PROM1 + VEGFA | 0,79 |
| GZMH + CXCL13 + GNLY + LAG3 + ITGAE + CCL5 + PF4 + IL17A + TSLP + REN + IHH + PROM1 + VEGFA | 0,79 |
| GZMH + IFNG + GNLY + LAG3 + CCL5 + CXCL9 + PF4 + IL17A + TSLP + REN + IHH + PROM1 + VEGFA | 0,79 |
| GZMH + CXCL13 + GNLY + LAG3 + ITGAE + CCL5 + CXCL9 + PF4 + IL17A + TSLP + REN + PROM1 + VEGFA | 0,79 |
| GZMH + IFNG + CXCL13 + GNLY + LAG3 + ITGAE + CCL5 + CXCL9 + IL17A + TSLP + REN + PROM1 + VEGFA | 0,79 |
| GZMH + CXCL13 + GNLY + LAG3 + ITGAE + CCL5 + CXCL9 + PF4 + IL17A + TSLP + REN + IHH + VEGFA | 0,79 |
| GZMH + IFNG + CXCL13 + GNLY + LAG3 + CCL5 + CXCL9 + PF4 + IL17A + TSLP + REN + IHH + VEGFA | 0,79 |
| GZMH + IFNG + CXCL13 + GNLY + LAG3 + ITGAE + CCL5 + CXCL9 + IL17A + TSLP + REN + IHH + VEGFA | 0,79 |
| GZMH + IFNG + CXCL13 + GNLY + LAG3 + ITGAE + CCL5 + CXCL9 + IL17A + REN + IHH + PROM1 + VEGFA | 0,79 |
| GZMH + IFNG + CXCL13 + GNLY + LAG3 + ITGAE + CCL5 + IL17A + TSLP + REN + IHH + PROM1 + VEGFA | 0,79 |
| IFNG + CXCL13 + GNLY + LAG3 + ITGAE + CCL5 + CXCL9 + IL17A + TSLP + REN + IHH + PROM1 + VEGFA | 0,79 |
| GZMH + IFNG + CXCL13 + GNLY + LAG3 + CCL5 + CXCL9 + PF4 + IL17A + REN + IHH + PROM1 + VEGFA | 0,79 |
| GZMH + CXCL13 + GNLY + LAG3 + CCL5 + CXCL9 + PF4 + IL17A + TSLP + REN + IHH + PROM1 + VEGFA | 0,79 |
| GZMH + IFNG + CXCL13 + GNLY + LAG3 + CCL5 + PF4 + IL17A + TSLP + REN + IHH + PROM1 + VEGFA | 0,79 |
| GZMH + IFNG + CXCL13 + GNLY + LAG3 + CCL5 + CXCL9 + PF4 + IL17A + TSLP + REN + IHH + PROM1 | 0,79 |
| GZMH + IFNG + CXCL13 + GNLY + CCL5 + CXCL9 + PF4 + IL17A + TSLP + REN + IHH + PROM1 + VEGFA | 0,79 |
| GZMH + IFNG + CXCL13 + GNLY + LAG3 + CCL5 + CXCL9 + PF4 + IL17A + TSLP + REN + PROM1 + VEGFA | 0,79 |
| GZMH + IFNG + CXCL13 + GNLY + LAG3 + ITGAE + CCL5 + CXCL9 + IL17A + TSLP + REN + IHH + PROM1 | 0,79 |
| GZMH + IFNG + CXCL13 + GNLY + ITGAE + CCL5 + CXCL9 + IL17A + TSLP + REN + IHH + PROM1 + VEGFA | 0,78 |
| IFNG + CXCL13 + GNLY + LAG3 + CCL5 + CXCL9 + PF4 + IL17A + TSLP + REN + IHH + PROM1 + VEGFA | 0,78 |
| GZMH + IFNG + GNLY + LAG3 + ITGAE + CXCL9 + PF4 + IL17A + TSLP + REN + IHH + PROM1 + VEGFA | 0,78 |
| GZMH + IFNG + CXCL13 + GNLY + LAG3 + ITGAE + CXCL9 + PF4 + IL17A + TSLP + REN + PROM1 + VEGFA | 0,78 |
| GZMH + IFNG + CXCL13 + GNLY + LAG3 + ITGAE + PF4 + IL17A + TSLP + REN + IHH + PROM1 + VEGFA | 0,78 |
| GZMH + IFNG + CXCL13 + GNLY + LAG3 + ITGAE + CXCL9 + PF4 + IL17A + REN + IHH + PROM1 + VEGFA | 0,78 |
| GZMH + IFNG + CXCL13 + GNLY + LAG3 + ITGAE + CXCL9 + PF4 + IL17A + TSLP + REN + IHH + VEGFA | 0,78 |
| GZMH + IFNG + CXCL13 + GNLY + LAG3 + ITGAE + CXCL9 + PF4 + IL17A + TSLP + REN + IHH + PROM1 | 0,78 |
| IFNG + CXCL13 + GNLY + LAG3 + ITGAE + CXCL9 + PF4 + IL17A + TSLP + REN + IHH + PROM1 + VEGFA | 0,78 |
| GZMH + IFNG + CXCL13 + GNLY + ITGAE + CXCL9 + PF4 + IL17A + TSLP + REN + IHH + PROM1 + VEGFA | 0,78 |
| GZMH + CXCL13 + GNLY + LAG3 + ITGAE + CCL5 + CXCL9 + IL17A + TSLP + REN + IHH + PROM1 + VEGFA | 0,78 |
| GZMH + CXCL13 + GNLY + LAG3 + ITGAE + CXCL9 + PF4 + IL17A + TSLP + REN + IHH + PROM1 + VEGFA | 0,78 |
| GZMH + IFNG + CXCL13 + GNLY + LAG3 + CXCL9 + PF4 + IL17A + TSLP + REN + IHH + PROM1 + VEGFA | 0,78 |
| GZMH + IFNG + CXCL13 + GNLY + LAG3 + CCL5 + CXCL9 + IL17A + TSLP + REN + IHH + PROM1 + VEGFA | 0,77 |
| GZMH + IFNG + GNLY + LAG3 + ITGAE + CCL5 + CXCL9 + PF4 + TSLP + REN + IHH + PROM1 + VEGFA | 0,77 |
| GZMH + IFNG + CXCL13 + GNLY + LAG3 + ITGAE + CCL5 + CXCL9 + PF4 + IL17A + IHH + PROM1 + VEGFA | 0,77 |
| GZMH + IFNG + CXCL13 + GNLY + LAG3 + ITGAE + CCL5 + CXCL9 + PF4 + IL17A + TSLP + IHH + PROM1 | 0,77 |
| GZMH + IFNG + CXCL13 + GNLY + LAG3 + ITGAE + CCL5 + PF4 + IL17A + TSLP + IHH + PROM1 + VEGFA | 0,77 |
| GZMH + IFNG + CXCL13 + GNLY + ITGAE + CCL5 + CXCL9 + PF4 + IL17A + TSLP + IHH + PROM1 + VEGFA | 0,77 |
| IFNG + CXCL13 + GNLY + LAG3 + ITGAE + CCL5 + CXCL9 + PF4 + IL17A + TSLP + IHH + PROM1 + VEGFA | 0,77 |
| GZMH + IFNG + CXCL13 + GNLY + LAG3 + ITGAE + CCL5 + CXCL9 + PF4 + IL17A + TSLP + PROM1 + VEGFA | 0,77 |
| GZMH + IFNG + CXCL13 + GNLY + LAG3 + ITGAE + CCL5 + PF4 + TSLP + REN + IHH + PROM1 + VEGFA | 0,77 |
| GZMH + IFNG + CXCL13 + GNLY + LAG3 + ITGAE + CCL5 + CXCL9 + PF4 + TSLP + REN + IHH + PROM1 | 0,77 |
| GZMH + IFNG + CXCL13 + GNLY + LAG3 + ITGAE + CCL5 + CXCL9 + PF4 + TSLP + REN + PROM1 + VEGFA | 0,77 |
| GZMH + CXCL13 + GNLY + LAG3 + ITGAE + CCL5 + CXCL9 + PF4 + IL17A + TSLP + IHH + PROM1 + VEGFA | 0,77 |
| GZMH + IFNG + CXCL13 + GNLY + ITGAE + CCL5 + CXCL9 + PF4 + TSLP + REN + IHH + PROM1 + VEGFA | 0,77 |
| GZMH + IFNG + GNLY + LAG3 + ITGAE + CCL5 + CXCL9 + PF4 + IL17A + TSLP + IHH + PROM1 + VEGFA | 0,77 |
| GZMH + IFNG + CXCL13 + GNLY + LAG3 + ITGAE + CCL5 + CXCL9 + PF4 + IL17A + TSLP + IHH + VEGFA | 0,77 |
| IFNG + CXCL13 + GNLY + LAG3 + ITGAE + CCL5 + CXCL9 + PF4 + TSLP + REN + IHH + PROM1 + VEGFA | 0,77 |
| GZMH + IFNG + CXCL13 + GNLY + LAG3 + ITGAE + CXCL9 + IL17A + TSLP + REN + IHH + PROM1 + VEGFA | 0,77 |
| GZMH + IFNG + CXCL13 + GNLY + LAG3 + ITGAE + CCL5 + CXCL9 + PF4 + REN + IHH + PROM1 + VEGFA | 0,77 |
| GZMH + CXCL13 + GNLY + LAG3 + ITGAE + CCL5 + CXCL9 + PF4 + TSLP + REN + IHH + PROM1 + VEGFA | 0,77 |
| GZMH + IFNG + CXCL13 + GNLY + LAG3 + ITGAE + CCL5 + CXCL9 + PF4 + TSLP + REN + IHH + VEGFA | 0,77 |
| GZMH + IFNG + CXCL13 + GNLY + LAG3 + ITGAE + CCL5 + CXCL9 + IL17A + TSLP + IHH + PROM1 + VEGFA | 0,77 |
| GZMH + IFNG + CXCL13 + GNLY + LAG3 + CCL5 + CXCL9 + PF4 + TSLP + REN + IHH + PROM1 + VEGFA | 0,77 |
| GZMH + IFNG + CXCL13 + GNLY + LAG3 + ITGAE + CXCL9 + PF4 + TSLP + REN + IHH + PROM1 + VEGFA | 0,76 |
| GZMH + IFNG + CXCL13 + GNLY + LAG3 + CCL5 + CXCL9 + PF4 + IL17A + TSLP + IHH + PROM1 + VEGFA | 0,76 |
| GZMH + IFNG + CXCL13 + GNLY + LAG3 + ITGAE + CCL5 + CXCL9 + TSLP + REN + IHH + PROM1 + VEGFA | 0,76 |
| GZMH + IFNG + CXCL13 + GNLY + LAG3 + ITGAE + CXCL9 + PF4 + IL17A + TSLP + IHH + PROM1 + VEGFA | 0,76 |
| GZMH + IFNG + CXCL13 + GNLY + LAG3 + ITGAE + CCL5 + CXCL9 + PF4 + TSLP + IHH + PROM1 + VEGFA | 0,75 |
| GZMH + IFNG + CXCL13 + LAG3 + CCL5 + CXCL9 + PF4 + IL17A + TSLP + REN + IHH + PROM1 + VEGFA | 0,75 |
| GZMH + CXCL13 + LAG3 + ITGAE + CCL5 + CXCL9 + PF4 + IL17A + TSLP + REN + IHH + PROM1 + VEGFA | 0,74 |
| IFNG + CXCL13 + LAG3 + ITGAE + CCL5 + CXCL9 + PF4 + IL17A + TSLP + REN + IHH + PROM1 + VEGFA | 0,74 |
| GZMH + IFNG + CXCL13 + LAG3 + ITGAE + CXCL9 + PF4 + IL17A + TSLP + REN + IHH + PROM1 + VEGFA | 0,74 |
| GZMH + IFNG + LAG3 + ITGAE + CCL5 + CXCL9 + PF4 + IL17A + TSLP + REN + IHH + PROM1 + VEGFA | 0,74 |
| GZMH + IFNG + CXCL13 + LAG3 + ITGAE + CCL5 + CXCL9 + PF4 + IL17A + REN + IHH + PROM1 + VEGFA | 0,74 |
| GZMH + IFNG + CXCL13 + LAG3 + ITGAE + CCL5 + CXCL9 + PF4 + IL17A + TSLP + REN + IHH + VEGFA | 0,74 |
| GZMH + IFNG + CXCL13 + LAG3 + ITGAE + CCL5 + CXCL9 + PF4 + IL17A + TSLP + REN + PROM1 + VEGFA | 0,74 |
| GZMH + IFNG + CXCL13 + LAG3 + ITGAE + CCL5 + CXCL9 + PF4 + IL17A + TSLP + REN + IHH + PROM1 | 0,74 |
| GZMH + IFNG + CXCL13 + ITGAE + CCL5 + CXCL9 + PF4 + IL17A + TSLP + REN + IHH + PROM1 + VEGFA | 0,74 |
| GZMH + IFNG + CXCL13 + LAG3 + ITGAE + CCL5 + PF4 + IL17A + TSLP + REN + IHH + PROM1 + VEGFA | 0,73 |
| GZMH + IFNG + CXCL13 + LAG3 + ITGAE + CCL5 + CXCL9 + IL17A + TSLP + REN + IHH + PROM1 + VEGFA | 0,73 |
| GZMH + IFNG + CXCL13 + LAG3 + ITGAE + CCL5 + CXCL9 + PF4 + IL17A + TSLP + IHH + PROM1 + VEGFA | 0,73 |
| GZMH + IFNG + CXCL13 + LAG3 + ITGAE + CCL5 + CXCL9 + PF4 + TSLP + REN + IHH + PROM1 + VEGFA | 0,73 |

| **Table 14: clusters of 14 genes:** | |
|---|---|
| **clusters of 14 genes** | **c_tau level** |
| GZMH + IFNG + GNLY + LAG3 + ITGAE + CCL5 + CXCL9 + PF4 + IL17A + TSLP + REN + IHH + PROM1 + VEGFA | 0,8 |
| GZMH + IFNG + CXCL13 + GNLY + LAG3 + ITGAE + CCL5 + PF4 + IL17A + TSLP + REN + IHH + PROM1 + VEGFA | 0,8 |
| GZMH + IFNG + CXCL13 + GNLY + LAG3 + ITGAE + CCL5 + CXCL9 + PF4 + IL17A + TSLP + REN + IHH + PROM1 | 0,79 |
| GZMH + IFNG + CXCL13 + GNLY + LAG3 + ITGAE + CCL5 + CXCL9 + PF4 + IL17A + TSLP + REN + PROM1 + VEGFA | 0,79 |
| GZMH + IFNG + CXCL13 + GNLY + LAG3 + ITGAE + CCL5 + CXCL9 + PF4 + IL17A + REN + IHH + PROM1 + VEGFA | 0,79 |
| GZMH + IFNG + CXCL13 + GNLY + ITGAE + CCL5 + CXCL9 + PF4 + IL17A + TSLP + REN + IHH + PROM1 + VEGFA | 0,79 |
| GZMH + IFNG + CXCL13 + GNLY + LAG3 + ITGAE + CCL5 + CXCL9 + PF4 + IL17A + TSLP + REN + IHH + VEGFA | 0,79 |
| IFNG + CXCL13 + GNLY + LAG3 + ITGAE + CCL5 + CXCL9 + PF4 + IL17A + TSLP + REN + IHH + PROM1 + VEGFA | 0,79 |
| GZMH + CXCL13 + GNLY + LAG3 + ITGAE + CCL5 + CXCL9 + PF4 + IL17A + TSLP + REN + IHH + PROM1 + VEGFA | 0,79 |
| GZMH + IFNG + CXCL13 + GNLY + LAG3 + ITGAE + CCL5 + CXCL9 + IL17A + TSLP + REN + IHH + PROM1 + VEGFA | 0,79 |
| GZMH + IFNG + CXCL13 + GNLY + LAG3 + CCL5 + CXCL9 + PF4 + IL17A + TSLP + REN + IHH + PROM1 + VEGFA | 0,79 |
| GZMH + IFNG + CXCL13 + GNLY + LAG3 + ITGAE + CXCL9 + PF4 + IL17A + TSLP + REN + IHH + PROM1 + VEGFA | 0,78 |
| GZMH + IFNG + CXCL13 + GNLY + LAG3 + ITGAE + CCL5 + CXCL9 + PF4 + IL17A + TSLP + IHH + PROM1 + VEGFA | 0,77 |
| GZMH + IFNG + CXCL13 + GNLY + LAG3 + ITGAE + CCL5 + CXCL9 + PF4 + TSLP + REN + IHH + PROM1 + VEGFA | 0,77 |
| GZMH + IFNG + CXCL13 + LAG3 + ITGAE + CCL5 + CXCL9 + PF4 + IL17A + TSLP + REN + IHH + PROM1 + VEGFA | 0,74 |

| **Table 15 :** | |
|---|---|
| **Top clusters** | **c_tau level** |
| GZMH + IFNG + CXCL13 + GNLY + LAG3 + ITGAE + CCL5 + CXCL9 + PF4 + IL17A + TSLP + REN + IHH + PROM1 + VEGFA | 0,79 |
| GZMH + IFNG + GNLY + LAG3 + ITGAE + CCL5 + CXCL9 + PF4 + IL17A + TSLP + REN + IHH + PROM1 + VEGFA | 0,8 |
| GZMH + IFNG + GNLY + LAG3 + ITGAE + CCL5 + PF4 + IL17A + TSLP + REN + IHH + PROM1 + VEGFA | 0,8 |
| GZMH + IFNG + GNLY + ITGAE + CCL5 + PF4 + IL17A + TSLP + REN + IHH + PROM1 + VEGFA | 0,8 |
| GZMH + IFNG + GNLY + ITGAE + CCL5 + PF4 + IL17A + REN + IHH + PROM1 + VEGFA | 0,8 |
| GZMH + IFNG + GNLY + ITGAE + CCL5 + PF4 + IL17A + REN + PROM1 + VEGFA | 0,8 |
| GZMH + IFNG + GNLY + ITGAE + CCL5 + PF4 + IL17A + REN + PROM1 | 0,8 |
| IFNG + GNLY + ITGAE + CCL5 + PF4 + IL17A + REN + PROM1 | 0,8 |
| IFNG + GNLY + ITGAE + CCL5 + PF4 + IL17A + REN | 0,79 |
| IFNG + GNLY + ITGAE + PF4 + IL17A + REN | 0,78 |
| IFNG + GNLY + PF4 + IL17A + REN | 0,78 |
| GNLY + PF4 + IL17A + REN | 0,77 |
| GNLY + PF4 + REN | 0,75 |
| GNLY + PF4 | 0,71 |
| GNLY | 0,68 |
| UICC | 0,52 |

### REFERENCES:

Throughout this application, various references describe the state of the art to which this invention pertains.

## Claims

1. A method for predicting the outcome of a cancer in a patient comprising a step consisting of determining the expression level of a gene cluster in a sample obtained from said patient, wherein said gene cluster is GZMH, IFNG, CXCL13, GNLY, LAG3, ITGAE, CCL5, CXCL9, PF4, IL17A, TSLP, REN, IHH, PROM1 and VEGFA.

2. Use of a kit comprising means for specifically measuring the expression level of fifteen genes in a biological sample for performing the method according to claim 1, wherein said fifteen genes are GZMH, IFNG, CXCL13, GNLY, LAG3, ITGAE, CCL5, CXCL9, PF4, IL17A, TSLP, REN, IHH, PROM1 and VEGFA.

## Patentansprüche

1. Ein Verfahren zur Vorhersage der Entwicklung von Krebs in einem Patienten, umfassend einen Schritt, bestehend aus der Bestimmung des Expressionsniveaus eines Genclusters in einer Probe, erhalten von dem Patienten, wobei das Gencluster GZMH, IFNG, CXCL13, GNLY , LAG3, ITGAE, CCL5, CXCL9, PF4, IL17A, TSLP, REN, IHH, PROM1 und VEGFA ist.

2. Verwendung eines Kits, umfassend Mittel zur spezifischen Messung des Expressionsniveaus von 15 Genen in einer biologischen Probe zur Durchführung des Verfahrens nach Anspruch 1, wobei diese 15 Gene GZMH, IFNG, CXCL13, GNLY, LAG3, ITGAE, CCL5, CXCL9, PF4, IL17A, TSLP, REN, IHH, PROM1 und VEGFA sind.

## Revendications

1. Procédé pour la prédiction de l'issue d'un cancer chez un patient comprenant une étape consistant à déterminer le niveau d'expression d'un groupe de gènes dans un échantillon obtenu dudit patient, dans lequel ledit groupe de gènes est GZMH, IFNG, CXCL13, GNLY, LAG3, ITGAE, CCL5, CXCL9, PF4, IL17A, TSLP, REN, IHH, PROM1 et VEGFA.

2. Utilisation d'un kit comprenant des moyens pour mesurer spécifiquement le niveau d'expression de quinze gènes dans un échantillon biologique pour réaliser le procédé selon la revendication 1, dans laquelle lesdits quinze gènes sont GZMH, IFNG, CXCL13, GNLY, LAG3, ITGAE, CCL5, CXCL9, PF4, IL17A, TSLP, REN, IHH, PROM1 et VEGFA.
